# EUROPEAN PATENT APPLICATION

(11) **EP 4 759 367 A1**
(43) Date of publication of application: **17.06.2026**
(21) Application number: 24307062.0
(22) Date of filing: 10.12.2024
(51) Int. Cl.: A61P 35/00, C07C 61/22, C07C 233/00

(54) **AMINO-ALCOHOL COMPOUNDS AND THEIR USE IN THERAPY**

(71) Applicant: Inventiva, 21121 Daix (FR)
(72) Inventor: The designation of the inventor has not yet been filed
(74) Representative: Cabinet Beau de Loménie

(57) **Abstract**

The present disclosure relates to compounds of formula (I): 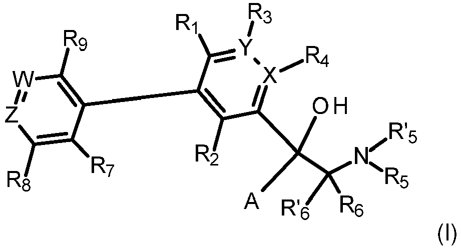 wherein A, W, X, Y, Z, R₁, R₂, R₃, R₄, R₅, R'₅, R₆, R'₆, R₇, R₈ and R₉ are as defined herein. The compounds of formula (I) are useful as modulators of the Hippo signalling pathway.

## Description

### Field of the invention

The present disclosure relates to compounds that modulate the YAP/TAZ-TEAD complex, to pharmaceutical compositions containing said compounds, and to the use of said compounds as medicaments, in particular for the treatment and/or prevention of cancers and/or fibrosis related diseases.

### Background

The hippo signalling pathway has key roles in organ size control and tumor suppression. Signal transduction in the hippo signalling pathway involves a core kinase cascade, leading to YAP/TAZ phosphorylation. Physiological or pathological inactivation of the hippo signalling pathway leads to dephosphorylation and nuclear accumulation of YAP and/or TAZ. Nuclear YAP/TAZ binds to transcriptional enhanced associate domains (TEADs) to mediate target genes expression. The YAP/TAZ-TEAD complex regulates organ development and amplification of oncogenic factors in many cancers (e.g., sarcoma, lung cancer, thyroid cancer, skin cancer, ovarian cancer, colorectal cancer, prostate cancer, pancreatic cancer, head and neck cancer, oesophageal cancer, kidney cancer, urogenital cancer, liver cancer, breast cancer) and in fibrosis related diseases. There is a need to find modulators of the Hippo signalling pathway useful as medicaments.

The present invention provides new compounds that disrupt the YAP/TAZ-TEAD complex.

### Summary of the invention

In one aspect, the present disclosure relates to a compound of formula (I): 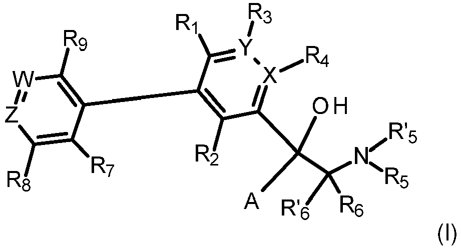
or a pharmaceutically acceptable salt, enantiomers, diastereomers, tautomers, solvates, isotope substituents, polymorphs, prodrugs or metabolites thereof,
wherein:
   A is a phenyl optionally substituted with 1 to 3 substituents selected from halogen, (C₁-C₆)alkyl and (C₁-C₆)alkoxy; or a 4- to 7-membered unsaturated, monocyclic group comprising from 1 to 3 heteroatoms, preferably 1 or 2 heteroatoms, chosen from nitrogen, oxygen and sulfur;
   W and Z are each independently CR₁₅ or N;
   X and Y are each independently C or N;
   R₁ is H, halogen, (C₁-C₆)alkyl or (C₁-C₆)alkoxy;
   R₂ is H, halogen, (C₁-C₆)alkyl or (C₁-C₆)alkoxy;
   R₃ is absent when Y is N, or R₃ is H, halogen, -OH, -COCH₃, (C₁-C₆)alkyl or (C₁-C₆)alkoxy when Y is C;
   R₄ is absent when X is N, or R₄ is H, halogen, -OH, -COCH₃, (C₁-C₆)alkyl or (C₁-C₆)alkoxy when X is C;
   R₅ and R'₅ are each independently H; (C₁-C₆)alkyl optionally substituted with halogen, -OH, - CONHR₁₂, -CONH₂, -NR₁₂R₁₃, (C₁-C₆)alkoxy or (C₃-C₆)cycloalkyl; or (C₃-C₆)cycloalkyl optionally substituted with -OR₁₂, -CONHR₁₂, -CONH₂, -NR₁₂R₁₃;
   or R₅ and R'₅, together with the nitrogen atom to which are bound, form a 4- to 7-membered ring;
   R₆ and R'₆ are each independently H or (C₁-C₆)alkyl; or R₆ and R'₆ together form a (C₃-C₆)cycloalkyl;
   or R₅ and R₆ together form a (C₃-C₆)cycloalkyl;
   R₇ is H, halogen, (C₁-C₆)alkyl or (C₁-C₆)alkoxy;
   R₈ is H, halogen, (C₁-C₆)alkyl, (C₁-C₆)alkyl optionally substituted with halogen, (C₁-C₆)alkoxy optionally substituted with halogen, (C₁-C₆)alkoxy optionally substituted with (C₁-C₆)alkoxy, -OR₁₀, -OCH₂R₁₀, -NHR₁₀, -N(CH₃)R₁₀, or 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₉ is (C₁-C₆)alkyl, (C₁-C₆)alkoxy, -CONHR₁₁, -C(NH)NHOH, -C(NH)NHOCH₃ or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₁₀ is H, (C₁-C₆)alkyl, (C₁-C₆)alkyl optionally substituted with halogen, (C₁-C₆)alkoxy or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₁₁ is H, -OH, -NH₂,(C₁-C₆)alkyl, (C₁-C₆)alkoxy, and -NHR₁₄ ;
   R₁₂ is H or (C₁-C₆)alkyl;
   R₁₃ is H or (C₁-C₆)alkyl;
   R₁₄ is (C₁-C₆)alkyl;
   R₁₅ is H, halogen, (C₁-C₆)alkyl or (C₁-C₆)alkoxy.

In one aspect, the present disclosure relates to a pharmaceutical composition comprising a compound of formula (I) or a pharmaceutically acceptable salt thereof, and one or more pharmaceutically acceptable excipients.

In one aspect, the present disclosure provides a method of modulating one or more of proteins encompassed by, or related to, the Hippo pathway in a subject, comprising administering to a subject in need thereof a compound of formula (I), or a pharmaceutically acceptable salt thereof.

In one aspect, the present disclosure relates to a method of treating or preventing a cancer which comprises administering an effective amount of a compound of formula (I) or a pharmaceutically acceptable salt thereof to a subject in need thereof.

In one aspect, the present disclosure relates to a method of treating or preventing a fibrosis-related disease which comprises administering an effective amount of a compound of formula (I) or a pharmaceutically acceptable salt thereof to a subject in need thereof.

In one aspect, the present disclosure relates to the use of a compound of formula (I) or a pharmaceutically acceptable salt thereof in the manufacture of a medicament for treating or preventing a cancer or a fibrosis-related disease.

In one aspect, the present disclosure relates to a compound of formula (I) or a pharmaceutically acceptable salt thereof for use in the treatment or prevention of a cancer or a fibrosis-related disease.

### Description of the invention

In one aspect, the present disclosure relates to a compound of formula (I): 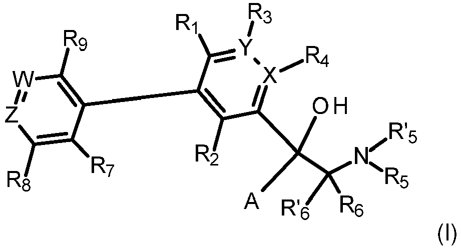
or a pharmaceutically acceptable salt, enantiomer, diastereomer, tautomer, solvate, isotope substituent, polymorph, prodrug or metabolite thereof,
wherein:
   A is a phenyl optionally substituted with 1 to 3 substituents selected from halogen, (C₁-C₆)alkyl and (C₁-C₆)alkoxy; or a 4- to 7-membered unsaturated, monocyclic group comprising from 1 to 3 heteroatoms, preferably 1 or 2 heteroatoms, chosen from nitrogen, oxygen and sulfur;;
   W and Z are each independently CR₁₅ or N;
   X and Y are each independently C or N;
   R₁ is H, halogen, (C₁-C₆)alkyl or (C₁-C₆)alkoxy;
   R₂ is H, halogen, (C₁-C₆)alkyl or (C₁-C₆)alkoxy;
   R₃ is absent when Y is N, or R₃ is H, halogen, -OH, -COCH₃, (C₁-C₆)alkyl or (C₁-C₆)alkoxy when Y is C;
   R₄ is absent when X is N, or R₄ is H, halogen, -OH, -COCH₃, (C₁-C₆)alkyl or (C₁-C₆)alkoxy when X is C;
   R₅ and R'₅ are each independently H; (C₁-C₆)alkyl optionally substituted with halogen, -OH, - CONHR₁₂, -CONH₂, -NR₁₂R₁₃, (C₁-C₆)alkoxy or (C₃-C₆)cycloalkyl; or (C₃-C₆)cycloalkyl optionally substituted with -OR₁₂, -CONHR₁₂, -CONH₂, -NR₁₂R₁₃;
   or R₅ and R'₅, together with the nitrogen atom to which are bound, form a 4- to 7-membered ring;
   R₆ and R'₆ are each independently H or (C₁-C₆)alkyl; or R₆ and R'₆ together form a (C₃-C₆)cycloalkyl;
   or R₅ and R₆ together form a (C₃-C₆)cycloalkyl;
   R₇ is H, halogen, (C₁-C₆)alkyl or (C₁-C₆)alkoxy;
   R₈ is H, halogen, (C₁-C₆)alkyl, (C₁-C₆)alkyl optionally substituted with halogen, (C₁-C₆)alkoxy, (C₁-C₆)alkoxy optionally substituted with halogen, (C₁-C₆)alkoxy optionally substituted with (C₁-C₆)alkoxy, -OR₁₀, -OCH₂R₁₀ , -NHR₁₀ , -N(CH₃)R₁₀, or 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₉ is (C₁-C₆)alkyl, (C₁-C₆)alkoxy, -CONHR₁₁, -C(NH)NHOH, -C(NH)NHOCH₃ or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₁₀ is H, (C₁-C₆)alkyl, (C₁-C₆)alkyl optionally substituted with halogen, (C₁-C₆)alkoxy or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₁₁ is H, -OH, -NH₂,(C₁-C₆)alkyl, (C₁-C₆)alkoxy, and -NHR₁₄ ;
   R₁₂ is H or (C₁-C₆)alkyl;
   R₁₃ is H or (C₁-C₆)alkyl;
   R₁₄ is (C₁-C₆)alkyl;
   R₁₅ is H, halogen, (C₁-C₆)alkyl or (C₁-C₆)alkoxy.

Any combination of the groups described above for the various variables is contemplated herein. Throughout the specification, groups and substituents thereof are chosen by one skilled in the field to provide stable moieties and compounds.

As used herein, the term "alkyl" means a linear or branched hydrocarbon moiety having the mentioned number of carbon atoms. In some embodiments, an alkyl comprises one to six carbon atoms (e.g., C₁-C₆ alkyl). In some embodiments, an alkyl comprises one to four carbon atoms (e.g., C₁-C₄ alkyl). In some embodiments, the alkyl group is selected from methyl, ethyl, propyl, 2-propyl, butyl, 2-methylpropyl, 2-butyl, tert-butyl, pentyl, 1,1-dimethylpropyl, 2,2-dimethylpropyl, 3-methylbutyl, 1-methylbutyl, 1-ethylpropyl, 1,2-dimethylpropyl, 1-methylethyl (isopropyl), 1-butyl (n-butyl), 1-methylpropyl (sec-butyl), pentyl or hexyl.

As used herein, the term "alkoxy" means a group -O-alkyl where alkyl is as defined above. As used herein, the term "cycloalkyl" means a 3- to 6-membered hydrocarbon cycle, which can be bridged, including cyclopropyl, cyclobutyl, cyclopentyl and cyclohexyl.

As used herein, the term "halogen" means F, Cl, Br or I, preferably F, Br or CI.

In some embodiments, A is a phenyl optionally substituted with 1 to 3 substituents selected from halogen, (C₁-C₆)alkyl and (C₁-C₆)alkoxy.

In some embodiments, A is a 4- to 7-membered unsaturated, monocyclic group comprising from 1 to 3 heteroatoms, preferably 1 or 2 heteroatoms, chosen from nitrogen, oxygen and sulfur. Examples of such monocyclic groups include the groups pyrrolyl, pyrazolyl, imidazolyl, oxazolyl, isoxazolyl, triazolyl, oxadiazolyl, furanyl, thienyl, thiazolyl, isothiazolyl, thiadiazolyl, pyridyl, pyridazinyl, pyrimidinyl, pyrazinyl, and triazinyl.

In some embodiments, W and Z are each CH.

In some embodiments, X and Y are each C.

In some embodiments, R₁ is halogen or (C₁-C₆)alkyl.

In some embodiments, R₂ is H, halogen or (C₁-C₆)alkyl.

In some embodiments, R₃ when present is H or halogen.

In some embodiments, R₄ when present is H.

In some embodiments, R'₅ is H or (C₁-C₆)alkyl or (C₃-C₆)cycloalkyl

In some embodiments, R₇ is H, halogen or (C₁-C₆)alkyl.

In some embodiments, R₈ is H or (C₁-C₆)alkoxy optionally substituted with (C₁-C₆)alkoxy or - OR₁₀

In some embodiments, R₁₁ is H or OH.

In some embodiments, the compound of formula (I) is selected from:
2-[2-chloro-5-[rel-(1S)-2-amino-1-hydroxy-1-phenyl-ethyl]phenyl]benzamide;
2-[2-chloro-5-[rel-(1R)-2-amino-1-hydroxy-1-phenyl-ethyl]phenyl]benzamide;
2-[5-(2-amino-1-hydroxy-1-phenyl-ethyl)-2-chloro-phenyl]-3-methyl-benzamide;
2-[5-(2-amino-1-hydroxy-1-phenyl-ethyl)-2-chloro-phenyl]-3-methyl-benzamide;
2-[5-(2-amino-1-hydroxy-1-phenyl-ethyl)-2-chloro-phenyl]-3-methyl-benzamide;
2-[5-(2-amino-1-hydroxy-1-phenyl-ethyl)-2-chloro-phenyl]-4-methoxy-3-methyl-benzamide;
2-[5-(2-amino-1-hydroxy-1-phenyl-ethyl)-2-chloro-phenyl]-4-(2-methoxyethoxy)-3-methyl-benzamide;
2-[5-(2-amino-1-hydroxy-1-phenyl-ethyl)-2-chloro-phenyl]-4-(2-methoxyethoxy)-3-methyl-benzamide;
rac2 2-[5-(2-amino-1-hydroxy-1-phenyl-ethyl)-2-chloro-phenyl]-4-(2-methoxyethoxy)-3-methyl-benzamide;
2-[5-(2-amino-1-hydroxy-1-phenyl-ethyl)-2-chloro-phenyl]-3-fluoro-benzamide;
2-[5-(2-amino-1-hydroxy-1-phenyl-ethyl)-2-chloro-phenyl]-3-fluoro-4-(2-methoxyethoxy)benzamide;
2-[2-chloro-5-[1-hydroxy-2-(methylamino)-1-phenyl-ethyl]phenyl]-3-fluoro-4-(2-methoxyethoxy)benzamide;
2-[5-(2-amino-1-hydroxy-1-phenyl-ethyl)-2-chloro-phenyl]-3-chloro-benzamide;
2-[3-(2-amino-1-hydroxy-1-phenyl-ethyl)-6-chloro-2-methyl-phenyl]benzamide;
2-[3-(2-amino-1-hydroxy-1-phenyl-ethyl)-6-chloro-2-methyl-phenyl]benzamide;
2-[3-(2-amino-1-hydroxy-1-phenyl-ethyl)-6-chloro-2-fluoro-phenyl]benzamide;
2-[5-(2-amino-1-hydroxy-1-phenyl-ethyl)-2-chloro-3-fluoro-phenyl]-3-methyl-benzamide;
2-[5-(2-amino-1-hydroxy-1-phenyl-ethyl)-2-methyl-phenyl]-3-fluoro-4-(2-methoxyethoxy)benzamide;
2-[5-[(1S)-2-amino-1-hydroxy-1-phenyl-ethyl]-2-chloro-phenyl]-3-fluoro-4-(2-methoxyethoxy)benzamide;
2-[5-[(1R)-2-amino-1-hydroxy-1-phenyl-ethyl]-2-chloro-phenyl]-3-fluoro-4-(2-methoxyethoxy)benzamide;
2-[5-[(1-aminocyclopropyl)-hydroxy-phenyl-methyl]-2-chloro-phenyl]-3-fluoro-4-(2-methoxyethoxy)benzamide;
2-[3-(2-amino-1-hydroxy-1-phenyl-ethyl)-2,6-difluoro-phenyl]-3-fluoro-4-(2-methoxyethoxy)benzamide;
2-[3-(2-amino-1-hydroxy-1-phenyl-ethyl)-2,6-difluoro-phenyl]-3-fluoro-4-(2-methoxyethoxy)benzamide;
2-[5-(2-amino-1-hydroxy-1-phenyl-ethyl)-2-chloro-phenyl]-3-chloro-4-methoxy-benzamide;
2-[2-chloro-5-[1-hydroxy-2-(methylamino)-1-phenyl-ethyl]phenyl]-3-fluoro-4-(2-methoxyethoxy)benzenecarbohydroxamic acid;
rel-(2aR)-2-[3-[(1S)-2-amino-1-hydroxy-1-phenyl-ethyl]-6-chloro-2-methyl-phenyl]-3-fluoro-4-(2-methoxyethoxy)benzamide;
rel-(2aS)-2-[3-[(1S)-2-amino-1-hydroxy-1-phenyl-ethyl]-6-chloro-2-methyl-phenyl]-3-fluoro-4-(2-methoxyethoxy)benzamide;
rel-(2-aS)-2-[3-[(1R)-2-amino-1-hydroxy-1-phenyl-ethyl]-6-chloro-2-methyl-phenyl]-3-fluoro-4-(2-methoxyethoxy)benzamide;
rel-(2aR)-2-[3-[(1R)-2-amino-1-hydroxy-1-phenyl-ethyl]-6-chloro-2-methyl-phenyl]-3-fluoro-4-(2-methoxyethoxy)benzamide;
2-[6-chloro-3-[2-(cyclohexylamino)-1-hydroxy-1-phenyl-ethyl]-2-fluoro-phenyl]-3-fluoro-4-(2-methoxyethoxy)benzamide;
rel-(2aS)-2-[6-chloro-2-fluoro-3-[(1S)-1-hydroxy-2-(methylamino)-1-phenyl-ethyl]phenyl]-3-fluoro-4-(2-methoxyethoxy)benzamide;
rel-(2aR)-2-[6-chloro-2-fluoro-3-[(1S)-1-hydroxy-2-(methylamino)-1-phenyl-ethyl]phenyl]-3-fluoro-4-(2-methoxyethoxy)benzamide;
rel-(2aR)-2-[6-chloro-2-fluoro-3-[(1R)-1-hydroxy-2-(methylamino)-1-phenyl-ethyl]phenyl]-3-fluoro-4-(2-methoxyethoxy)benzamide;
rel-(2aS)-2-[6-chloro-2-fluoro-3-[(1R)-1-hydroxy-2-(methylamino)-1-phenyl-ethyl]phenyl]-3-fluoro-4-(2-methoxyethoxy)benzamide;
2-[2-chloro-5-[(R)-hydroxy-phenyl-[(2S)-pyrrolidin-2-yl]methyl]phenyl]-3-methyl-benzamide;
2-[rel-(5R)-5-[[(2S)-azetidin-2-yl]-hydroxy-phenyl-methyl]-2-chloro-phenyl]-3-fluoro-4-(2-methoxyethoxy)benzamide;
2-[3-(2-amino-1-hydroxy-1-phenyl-ethyl)-2,6-dichloro-phenyl]-3-fluoro-4-(2-methoxyethoxy)benzamide;
trans-2-[2-chloro-5-[1-hydroxy-2-[(4-hydroxycyclohexyl)amino]-1-phenyl-ethyl]phenyl]-3-fluoro-4-(2-methoxyethoxy)benzamide;
rel-(2aR)-2-[3-[(1R)-2-amino-1-hydroxy-1-phenyl-ethyl]-6-chloro-2-fluoro-phenyl]-3-fluoro-4-(2-methoxyethoxy)benzamide;
rel-(2aS)-2-[3-[(1R)-2-amino-1-hydroxy-1-phenyl-ethyl]-6-chloro-2-fluoro-phenyl]-3-fluoro-4-(2-methoxyethoxy)benzamide;
rel-(2aR)-2-[3-[(1S)-2-amino-1-hydroxy-1-phenyl-ethyl]-6-chloro-2-fluoro-phenyl]-3-fluoro-4-(2-methoxyethoxy)benzamide;
rel-(2aS)-2-[3-[(1S)-2-amino-1-hydroxy-1-phenyl-ethyl]-6-chloro-2-fluoro-phenyl]-3-fluoro-4-(2-methoxyethoxy)benzamide;
2-[6-chloro-3-[2-(cyclopropylmethylamino)-1-hydroxy-1-phenyl-ethyl]-2-fluoro-phenyl]-3-fluoro-4-(2-methoxyethoxy)benzamide;
2-[6-chloro-2-fluoro-3-[1-hydroxy-2-(2-methoxyethylamino)-1-phenyl-ethyl]phenyl]-3-fluoro-4-(2-methoxyethoxy)benzamide;
2-[6-chloro-3-[2-(dimethylamino)-1-hydroxy-1-phenyl-ethyl]-2-fluoro-phenyl]-3-fluoro-4-(2-methoxyethoxy)benzamide;
2-[2-chloro-5-(1-hydroxy-1-phenyl-2-pyrrolidin-1-yl-ethyl)phenyl]-3-fluoro-4-(2-methoxyethoxy)benzamide;
2-[2-chloro-5-[1-hydroxy-1-phenyl-2-(1-piperidyl)ethyl]phenyl]-3-fluoro-4-(2-methoxyethoxy)benzamide;
2-[5-(2-amino-1-hydroxy-1-phenyl-ethyl)-2,4-dichloro-phenyl]-3-methyl-benzamide;
2-[5-(2-amino-1-hydroxy-1-phenyl-ethyl)-2-chloro-4-fluoro-phenyl]-3-methyl-benzamide rel-(2aS)-2-[6-chloro-3-[(1S)-2-(cyclohexylamino)-1-hydroxy-1-phenyl-ethyl]-2-fluoro-phenyl]-3-fluoro-4-(2-methoxyethoxy)benzamide;
rel-(2aR)-2-[6-chloro-3-[(1S)-2-(cyclohexylamino)-1-hydroxy-1-phenyl-ethyl]-2-fluoro-phenyl]-3-fluoro-4-(2-methoxyethoxy)benzamide;
rel-(2aR)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide;
rel-(2aS)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide;
2-[6-chloro-2-fluoro-3-[1-hydroxy-2-(methylamino)-1-phenyl-ethyl]phenyl]-3-fluoro-4-(5-methylisoxazol-3-yl)oxy-benzamide;
2-[5-(2-amino-1-hydroxy-1-pyrimidin-2-yl-ethyl)-2-chloro-phenyl]-3-fluoro-4-(2-methoxyethoxy)benzamide;
and pharmaceutically acceptable salts, enantiomers, diastereomers, tautomers, solvates, isotope substituents, polymorphs, prodrugs or metabolites thereof.

In some embodiments, the compound of formula (I) is selected from:
2-[5-(2-amino-1-hydroxy-1-phenyl-ethyl)-2-chloro-phenyl]-3-methyl-benzamide;
2-[5-(2-amino-1-hydroxy-1-phenyl-ethyl)-2-chloro-phenyl]-3-fluoro-4-(2-methoxyethoxy)benzamide;
2-[2-chloro-5-[1-hydroxy-2-(methylamino)-1-phenyl-ethyl]phenyl]-3-fluoro-4-(2-methoxyethoxy)benzamide;
2-[3-(2-amino-1-hydroxy-1-phenyl-ethyl)-6-chloro-2-methyl-phenyl]benzamide;
2-[5-[(1R)-2-amino-1-hydroxy-1-phenyl-ethyl]-2-chloro-phenyl]-3-fluoro-4-(2-methoxyethoxy)benzamide;
2-[5-[(1-aminocyclopropyl)-hydroxy-phenyl-methyl]-2-chloro-phenyl]-3-fluoro-4-(2-methoxyethoxy)benzamide;
2-[3-(2-amino-1-hydroxy-1-phenyl-ethyl)-2,6-difluoro-phenyl]-3-fluoro-4-(2-methoxyethoxy)benzamide;
2-[2-chloro-5-[1-hydroxy-2-(methylamino)-1-phenyl-ethyl]phenyl]-3-fluoro-4-(2-methoxyethoxy)benzenecarbohydroxamic acid;
rel-(2aS)-2-[3-[(1S)-2-amino-1-hydroxy-1-phenyl-ethyl]-6-chloro-2-methyl-phenyl]-3-fluoro-4-(2-methoxyethoxy)benzamide;
rel-(2-aS)-2-[3-[(1R)-2-amino-1-hydroxy-1-phenyl-ethyl]-6-chloro-2-methyl-phenyl]-3-fluoro-4-(2-methoxyethoxy)benzamide;
2-[6-chloro-3-[2-(cyclohexylamino)-1-hydroxy-1-phenyl-ethyl]-2-fluoro-phenyl]-3-fluoro-4-(2-methoxyethoxy)benzamide;
rel-(2aS)-2-[6-chloro-2-fluoro-3-[(1S)-1-hydroxy-2-(methylamino)-1-phenyl-ethyl]phenyl]-3-fluoro-4-(2-methoxyethoxy)benzamide;
rel-(2aS)-2-[6-chloro-2-fluoro-3-[(1R)-1-hydroxy-2-(methylamino)-1-phenyl-ethyl]phenyl]-3-fluoro-4-(2-methoxyethoxy)benzamide;
2-[3-(2-amino-1-hydroxy-1-phenyl-ethyl)-2,6-dichloro-phenyl]-3-fluoro-4-(2-methoxyethoxy)benzamide;
trans-2-[2-chloro-5-[1-hydroxy-2-[(4-hydroxycyclohexyl)amino]-1-phenyl-ethyl]phenyl]-3-fluoro-4-(2-methoxyethoxy)benzamide;
rel-(2aS)-2-[3-[(1R)-2-amino-1-hydroxy-1-phenyl-ethyl]-6-chloro-2-fluoro-phenyl]-3-fluoro-4-(2-methoxyethoxy)benzamide;
rel-(2aS)-2-[3-[(1S)-2-amino-1-hydroxy-1-phenyl-ethyl]-6-chloro-2-fluoro-phenyl]-3-fluoro-4-(2-methoxyethoxy)benzamide;
2-[6-chloro-3-[2-(cyclopropylmethylamino)-1-hydroxy-1-phenyl-ethyl]-2-fluoro-phenyl]-3-fluoro-4-(2-methoxyethoxy)benzamide;
2-[6-chloro-2-fluoro-3-[1-hydroxy-2-(2-methoxyethylamino)-1-phenyl-ethyl]phenyl]-3-fluoro-4-(2-methoxyethoxy)benzamide;
rel-(2aS)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide;
2-[6-chloro-2-fluoro-3-[1-hydroxy-2-(methylamino)-1-phenyl-ethyl]phenyl]-3-fluoro-4-(5-methylisoxazol-3-yl)oxy-benzamide;
and pharmaceutically acceptable salts, enantiomers, diastereomers, tautomers, solvates, isotope substituents, polymorphs, prodrugs or metabolites thereof.

In the context of the present disclosure, the various embodiments described herein can be combined.

Pharmaceutically acceptable salts of the compounds of formula (I) include those disclosed in the Handbook of Pharmaceutical Salts: Properties, Selection and Use (P. H. Stahl and C. G. Wermuth, Weinheim/Zürich:Wiley-VCH/VHCA, 2002).

In one aspect, the present disclosure relates to enantiomers, diastereomers, tautomers, solvates, isotope substituents, polymorphs, prodrugs or metabolites of the compounds of formula (I).

As used herein, the term "prodrug" is meant to indicate a compound that is converted under physiological conditions or by solvolysis to a biologically active compound described herein. Thus, the term "prodrug" refers to a precursor of a biologically active compound that is pharmaceutically acceptable. In some embodiments, a prodrug is inactive when administered to a subject, but is converted *in vivo* to an active compound, for example, by hydrolysis. The prodrug compound often offers advantages of solubility, tissue compatibility or delayed release in a mammalian organism. The term "prodrug" is also meant to include any covalently bonded carriers, which release the active compound *in vivo* when such prodrug is administered to a mammalian subject. In some embodiments, prodrugs of an active compound, as described herein, are prepared by modifying functional groups present in the active compound in such a way that the modifications are cleaved, either in routine manipulation or *in vivo,* to the parent active compound.

As used herein, the term "solvate" refers to an aggregate of molecules comprising one or more compounds of the present disclosure and one or more solvent molecules. The solvent may be water, in which case the solvate may be a hydrate. Alternatively, the solvent may be an organic solvent. Thus, the compounds of the present disclosure may exist as hydrates, including monohydrate, dihydrate, hemihydrate, sesquihydrate, trihydrate, tetrahydrate, and the like, and as corresponding solvated forms. The disclosed compounds may be true solvates, while in other cases, the disclosed compounds may only remain in water or a mixture of water plus some solvent.

The compounds disclosed herein, or pharmaceutically acceptable salts thereof, may contain one or more chiral carbon atoms, and thus may yield enantiomers, diastereomers, and other stereoisomeric forms. Each chiral carbon atom may be defined as (R)- or (S)- based on stereochemistry. Unless stated otherwise, it is intended that all stereoisomeric forms of the compounds disclosed herein are contemplated by this disclosure. When the compounds described herein contain alkene double bonds, and unless specified otherwise, it is intended that this disclosure includes both E and Z geometric isomers (e.g., cis or trans). Likewise, all possible isomers, as well as their racemic and optically pure forms, and all tautomeric forms are also intended to be included. The term "geometric isomer" refers to E or Z geometric isomers (e.g., cis or trans) of an alkene double bond. The disclosure is intended to include all possible isomers, as well as racemates and optically pure forms thereof. The preparation of the compounds disclosed herein may select racemates, diastereomers or enantiomers as starting materials or intermediates. Optically active isomers may be prepared using chiral synthesizers or chiral reagents, or resolved using conventional techniques, such as crystallization and chiral chromatography.

Conventional techniques for preparing/separating individual isomers include chiral synthesis from suitable optically pure precursors or using chiral reagents/catalysts, separation of racemates/diastereoisomers (or racemates/diastereoisomers of salts or derivatives) using, for example, chiral high-performance liquid chromatography, chiral Supercritical Fluid chromatography (SFC) or recrystallization

As used herein, the term "stereoisomer" refers to a compound consisting of the same atom, bonded by the same bond, but having a different three-dimensional structure. The present disclosure will cover various stereoisomers and mixtures thereof.

As used herein, the term "tautomer" refers to a molecule wherein a proton shift from one atom of a molecule to another atom of the same molecule is possible. The compounds presented herein, in certain embodiments, exist as tautomers. In circumstances where tautomerization is possible, a chemical equilibrium of the tautomers will exist. The exact ratio of the tautomers depends on several factors, including physical state, temperature, solvent, and pH. All tautomeric forms of the compounds disclosed herein will also be included within the scope of the present disclosure.

The present disclosure also encompasses all suitable isotopic substitutions (or isotopic variations) of the compounds of the present disclosure, or pharmaceutically acceptable salts thereof. Isotopic variations of the compounds disclosed herein, or pharmaceutically acceptable salts thereof, are defined as those in which at least one atom is replaced by an atom having the same number of atoms but different in atomic mass from that found frequently in nature. Isotopes that may be incorporated into the compounds of the present disclosure and pharmaceutically acceptable salts thereof include, but are not limited to, isotopes of H, C, N, and O, such as ²H, ³H, ¹¹C, ¹³C, ¹⁴C, ¹⁵N, ¹⁷O, ¹⁸O, ³⁵S, ¹⁸F, ³⁶Cl, and ¹²⁵I. Isotopic variations of the compounds described herein, may be prepared by conventional techniques using suitable isotopic variations of suitable reagents.

As used herein, the terms "crystalline form" and "polymorph" are used interchangeably and refer to crystalline structures in which a compound (or a salt, enantiomer, diastereomer, tautomer, solvate, isotope substituent, polymorph, prodrug or metabolite) can crystallize in different crystal stacking arrangements (all having the same elemental composition). Different crystal forms generally have different X-ray diffraction patterns, infrared spectra, melting points, density hardness, crystal shape, optical and electrical properties, stability and solubility. Recrystallization solvents, crystallization rates, storage temperatures, or other factors may lead to dominance of one of the crystal forms. Polymorphs of compounds can be prepared by crystallization under different conditions.

As used herein, the term "metabolite" refers to derivatives that are also pharmacologically active resulting from the metabolic process of the parent pharmaceutical active ingredient in a subject, for example, such metabolites may result from oxidation, reduction, hydrolysis, amidation, deamidation, esterification, enzymatic cleavage, and the like of the administered compound or salt or prodrug.

In one aspect the present disclosure relates to a compound of formula (I) as defined above, or a pharmaceutically acceptable salt, enantiomer, diastereomer, tautomer, solvate, isotope substituent, polymorph, prodrug or metabolite thereof, for use as a medicament.

The compounds of formula (I) can be administered in the form of a pharmaceutical composition. Accordingly, in one aspect, the present disclosure relates to a pharmaceutical composition comprising a compound of formula (I) or one of its pharmaceutically acceptable salts, enantiomer, diastereomer, tautomer, solvate, isotope substituent, polymorph, prodrug or metabolite thereof. In some embodiments, the compound of formula (I) as described herein is substantially pure, in that it contains less than about 5%, or less than about 1%, or less than about 0.1%, of other organic small molecules, such as contaminating intermediates or by-products that are created, for example, in one or more of the steps of a synthesis method. Administration *in vivo* may be by any routes, including oral, parenteral, topical, buccal, parenteral (e.g. intramuscular, subcutaneous, intradermal, or intravenous), intranasal, sublingual, intratracheal, inhalation, ocular, vaginal and rectal routes. The most suitable form of administration in any given case will depend on the degree and severity of the condition being treated and on the nature of the particular compound being used. For example, disclosed compositions are formulated as a unit dose, and/or are formulated for oral or subcutaneous administration. Depending on the intended mode of *in vivo* administration, said composition may be in a solid dosage form, a semi-solid dosage form or a liquid dosage form, the list being not limitative. In some embodiments, the pharmaceutical composition is a solid dosage form. Exemplary solid dosage forms include tablets, capsules, stick-packs, dragees, sachets, lozenges, powders, pills, or granules. Preferred solid dosage forms include tablets, capsules and stick-packs, tablets being especially preferred.

In some embodiments, the pharmaceutical composition is a liquid dosage form. Exemplary liquid dosage forms include emulsions, microemulsions, solutions, suspensions, syrups and elixirs.

Advantageously, the compositions are administered in unit dosage forms suitable for single administration of precise dosage amounts.

The pharmaceutical compositions may also include, depending on the formulation desired, one or more pharmaceutically acceptable excipient(s). The choice of excipient(s) will to a large extent depend on factors such as the particular mode of administration, the effect of the excipient on solubility and stability, and the nature of the dosage form. The pharmaceutical compositions of the invention can be prepared by conventional methods, as described e.g. in Remington's Pharmaceutical Sciences, 19th Edition (Mack Publishing Company, 1995), incorporated herein by reference.

In some embodiments, the pharmaceutical composition comprises from 0.01 mg to 1000 mg of compound of formula (I) or a pharmaceutically acceptable salt, enantiomer, diastereomer, tautomer, solvate, isotope substituent, polymorph, prodrug or metabolite thereof.

In some embodiments, the pharmaceutical composition further comprises at least one other active pharmaceutical ingredient or its derivatives, prodrugs, solvates, tautomers or stereoisomers thereof as well as the pharmaceutically acceptable salts of each of the foregoing, including mixtures thereof in all ratios, wherein the at least one other active pharmaceutical ingredient is other than a compound of formula (I); preferably, at least one other active pharmaceutical ingredient that is useful in the treatment, prevention, suppression and/or amelioration of medicinal conditions or pathologies for which the compounds of the present disclosure are useful.

In some embodiments, the at least one other active pharmaceutical ingredient is selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and a combination thereof.

As used herein, the term "chemotherapeutic agent" includes, for example, alkylating agents such as nitrogen mustards, alkylsulfonates, nitrosoureas, triazines, ethylenimines and platinum derivatives; antimetabolites such as 5-fluorouracil (5-FU), 6-mercaptopurine (6-MP), cytarabine, capecitabine, fludarabine, gemcitabine, methotrexate, pemetrexed, pentostatin and thioguanine; anthracyclines, such as doxorubicin, daunorubicin, idarubicin and epirubicin; topoisomerase inhibitors, such as topotecan, irinotecan, etoposide and teniposide; mitotic spindle inhibitors, such as plant alkaloids and taxanes; and hormonal agents, such as corticosteroid hormones and sex hormones, the list of chemotherapeutic agent being not limitative.

Non-limiting examples of nitrogen mustards are chlorambucil, cyclophosphamide, ifosfamide, and melphalan. Non-limiting example of alkylsulfonates is busulfan. Non-limiting examples of nitrosoureas are streptozotocin, carmustine, and lomustine. Non-limiting example of triazines is dacarbazine. Non-limiting examples of ethylenimines are thiotepa and altretamine. Non-limiting examples of platinum derivatives are cisplatin, carboplatin and oxaliplatin. Non-limiting examples of plant alkaloids are vinblastine, vincristine and vinorelbine. Non-limiting examples of taxanes are paclitaxel and docetaxel. Non-limiting examples of corticosteroid hormones are prednisone, methylprednisolone and dexamethasone. Non-limiting examples of sex hormones are tamoxifen and leuprolide.

As used herein, "radiotherapeutic treatment" may consist of gamma-radiation, X-ray radiation, electrons or photons, external radiotherapy or internal radiotherapy. As used herein, the term "radiotherapeutic treatment", is also intended to refer to any radiotherapeutic treatment known to one of skill in the art to be effective to treat or ameliorate cancer, without limitation. For instance, the radiotherapeutic treatment can be an agent such as those administered in brachytherapy or radionuclide therapy. Such methods can optionally further comprise the administration of one or more additional cancer therapies, such as, but not limited to, chemotherapies. For instance, the radiotherapeutic treatment can include radioligand therapy (vectorized internal radiotherapy), such as for example but without limitations lutecium-177 (Lu177), vipivotide tetraxetan, andibritumomab tiuxetan.

Examples of immunotherapy agents include without limitations monoclonal antibodies, antibody-drug conjugates, bispecific antibodies, immunoconjugates, checkpoint modulators, cytokines and photoimmunotherapy.

Examples of immunotherapy drug based on oncolytic viruses include without limitations T-VEC (Imlygic^{®}), and other oncolityc virus such as for example: adenovirus, herpes simplex virus, maraba virus, measles, newcastle disease virus, picornavirus, reovirus, vaccinia virus and vesicular stomatitis virus.

Such combination of two or more active pharmaceutical ingredients may be safer or more effective than either compound of formula (I) alone, or the combination is safer or more effective than it would be expected based on the additive properties of the individual drugs (at least one other active pharmaceutical ingredient and compound of formula (I)). Such at least one other active pharmaceutical ingredient may be administered, by a route and in an amount commonly used contemporaneously or sequentially with a compound of formula (I) of the present disclosure. When a compound of formula (I) of the present disclosure is used contemporaneously with at least one other active pharmaceutical ingredient, a combination product containing such at least one other active pharmaceutical ingredient and the compound of formula (I) of the present disclosure - also referred to as "fixed dose combination" - is preferred. However, combination therapy also includes therapies in which the compound of formula (I) of the present disclosure and at least one other active pharmaceutical ingredient are administered on different overlapping schedules. It is contemplated that when used in combination with at least one other active pharmaceutical ingredient, the compound of formula (I) of the present disclosure or the at least one other active pharmaceutical ingredient or both may be used effectively in lower doses than when each is used alone. Accordingly, the pharmaceutical compositions of the present invention include those that contain at least one other active pharmaceutical ingredient, in addition to a compound of formula (I) of the invention.

Consequently, in one aspect, the present disclosure relates to a pharmaceutical composition which comprises a compound of formula (I) according to the invention or a pharmaceutically acceptable salt, enantiomer, diastereomer, tautomer, solvate, isotope substituent, polymorph, prodrug or metabolite thereof, and at least one of the active pharmaceutical ingredients described above as combination partners, optionally together with one or more pharmaceutically acceptable excipients.

The compounds of formula (I) of the invention or pharmaceutically acceptable salts, enantiomers, diastereomers, tautomers, solvates, isotope substituents, polymorphs, prodrugs or metabolite thereof, and the additional active pharmaceutical ingredient(s) to be combined therewith may both be present together in one formulation, for example a tablet or capsule, or separately in two identical or different formulations, for example as so-called kit-of-parts.

In one aspect the present disclosure relates to a compound of formula (I) as defined above, or a pharmaceutically acceptable salt, enantiomer, diastereomer, tautomer, solvate, isotope substituent, polymorph, prodrug or metabolite thereof, for use for the treatment or prevention of a cancer.

In one aspect, the present disclosure relates to the use of a compound of formula (I) as defined above, or a pharmaceutically acceptable salt, enantiomer, diastereomer, tautomer, solvate, isotope substituent, polymorph, prodrug or metabolite thereof, in the manufacture of a medicament for the treatment or prevention of a cancer.

In one aspect, the present disclosure relates to a method of treating or preventing a cancer in a patient, comprising administering to the patient, a therapeutically effective amount of a compound of formula (I) as defined above, or a pharmaceutically acceptable salt, enantiomer, diastereomer, tautomer, solvate, isotope substituent, polymorph, prodrug or metabolite thereof.

In one aspect, the present disclosure relates to a pharmaceutical composition comprising a compound of formula (I) as defined above, or a pharmaceutically acceptable salt, enantiomer, diastereomer, tautomer, solvate, isotope substituent, polymorph, prodrug or metabolite thereof, for use for the treatment or prevention of a cancer.

In one aspect, the present disclosure relates to the use of a pharmaceutical composition comprising a compound of formula (I) as defined above, or a pharmaceutically acceptable salt, enantiomer, diastereomer, tautomer, solvate, isotope substituent, polymorph, prodrug or metabolite thereof, in the manufacture of a medicament for the treatment or prevention of a cancer.

In one aspect, the present disclosure relates to a method of treating or preventing a cancer in a patient, comprising administering to the patient a pharmaceutical composition comprising a therapeutically effective amount of a compound of formula (I) as defined above, or a pharmaceutically acceptable salt, enantiomer, diastereomer, tautomer, solvate, isotope substituent, polymorph, prodrug or metabolite thereof.

In some embodiments, the cancer is a solid tumor. In some embodiments, the cancer is a liquid tumor. In some embodiments, the cancer is selected from adrenal cancer, adrenocortical cancer, anal cancer, bile duct cancer, bladder cancer, blood related cancer, bone cancer, brain cancer, breast cancer, male breast cancer, cervical cancer, colon cancer, colorectal cancer, embryonal cancer, endometrial cancer, uterine cancer, esophageal cancer, eye cancer, gastric cancer, head and neck cancer, kidney cancer, liver cancer, lung cancer, mesothelioma, ovarian cancer, pancreatic cancer, peripheral nervous system cancer, prostate cancer, gallbladder cancer, rhabdoid tumors, soft tissue sarcoma and sarcoma, skin cancer, melanoma, penile cancer, rectal cancer, small intestine cancer, testicular cancer, urethral cancer, germ cell tumors, childhood cancer and thyroid cancer.

In some embodiments, the pharmaceutical composition useful for the treatment or prevention of a cancer further comprises at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and a combination thereof.

In embodiments that involve administering to a patient a compound of formula (I) as defined above, or a pharmaceutically acceptable salt, enantiomer, diastereomer, tautomer, solvate, isotope substituent, polymorph, prodrug or metabolite thereof, the expressions "effective amount", "therapeutically effective amount", "amount effective to treat" or "pharmaceutically effective amount" denote the amount of compound of formula (I) as defined above, or a pharmaceutically acceptable salt, enantiomer, diastereomer, tautomer, solvate, isotope substituent, polymorph, prodrug or metabolite thereof, which is needed to inhibit or reverse a disease condition (e.g., to treat cancer or fibrosis related diseases). Determining an effective amount specifically depends on such factors as toxicity and efficacy of the medicament. These factors will differ depending on other factors such as potency, relative bioavailability, patient body weight, severity of adverse side-effects and preferred mode of administration. Toxicity may be determined using methods well known in the art. Efficacy may be determined utilizing the same guidance. An effective amount, therefore, is an amount that is deemed by the clinician to be toxicologically tolerable, yet efficacious. This amount can be a fixed dose for all subjects being treated, or can vary depending upon the physical condition of the subject to be treated, a professional assessment of the medical situation, and other relevant factors.

As used herein, the term "treatment" or "treating" refers to any process, action, application, therapy, or the like, wherein the patient is under aid, in particular, medical, or veterinarian aid with the object of improving the patient's condition, in particular leading to a cure or a treatment which alleviates, improves and/or eliminates, reduces and/or stabilizes the symptoms of a disease or the suffering that it causes, either directly or indirectly.

As used herein, the term "prevent", "prevention" or "prophylaxis" or "preventive treatment" or "prophylactic treatment" includes a treatment leading to the prevention of a disease as well as a treatment reducing and/or delaying the incidence of a disease or the risk of the disease occurring.

As used herein, "patient" or "subject" refers to a human individual or an animal different from a human. The patient is for example a human or an animal liable to have cancer and/or fibrosis related disease or suffering from such diseases. The patient is advantageously a human being. The patient may be a child (human patient aged 18 or under) or an adult (human patient over 18). In another embodiment, the subject is a non-human animal including, but are not limited to dog, cat, guinea pig, rabbit, rat, mouse, horse, cattle, bear, cow, ape, monkey, orangutan, and chimpanzee, and so on. In the context of the present disclosure the terms "patient" and "subject" are used interchangeably

In one aspect the present disclosure relates to a compound of formula (I) as defined above, or a pharmaceutically acceptable salt, enantiomer, diastereomer, tautomer, solvate, isotope substituent, polymorph, prodrug or metabolite thereof, for use for the treatment or prevention of a fibrosis related disease.

In one aspect, the present disclosure relates to the use of a compound of formula (I) as defined above, or a pharmaceutically acceptable salt, enantiomer, diastereomer, tautomer, solvate, isotope substituent, polymorph, prodrug or metabolite thereof, in the manufacture of a medicament for the treatment or prevention of a fibrosis related disease.

In one aspect, the present disclosure relates to a method of treating or preventing a fibrosis related disease in a patient, comprising administering to the patient an effective amount of a compound of formula (I) as defined above, or a pharmaceutically acceptable salt, enantiomer, diastereomer, tautomer, solvate, isotope substituent, polymorph, prodrug or metabolite thereof.

In one aspect the present disclosure relates to a pharmaceutical composition comprising a compound of formula (I) as defined above, or a pharmaceutically acceptable salt, enantiomer, diastereomer, tautomer, solvate, isotope substituent, polymorph, prodrug or metabolite thereof, for use for the treatment or prevention of a fibrosis related disease.

In one aspect, the present disclosure relates to the use of a pharmaceutical composition comprising a compound of formula (I) as defined above, or a pharmaceutically acceptable salt, enantiomer, diastereomer, tautomer, solvate, isotope substituent, polymorph, prodrug or metabolite thereof, in the manufacture of a medicament for the treatment or prevention of a fibrosis related disease.

In one aspect, the present disclosure relates to a method of treating or preventing a fibrosis related disease in a patient, comprising administering to the patient a pharmaceutical composition comprising an effective amount of a compound of formula (I) as defined above, or a pharmaceutically acceptable salt, enantiomer, diastereomer, tautomer, solvate, isotope substituent, polymorph, prodrug or metabolite thereof.

In some embodiments, fibrosis related diseases include, without limitations, skin fibrosis (such as for example hypertrophic scar or systemic sclerosis), heart fibrosis (such as for example cardiac fibrosis, hypertrophic cardiomyopathy, valvular diseases), bone marrow fibrosis (such as for example myelofibrosis, myelodysplatic syndrome), liver fibrosis (such as for example non-alcoholic steatohepatitis, Alcoholic liver disease, cirrhosis, portal hypertension), retroperitoneum fibrosis, gut fibrosis (such as for example intestinal fibrosis, Inflammatory bowel disease, enteropathies), joint fibrosis (such as for example arthrofibrosis), brain fibrosis (such as glial scar), nervous system fibrosis, eye fibrosis (such as for example subretinal fibrosis, epiretinal fibrosis, vision loss), lung fibrosis (such as for example idiopathic pulmonary fibrosis, ANCA-associated vasculitis, pulmonary hypertension), cystic fibrosis, mediastinum fibrosis, pancreas fibrosis (such as for example pancreatic fibrosis, chronic pancreatitis, duct obstruction) and kidney fibrosis (such as for example renal fibrosis, ANCA-associated vasculitis, nephrogenic systemic fibrosis, chronic kidney disease)..

In some embodiments, the doses of the composition comprising at least one compound of formula (I) as described herein differ, depending upon the patient's (e.g., human) condition, that is, stage of the disease, general health status, age, and other factors that a person skilled in the medical art will use to determine dose. In some instances, pharmaceutical compositions are administered in a manner appropriate to the disease to be treated as determined by skilled practitioner. An appropriate dose and a suitable duration and frequency of administration will be determined by such factors as the condition of the patient, the type and severity of the patient's disease, the particular form of the active ingredient, and the method of administration. In general, an appropriate dose and treatment regimen provides the composition(s) in an amount sufficient to provide therapeutic and/or prophylactic benefit (e.g., an improved clinical outcome, such as more frequent complete or partial remissions, or longer disease-free and/or overall survival, or a lessening of symptom severity). Optimal doses are generally determined using experimental models and/or clinical trials. In some embodiments, the optimal dose depends upon the body mass, weight, or blood volume of the patient and on the activity of the compound of formula (I) - or pharmaceutically acceptable salt, enantiomer, diastereomer, tautomer, solvate, isotope substituent, polymorph, prodrug or metabolite thereof.

In one aspect, the present disclosure provides a method of modulating one or more of proteins encompassed by, or related to, the Hippo pathway in a subject, comprising administering to a subject in need thereof a compound of formula (I), or a pharmaceutically acceptable salt thereof.

In some embodiments, a modulator of the Hippo pathway is a modulator of a core protein of the Hippo pathway. In some embodiments, a modulator of the Hippo pathway is a modulator of YAP. In some embodiments, a modulator of the Hippo pathway is a modulator of TAZ. In some embodiments, a modulator of the Hippo pathway is a modulator of TEAD. In some embodiments, the compound of formula (I) is a modulator of the Hippo pathway, in particular a modulator of a core protein of the Hippo pathway. In some embodiments, the compound of formula (I) is a modulator of YAP. In some embodiments, the compound of formula (I) is a modulator of TAZ. In some embodiments, the compound of formula (I) is a modulator of TEAD.

In one aspect, the present disclosure provides a method of inhibiting one or more of proteins encompassed by, or related to, the Hippo pathway in a subject, comprising administering to a subject in need thereof a compound of formula (I), or a pharmaceutically acceptable salt thereof.

In some embodiments, an inhibitor of the Hippo pathway is an inhibitor of a core protein of the Hippo pathway. In some embodiments, an inhibitor of the Hippo pathway is an inhibitor of YAP. In some embodiments, an inhibitor of the Hippo pathway is an inhibitor of TAZ. In some embodiments, an inhibitor of the Hippo pathway is an inhibitor of TEAD. In some embodiments, the compound of formula (I) is an inhibitor of the Hippo pathway, in particular an inhibitor of a core protein of the Hippo pathway. In some embodiments, the compound of formula (I) is an inhibitor of YAP. In some embodiments, the compound of formula (I) is an inhibitor of TAZ. In some embodiments, the compound of formula (I) is an inhibitor of TEAD.

In one aspect, the present disclosure provides a method of activating one or more of proteins encompassed by, or related to, the Hippo pathway in a subject, comprising administering to a subject in need thereof a compound of formula (I), or a pharmaceutically acceptable salt thereof.

In some embodiments, an activator of the Hippo pathway is an activator of a core protein of the Hippo pathway. In some embodiments, an activator of the Hippo pathway is an activator of YAP. In some embodiments, an activator of the Hippo pathway is an activator of TAZ. In some embodiments, an activator of the Hippo pathway is an activator of TEAD. In some embodiments, the compound of formula (I) is an activator of the Hippo pathway, in particular an activator of a core protein of the Hippo pathway. In some embodiments, the compound of formula (I) is an activator of YAP. In some embodiments, the compound of formula (I) is an activator of TAZ. In some embodiments, the compound of formula (I) is an activator of TEAD.

In one aspect, provided herein are compound of formula (I) as disclosed herein or a pharmaceutically acceptable salt thereof, or the pharmaceutical composition thereof as described herein, for treating or preventing a disease or disorder by modulating or inhibiting or activating one or more of proteins encompassed by, or related to, the Hippo pathway, in a subject in need thereof. In yet another aspect, provided herein are uses of a compound of formula (I) as disclosed herein or a pharmaceutically acceptable salt thereof, or the pharmaceutical composition thereof as described herein, in the preparation of a medicament for treating or preventing a disease or disorder by modulating or inhibiting or activating one or more of proteins encompassed by, or related to, the Hippo pathway, in a subject in need thereof.

In some embodiments, a modulator of the Hippo pathway is a modulator of a core protein of the Hippo pathway. In some embodiments, a modulator of the Hippo pathway is a modulator of YAP. In some embodiments, a modulator of the Hippo pathway is a modulator of TAZ. In some embodiments, a modulator of the Hippo pathway is a modulator of TEAD. In some embodiments, the compound of formula (I) is a modulator of the Hippo pathway, in particular a modulator of a core protein of the Hippo pathway. In some embodiments, the compound of formula (I) is a modulator of YAP. In some embodiments, the compound of formula (I) is a modulator of TAZ. In some embodiments, the compound of formula (I) is a modulator of TEAD.

In some embodiments, an inhibitor of the Hippo pathway is an inhibitor of a core protein of the Hippo pathway. In some embodiments, an inhibitor of the Hippo pathway is an inhibitor of YAP. In some embodiments, an inhibitor of the Hippo pathway is an inhibitor of TAZ. In some embodiments, an inhibitor of the Hippo pathway is an inhibitor of TEAD. In some embodiments, the compound of formula (I) is an inhibitor of the Hippo pathway, in particular an inhibitor of a core protein of the Hippo pathway. In some embodiments, the compound of formula (I) is an inhibitor of YAP. In some embodiments, the compound of formula (I) is an inhibitor of TAZ. In some embodiments, the compound of formula (I) is an inhibitor of TEAD. In some embodiments, an activator of the Hippo pathway is an activator of a core protein of the Hippo pathway. In some embodiments, an activator of the Hippo pathway is an activator of YAP. In some embodiments, an activator of the Hippo pathway is an activator of TAZ. In some embodiments, an activator of the Hippo pathway is an activator of TEAD. In some embodiments, the compound of formula (I) is an activator of the Hippo pathway, in particular an activator of a core protein of the Hippo pathway. In some embodiments, the compound of formula (I) is an activator of YAP. In some embodiments, the compound of formula (I) is an activator of TAZ. In some embodiments, the compound of formula (I) is an activator of TEAD.

### General synthetic schemes

The compounds of formula (I) can be prepared using the synthetic transformations illustrated in schemes I and II. Starting materials are commercially available or may be prepared by procedures described herein (schemes III, IV, V), by procedures described in the literature, or by procedures well known to one skilled in the art of organic chemistry. All general synthetic schemes are illustrated with A = phenyl and R₄ = H.

In scheme I, the coupling reaction, starting from the bromo derivative (prepared following scheme III) can be performed by a Suzuki or Stille reaction using either the free amino-alcohol derivative (step a) or the corresponding protected version (step c). The Suzuki coupling can be performed with the corresponding boronate or boronic acid (commercially available or prepared following scheme V) using classical palladium catalysts (such as Pd(dppf)Cl₂, XPHOS PD G2/RUPHOS, APHOS PD G3, Pd₂dba₃/N-XANTPHOS, etc.) in the presence of a base (such as potassium carbonate, tri-potassium phosphate, etc.). The Stille coupling can be performed with the corresponding stannyl derivative (commercially available or prepared following scheme V) using classical palladium catalysts (such as Pd(P(tBu)₃)₂, etc.). In steps b and c, when R₉ is nitrile, the hydrolysis is performed using Ghaffar-Parkins reagent to give the corresponding amide group. If needed a final cleavage of the protecting group is performed using either acidic or basic medium (step e).

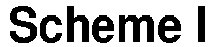

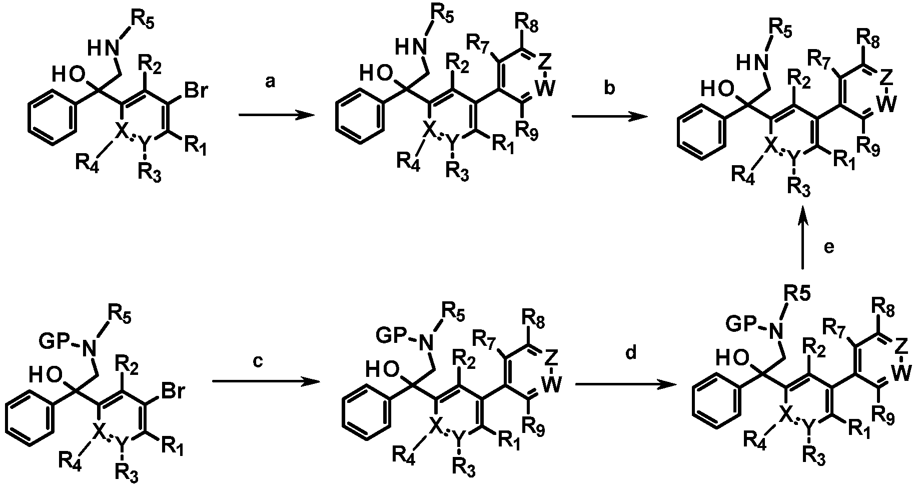

In scheme II, step a, the bromo derivative (prepared in scheme III) is converted into the corresponding boronate or boronic acid using either bis-pinacol-borane or di-boric acid. In step b, the Suzuki coupling with the appropriate halogeno-aryl can be performed using classical palladium catalysts (such as Pd(dppf)Cl₂, XPHOS PD G2/RUPHOS, APHOS PD G3, Pd2dba3/N-XANTPHOS etc.). In step c, if needed, hydrolysis of the nitrile is performed using Ghaffar-Parkins reagent. The same route can also be used starting from the protected version of the amino-alcohol derivative (step d - f). Then a final deprotection is performed using either acidic or basic medium (step g).

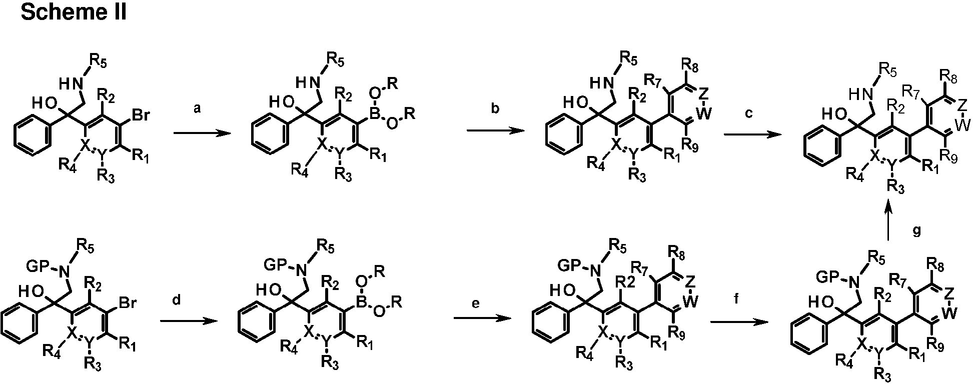

In Scheme III, step a, the formation of acylchloride derivative is done from benzoic acid analogs (commercially available or described in literature) using either sulfonylchloride or oxalyl-chloride. In step b, Fridel-Craft reaction is performed between benzoyl chloride (either obtained from step a or commercially available) and benzene using aluminum chloride to give the corresponding di-aryl ketone. In step c, the formation of the epoxide is performed using trimethylsulfonium iodide in a mixture of dimethylsulfoxide / tetrahydrofuran. In step d, the epoxide ring is opened with the appropriate amine. The amino-alcohol derivative can be protected either on the amino group (such as Boc, Fmoc, phtalimide) (step e) or on the amino-alcohol group (such as cyclic carbamate or oxazolidine) (step f) using well known procedures.

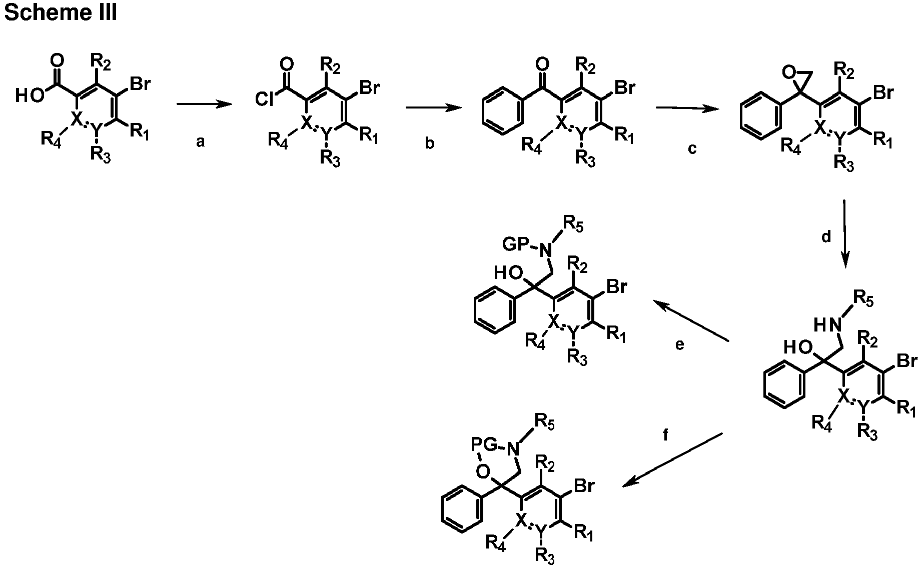

Scheme IV describes the general procedure for the chiral resolution of the starting amino-alcohol derivatives. In step a, the carbamate is formed using either the (+) or (-) menthol chloroformate; the two diastereoisomers obtained can be separated either by crystallization or by column chromatography on silica gel. In step c, menthol is removed in basic conditions.

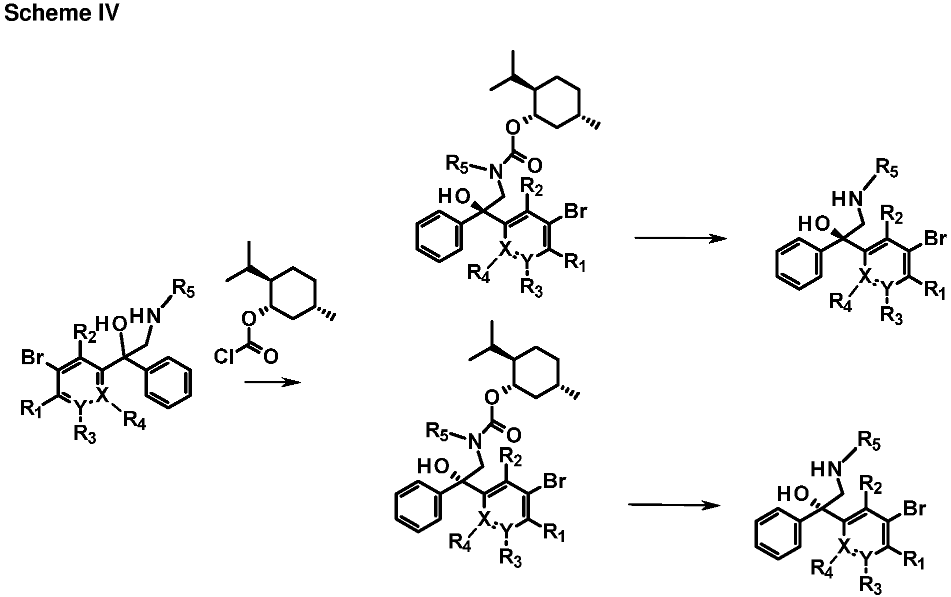

Scheme V describes the general procedure for the preparation of the synthons used in the Suzuki (step a) or Stille coupling (step b). In step a, the boronate derivative is prepared with bis(pinacolato)diboron, using Pd(dppf)Cl₂ as catalyst, in the presence of potassium acetate. In step b, the Stille reactant is formed with MeSnCI using n-butyl-lithium as base.

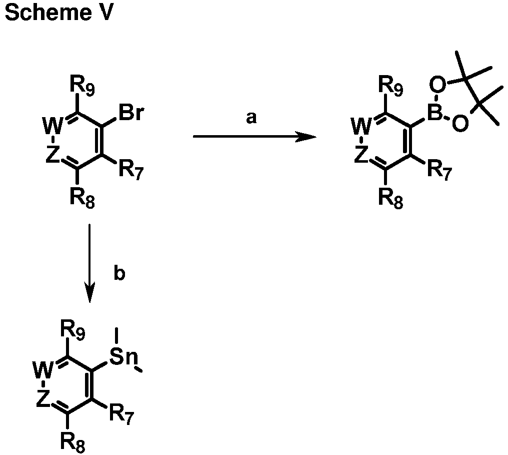

### EXPERIMENTAL PROCEDURES

### ABBREVIATIONS

AcOH = Acetic acid; ACN = Acetonitrile; AlCl₃ = Aluminum chloride; APHOS PD G3 = [4-(Di-tert-butylphosphino)-N,N-dimethylaniline-2-(2'-aminobiphenyl)]palladium(II) methanesulfonate; Boc = t-Butoxycarbonyl; Boc₂O = Di-*tert*-butyl-dicarbonate; br = broad; n-BuLi = n-Butyllithium; CDCl₃ = Chloroform deutered; CV = column volume; d = Doublet; DCM = Dichloromethane; dd = Doublet of doublets; DIPEA = Diisopropylethylamine; DMF = N,N-Dimethylformamide; DMSO = Dimethylsulfoxide; EDCI = 1-Ethyl-3-(3-dimethylaminopropyl)carbodiimide hydrochloride; eq = Equivalent(s); ESI = Electrospray ionization; EtOAc = Ethyl acetate; Et₂O = Diethyl-Ether; EtOH = Ethanol; g = Gram(s); h = Hour(s); HCl = Hydrochloric acid; Hz = Hertz; HCOH = formaldehyde; HCOOH = formic acid; iPrOH = Isopropanol; KOH = potassium hydroxide; K₃PO₄ = Potassium phosphate; LC/MS = Liquid chromatography/mass spectrometry; LC/UV = Liquid chromatography/ultra-violet; LDA = Lithium diisopropylamide; LiAlH₄ = m = Multiplet; min = Minute; MeOH = Methanol; mg = Milligram; MgSO₄ = Magnesium sulfate; min = Minute(s); mL = milliliter; µL = microliter, mmol = Millimole; mp = Melting point; MW = Microwave; N = Normal; NaCl = Sodium chloride; NaH = Sodium hydride; NaHCO₃ = Sodium bicarbonate; NaBH₄ = Sodium borihydride; NaOH = Sodium hydroxide; NaOMe = Sodium methylate; Na₂SO₄ = Sodium sulfate; NH₃ = Ammonia; NH₄Cl = Ammonium chloride; NMR = Nuclear Magnetic Resonance; PBS = Phosphate buffered saline; Pd₂(dba)₃ = Bis(dibenzylidenacetone)palladium(0); Ph = Phenyl; ppm = Parts per million; q = Quadruplet; quint = Quintuplet ; Rt = Retention time ; rt = Room temperature; (Pd) RuPhos = Palladium 2-dicyclohexylphosphanyl-2',6'-diisopropoxybiphenyl; s = singlet; SFC = supercritical fluid chromatography; SiO₂ = Silica; SnCl₂ = tin chloride; t = triplet; TEA = Triethylamine; THF = Tetrahydrofuran; XPHOS PD G2 = Chloro(2-dicyclohexylphosphino-2',4',6'-triisopropyl-1,1'-biphenyl)[2-(2'-amino-1,1'-biphenyl)]-palladium(II).

### Analytical and semi-preparative Methods

| **Method** | **Description** |
|---|---|
| *Analytical A* | *Column: KINETEX XB-C18 core-shell (Dimensions: 30 × 3 mm, 2.6 µm)* |
| | *Column temperature: 45 °C* |
| | *Mobile Phase: A 1: Water (0.1 % v*/*v AcOH) B1: ACN (0.1 % v*/*v AcOH)* |
| | *Gradient: from 10% to 100% B1 within 3.15 min* |
| | *Flow rate: 1.4 ml*/*min, UV detection: DAD 210-260 nm, MS detection: ESI positive* |
| *Semi-preparative B* | *Column: Kinetex C18,30 × 150mm 5µm (phenomenex; 330mg),* |
| | *Flowrate : 42 ml*/*min,* |
| | *Mobile phase: H₂O with 0.1 % HCOOH* / *ACN + 0.1* % *HCOOH (% ACN : 0 to 0.5 min, 10% ; 0.5 to 1 min, 10 to 30% ; 1 to 8 min, 30 to 45%; 8 to 9 min,45 to 90%; 9 to 9.5 min, 90 to 100%)* |
| *Analytical C* | *Column : ACQUITY BEH Shield RP18* |
| | *Column temperature: 45 °C* |
| | *Mobile Phase: A: Water (0.1 % v*/*v AcOH) B: ACN (0.1 % v*/*v AcOH)* |
| | *Gradient: from 5% to 95% B within 2.5 min* |
| | *Flow rate: 0.8 ml*/*min, UV detection: DAD 210-260 nm, MS detection: ESI positive* |
| *Chiral D* | *Column : Chiralpak I8, 250 *4.6mm *5pm* |
| | *Column temperature: -4 °C* |
| | *Mobile phase : 97% Heptane* / *3% iPrOH +0.1*% *Ethanolamine* |
| | *Flowrate : 1ml*/*min* , *UV detection λ = 240nm* |
| *Analytical E* | *Column: Waters X bridge C18 50 × 2.1 mm × 3.5 µm* |
| | *Column temperature: 45 °C* |
| | *Mobile phase: A = H₂O + 0.1% NH₃ B = ACN + 0.1% NH3* |
| | *Flowrate : 0.5ml*/*min* , *UV detection A = 210-260nm* |

### Example 1: 2-[5-(2-amino-1-hydroxy-1-phenyl-ethyl)-2-chloro-phenyl]benzamide

### Step a: (3-bromo-4-chloro-phenyl)-phenyl-methanone

In a 100ml round bottom flask, 3-bromo-4-chlorobenzoyl chloride (3.13 g, 12.3 mmol, 1 eq.) was dissolved in benzene (41.0 mL, 462 mmol). Aluminum chloride (1.74 g, 13.1 mmol, 1.1 eq.) was added portionwise and the reaction mixture was heated at reflux for 1 h (80°C). The reaction mixture was poured onto a mixture of ice (500 g) and concentrated HCl (1M, 60 mL). The mixture was extracted with ethyl acetate (3 times). The combined organic layers were washed with brine and dried over MgSO₄. The reaction mixture was filtered and concentrated to dryness to give (3-bromo-4-chloro-phenyl)-phenyl-methanone (3.51 g, 96.2%) as a white solid. ¹H NMR (400 MHz, DMSO-d6) δ ppm 8.04 (d, J=1.98 Hz, 1 H), 7.82 (d, J=8.25 Hz, 1 H), 7.78 (m, 1 H), 7.75 (d, J=1.43 Hz, 1 H), 7.73 (m, 1 H), 7.70 (m, 1 H), 7.59 (s, 2 H)

### Step b: 2-(3-bromo-4-chloro-phenyl)-2-phenyl-oxirane

In a 100 ml round-bottomed flask, under an argon atmosphere, trimethylsulfoxonium iodide 98% (5.21 g, 23.7 mmol, 2 eq.) was dissolved in DMSO (11.3 mL, 159 mmol) (Dry, Acroseal). NaH (60% in oil) (0.947 g, 23.7 mmol, 2 eq.) was added portionwise and the mixture was stirred for 30 min at rt until rapid evolution of hydrogen has ceased. A solution of (3-bromo-4-chloro-phenyl)-phenyl-methanone (3.50 g, 11.8 mmol. 1 eq.) in THF (6.78 mL)/DMSO (2.90 mL) was added, then the mixture was stirred at rt for 1 h. The mixture was poured onto water and extracted three times with Et₂O. The combined organic layers were washed with water (twice), dried over MgSO₄, filtered and concentrated in vacuo to give 2-(3-bromo-4-chloro-phenyl)-2-phenyl-oxirane (2.888 g, 78.8%) as a yellow liquid. ¹H NMR (400 MHz, DMSO-*d*₆) δ ppm 7.66 (d, *J*=1.98 Hz, 1 H), 7.63 (d, *J*=8.36 Hz, 1 H), 7.37 (m, 5 H), 7.32 (dd, *J*=8.36, 2.09 Hz, 1 H), 3.34 (d, *J*=4.80 Hz, 1 H), 3.31 (d, *J*=4.80 Hz, 1 H).

### Step c: 2-amino-1-(3-bromo-4-chloro-phenyl)-1-phenyl-ethanol

Into 2 big Q-tube, under argon atmosphere, 2-(3-bromo-4-chloro-phenyl)-2-phenyl-oxirane (2.888 g, 9.3 mmol, 1 eq.) was dissolved in a 7N solution of ammoniac in methanol (26.7 mL, 187 mmol, 20 eq.). The reaction mixture was stirred at 60°C for 20h. The reaction mixture was concentrated in vacuo to give crude as a white gum. The crude was purified via chromatography (Cartridge Biotage Sfar 25 g / 20 µm, DCM/(DCM/MeOH+NH3 9:1) 100:0 over 5 CV, 100:0 to 60:40 over 15 CV, 60:40 over 5 CV, Flow rate 80 ml / min). The fractions were collected, concentrated, to give 2-amino-1-(3-bromo-4-chloro-phenyl)-1-phenyl-ethanol (2.22 g, 59.2%) as a beige solid. ¹H NMR (400 MHz, DMSO-d6) δ ppm 7.79 (d, J=2.09 Hz, 1 H), 7.52 (d, J=8.10 Hz, 1 H), 7.43 (m, 3 H), 7.31 (m, 2 H), 7.20 (tt, J=7.30, 1.00 Hz, 1 H), 5.78 (br s, 1 H), 3.26 (d, J=13.40 Hz, 1 H), 3.20 (d, J=13.30 Hz, 1 H), 1.37 (br s, 2 H).

### Step d: 2-[5-(2-amino-1-hydroxy-1-phenyl-ethyl)-2-chloro-phenyl]benzamide

In a MW vial, under argon, a solution of 2-amino-1-(3-bromo-4-chloro-phenyl)-1-phenyl-ethanol (250 mg, 0.77 mmol, 1 eq.) and K₃PO₄ (650 mg, 3.1 mmol, 4 eq.) in 1,4-dioxane (2.75 mL)/water (0.875 mL) was degassed for 2 min. Then Pd(dppf)Cl₂, CH₂Cl₂ COMPLEX (61 mg, 0.075 mmol, 0.10 eq.) and 2-boronobenzamide (457.5 mg, 2.77 mmol, 3.6 eq.) were added and the mixture was degassed for 2 min before being irradiated at 100°C for 2 h. The reaction mixture was diluted with EtOAc and saturated NaHCO₃ then extracted with EtOAc. The combined organic layers were washed with brine (twice), then dried over MgSO₄, filtered and concentrated to give 46.5 mg as a brown solid. The crude was purified by chromatography (Biotage Isolera. Solid deposit in DCM. Monitor 220+254 nm, Cartridge Biotage Sfar Duo 10 g / 20 µm, DCM/(DCM:MeOH+NH3 9:1) 100:0 over 5 CV, 100:0 to 0:100 over 15 CV, 0:100 over 5 CV, Flow rate 40 ml / min). The fractions were collected, concentrated, and dried overnight (44°C) to give 2-[5-(2-amino-1-hydroxy-1-phenyl-ethyl)-2-chloro-phenyl]benzamide (110.7 mg, 39%) as a beige solid. ¹H NMR (500 MHz, DMSO-d6) δ ppm 7.58 (br d, J=1.38 Hz, 1 H), 7.46 (m, 1 H), 7.42 (m, 4 H), 7.35 (s, 2 H), 7.28 (t, J=7.70 Hz, 2 H), 7.22 (dd, J=7.63, 1.17 Hz, 1 H), 7.17 (m, 1 H), 6.91 (m, 2 H), 5.35 (m, 1 H), 3.25 (m, 1 H), 3.17 (m, 1 H), 1.37 (m, 2 H).

### Examples 1a and 1b: 2-[2-chloro-5-[rel-(1S)-2-amino-1-hydroxy-1-phenylethyl]phenyl]benzamide and 2-[2-chloro-5-[rel-(1R)-2-amino-1-hydroxy-1-phenylethyl]phenyl]benzamide

The two title compounds were isolated by SFC chiral chromatography. The racemate mixture was dissolved to 20.3 mg/mL in EtOH and was then purified by SFC (Column Details ChiralPak AD-H (30x250mm, 5µm), Column Temperature 40°C, Flow Rate 120 mL/min, BPR 100 Bar, Detector Wavelength 226 nm, Injection Volume 2700 uL, Isocratic Conditions MeOH:EtOH:iPrOH(1:1:1):CO₂ 20% (0.2%% v/v isopropylamine)). The combined fractions of each were collected, then evaporated and dried in a vacuum oven at 30°C and 20mbar until constant weight to afford 2-[2-chloro-5-[rel-(1S)-2-amino-1-hydroxy-1-phenylethyl]phenyl]benzamide (compound 1a) (24.4 mg, ee 100 %) as a white solid, ¹H NMR (500 MHz, DMSO-*d*₆) δ ppm 7.59 (s, 1 H), 7.56 (d, *J*=7.01 Hz, 1 H), 7.50 (m, 1 H), 7.46 (m, 1 H), 7.44 (m, 3 H), 7.37 (m, 2 H), 7.30 (m, 2 H), 7.26 (m, 1 H), 7.19 (m, 2 H), 5.67 (m, 1 H), 3.23 (m, 1 H), 3.13 (d, *J*=13.30 Hz, 1 H) and 1b 2-[2-chloro-5-[rel-(1R)-2-amino-1-hydroxy-1-phenyl-ethyl]phenyl]benzamide (compound 1b) (18.6 mg, ee 100%) as a white solid, ¹H NMR (500 MHz, DMSO-*d*₆) δ ppm 7.59 (s, 1 H), 7.56 (d, *J*=7.15 Hz, 1 H), 7.49 (s, 1 H), 7.47 (d, *J*=7.98 Hz, 1 H), 7.44 (m, 3 H), 7.37 (m, 2 H), 7.30 (m, 2 H), 7.26 (m, 1 H), 7.20 (m, 2 H), 5.67 (m, 1 H), 3.23 (m, 1 H), 3.13 (d, *J*=13.30 Hz, 1 H).

### Example 2: 2-[5-(2-amino-1-hydroxy-1-phenyl-ethyl)-2-chloro-phenyl]-3-methyl-benzamide

### Step a: 3-methyl-2-(4,4,5,5-tetramethyl-1,3,2-dioxaborolan-2-yl)benzonitrile

In a 250 mL round bottom flask under argon, 2-bromo-3-methylbenzonitrile (2.50 g, 12.8 mmol, 1 eq.) was dissolved in 1,4-dioxane (50.0 mL). The mixture was degassed under argon for 5 min, then bis(pinacolato)diboron (4.86 g, 19.1 mmol, 1.5 eq.), Pd(dppf)Cl₂, CH₂Cl₂ COMPLEX (1.04 g, 1.27 mmol, 0.1 eq.) and potassium acetate (3.75 g, 38.2 mmol, 3 eq.) were added. The mixture was stirred at 100°C overnight. The mixture was filtered. The solid was washed twice with DCM and twice with EtOAc. The filtrate was evaporated to give a brown oil. The crude was purified by chromatography (Biotage Isolera, Cartridge Biotage Sfär 100 g 20 µm silica, cyclohexane-EtOAc 100-0 / 6 CV (column volume) 100-0 to 92-8 / 15 CV, 92-8/ 6 CV, flow rate 80 ml / min). Fractions were collected and evaporated under reduced pressure to give 3-methyl-2-(4,4,5,5-tetramethyl-1,3,2-dioxaborolan-2-yl)benzonitrile (1.9 g, 45%) as a white powder. ¹H NMR (400 MHz, DMSO-*d*₆) δ ppm 7.63 (m, 1 H), 7.51 (m, 2 H), 3.31 (s, 6 H), 2.41 (s, 3 H).

### Step b: 2-[5-(2-amino-1-hydroxy-1-phenyl-ethyl)-2-chloro-phenyl]-3-methyl-benzonitrile

In a MW vial, under argon, a solution of 2-amino-1-(3-bromo-4-chloro-phenyl)-1-phenyl-ethanol (50.0 mg, 0.153 mmol, 1 eq.) and 3-methyl-2-(4,4,5,5-tetramethyl-1,3,2-dioxaborolan-2-yl)benzonitrile (153 mg, 0.46 mmol, 3 eq.) in toluene (1.5 mL)/water (273 µL) was degassed for 2 min. Then K₃PO₄ (130 mg, 0.6124 mmol, 4 eq.), XPHOS PD G2 (12.1 mg, 0.0154 mmol, 0.1 eq.) and 2-dicyclohexylphosphino-2',6'-di-i-propoxy-1,1'-biphenyl (7.2 mg, 0.015 mmol, 0.1 eq.) were added and the mixture was degassed for 5 min before being irradiated at 100°C for 3 h. The mixture was diluted with a saturated solution of NaHCO₃ and extracted with EtOAc (3x). The combined organic layers were dried over anhydrous Na₂SO₄. After filtration, the filtrate was concentrated under reduced pressure. The crude product was purified by preparative LCMS (Column: Kinetex C18,30 × 150mm 5µm (phenomenex), flowrate : 42 ml/min, Mobile phase: H₂O with 0,1% FA / ACN + 0,1 % FA, sample diluted in MeOH / ACN / Water). Fractions were collected. ACN was evaporated and the mixture was diluted with a saturated solution of NaHCO₃. The aqueous phase was extracted with DCM (3 times). The organic layer was dried over Na₂SO₄, filtered and concentrated to give 2-[5-(2-amino-1-hydroxy-1-phenyl-ethyl)-2-chloro-phenyl]-3-methyl-benzonitrile (24.0 mg, 43.2%) as a pale yellow solid. ¹H NMR (400 MHz, DMSO-d6) δ ppm 7.77 (m, 1 H), 7.68 (m, 1 H), 7.55 (m, 2 H), 7.49 (m, 1 H), 7.43 (m, 3 H), 7.29 (m, 2 H), 7.20 (m, 1 H), 5.75 (br s, 1 H), 3.28 (m, 1 H), 3.18 and 3.17 (d, J=13.50, 1 H), 2.02 and 2.01 (s, 3 H).

### Step c: 2-[5-(2-amino-1-hydroxy-1-phenyl-ethyl)-2-chloro-phenyl]-3-methyl-benzamide

Into a 5 mL round bottom flask, under argon atmosphere, 2-[5-(2-amino-1-hydroxy-1-phenylethyl)-2-chloro-phenyl]-3-methyl-benzonitrile (22 mg, 0.06 mmol, 1 eq.) was dissolved in ethanol (660 µL) and water (220 µL), then Ghaffar-Parkins catalyst (2.6 mg, 0.0061 mmol, 0.1 eq.) was added. The reaction mixture was heated and stirred at 80°C overnight. The mixture was concentrated under reduced pressure. The crude was purified by chromatography (Cartridge Biotage Sfar Duo 5 g / 20 µm, DCM/MeOH/1%NH₃ 100:0 over 4 CV, 100:0 to 90/10 over 15 CV, 90/10 over 4 CV, Flow rate 18 ml / min). The collected fractions were evaporated to give 2-[5-(2-amino-1-hydroxy-1-phenyl-ethyl)-2-chloro-phenyl]-3-methyl-benzamide (15.0 mg, 65.0%) as a white powder. ¹H NMR (500 MHz, DMSO-*d*₆) δ ppm 7.45 - 7.25 (m, 11 H), 7.19 (m, 1 H), 7.08 and 7.06 (br s, 1 H), 5.66 and 5.65 (br s, 1 H), 3.24 (m, 2 H), 3.11 and 3.07 (d, *J*=13.62 Hz, 1 H), 1.95 and 1.91 (s, 3 H).

### Example 2a and 2b: two racemates of 2-[5-(2-amino-1-hydroxy-1-phenyl-ethyl)-2-chloro-phenyl]-3-methyl-benzamide

2-[5-(2-amino-1-hydroxy-1-phenyl-ethyl)-2-chloro-phenyl]-3-methyl-benzamide (35 mg) was separated by chromatography LC (Column: Xbridge C18, 30 × 100mm 5µm, Flowrate : 42 ml/min, Mobile phase: H2O+0,1%NH3/ MeOH + 0.1% NH3, Temperature : 45°C, Gradient: from 10 to 95% of MeOH/NH₃ over 30 min, solvent of solubilization before prep injection: MeOH/DMSO/CAN) to give a mixture of racemates 2a and 2b. Racemate **2a:** (Rt=25.854 min) (17.47 mg, 35.4%) ¹H NMR (500 MHz, DMSO-*d*₆) δ ppm 7.42 (m, 3 H), 7.36 (m, 5 H), 7.30 (t, *J*=7.70 Hz, 2 H), 7.26 (dd, *J*=8.60, 2.13 Hz, 1 H), 7.19 (m, 1 H), 7.08 (s, 1 H), 5.65 (br s, 1 H), 3.20 (d, *J*=13.34 Hz, 1 H), 3.10 (d, *J*=13.30 Hz, 1 H), 1.95 (s, 3 H) and Racemate **2b:** (Rt=26.035min) (13.75 mg, 27.9%) ¹H NMR (500 MHz, DMSO-*d*₆) δ ppm 7.45 (dd, *J*=8.40, 2.20 Hz, 1 H), 7.41 (m, 4 H), 7.33 (m, 3 H), 7.27 (m, 3 H), 7.18 (t, *J*=7.00 Hz, 1 H), 7.06 (br s, 1 H), 5.63 (s, 1 H), 3.28 (d, *J*=13.80 Hz, 2 H), 3.06 (d, *J*=13.48 Hz, 1 H), 1.91 (s, 3 H).

### Example 3: 2-[5-(2-amino-1-hydroxy-1-phenyl-ethyl)-2-chloro-phenyl]-4-methoxy-3-methyl-benzamide

### Step a: 2-bromo-4-fluoro-3-methyl-benzonitrile

In a 250 mL round bottom flask under argon, 2-bromo-4-fluorobenzenecarbonitrile (2.50 g, 12.5 mmol, 1 eq.) was dissolved in THF (59 mL) then the mixture was cooled to -78°C. LDA (9.40 mL) (2M in THF) was added dropwise and the reaction mixture was stirred for 45 minutes at -78°C, methyl-iodide (1.56 mL) was added dropwise, and the reaction mixture was stirred at -78°C for 1h30. The mixture was quenched with diluted 1M HCl, extracted with EtOAc x3. The combined organic layers were washed with brine, dried over MgSO₄, filtered and concentrated under reduced pressure to give a brown solid (2.9 g). The crude was purified via flash chromatography (Cartridge Biotage Sfar 50 g / 60 µm, Cyclohexane/EtOAc 100:0 over 5 CV, 100:0 to, 90:10 over 15 CV , 0:100 over 3 CV, Flow rate 100 ml / min). Fractions were collected, concentrated to give 2-bromo-4-fluoro-3-methyl-benzonitrile (2.4 g, 90.6%) as a light yellow solid. ¹H NMR (400 MHz, DMSO-*d*₆) δ ppm 7.91 (ddq, *J*=8.69, 5.61, 0.55 Hz, 1 H), 7.48 (t, *J*=8.91 Hz, 1 H), 2.34 (d, *J*=2.53 Hz, 3 H).

### Step b: 2-bromo-4-methoxy-3-methyl-benzonitrile

To a stirred solution of 2-bromo-4-fluoro-3-methyl-benzonitrile (1 g, 4.67 mmol, 1 eq.) in MeOH (12.0 mL) under argon, was added NaOMe (379 mg, 7.02 mmol, 1.5 eq). The reaction mixture was stirred at 65°C for 24 h. NaOMe (260 mg, 4.81 mmol, 1.03 eq) was added to the solution and the mixture was stirred for an additional 48 h. The reaction mixture was quenched with a saturated solution of NaHCO₃ and extracted with EtOAc. The organic layers were combined and washed with a saturated solution of NaHCO₃, dried over MgSO₄ and concentrated to give a beige solid. The crude was purified via flash chromatography (Cartridge Biotage Sfar 25 g / 60 µm, Cyclohexane:(cyclohexane/EtOAc 9:1) 100:0 over 5 CV, 100:0 to 0:100 over 15 CV,0:100 over 2CV, Flow rate 80 ml / min). Fractions were collected, concentrated to give 2-bromo-4-methoxy-3-methyl-benzonitrile (597 mg, 56.5%) as a white solid. ¹H NMR (400 MHz, DMSO-*d*₆) δ ppm 7.81 (d, *J*=8.69 Hz, 1 H), 7.18 (d, *J*=8.80 Hz, 1 H), 3.28 (s, 3 H), 2.28 (s, 3 H).

Step c: 4-methoxy-3-methyl-2-(4,4,5,5-tetramethyl-1,3,2-dioxaborolan-2-yl)benzonitrile The compound was prepared using the same procedure as detailed in example 2 step a starting from 2-bromo-4-methoxy-3-methyl-benzonitrile (595 mg, 2.63 mmol, 1 eq.) giving 4-methoxy-3-methyl-2-(4,4,5,5-tetramethyl-1,3,2-dioxaborolan-2-yl)benzonitrile (374 mg, 52.0%) as a white solid. ¹H NMR (400 MHz, DMSO-*d*₆) δ ppm 7.67 (d, *J*=8.58 Hz, 1 H), 7.14 (d, *J*=8.58 Hz, 1 H), 3.86 (s, 3 H), 2.21 (s, 3 H), 1.35 (s, 12 H).

### Step d: 2-[5-(2-amino-1-hydroxy-1-phenyl-ethyl)-2-chloro-phenyl]-4-methoxy-3-methyl-benzonitrile

The compound was prepared using the same procedure as detailed in example 2 step b starting from 2-amino-1-(3-bromo-4-chloro-phenyl)-1-phenyl-ethanol (example 1, step c) (200 mg, 0.551 mmol, 1 eq.) and 4-methoxy-3-methyl-2-(4,4,5,5-tetramethyl-1,3,2-dioxaborolan-2-yl)benzonitrile (374 mg, 1.37 mmol, 2.49 eq.) giving 2-[5-(2-amino-1-hydroxy-1-phenyl-ethyl)-2-chloro-phenyl]-4-methoxy-3-methyl-benzonitrile (88.8 mg, 41.0%) as a white solid. ¹H NMR (500 MHz, DMSO-*d*₆) δ ppm 7.81 and 7.79 (s, 1 H), 7.55 (s, 1 H), 7.55 and 7.54 (m, 1 H), 7.48 and 7.46 (d, *J*=2.34 Hz, 1 H), 7.44 and 7.43 (m, 3 H), 7.40 (d, *J*=2.34 Hz, 0.5 H), 7.39 (t, *J*=1.31 Hz, 0.5 H), 7.28 (m, 2 H), 7.21 and 7.19 (m, 2 H), 5.78 and 5.76 (s, 1 H), 3.93 and 3.92 (s, 3 H), 3.29 and 3.24 (d, *J*=13.50 Hz, 1 H), 3.19 and 3.17 (d, *J*=13.50 Hz, 1 H), 1.82 and 1.81 (s, 3 H).

### Step e: 2-[5-(2-amino-1-hydroxy-1-phenyl-ethyl)-2-chloro-phenyl]-4-methoxy-3-methyl-benzamide

The compound was prepared using the same procedure as detailed in example 2 step c starting from 2-[5-(2-amino-1-hydroxy-1-phenyl-ethyl)-2-chloro-phenyl]-4-methoxy-3-methyl-benzonitrile (77.7 mg, 0.198 mmol, 1 eq.) giving 2-[5-(2-amino-1-hydroxy-1-phenyl-ethyl)-2-chloro-phenyl]-4-methoxy-3-methyl-benzamide (63.3 mg, 77.9%) as a white solid. ¹H NMR (500 MHz, DMSO-*d*₆) δ ppm 7.45 - 7.35 (m, 4 H), 7.31 -7.23 (m, 4 H), 7.19 (m, 2 H), 7.03 and 7.01 (d, *J*=4.68 Hz, 1 H), 6.95 and 6.93 (br s, 1 H), 5.64 (m, 1 H), 3.86 and 3.85 (s, 3 H), 3.26 and 3.17 (d, *J*=13.62 Hz, 1 H), 3.09 and 3.05 (d, *J*=13.62 Hz, 1 H), 1.76 and 1.72 (s, 3 H).

### Example 4: 2-[5-(2-amino-1-hydroxy-1-phenyl-ethyl)-2-chloro-phenyl]-4-(2-methoxyethoxy)-3-methyl-benzamide

### Step a: 2-bromo-4-(2-methoxyethoxy)benzonitrile

At 0°C, NaH (60% in oil) (1.3 g, 33 mmol, 1.3 eq.) was added to a solution of 2-methoxyethanol (1.98 mL, 25.1 mmol, 1 eq.) in DMF (100 mL) under argon. After 5 min, 2-bromo-4-fluorobenzenecarbonitrile (5.00 g, 25.0 mmol, 1.00 eq.) was added portionwise and the reaction was stirred at 0°C for 90min. The mixture was quenched with a solution of NH₄Cl sat and extracted with EtOAc. The combined organic layers were washed with NH₄Cl and dried over anhydrous Na₂SO₄. After filtration, the filtrate was concentrated under reduced pressure. The crude was purified by chromatography (Cartridge Biotage Sfär 100 g 20 µm silica, cyclohexane/EtOAc 100-0 / 3 CV (column volume) 100-0 to 80-20 / 12 CV, 80-20 / 6 CV, flow rate 80 ml / min) to give 2-bromo-4-(2-methoxyethoxy)benzonitrile (5.72 g, 89.3%) as a purple solid. ¹H NMR (400 MHz, DMSO-*d*₆) δ ppm 7.85 (d, *J*=8.80 Hz, 1 H), 7.48 (d, *J*=2.42 Hz, 1 H), 7.14 (dd, *J*=8.75, 2.48 Hz, 1 H), 4.23 (m, 2 H), 3.66 (m, 2 H), 3.30 (s, 3 H).

### Step b: 2-bromo-4-(2-methoxyethoxy)-3-methyl-benzonitrile

In a 250 mL three necked flask, dried under argon, 2-bromo-4-(2-methoxyethoxy)benzonitrile (5.91 g, 23.1 mmol, 1 eq.) was dissolved in THF (120 mL). Under argon at -78°C, LDA (17.3 mL, 34.6 mmol, 1.5 eq.) in THF was added dropwise and stirred for 60 minutes. At -78°C, methyl-iodide (2.9 mL, 47 mmol, 2 eq.) was added dropwise. The reaction mixture was stirred at -78°C for 1h. The mixture was quenched with diluted NH₄Cl and extracted with EtOAc (x3). The organic layer was dried over Na₂SO₄, filtered and concentrated to give crude. The crude was purified by chromatography (Cartridge Biotage Sfär 100 g 20 µm silica, Cyclohexane/ DCM 100-0 / 4 CV (column volume) 100-0 to 0-100 / 20 CV, 0-10 / 6 CV, flow rate 100 ml / min) to give 2-bromo-4-(2-methoxyethoxy)-3-methyl-benzonitrile (5.58 g, 86.8%) as a white powder. ¹H NMR (400 MHz, DMSO-*d*₆) δ ppm 7.78 (dd, *J*=8.69, 0.55 Hz, 1 H), 7.19 (d, *J*=8.80 Hz, 1 H), 4.24 (m, 2 H), 3.71 (m, 2 H), 3.32 (s, 3 H), 2.29 (s, 3 H).

### Step c: 4-(2-methoxyethoxy)-3-methyl-2-(4,4,5,5-tetramethyl-1,3,2-dioxaborolan-2-yl)benzonitrile

The compound was prepared using the same procedure as detailed in example 2 step a starting from 2-bromo-4-(2-methoxyethoxy)-3-methyl-benzonitrile (5.54 g, 19.9 mmol, 1 eq) giving 4-(2-methoxyethoxy)-3-methyl-2-(4,4,5,5-tetramethyl-1,3,2-dioxaborolan-2-yl)benzonitrile (3.9 g, 38%) as a pale yellow powder. ¹H NMR (400 MHz, DMSO-*d*₆) δ ppm 7.64 (m, 1 H), 7.15 (d, *J*=8.69 Hz, 1 H), 4.20 (m, 2 H), 3.69 (m, 2 H), 3.32 (s, 3 H), 2.21 (s, 3 H), 1.35 (s, 12 H)

### Step d: 2-[5-(2-amino-1-hydroxy-1-phenyl-ethyl)-2-chloro-phenyl]-4-(2-methoxyethoxy)-3-methyl-benzonitrile

The compound was prepared using the same procedure as detailed in example 2 step b starting from 2-amino-1-(3-bromo-4-chloro-phenyl)-1-phenyl-ethanol (example 1, step c) (400 mg, 1.22 mmol, 1 eq.) and 4-(2-methoxyethoxy)-3-methyl-2-(4,4,5,5-tetramethyl-1,3,2-dioxaborolan-2-yl)benzonitrile (1.910 g, 3.673 mmol, 3 eq.) giving 2-[5-(2-amino-1-hydroxy-1-phenyl-ethyl)-2-chloro-phenyl]-4-(2-methoxyethoxy)-3-methyl-benzonitrile (319 mg, 59.6%) as a white powder. ¹H NMR (500 MHz, DMSO-*d*₆) δ ppm 7.77 (d, *J*=8.67 Hz, 1 H), 7.55 (s, 1 H), 7.54 and 7.48 (3, 1 H), 7.42 (m, 3 H), 7.29 (t, *J*=7.70 Hz, 2 H), 7.20 (m, 2 H), 5.74 (br s, 1 H), 4.25 (m, 2 H), 3.72 (t, *J*=4.40 Hz, 2 H), 3.3 (s, 3 H), 3.29 and 3.23 (d, *J*=13.30 Hz, 1 H), 3.18 and 3.16 (d, *J*=13.90 Hz, 1 H), 1.83 and 1.82 (s, 3 H).

### Step e: 2-[5-(2-amino-1-hydroxy-1-phenyl-ethyl)-2-chloro-phenyl]-4-(2-methoxyethoxy)-3-methyl-benzamide

The compound was prepared using the same procedure as detailed in example 2 step c starting from 2-[5-(2-amino-1-hydroxy-1-phenyl-ethyl)-2-chloro-phenyl]-4-(2-methoxyethoxy)-3-methyl-benzonitrile (286 mg, 0.655 mmol, 1 eq.) giving 2-[5-(2-amino-1-hydroxy-1-phenylethyl)-2-chloro-phenyl]-4-(2-methoxyethoxy)-3-methyl-benzamide (190 mg, 63.8%) as a white powder. ¹H NMR (500 MHz, DMSO-*d*₆) δ ppm 7.39 (m, 4 H), 7.28 (m, 4 H), 7.19 (m, 2 H), 7.03 and 7.01 (d, *J*=4.54 Hz, 1 H), 6.94 and 6.91 (br s, 1 H), 5.63 (br s, 1 H), 4.17 (m, 2 H), 3.71 (m, 2 H), 3.33 and 3.32 (s, 3 H), 3.27 and 3.18 (d, *J*=13.34 Hz, 1 H), 3.10 and 3.05 (d, *J*=13.62 Hz, 1 H), 1.77 and 1.73 (s, 3 H).

### Example 4a and 4b: two racemates of 2-[5-(2-amino-1-hydroxy-1-phenyl-ethyl)-2-chloro-phenyl]-4-(2-methoxyethoxy)-3-methyl-benzamide

The two racemates were separated by chromatography LC-UV (Column: Xbridge C18, 30 x 100mm 5µm, Flowrate: 40 mL/min, Mobile phase: H2O + 0.1% NH4OH / MeOH/ACN (1:1) + 0.1% NH4OH, Temperature: 45°C, Gradient: 20 to 95 % in 35 min) (128 mg, 0.281 mmol) to give racemates 4a and 4b. **4a** (Rt: 18 min) (31.7 mg, 0.0348 mmol, 0.124, 12.4% Yield) ¹H NMR (500 MHz, DMSO-*d*₆) δ ppm 7.40 (dt, *J*=8.42, 1.98 Hz, 3 H), 7.36 (d, *J*=8.53 Hz, 1 H), 7.30 (m, 3 H), 7.25 (dd, *J*=8.39, 2.34 Hz, 2 H), 7.19 (tt, *J*=7.40, 5.60 Hz, 1 H), 7.03 (d, *J*=8.53 Hz, 1 H), 6.93 (br s, 1 H), 5.63 (s, 1 H), 4.17 (m, 2 H), 3.71 (t, *J*=4.61 Hz, 2 H), 3.33 (s, 3 H), 3.18 (d, *J*=13.20 Hz, 1 H), 3.09 (d, *J*=13.50 Hz, 1 H), 1.77 (s, 3 H), **4b** (Rt: 20 min) (38.6 mg, 15.1%) ¹H NMR (500 MHz, DMSO-*d*₆) δ ppm 7.44 (dd, *J*=8.50, 2.20 Hz, 1 H), 7.41 (m, 2 H), 7.38 (d, *J*=8.53 Hz, 2 H), 7.28 (m, 2 H), 7.26 (br s, 1 H), 7.19 (m, 2 H), 7.02 (d, *J*=8.67 Hz, 1 H), 6.91 (br s, 1 H), 5.62 (s, 1 H), 4.16 (m, 2 H), 3.70 (t, *J*=4.61 Hz, 2 H), 3.33 (s, 3 H), 3.26 (d, *J*=13.62 Hz, 1 H), 3.05 (d, *J*=13.62 Hz, 1 H), 1.73 (s, 3 H).

### Example 5: 2-[5-(2-amino-1-hydroxy-1-phenyl-ethyl)-2-chloro-phenyl]-3-fluoro-benzamide

### Step a: 3-fluoro-2-(4,4,5,5-tetramethyl-1,3,2-dioxaborolan-2-yl)benzonitrile

The compound was prepared using the same procedure as detailed in example 2 step a starting from 2-bromo-3-fluoro-benzonitrile (5.00 g, 25.0 mmol, 1 eq.) giving 3-fluoro-2-(4,4,5,5-tetramethyl-1,3,2-dioxaborolan-2-yl)benzonitrile (6.63 g, 80.5%) as a white powder. ¹H NMR (400 MHz, DMSO-*d*₆) δ ppm 7.73 (m, 2 H), 7.57 (m, 1 H), 1.34 (s, 12 H).

Step b: 2-[5-(2-amino-1-hydroxy-1-phenyl-ethyl)-2-chloro-phenyl]-3-fluoro-benzonitrile The compound was prepared using the same procedure as detailed in example 2 step b starting from 2-amino-1-(3-bromo-4-chloro-phenyl)-1-phenyl-ethanol (example 1, step c) (300 mg, 0.919 mmol, 1 eq.) and 3-fluoro-2-(4,4,5,5-tetramethyl-1,3,2-dioxaborolan-2-yl)benzonitrile (1.50 g, 4.55 mmol, 4.96 eq.) giving 2-[5-(2-amino-1-hydroxy-1-phenyl-ethyl)-2-chloro-phenyl]-3-fluoro-benzonitrile (97.0 mg, 28.8%) as a white powder. ¹H NMR (400 MHz, DMSO-*d*₆) δ ppm 7.87 (m, 1 H), 7.73 (m, 2 H), 7.61 (m, 2 H), 7.57 and 7.52 (m, 1 H), 7.45 (m, 2 H), 7.29 (m, 2 H), 7.21 (dd, *J*=7.15, 5.72 Hz, 1 H), 5.78 (br s, 1 H), 3.29 (t, *J*=13.53 Hz, 1 H), 3.20 (d, *J*=13.53 Hz, 1 H).

### Step c: 2-[5-(2-amino-1-hydroxy-1-phenyl-ethyl)-2-chloro-phenyl]-3-fluoro-benzamide

The compound was prepared using the same procedure as detailed in example 2 step c starting from 2-[5-(2-amino-1-hydroxy-1-phenyl-ethyl)-2-chloro-phenyl]-3-fluoro-benzonitrile (80.0 mg, 0.218 mmol, 1 eq.) giving 2-[5-(2-amino-1-hydroxy-1-phenyl-ethyl)-2-chlorophenyl]-3-fluoro-benzamide (24.0 mg, 28.6%) as a white powder. ¹H NMR (500 MHz, DMSO-*d*₆) δ ppm 7.71 and 7.70 (br s, 1 H), 7.51 (m, 2 H), 7.40 (m, 5 H), 7.31 (m, 4 H), 7.20 (m, 1 H), 5.68 and 5.66 (br s, 1 H), 3.28 and 3.18 (d, *J*=13.62 Hz, 1 H), 3.13 and 3.11 (d, J=13.62 Hz, 1 H)

### Example 6: 2-[5-(2-amino-1-hydroxy-1-phenyl-ethyl)-2-chloro-phenyl]-3-fluoro-4-(2-methoxyethoxy)benzamide

### Step a: 2-bromo-3-fluoro-4-(2-methoxyethoxy)benzonitrile

The compound was prepared using the same procedure as detailed in example 4 step a starting from 2-bromo-3,4-difluorobenzonitrile (1.5 g, 6.88 mmol, 1 eq.) giving 2-bromo-3-fluoro-4-(2-methoxyethoxy)benzonitrile (1.20 g, 63.6%) as a white powder. ¹H NMR (400 MHz, DMSO-*d*₆) δ ppm 7.80 (dd, *J*=8.80, 1.87 Hz, 1 H), 7.40 (dd, *J*=8.80, 8.03 Hz, 1 H), 4.33 (m, 2 H), 3.70 (m, 2 H), 3.31 (s, 3 H).

### Step b: 3-fluoro-4-(2-methoxyethoxy)-2-(4,4,5,5-tetramethyl-1,3,2-dioxaborolan-2-yl)benzonitrile

The compound was prepared using the same procedure as detailed in example 2 step a starting from 2-bromo-3-fluoro-4-(2-methoxyethoxy)benzonitrile (600 mg, 2.19 mmol, 1 eq.) giving 3-fluoro-4-(2-methoxyethoxy)-2-(4,4,5,5-tetramethyl-1,3,2-dioxaborolan-2-yl)benzonitrile (382 mg, 54.3%) as a pale yellow solid. ¹H NMR (400 MHz, DMSO-*d*₆) δ ppm 7.68 (dd, *J*=8.47, 0.99 Hz, 1 H), 7.42 (t, *J*=8.64 Hz, 1 H), 4.28 (m, 2 H), 3.68 (m, 2 H), 3.30 (s, 3 H), 1.33 (s, 12 H)

### Step c: 2-[5-(2-amino-1-hydroxy-1-phenyl-ethyl)-2-chloro-phenyl]-3-fluoro-4-(2-methoxyethoxy)benzonitrile

The compound was prepared using the same procedure as detailed in example 2 step b starting from 2-amino-1-(3-bromo-4-chloro-phenyl)-1-phenyl-ethanol (example 1, step c) (50.0 mg, 0.153 mmol, 1 eq.) and 3-fluoro-4-(2-methoxyethoxy)-2-(4,4,5,5-tetramethyl-1,3,2-dioxaborolan-2-yl)benzonitrile (240 mg, 0.471 mmol, 3.08 eq.) giving 2-[5-(2-amino-1-hydroxy-1-phenyl-ethyl)-2-chloro-phenyl]-3-fluoro-4-(2-methoxyethoxy)benzonitrile (40.0 mg, 59.3%) as a yellow oil. ¹H NMR (400 MHz, DMSO-*d*₆) δ ppm 7.81 (dd, *J*=8.64, 1.38 Hz, 1 H), 7.53-7.51 (m, 3 H), 7.47-7.42 (m, 3 H), 7.29 (t, J=7.76 Hz, 2 H), 7.20 (m, 1 H), 5.76 (br s, 1 H), 4.34 (m, 2 H), 3.72 (t, *J*=4.29 Hz, 2 H), 3.34 (s, 3 H), 3.26 (d, *J*=13.31 Hz, 1 H), 3.19 (d, J=13.31 Hz, 1 H).

### Step d: 2-[5-(2-amino-1-hydroxy-1-phenyl-ethyl)-2-chloro-phenyl]-3-fluoro-4-(2-methoxyethoxy)benzamide

The compound was prepared using the same procedure as detailed in example 2 step c starting from 2-[5-(2-amino-1-hydroxy-1-phenyl-ethyl)-2-chloro-phenyl]-3-fluoro-4-(2-methoxyethoxy)benzonitrile (40 mg, 0.0907 mmol, 1 eq.) giving 2-[5-(2-amino-1-hydroxy-1-phenyl-ethyl)-2-chloro-phenyl]-3-fluoro-4-(2-methoxyethoxy)benzamide (25.0 mg, 60.0%) as a white powder. ¹H NMR (400 MHz, DMSO-*d*₆) δ ppm 7.50-7.47 (m, 1.5 H), 7.44-7.37 (m, 4.5 H), 7.35-7.25 (m, 4 H), 7.20 (m, 1 H), 7.10 (m, 1 H), 5.71 (br s, 1 H), 4.25 (m, 2 H), 3.70 (m, 2 H), 3.32 and 3.31 (s, H), 3.31 (s, 2 H), 3.27 and 3.22 (d, J=13.42 Hz, 1 H), 3.16 and 3.12 (d, J=13.42 Hz, 1 H).

### Example 7: 2-[2-chloro-5-[1-hydroxy-2-(methylamino)-1-phenyl-ethyl]phenyl]-3-fluoro-4-(2-methoxyethoxy)benzamide

### Step a: 5-(3-bromo-4-chloro-phenyl)-5-phenyl-oxazolidin-2-one

To a well-stirred solution of 2-amino-1-(3-bromo-4-chloro-phenyl)-1-phenyl-ethanol (example 1, step c) (500 mg, 1.50 mmol, 1 eq.) in DCM (10.9 mL) under an inert atmosphere, cooled to 0°C (ice-bath), were added successively TEA (417 µL, 3.00 mmol, 2 eq.) and triphosgene (147 mg, 0.49 mmol, 0.33 eq.). The reaction mixture was stirred for 30 min at 0°C. The reaction was then quenched by the addition of water and extracted with DCM (twice). The organic layer was dried over Na₂SO₄, filtered and concentrated to give a white solid. The crude was purified by flash chromatography (Cartridge Biotage Sfar 10 g / 20 µm, Cyclohexane/EtOAc, 100:0 over 4 CV, 100:0 to 60:40 over 15 CV, 60:40 over 6 CV, Flow rate 40 ml / min) to give 5-(3-bromo-4-chloro-phenyl)-5-phenyl-oxazolidin-2-one (395 mg, 74.7%) as a white powder. ¹H NMR (400 MHz, DMSO-*d*₆) δ ppm 7.94 (s, 1 H), 7.82 (d, *J*=2.20 Hz, 1 H), 7.65 (d, *J*=8.47 Hz, 1 H), 7.49 (dd, *J*=8.50, 2.10 Hz, 2 H), 7.46 (m, 1 H), 7.41 (m, 2 H), 7.34 (m, 1 H), 4.16 (d, *J*=9.90 Hz, 1 H), 4.13 (d, *J*=10.00 Hz, 1 H)

### Step b: 5-(3-bromo-4-chloro-phenyl)-3-methyl-5-phenyl-oxazolidin-2-one

In a 25 mL round bottom flask under argon, 5-(3-bromo-4-chloro-phenyl)-5-phenyl-oxazolidin-2-one (342 mg, 0.970 mmol, 1 eq.) was dissolved in DMF (10 mL). The reaction mixture was cooled to 0°C and NaH (60% in oil) (58.2 mg, 1.46 mmol, 1.5 eq.) was added portionwise. The reaction mixture was stirred at 0°C for 30min. Then methyl iodide (91 µL, 1.46 mmol, 1.51 eq.) was added dropwise at 0°C and the mixture was stirred at rt for 15 min. The mixture was diluted with a saturated solution of NH₄Cl and extracted with EtOAc (x3). The combined organic layers were washed with brine and dried over anhydrous Na₂SO₄. After filtration, the filtrate was concentrated under reduced pressure. The crude product was purified by chromatography (Cartridge Biotage Sfär 10 g 20 µm silica, Cyclohexane/ EtOAc 100-0 / 4 CV (column volume), 100-0 to 60-40 / 10 CV, 60-40 / 6 CV, flow rate 40 ml / min) to give 5-(3-bromo-4-chloro-phenyl)-3-methyl-5-phenyl-oxazolidin-2-one (324 mg, 91.1%) as a white powder. ¹H NMR (400 MHz, DMSO-*d*₆) δ ppm 7.83 (d, *J*=2.20 Hz, 1 H), 7.65 (d, *J*=8.47 Hz, 1 H), 7.47 (m, 3 H), 7.42 (m, 2 H), 7.35 (m, 1 H), 4.22 (s, 2 H), 2.79 (s, 3 H).

### Step c: 1-(3-bromo-4-chloro-phenyl)-2-(methylamino)-1-phenyl-ethanol

In a Q-tube reactor, 5-(3-bromo-4-chloro-phenyl)-3-methyl-5-phenyl-oxazolidin-2-on (350 mg, 0.95 mmol, 1 eq.) was dissolved in 1,4-dioxane (7 mL) / water (1.75 mL). KOH (1.10 g, 19.6 mmol, 20.5 eq.) was added, then the reactor was closed, and the mixture was stirred at 100°C for 2 days. The mixture was concentrated, and water was added to the mixture. The aqueous layer was extracted with EtOAc (3x). The organic layer was dried over Na₂SO₄, filtered and concentrated to give 1-(3-bromo-4-chloro-phenyl)-2-(methylamino)-1-phenyl-ethanol (318 mg, 97.8%). ¹H NMR (400 MHz, DMSO-*d*₆) δ ppm 7.79 (d, *J*=2.09 Hz, 1 H), 7.51 (m, 1 H), 7.42 (m, 3 H), 7.30 (m, 2 H), 7.20 (m, 1 H), 5.80 (br d, *J*=0.55 Hz, 1 H), 3.25 (d, *J*=12.20 Hz, 1 H), 3.14 (d, *J*=12.00 Hz, 1 H), 2.28 (s, 3 H).

### Step d: 2-[2-chloro-5-[1-hydroxy-2-(methylamino)-1-phenyl-ethyl]phenyl]-3-fluoro-4-(2-methoxyethoxy)benzonitrile

The compound was prepared using the same procedure as detailed in example 2 step b starting from 1-(3-bromo-4-chloro-phenyl)-2-(methylamino)-1-phenyl-ethanol (150.0 mg, 0.44 mmol, 1 eq.) and 3-fluoro-4-(2-methoxyethoxy)-2-(4,4,5,5-tetramethyl-1,3,2-dioxaborolan-2-yl)benzonitrile (example 6, step b) (424.3 mg, 1.32 mmol, 3 eq.) giving 2-[2-chloro-5-[1-hydroxy-2-(methylamino)-1-phenyl-ethyl]phenyl]-3-fluoro-4-(2-methoxyethoxy)benzonitrile (85 mg, 42.4%) as a yellow solid. ¹H NMR (400 MHz, DMSO-*d*₆) δ ppm 7.80 (m, 1 H), 7.57 (m, 3 H), 7.44 (m, 3 H), 7.27 (m, 2 H), 7.19 (m, 1 H), 5.75 and 5.73 (m, 1 H), 4.34 (q, *J*=3.78 Hz, 2 H), 3.71 (dd, *J*=4.84, 3.96 Hz, 2 H), 3.31 and 3.28 (s, 3 H), 3.26 - 3.14 (m, 2 H), 2.27 and 2.25 (s, 3 H).

### Step e: 2-[2-chloro-5-[1-hydroxy-2-(methylamino)-1-phenyl-ethyl]phenyl]-3-fluoro-4-(2-methoxyethoxy)benzamide

The compound was prepared using the same procedure as detailed in example 2 step c starting from 2-[2-chloro-5-[1-hydroxy-2-(methylamino)-1-phenyl-ethyl]phenyl]-3-fluoro-4-(2-methoxyethoxy)benzonitrile (84.0 mg, 0.185 mmol, 1 eq) giving 2-[2-chloro-5-[1-hydroxy-2-(methylamino)-1-phenyl-ethyl]phenyl]-3-fluoro-4-(2-methoxyethoxy)benzamide (63 mg, 71.5%) as a white powder. ¹H NMR (500 MHz, DMSO-*d*₆) δ ppm 7.49 -7.37 (m, 6 H), 7.33 - 7.24 (m, 4 H), 7.18 (m, 1 H), 7.12 and 7.09 (br s, 1 H), 5.64 and 5.61 (br s, 1 H), 4.24 (m, 2 H), 3.70 (td, *J*=4.40, 2.89 Hz, 2 H), 3.31 and 3.30 (s, 3 H), 3.21 and 3.16 (d, *J*=11.80 Hz, 1 H), 3.11 and 3.10 (d, *J*=11.80 Hz, 1 H), 2.27 and 2.25 (s, 3 H).

### Example 8: 2-[5-(2-amino-1-hydroxy-1-phenyl-ethyl)-2-chloro-phenyl]-3-chloro-benzamide

### Step a: 5-[4-chloro-3-(4,4,5,5-tetramethyl-1,3,2-dioxaborolan-2-yl)phenyl]-5-phenyl-oxazolidin-2-one

The compound was prepared using the same procedure as detailed in example 2 step a starting from 5-(3-bromo-4-chloro-phenyl)-5-phenyl-oxazolidin-2-one (example 7, step a)

(784 mg, 2.09 mmol, 1 eq.) giving 5-[4-chloro-3-(4,4,5,5-tetramethyl-1,3,2-dioxaborolan-2-yl)phenyl]-5-phenyl-oxazolidin-2-one (872 mg, 51.2%) as an orange oil, used without further purification.

### Step b: 3-chloro-2-[2-chloro-5-(2-oxo-5-phenyl-oxazolidin-5-yl)phenyl]benzonitrile

The compound was prepared using the same procedure as detailed in example 2 step b starting from 5-[4-chloro-3-(4,4,5,5-tetramethyl-1,3,2-dioxaborolan-2-yl)phenyl]-5-phenyl-oxazolidin-2-one (310 mg, 0.380 mmol, 1 eq) and 2-bromo-3-chlorobenzonitrile (99.0 mg, 0.457 mmol, 1.2 eq.) giving 3-chloro-2-[2-chloro-5-(2-oxo-5-phenyl-oxazolidin-5-yl)phenyl]benzonitrile (98 mg, 40.3%) as a pale yellow gum, used without further purification. Step c: 3-chloro-2-[2-chloro-5-(2-oxo-5-phenyl-oxazolidin-5-yl)phenyl]benzamide The compound was prepared using the same procedure as detailed in example 2 step c starting from 3-chloro-2-[2-chloro-5-(2-oxo-5-phenyl-oxazolidin-5-yl)phenyl]benzonitrile (95.0 mg, 0.149 mmol, 1 eq.) giving 3-chloro-2-[2-chloro-5-(2-oxo-5-phenyl-oxazolidin-5-yl)phenyl]benzamide (63 mg, 98.2%) as a white gum. ¹H NMR (400 MHz, DMSO-*d*₆) δ ppm 7.90 and 7.88 (s, 1 H), 7.70 and 7.69 (br s, 1 H), 7.63 (m, 1 H), 7.50 (m, 3 H), 7.41 (m, 5 H), 7.33 (m, 2 H), 7.25 (s, 1 H), 4.18 and 4.10 (d, *J*=10.20 Hz, 1 H), 4.05 and 4.04 (d, J=10.20 Hz, 1 H).

### Step e: 2-[5-(2-amino-1-hydroxy-1-phenyl-ethyl)-2-chloro-phenyl]-3-chloro-benzamide

In a 5 mL round bottom flask with a condenser, 3-chloro-2-[2-chloro-5-(2-oxo-5-phenyl-oxazolidin-5-yl)phenyl]benzamide (53.0 mg, 0.124 mmol, 1 eq) was dissolved in a mixture of 1,4-dioxane (1.60 mL) and water (265 µL). KOH (139 mg, 2.48 mmol, 20 eq.) was added and the mixture was stirred at 90°C for 24h. The mixture was cooled to rt, diluted with 15 mL of water, acidified with aqueous HCl (1N) until pH 1 and extracted twice with Et₂O. The aqueous layer was basified until pH 8 with a saturated aqueous NaHCO₃ and extracted three times with DCM. The combined organic layers were dried over MgSO₄, filtered and concentrated under reduced pressure to give 53 mg of a white solid. The crude product was purified by chromatography (Cartridge Biotage Sfär 5 g 60 µm silica, DCM-MeOH + NH4OH 100-0 / 2 CV (column volume) 100-0 to 90-10 / 20 CV 90-10 / 5 CV, flow rate 18 ml / min) to give 2-[5-(2-amino-1-hydroxy-1-phenyl-ethyl)-2-chloro-phenyl]-3-chloro-benzamide (24.0 mg, 48.2%). ¹H NMR (500 MHz, DMSO-*d*₆) δ ppm 7.65 (br s, 1 H), 7.62 (m, 1 H), 7.48 (m, 2 H), 7.41 (m, 4 H), 7.30 (m, 4 H), 7.20 (m, 1 H), 5.68 and 5.66 (br s, 1 H), 3.27 and 3.19 (d, *J*=13.50 Hz, 1 H), 3.10 and 3.07 (d, J=13.50 Hz, 1 H).

### Example 9a et 9b: two racemates of 2-[3-(2-amino-1-hydroxy-1-phenyl-ethyl)-6-chloro-2-methyl-phenyl]benzamide, formate salt

### Step a: (3-bromo-4-chloro-2-methyl-phenyl)-phenyl-methanone

The compound was prepared using the same procedure as detailed in example 1 step a starting from 3-bromo-4-chloro-2-methyl-benzoyl chloride (3.71 g, 13.8 mmol, 1 eq.) giving (3-bromo-4-chloro-2-methyl-phenyl)-phenyl-methanone (4.19 g, 97.9%) as an oil. ¹H NMR (400 MHz, DMSO-*d*₆) δ ppm 7.73 (m, 3 H), 7.62 (dd, *J*=8.20, 0.61 Hz, 1 H), 7.56 (m, 2 H), 7.36 (d, *J*=8.25 Hz, 1 H), 2.28 (s, 3 H).

### Step b: 2-(3-bromo-4-chloro-2-methyl-phenyl)-2-phenyl-oxirane

The compound was prepared using the same procedure as detailed in example 1 step b starting from (3-bromo-4-chloro-2-methyl-phenyl)-phenyl-methanone (4.19 g, 13.5 mmol, 1 eq.) giving 2-(3-bromo-4-chloro-2-methyl-phenyl)-2-phenyl-oxirane (4.69 g, quant) as an oil. ¹H NMR (400 MHz, DMSO-*d*₆) δ ppm 7.57 (dd, *J*=8.30, 0.77 Hz, 1 H), 7.52 (d, *J*=8.40 Hz, 1 H), 7.33 (m, 3 H), 7.11 (m, 2 H), 3.37 (d, *J*=5.06 Hz, 1 H), 3.34 (d, *J*=4.95 Hz, 1 H), 2.31 (s, 3 H).

### Step c: 2-amino-1-(3-bromo-4-chloro-2-methyl-phenyl)-1-phenyl-ethanol

The compound was prepared using the same procedure as detailed in example 1 step c starting from 2-(3-bromo-4-chloro-2-methyl-phenyl)-2-phenyl-oxirane (4.69 g, 14.5 mmol, 1eq.) giving 2-amino-1-(3-bromo-4-chloro-2-methyl-phenyl)-1-phenyl-ethanol (2.28 g, 46.2%) as a brown powder. ¹H NMR (400 MHz, DMSO-*d*₆) δ ppm 7.67 (d, *J*=8.47 Hz, 1 H), 7.50 (d, *J*=8.47 Hz, 1 H), 7.28 (m, 2 H), 7.20 (m, 3 H), 5.76 (br s, 1 H), 3.29 (d, J=13.31 Hz, 1 H), 3.00 (d, *J*=13.31 Hz, 1 H), 2.13 (s, 3 H).

### Step d: 2-[3-(2-amino-1-hydroxy-1-phenyl-ethyl)-6-chloro-2-methyl-phenyl]benzonitrile

The compound was prepared using the same procedure as detailed in example 2 step b starting from 2-amino-1-(3-bromo-4-chloro-2-methyl-phenyl)-1-phenyl-ethanol (200 mg, 0.59 mmol, 1 eq.) and 2-(4,4,5,5-tetramethyl-1,3,2-dioxaborolan-2-yl)benzonitrile (405 mg, 1.77 mmol, 3.0 eq.) giving 2-[3-(2-amino-1-hydroxy-1-phenyl-ethyl)-6-chloro-2-methylphenyl]benzonitrile (75 mg, 31.2%) as a yellow powder. ¹H NMR (400 MHz, DMSO-*d*₆) δ ppm 7.95 (d, *J*=7.81 Hz, 1 H), 7.91 (m, 1 H), 7.79 (m, 1 H), 7.73 (d, *J*=8.25 Hz, 1 H), 7.58 (m, 1 H), 7.52 (m, 1 H), 7.26 (m, 5 H), 5.75 (s, 1 H), 3.25 (d, J=13.09 Hz, 1 H), 3.14 (d, *J*=13.09 Hz, 1 H), 1.64 and 1.63 (s, 3 H).

### Step e: two racemates of 2-[3-(2-amino-1-hydroxy-1-phenyl-ethyl)-6-chloro-2-methylphenyl]benzamide, formate salt

The compound was prepared using the same procedure as detailed in example 2 step c starting from 2-[3-(2-amino-1-hydroxy-1-phenyl-ethyl)-6-chloro-2-methyl-phenyl]benzonitrile; (187 mg, 0.457 mmol, 1 eq.) and then the same procedure as detailed in examples 2a and 2b giving after separation by semi-preparative HPLC (Method B) a mixture of racemates 9a and 9b. **9a** : 2-[4-chloro-3-(2-cyanophenyl)-2-methyl-phenyl]-2-hydroxy-2-phenylethyl]ammonium; formate Rt 16.48 min ¹H NMR (500 MHz, DMSO-d6) δ ppm 8.25 (s, 1 H), 7.58 (m, 2 H), 7.50 (m, 1 H), 7.41 (m, 1 H), 7.33 (d, J=8.53 Hz, 1 H), 7.24 (m, 6 H), 7.05 (m, 1 H), 7.02 (d, J=7.43 Hz, 1 H), 3.37 (d, J=13.07 Hz, 3 H), 3.09 (d, J=13.20 Hz, 1 H), 1.63 (s, 3 H). and **9b** 2-[4-chloro-3-(2-cyanophenyl)-2-methyl-phenyl]-2-hydroxy-2-phenylethyl]ammonium; formate: Rt 27.88 min ¹H NMR (500 MHz, DMSO-*d*₆) δ ppm 8.48 (s, 1 H), 7.63 (d, *J*=7.57 Hz, 1 H), 7.57 (d, *J*=8.67 Hz, 1 H), 7.40 (m, 2 H), 7.29 (d, *J*=8.53 Hz, 1 H), 7.24 (m, 5 H), 7.16 (m, 1 H), 6.88 (dd, *J*=7.50, 0.89 Hz, 1 H), 6.77 (br s, 2 H), 3.34 (d, *J*=13.07 Hz, 1 H), 3.09 (brd, *J*=13.20 Hz, 1 H), 1.67 (s, 3 H).

### Example 10: 2-[3-(2-amino-1-hydroxy-1-phenyl-ethyl)-6-chloro-2-fluorophenyl]benzamide

### Step a: (3-bromo-4-chloro-2-fluoro-phenyl)-phenyl-methanone

DMF (75 µL) was added to a solution of 3-bromo-4-chloro-2-fluorobenzoic acid (7.5 g, 30 mmol, 1 eq.) in thionyl chloride (38 mL, 524 mmol, 17 eq.). The reaction mixture was stirred under reflux for 1h30. The reaction mixture was concentrated to give a broken white solid. The residue was taken in benzene (75 mL) and AlCl₃ (4.7 g, 35 mmol, 1.2 eq.) was added. The reaction mixture was stirred under reflux for 15 min. The reaction mixture was cooled to rt overnight, diluted with aqueous NaOH 1N and extracted with toluene (2x). The organic layer was washed with aqueous NaOH 1N, brine, dried over anhydrous MgSO₄, filtered and concentrated to give a beige solid. The crude was purified by chromatography over silica gel (dry loading, column: 100 g of silica, flow rate 100 mL/min, eluant: 2CV of 100% cyclohexane then 10CV gradient from 100% cyclohexane to 90% cyclohexane - 10% EtOAc) to give (3-bromo-4-chloro-2-fluoro-phenyl)-phenyl-methanone (7.9 g, 85%) as a white solid. ¹H NMR (400 MHz, DMSO-*d*₆) δ ppm 7.81 (dt, *J*=8.34, 1.11 Hz, 2 H), 7.74 (m, 1 H), 7.69 (dd, *J*=8.47, 1.10 Hz, 1 H), 7.63 (dd, *J*=8.80, 6.82 Hz, 1 H), 7.58 (m, 2 H).

### Step b: 2-(3-bromo-4-chloro-2-fluoro-phenyl)-2-phenyl-oxirane

The compound was prepared using the same procedure as detailed in example 1 step b starting from (3-bromo-4-chloro-2-fluoro-phenyl)-phenyl-methanone (5.7 g, 18 mmol, 1 eq.) giving 2-(3-bromo-4-chloro-2-fluoro-phenyl)-2-phenyl-oxirane (5.2 g, 73%) as a yellow gum. ¹H NMR (400 MHz, DMSO-*d*₆) δ ppm 7.64 (dd, *J*=8.36, 7.04 Hz, 1 H), 7.59 (dd, *J*=8.47, 1.10 Hz, 1 H), 7.35 (m, 3 H), 7.22 (m, 2 H), 3.38 (d, *J*=5.28 Hz, 1 H), 3.34 (d, *J*=5.06 Hz, 1 H).

### Step c: 2-amino-1-(3-bromo-4-chloro-2-fluoro-phenyl)-1-phenyl-ethanol

The compound was prepared using the same procedure as detailed in example 1 step c starting from 2-(3-bromo-4-chloro-2-fluoro-phenyl)-2-phenyl-oxirane (5.2 g, 16 mmol, 1 eq.) giving 2-amino-1-(3-bromo-4-chloro-2-fluoro-phenyl)-1-phenyl-ethanol (2.76 g, 94%) as a yellow powder. ¹H NMR (400 MHz, DMSO-*d*₆) δ ppm 7.77 (t, *J*=8.42 Hz, 1 H), 7.50 (dd, *J*=8.64, 1.49 Hz, 1 H), 7.32 (m, 4 H), 7.23 (m, 1 H), 5.92 (br s, 1 H), 3.38 (d, *J*=12.43 Hz, 1 H), 3.25 (d, *J*=13.20 Hz, 1 H).

### Step d: 2-[3-(2-amino-1-hydroxy-1-phenyl-ethyl)-6-chloro-2-fluoro-phenyl]benzonitrile

The compound was prepared using the same procedure as detailed in example 2 step b starting from 2-amino-1-(3-bromo-4-chloro-2-fluoro-phenyl)-1-phenyl-ethanol (350 mg, 1.02 mmol, 1 eq.) and 2-(4,4,5,5-tetramethyl-1,3,2-dioxaborolan-2-yl)benzonitrile (465 mg, 2.03 mmol, 2 eq.) giving 2-[3-(2-amino-1-hydroxy-1-phenyl-ethyl)-6-chloro-2-fluorophenyl]benzonitrile (26 mg, 6.7%) as a white powder. ¹H NMR (400 MHz, DMSO-*d*₆) δ ppm 7.98 (td, *J*=7.15, 1.87 Hz, 1 H), 7.90 (m, 1 H), 7.81 (m, 1 H), 7.65 (td, *J*=7.70, 1.21 Hz, 1 H), 7.55 (m, 2 H), 7.36 (m, 2 H), 7.27 (m, 2 H), 7.21 (m, 1 H), 5.93 (br s, 1 H), 3.37 (m, 1 H), 3.24 (m, 1 H).

### Step e: 2-[3-(2-amino-1-hydroxy-1-phenyl-ethyl)-6-chloro-2-fluoro-phenyl]benzamide

The compound was prepared using the same procedure as detailed in example 2 step c starting from 2-[3-(2-amino-1-hydroxy-1-phenyl-ethyl)-6-chloro-2-fluoro-phenyl]benzonitrile (25 mg, 0.068 mmol, 1 eq.) giving 2-[3-(2-amino-1-hydroxy-1-phenyl-ethyl)-6-chloro-2-fluorophenyl]benzamide (15 mg, 57.1%) as a white solid. ¹H NMR (500 MHz, DMSO-*d*₆) δ ppm 7.68 (m, 3 H), 7.50 (m, 2 H), 7.31 (m, 5 H), 7.21 (m, 2 H), 5.83 and 5.75 (s, 1 H), 3.40 and 3.24 (d, *J*=15.82 Hz, 1 H), 3.10 (m, 1 H).

### Example 11: 2-[5-(2-amino-1-hydroxy-1-phenyl-ethyl)-2-chloro-3-fluoro-phenyl]-3-methyl-benzamide

### Step a: (3-bromo-4-chloro-5-fluoro-phenyl)-phenyl-methanone

The compound was prepared using the same procedure as detailed in example 10 step a starting from 3-bromo-4-chloro-5-fluoro-benzoic acid (4.5 g, 18 mmol, 1 eq.) giving (3-bromo-4-chloro-5-fluoro-phenyl)-phenyl-methanone (1.58 g, 28%) as an off-white powder. ¹H NMR (400 MHz, DMSO-*d*₆) δ ppm 7.89 (m, 1 H), 7.80 (m, 2 H), 7.76 (dd, *J*=8.97, 1.82 Hz, 1 H), 7.72 (m, 1 H), 7.60 (m, 2 H).

### Step b: 2-(3-bromo-4-chloro-5-fluoro-phenyl)-2-phenyl-oxirane

The compound was prepared using the same procedure as detailed in example 1 step b starting from (3-bromo-4-chloro-5-fluoro-phenyl)-phenyl-methanone (1.58 g, 4.89 mmol, 1 eq.) giving 2-(3-bromo-4-chloro-5-fluoro-phenyl)-2-phenyl-oxirane (604 mg, 33.2%) as a pale-yellow oil. ¹H NMR (400 MHz, DMSO-*d*₆) δ ppm 7.52 (m, 1 H), 7.39 (m, 6 H), 3.37 (d, *J*=4.62 Hz, 1 H), 3.33 (d, *J*=4.84 Hz, 1 H).

### Step c: 2-amino-1-(3-bromo-4-chloro-5-fluoro-phenyl)-1-phenyl-ethanol

The compound was prepared using the same procedure as detailed in example 1 step c starting from 2-(3-bromo-4-chloro-5-fluoro-phenyl)-2-phenyl-oxirane (604 mg, 1.62 mmol, 1 eq.) giving 2-amino-1-(3-bromo-4-chloro-5-fluoro-phenyl)-1-phenyl-ethanol (352 mg, 53.5%) as a pale yellow oil. ¹H NMR (400 MHz, DMSO-*d*₆) δ ppm 7.65 (dd, *J*=1.98, 1.43 Hz, 1 H), 7.50 (dd, *J*=10.45, 1.98 Hz, 1 H), 7.46 (m, 2 H), 7.32 (m, 2 H), 7.22 (tt, *J*=7.20, 1.20 Hz, 1 H), 5.89 (br s, 1 H), 3.33 (d, *J*=13.20 Hz, 1 H), 3.19 (d, *J*=13.00 Hz, 1 H).

### Step d: 2-[5-(2-amino-1-hydroxy-1-phenyl-ethyl)-2-chloro-3-fluoro-phenyl]-3-methyl-benzonitrile

The compound was prepared using the same procedure as detailed in example 2 step b starting from 2-amino-1-(3-bromo-4-chloro-5-fluoro-phenyl)-1-phenyl-ethanol (150 mg, 0.370 mmol, 1 eq.) and 3-methyl-2-(4,4,5,5-tetramethyl-1,3,2-dioxaborolan-2-yl)benzonitrile (270 mg, 1.11 mmol, 3 eq.) giving 2-[5-(2-amino-1-hydroxy-1-phenyl-ethyl)-2-chloro-3-fluorophenyl]-3-methyl-benzonitrile (35 mg, 23%) as a white powder. The product was used without further purification.

### Step e: 2-[5-(2-amino-1-hydroxy-1-phenyl-ethyl)-2-chloro-3-fluoro-phenyl]-3-methyl-benzamide

The compound was prepared using the same procedure as detailed in example 2 step c starting from 2-[5-(2-amino-1-hydroxy-1-phenyl-ethyl)-2-chloro-3-fluoro-phenyl]-3-methyl-benzonitrile (35 mg, 0.085 mmol, 1 eq.) giving 2-[5-(2-amino-1-hydroxy-1-phenyl-ethyl)-2-chloro-3-fluoro-phenyl]-3-methyl-benzamide (16 mg, 47%) as a white powder. ¹H NMR (500 MHz, DMSO-*d*₆) δ ppm 7.54 (br s, 1 H), 7.42 (m, 3 H), 7.37 (d, *J*=6.60 Hz, 3 H), 7.29 (m, 2 H), 7.20 (m, 1 H), 7.18 and 7.10 (br s, 1 H), 7.10 (m, 1 H), 5.77 and 5.76(br s, 1 H), 3.32 and 3.22 (d, *J*=13.48 Hz, 1 H), 3.10 and 3.08 (d, *J*=13.55 Hz, 1 H), 1.95 and 1.91 (s, 3 H).

### Example 12: 2-[5-(2-amino-1-hydroxy-1-phenyl-ethyl)-2-methyl-phenyl]-3-fluoro-4-(2-methoxyethoxy)benzamide

### Step a: (3-bromo-4-methyl-phenyl)-phenyl-methanone

The compound was prepared using the same procedure as detailed in example 10 step a starting from 3-bromo-4-methylbenzoic acid (6.00 g, 27.9 mmol, 1 eq.) giving (3-bromo-4-methyl-phenyl)-phenyl-methanone (6.026 g, 78.5%) as a white powder. ¹H NMR (400 MHz, DMSO-*d*₆) δ ppm 7.88 (d, *J*=1.76 Hz, 1 H), 7.73 (m, 2 H), 7.70 (t, *J*=7.37 Hz, 1 H), 7.64 (dd, *J*=7.70, 1.65 Hz, 1 H), 7.57 (m, 3 H), 2.45 (s, 3 H).

### Step b: 2-(3-bromo-4-methyl-phenyl)-2-phenyl-oxirane

The compound was prepared using the same procedure as detailed in example 1 step b starting from (3-bromo-4-methyl-phenyl)-phenyl-methanone (6.026 g, 21.9 mmol, 1 eq.) giving 2-(3-bromo-4-methyl-phenyl)-2-phenyl-oxirane (6.081 g, 68.2%) as a yellow oil. ¹H NMR (400 MHz, DMSO-*d*₆) δ ppm 7.47 (d, *J*=1.87 Hz, 1 H), 7.35 (m, 6 H), 7.21 (dd, *J*=7.81, 1.87 Hz, 1 H), 3.29 (s, 2 H), 2.34 (s, 3 H).

### Step c: 2-amino-1-(3-bromo-4-methyl-phenyl)-1-phenyl-ethanol

The compound was prepared using the same procedure as detailed in example 1 step c starting from 2-(3-bromo-4-methyl-phenyl)-2-phenyl-oxirane (6.08 g, 14.9 mmol, 1 eq.) giving 2-amino-1-(3-bromo-4-methyl-phenyl)-1-phenyl-ethanol (3.05 g, 32.3%) as a yellow oil. ¹H NMR (400 MHz, DMSO-*d*₆) δ ppm 7.61 (d, *J*=1.65 Hz, 1 H), 7.43 (m, 2 H), 7.30 (m, 3 H), 7.25 (d, *J*=8.25 Hz, 1 H), 7.19 (m, 1 H), 5.65 (br s, 1 H), 3.17 (s, 2 H), 2.28 (s, 3 H).

### Step d: 2-[5-(2-amino-1-hydroxy-1-phenyl-ethyl)-2-methyl-phenyl]-3-fluoro-4-(2-methoxy-ethoxy)benzonitrile

The compound was prepared using the same procedure as detailed in example 2 step b starting from 2-amino-1-(3-bromo-4-methyl-phenyl)-1-phenyl-ethanol (170 mg, 0.555 mmol, 1 eq.) and 3-fluoro-4-(2-methoxyethoxy)-2-(4,4,5,5-tetramethyl-1,3,2-dioxaborolan-2-yl)benzonitrile (example 6, step b) (581 mg, 1.66 mmol, 3 eq.) giving 2-[5-(2-amino-1-hydroxy-1-phenyl-ethyl)-2-methyl-phenyl]-3-fluoro-4-(2-methoxyethoxy)benzonitrile (190 mg, 81.4%) as a yellow powder. The product was used without further purification.

### Step e: 2-[5-(2-amino-1-hydroxy-1-phenyl-ethyl)-2-methyl-phenyl]-3-fluoro-4-(2-methoxyethoxy)benzamide

The compound was prepared using the same procedure as detailed in example 2 step c starting from 2-[5-(2-amino-1-hydroxy-1-phenyl-ethyl)-2-methyl-phenyl]-3-fluoro-4-(2-methoxy-ethoxy)benzonitrile (184 mg, 0.438 mmol, 1 eq.) giving 2-[5-(2-amino-1-hydroxy-1-phenyl-ethyl)-2-methyl-phenyl]-3-fluoro-4-(2-methoxyethoxy)benzamide (83.0 mg, 43.3%) as a white solid. ¹H NMR (500 MHz, DMSO-*d*₆) δ ppm 7.40 (m, 2 H), 7.33 (m, 2 H), 7.25 (m, 4 H), 7.16 (m, 3 H), 7.10 and 7.08 (br s, 1 H), 5.52 (br s, 1 H), 4.23 (m, 2 H), 3.69 (m, 2 H), 3.31 and 3.30 (s, 3 H), 3.25 and 3.17 (d, *J*=13.48 Hz, 1 H), 3.10 and 3.05 (d, *J*=13.62 Hz, 1 H), 2.01 (s, 3 H).

### Example 13: 2-[5-[(1S)-2-amino-1-hydroxy-1-phenyl-ethyl]-2-chloro-phenyl]-3-fluoro-4-(2-methoxyethoxy)benzamide

### Step a: [(1S,2R,5S)-2-isopropyl-5-methyl-cyclohexyl] N-[(2S)-2-(3-bromo-4-chloro-phenyl)-2-hydroxy-2-phenyl-ethyl]carbamate and [(1S,2R,5S)-2-isopropyl-5-methyl-cyclohexyl] N-[(2R)-2-(3-bromo-4-chloro-phenyl)-2-hydroxy-2-phenyl-ethyl]carbamate

Under N₂ atmosphere, 2-amino-1-(3-bromo-4-chloro-phenyl)-1-phenyl-ethanol as HCl salt (example 1, step c) (23.8 g, 65.6 mmol, 1 eq.) was solubilized in DCM (470 mL) and TEA (27.3 mL, 196 mmol, 3 eq.) was added. The solution was cooled to 0°C and (+)-menthyl chloroformate (17.2 g, 78.6 mmol, 1.2 eq.) in 20 ml of DCM was added dropwise at 0°C. The solution was diluted with DCM and washed with NaCl (aq). The resulting organic layer was dried over MgSO₄, filtered and concentrated to afford a white foam (43g). The crude was suspended in 1260 ml of ACN, heated to reflux until complete dissolution and let cooled down overnight to afford after filtration 11.6 g of a white solid (Ratio S/R 90/10). The solid was suspended in 340 ml of ACN and heated to reflux for 1h (it was not totally soluble), it was cooled down overnight to afford after filtration [(1S,2R,5S)-2-isopropyl-5-methylcyclohexyl] N-[(2S)-2-(3-bromo-4-chloro-phenyl)-2-hydroxy-2-phenyl-ethyl]carbamate (9.95 g) (ratio S/R: 100/0). The absolute configuration was determined by XR. ¹H NMR (500 MHz, DMSO-*d*₆) δ ppm 7.77 (d, *J*=1.93 Hz, 1 H), 7.50 (d, *J*=8.53 Hz, 1 H), 7.42 (m, 2 H), 7.31 (m, 3 H), 7.24 (t, *J*=7.70 Hz, 1 H), 6.74 (br t, *J*=5.78 Hz, 1 H), 6.05 (s, 1 H), 4.30 (td, *J*=10.83, 4.06 Hz, 1 H), 3.88 (dd, *J*=13.82, 6.53 Hz, 1 H), 3.72 (dd, *J*=13.96, 5.16 Hz, 1 H), 1.69 (m, 2 H), 1.57 (m, 2 H), 1.36 (dt, *J*=6.33, 3.03 Hz, 1 H), 1.17 (br t, *J*=11.55 Hz, 1 H), 0.97 (qd, *J*=12.81, 2.54 Hz, 1 H), 0.86 (d, *J*=6.46 Hz, 3 H), 0.78 (m, 5 H), 0.66 (d, *J*=6.88 Hz, 3 H).

The filtrates were mixed and evaporated to afford after evaporation 21.6 g of an orange oil (with a ratio S/R 18/82). The oil was suspended in 105 ml (5V) of ACN and heated until complete dissolution. It was cooled down overnight, a precipitate appeared, it was filtered off. The filtrate was evaporated to afford 13 g of an oil. (ratio S/R 10/90). To this oil was added 65 ml of ACN. After 18h a precipitate appeared, it was filtered off and dried to afford [(1S,2R,5S)-2-isopropyl-5-methyl-cyclohexyl] N-[(2R)-2-(3-bromo-4-chloro-phenyl)-2-hydroxy-2-phenyl-ethyl]carbamate (3.45 g) (ratio S/R 5/95). ¹H NMR (400 MHz, DMSO-*d*₆) δ ppm 7.77 (d, *J*=1.98 Hz, 1 H), 7.48 (d, *J*=8.69 Hz, 1 H), 7.42 (d, *J*=7.59 Hz, 2 H), 7.33 (m, 4 H), 7.24 (br d, *J*=7.26 Hz, 1 H), 6.68 (br dd, *J*=6.49, 4.07 Hz, 1 H), 6.02 (s, 1 H), 4.31 (td, J=10.83, 4.5 Hz, 1 H), 4.06 (dd, *J*=13.75, 7.59 Hz, 1 H), 3.60 (dd, *J*=13.97, 4.18 Hz, 1 H), 1.75 (m, 1 H), 1.56 (m, 3 H), 1.37 (m, 1 H), 1.16 (m, 1 H), 0.98 (m, 1 H), 0.85 (d, *J*=7.04 Hz, 3 H), 0.76 (br d, *J*=7.04 Hz, 5 H), 0.59 (d, *J*=7.04 Hz, 3 H).

### Step b: (1S)-2-amino-1-(3-bromo-4-chloro-phenyl)-1-phenyl-ethanol

[(1S,2R,5S)-2-isopropyl-5-methyl-cyclohexyl] N-[(2S)-2-(3-bromo-4-chloro-phenyl)-2-hydroxy-2-phenyl-ethyl]carbamate (9.9 g, 19 mmol, 1 eq.) was suspended in EtOH (42 mL) .KOH (2600 mg, 46.3412 mmol, 20 eq.) in water (8 mL) was added. The reaction mixture was stirred at 100°C for 20h. The aqueous layer was extracted with EtOAc. The organic layer was dried over Na₂SO₄ and evaporated to give the crude product which was purified by chromatography (from 0% to 100 % DCM/MeOH/NH₃: 9/1/0.05 in 8 CV) to afford (1S)-2-amino-1-(3-bromo-4-chloro-phenyl)-1-phenyl-ethanol (5.2 g, 82%) as a white solid. ¹H NMR (500 MHz, DMSO-*d*₆) δ ppm 7.79 (d, *J*=2.06 Hz, 1 H), 7.52 (d, *J*=8.39 Hz, 1 H), 7.44 (m, 3 H), 7.31 (t, *J*=7.70 Hz, 2 H), 7.21 (t, *J*=7.57 Hz, 1 H), 5.80 (m, 1 H), 3.26 (d, *J*=13.34 Hz, 1 H), 3.20 (d, *J*=13.75 Hz, 1 H). [α]_{d}²² = +18.8° (C=0.42, DCM).

### Step c: 2-[5-[(1S)-2-amino-1-hydroxy-1-phenyl-ethyl]-2-chloro-phenyl]-3-fluoro-4-(2-methoxyethoxy)benzonitrile

The compound was prepared using the same procedure as detailed in example 2 step b starting from (1S)-2-amino-1-(3-bromo-4-chloro-phenyl)-1-phenyl-ethanol (200 mg, 0.61 mmol, 1 eq.) and 3-fluoro-4-(2-methoxyethoxy)-2-(4,4,5,5-tetramethyl-1,3,2-dioxaborolan-2-yl)benzonitrile (590 mg, 1.84 mmol, 3 eq.) giving 2-[5-[(1S)-2-amino-1-hydroxy-1-phenylethyl]-2-chloro-phenyl]-3-fluoro-4-(2-methoxyethoxy)benzonitrile (77.0 mg, 0.175 mmol, 28%) as a yellow powder. ¹H NMR (400 MHz, DMSO-*d*₆) δ ppm 7.81 (dd, *J*=8.69, 1.21 Hz, 1 H), 7.57 (m, 3 H), 7.45 (m, 3 H), 7.29 (t, *J*=0.33 Hz, 2 H), 7.20 (m, 1 H), 5.76 (br s, 1 H), 4.34 (m, 2 H), 3.72 (t, *J*=4.35 Hz, 2 H), 3.34 and 3.33 (s, 3 H), 3.26 (d, *J*=13.53 Hz, 1 H), 3.19 (d, *J*=13.50 Hz, 1 H).

### Step d: 2-[5-[(1S)-2-amino-1-hydroxy-1-phenyl-ethyl]-2-chloro-phenyl]-3-fluoro-4-(2-methoxyethoxy)benzamide

The compound was prepared using the same procedure as detailed in example 2 step c starting from 2-[5-[(1S)-2-amino-1-hydroxy-1-phenyl-ethyl]-2-chloro-phenyl]-3-fluoro-4-(2-methoxyethoxy)benzonitrile (75 mg, 0.17 mmol, 1 eq.) giving 2-[5-[(1S)-2-amino-1-hydroxy-1-phenyl-ethyl]-2-chloro-phenyl]-3-fluoro-4-(2-methoxyethoxy)benzamide (55.0 mg, 70.5%) as a white powder. ¹H NMR (500 MHz, DMSO-*d*₆) δ ppm 7.52 (m, 1 H), 7.48 and 7.72 (dd, *J*=8.50, 2.20 Hz, 1 H), 7.39 (m, 1 H), 7.40 (m, 3 H), 7.34 - 7.24 (m, 4 H), 7.19 (q, *J*=7.43 Hz, 1 H), 7.12 (br d, *J*=0.96 Hz, 1 H), 5.67 (br s, 1 H), 4.24 (m, 2 H), 3.70 (m, 2 H), 3.32 and 3.31 (s, 3 H), 3.28 and 3.17 (d, *J*=13.48 Hz, 1 H), 3.13 and 3.08 (d, J=13.62 Hz, 1 H). [α]_{d}^{22.5} = -11° (C=0.18, MeOH).

### Example 14: 2-[5-[(1R)-2-amino-1-hydroxy-1-phenyl-ethyl]-2-chloro-phenyl]-3-fluoro-4-(2-methoxyethoxy)benzamide

### Step a: (1R)-2-amino-1-(3-bromo-4-chloro-phenyl)-1-phenyl-ethanol

The compound was prepared using the same procedure as detailed in example 13 step b starting from [(1S,2R,5S)-2-isopropyl-5-methyl-cyclohexyl] N-[(2R)-2-(3-bromo-4-chlorophenyl)-2-hydroxy-2-phenyl-ethyl]carbamate (3.45 g, 6.78 mmol, 1 eq) giving (1R)-2-amino-1-(3-bromo-4-chloro-phenyl)-1-phenyl-ethanol (1.89 g, 85.4%) as a white solid. ¹H NMR (500 MHz, DMSO-*d*₆) δ ppm 7.79 (d, *J*=2.06 Hz, 1 H), 7.53 (d, *J*=8.39 Hz, 1 H), 7.43 (m, 3 H), 7.31 (t, *J*=7.70 Hz, 2 H), 7.21 (t, *J*=7.15 Hz, 1 H), 5.80 (m, 1 H), 3.26 (d, *J*=13.20 Hz, 1 H), 3.20 (d, *J*=13.20 Hz, 1 H). [α]_{d}²² = -19.1° (C=0.48, DCM).

### Step b: 2-[5-[(1R)-2-amino-1-hydroxy-1-phenyl-ethyl]-2-chloro-phenyl]-3-fluoro-4-(2-methoxyethoxy)benzonitrile

The compound was prepared using the same procedure as detailed in example 2 step b starting from (1R)-2-amino-1-(3-bromo-4-chloro-phenyl)-1-phenyl-ethanol (200 mg, 0.61 mmol, 1 eq.) and 3-fluoro-4-(2-methoxyethoxy)-2-(4,4,5,5-tetramethyl-1,3,2-dioxaborolan-2-yl)benzonitrile (590 mg, 1.84 mmol, 3 eq.) giving 2-[5-[(1R)-2-amino-1-hydroxy-1-phenylethyl]-2-chloro-phenyl]-3-fluoro-4-(2-methoxyethoxy)benzonitrile (178 mg, 64.6%) as a yellow powder. ¹H NMR (400 MHz, DMSO-*d*₆) δ ppm 7.81 (dd, *J*=8.69, 1.32 Hz, 1 H), 7.58 (m, 3 H), 7.45 (m, 3 H), 7.29 (t, *J*=7.65 Hz, 2 H), 7.20 (m, 1 H), 5.79 (br s, 1 H), 4.34 (m, 2 H), 3.72 (t, *J*=4.40 Hz, 2 H), 3.31 and 3.30 (s, 3 H), 3.27 (d, *J*=13.53 Hz, 1 H), 3.20 (d, *J*=13.53 Hz, 1 H).

### Step c: 2-[5-[(1R)-2-amino-1-hydroxy-1-phenyl-ethyl]-2-chloro-phenyl]-3-fluoro-4-(2-methoxyethoxy)benzamide

The compound was prepared using the same procedure as detailed in example 2 step c starting from 2-[5-[(1R)-2-amino-1-hydroxy-1-phenyl-ethyl]-2-chloro-phenyl]-3-fluoro-4-(2-methoxyethoxy)benzonitrile (0.178 g, 0.404 mmol, 1 eq.) giving 2-[5-[(1R)-2-amino-1-hydroxy-1-phenyl-ethyl]-2-chloro-phenyl]-3-fluoro-4-(2-methoxyethoxy)benzamide (54.0 mg, 29.1%) as a white powder. ¹H NMR (500 MHz, DMSO-*d*₆) δ ppm 7.52 (br s, 1 H), 7.48 and 7.43 (dd, *J*=8.50, 2.50 Hz, 1 H), 7.41 (m, 4 H), 7.29 (m, 4 H), 7.19 (q, *J*=7.84 Hz, 1 H), 7.12 (br s, 1 H), 5.65 (br s, 1 H), 4.24 (m, 2 H), 3.70 (m, 2 H), 3.32 and 3.31 (s, 3 H), 3.27 and 3.17 (d, *J*=13.62 Hz, 1 H), 3.12 and 3.08 (d, *J*=13.62 Hz, 1 H). [α]_{d}^{22.7} = +3° (C=0.2, MeOH).

### Example 15: 2-[5-[(1-aminocyclopropyl)-hydroxy-phenyl-methyl]-2-chloro-phenyl]-3-fluoro-4-(2-methoxyethoxy)benzamide

### Step a: tert-butyl N-(1-benzoylcyclopropyl)carbamate

To a 100 mL round bottom flask, flame dried, under argon, *tert-butyl* (1-(methoxy(methyl)carbamoyl)cyclopropyl)carbamate (2 g, 8.19 mmol, 1 eq.) was dissolved in THF (20 mL) (dry, Acroseal) and the mixture was cooled to 5°C. Phenylmagnesium bromide (41 mL, 41.0 mmol, 5 eq.) was added dropwise (the mixture was kept at 5°C, 5 minutes of addition), the mixture was left to return to rt and stirred at rt for1h. The mixture was slowly quenched with an aqueous saturated solution of NH₄Cl at rt and extracted with EtOAc. The combined organic layers were dried over anhydrous MgSO₄ and concentrated under reduced pressure to give a crude which was purified by chromatography (Cartridge Biotage Sfär 50 g 20 µm silica, Cyclohexane/EtOAc 100-0 / 4 CV (column volume), 100-0 to 0-100 / 20 CV, 0-100 / 6 CV, flow rate 100 ml / min) to give *tert*-butyl *N*-(1-benzoylcyclopropyl)carbamate (1.19 g, 54.5%) as a white powder. ¹H NMR (400 MHz, DMSO-*d*₆) δ ppm 8.02 (s, 1 H), 7.73 (d, *J*=7.48 Hz, 2 H), 7.52 (m, 1 H), 7.44 (t, *J*=7.59 Hz, 2 H), 1.45 (q, *J*=4.07 Hz, 2 H), 1.12 (s, 9 H), 1.09 (m, 2 H).

### Step b: tert-butyl N-[1-[(3-bromo-4-chloro-phenyl)-hydroxy-phenylmethyl]cyclopropyl]carbamate

In a dried 250 mL round-bottomed flask, under argon atmosphere, 2-bromo-1-chloro-4-iodobenzene (6.7 g, 21 mmol, 5 eq.) was dissolved in anhydrous THF (21 mL). The reaction mixture was cooled down to 0°C. Isopropylmagnesium chloride lithium chloride complex (16.2 mL, 21.1 mmol, 5 eq.) was added dropwise at 0°C. The reaction mixture was stirred at 0°C for 1h (yellow solution). Separately, *Tert*-butyl *N*-(1-benzoylcyclopropyl)carbamate (1.1 g, 4.21 mmol, 1 eq) was solubilized in anhydrous Et₂O (21 mL), and the formed solution was added dropwise to the reaction mixture at rt. The reaction mixture was stirred at 35°C for 45 min. The mixture was diluted with a saturated solution of NH₄Cl and extracted with EtOAc. The combined organic layers were dried over anhydrous MgSO₄ and concentrated under reduced pressure. The crude product was purified by chromatography (Cartridge Biotage Sfär 100 g 20 µm silica, Cyclohexane/ DCM 100-0 / 4 CV (column volume), 100-0 to 50-50 / 15 CV, 50-50 / 6 CV, flow rate 100 ml / min) to give *tert*-butyl *N*-[1-[(3-bromo-4-chlorophenyl)-hydroxy-phenyl-methyl]cyclopropyl]carbamate (709 mg, 37.2%) as a white powder. ¹H NMR (400 MHz, DMSO-*d*₆) δ ppm 7.74 (d, *J*=1.32 Hz, 1 H), 7.51 (d, *J*=8.47 Hz, 2 H), 7.43 (m, 3 H), 7.29 (m, 2 H), 7.23 (m, 1 H), 6.54 (s, 1 H), 1.25 (s, 9 H), 0.91 (m, 1 H), 0.76 (m, 3 H).

### Step c: tert-butyl N-[1-[[4-chloro-3-[6-cyano-2-fluoro-3-(2-methoxyethoxy)phenyl]phenyl]-hydroxy-phenyl-methyl]cyclopropyl]carbamate

The compound was prepared using the same procedure as detailed in example 2 step b starting from *tert*-butyl *N*-[1-[(3-bromo-4-chloro-phenyl)-hydroxy-phenylmethyl]cyclopropyl]carbamate (180 mg, 0.398 mmol, 1 eq.) and 3-fluoro-4-(2-methoxyethoxy)-2-(4,4,5,5-tetramethyl-1,3,2-dioxaborolan-2-yl)benzonitrile (383 mg, 1.19 mmol, 3 eq.) giving *tert*-butyl *N*-[1-[[4-chloro-3-[6-cyano-2-fluoro-3-(2-methoxyethoxy)phenyl]phenyl]-hydroxy-phenyl-methyl]cyclopropyl]carbamate (157 mg, 68.3%) as a white powder. ¹H NMR (400 MHz, DMSO-*d*₆) δ ppm 7.81 (m, 1 H), 7.51 (m, 7 H), 7.25 (m, 3 H), 6.80 (s, 1 H), 4.34 (m, 2 H), 3.72 (t, *J*=4.40 Hz, 2 H), 3.30 (s, 3H), 1.25 (s, 9 H), 0.83 (m, 3 H), 0.66 (m, 1 H).

### Step d: tert-butyl N-[1-[[3-[6-carbamoyl-2-fluoro-3-(2-methoxyethoxy)phenyl]-4-chlorophenyl]-hydroxy-phenyl-methyl]cyclopropyl]carbamate

The compound was prepared using the same procedure as detailed in example 12 step e starting from *tert*-butyl *N*-[1-[[4-chloro-3-[6-cyano-2-fluoro-3-(2-methoxyethoxy)phenyl]phenyl]-hydroxy-phenyl-methyl]cyclopropyl]carbamate (180 mg, 0.311 mmol, 1 eq.) giving *tert*-butyl *N*-[1-[[3-[6-carbamoyl-2-fluoro-3-(2-methoxyethoxy)phenyl]-4-chloro-phenyl]-hydroxy-phenyl-methyl]cyclopropyl]carbamate (147 mg, 80.8%) as a white powder. ¹H NMR (400 MHz, DMSO-*d*₆) δ ppm 7.51 (m, 4 H), 7.39 (d, *J*=8.47 Hz, 2 H), 7.28 (m, 3 H), 7.21 (br d, *J*=7.92 Hz, 2 H), 7.18 and 7.13 (m, 1 H), 7.10 (m, 1 H), 6.72 and 6.69 (s, 1 H), 4.24 (m, 2 H), 3.69 (dt, *J*=9.38, 4.61 Hz, 2 H), 3.32 and 3.30 (s, 3 H), 1.26 (s, 9 H), 0.79 (m, 3 H), 0.67 (m, 1 H).

### Step e: 2-[5-[(1-aminocyclopropyl)-hydroxy-phenyl-methyl]-2-chloro-phenyl]-3-fluoro-4-(2-methoxyethoxy)benzamide

In a 25 mL round bottom flask, *tert*-butyl *N*-[1-[[3-[6-carbamoyl-2-fluoro-3-(2-methoxyethoxy)phenyl]-4-chloro-phenyl]-hydroxy-phenyl-methyl]cyclopropyl]carbamate (126 mg, 0.215 mmol, 1 eq.) was dissolved in 1,4-dioxane (5 mL) and a 4.0M hydrochloric acid solution in 1,4-dioxane (2.5 mL, 10 mmol, 46 eq.) was added, the mixture was stirred at rt for 6h. The mixture was concentrated under reduced pressure. The crude product was purified by chromatography semi-preparative (method B) to give 2-[5-[(1-aminocyclopropyl)-hydroxyphenyl-methyl]-2-chloro-phenyl]-3-fluoro-4-(2-methoxyethoxy)benzamide (54.0 mg, 51.7%) as a white powder. ¹H NMR (500 MHz, DMSO-*d*₆) δ ppm 7.58 (br s, 1 H), 7.51 (dd, *J*=9.70, 2.27 Hz, 1 H), 7.44 (m, 3 H), 7.37 (m, 1 H), 7.29 (m, 2 H), 7.21 (m, 3 H), 7.16 and 7.12 (br s, 1 H), 5.70 and 5.69 (s, 1 H), 4.24 (m, 2 H), 3.70 (q, *J*=4.31 Hz, 2 H), 3.33 and 3.31 (s, 3 H), 0.57 (m, 3 H), 0.44 (m, 1 H).

### Example 16a and 16b: two racemates of 2-[3-(2-amino-1-hydroxy-1-phenyl-ethyl)-2,6-difluoro-phenyl]-3-fluoro-4-(2-methoxyethoxy)benzamide

### Step a: (3-bromo-2,4-difluoro-phenyl)-phenyl-methanone

The compound was prepared using the same procedure as detailed in example 10 step a starting from 3-bromo-2,4-difluorobenzoic acid (2.0 g, 8.4 mmol, 1 eq.) giving (3-bromo-2,4-difluoro-phenyl)-phenyl-methanone (2.5 g, quant) as a beige powder. ¹H NMR (400 MHz, DMSO-*d*₆) δ ppm 7.80 (m, 2 H), 7.73 (tt, *J*=7.48, 1.32 Hz, 1 H), 7.69 (m, 1 H), 7.58 (m, 2 H), 7.45 (m, 1 H).

### Step b: 2-(3-bromo-2,4-difluoro-phenyl)-2-phenyl-oxirane

The compound was prepared using the same procedure as detailed in example 1 step b starting from (3-bromo-2,4-difluoro-phenyl)-phenyl-methanone (2.31 g, 7.78 mmol, 1 eq.) giving 2-(3-bromo-2,4-difluoro-phenyl)-2-phenyl-oxirane (2.24 g, 64.8%) as an orange oil. ¹H NMR (400 MHz, DMSO-*d*₆) δ ppm 7.68 (m, 1 H), 7.35 (m, 4 H), 7.21 (m, 2 H), 3.38 (d, *J*=5.06 Hz, 1 H), 3.31 (d, *J*=4.95 Hz, 1 H).

### Step c: 2-amino-1-(3-bromo-2,4-difluoro-phenyl)-1-phenyl-ethanol

The compound was prepared using the same procedure as detailed in example 1 step c starting from 2-(3-bromo-2,4-difluoro-phenyl)-2-phenyl-oxirane (2.03 g, 4.57 mmol, 1 eq.) giving 2-amino-1-(3-bromo-2,4-difluoro-phenyl)-1-phenyl-ethanol (931 mg, 59.6%) as an orange powder. ¹H NMR (400 MHz, DMSO-*d*₆) δ ppm 7.79 (td, *J*=8.86, 6.60 Hz, 1 H), 7.28 (m, 6 H), 5.87 (br s, 1 H), 3.36 (d, *J*=13.20 Hz, 1 H), 3.24 (d, *J*=13.20 Hz, 1 H).

### Step d: 2-[2-(3-bromo-2,4-difluoro-phenyl)-2-hydroxy-2-phenyl-ethyl]isoindoline-1,3-dione

In a 25mL round bottom flask, 2-amino-1-(3-bromo-2,4-difluoro-phenyl)-1-phenyl-ethanol (626 mg, 1.91 mmol, 1 eq) and phtalic anhydride (339 mg, 2.29 mmol, 1.2 eq.) were dissolved in toluene (6.3 mL). The reaction mixture was heated at 120°C for 2h. The reaction mixture was cooled down to rt and was concentrated under vacuum. Isopropanol was added and a precipitate was formed. The precipitate was sonicated for 2 min, filtered, washed twice with cold isopropanol and dried under vacuum at 40°C for 1h to give 2-[2-(3-bromo-2,4-difluoro-phenyl)-2-hydroxy-2-phenyl-ethyl]isoindoline-1,3-dione (566 mg, 64.7%) as a white powder. ¹H NMR (400 MHz, DMSO-*d*₆) δ ppm 7.79 (s, 4 H), 7.63 (td, *J*=8.83, 6.55 Hz, 1 H), 7.42 (d, *J*=7.59 Hz, 2 H), 7.35 (t, *J*=6.93 Hz, 2 H), 7.29 (t, *J*=6.93 Hz, 1 H), 7.12 (m, 1 H), 6.18 (s, 1 H), 4.62 (d, *J*=13.86 Hz, 1 H), 4.49 (d, *J*=13.76 Hz, 1 H).

### Step e: 2-[3-[2-(1,3-dioxoisoindolin-2-yl)-1-hydroxy-1-phenyl-ethyl]-2,6-difluoro-phenyl]-3-fluoro-4-(2-methoxyethoxy)benzonitrile

The compound was prepared using the same procedure as detailed in example 2 step b starting from 2-[2-(3-bromo-2,4-difluoro-phenyl)-2-hydroxy-2-phenyl-ethyl]isoindoline-1,3-dione (300 mg, 0.65 mmol, 1 eq) and 3-fluoro-4-(2-methoxyethoxy)-2-(4,4,5,5-tetramethyl-1,3,2-dioxaborolan-2-yl)benzonitrile (Ex 6, step b) (717 mg, 1.96 mmol, 3 eq.) giving 2-[3-[2-(1,3-dioxoisoindolin-2-yl)-1-hydroxy-1-phenyl-ethyl]-2,6-difluoro-phenyl]-3-fluoro-4-(2-methoxyethoxy)benzonitrile (171 mg, 36.5%) as a pale yellow powder used in the next step without further purification.

### Step f: 2-[3-(2-amino-1-hydroxy-1-phenyl-ethyl)-2,6-difluoro-phenyl]-3-fluoro-4-(2-methoxyethoxy)benzonitrile

In a MW vial (0.5-2mL), 2-[3-[2-(1,3-dioxoisoindolin-2-yl)-1-hydroxy-1-phenyl-ethyl]-2,6-difluoro-phenyl]-3-fluoro-4-(2-methoxyethoxy)benzonitrile (75 mg, 0.13 mmol, 1 eq) and hydrazine hydrate (13 µL, 0.27 mmol, 2 eq.) were dissolved in EtOH (1.3 mL). The reaction mixture was heated at 60°C overnight. The reaction mixture was cooled down to rt and was concentrated under vacuum. The crude product was purified by chromatography (Cartridge Biotage Sfär HC 5g / 20µm silica, DCM-(DCM/MeOH:90/10 + 1%NH3), 100-0% : 3 CV (column volume), 100-0% to 65-35% : 7 CV, 65-35% : 9 CV, 65-35% to 0-100% : 13 CV, 0-100% : 5 CV, Flow rate 18ml/min) to give DLVT0645-001 (84 mg, quant) as a pale-yellow powder. ¹H NMR (400 MHz, DMSO-*d*₆) δ ppm 7.94 (m, 1 H), 7.84 (m, 1 H), 7.48 (t, *J*=8.42 Hz, 1 H), 7.36 (m, 3 H), 7.27 (m, 2 H), 7.21 (m, 1 H), 5.94 (br s, 1 H), 4.34 (m, 2 H), 3.70 (m, 2 H), 3.34 (m, 1 H), 3.30 and 3.30 (s, 3 H), 3.25 (m, 1 H).

### Step g: 2-[3-(2-amino-1-hydroxy-1-phenyl-ethyl)-2,6-difluoro-phenyl]-3-fluoro-4-(2-methoxyethoxy)benzamide

The compound was prepared using the same procedure as detailed in example 2 step c starting from 2-[3-(2-amino-1-hydroxy-1-phenyl-ethyl)-2,6-difluoro-phenyl]-3-fluoro-4-(2-methoxyethoxy)benzonitrile (84 mg, 0.19 mmol, 1 eq.) giving 2-[3-(2-amino-1-hydroxy-1-phenyl-ethyl)-2,6-difluoro-phenyl]-3-fluoro-4-(2-methoxyethoxy)benzamide (94 mg) as a mixture of four diastereomers. The crude product was purified and separated (Column: Xbridge C18, 30 × 100mm 5µm, Flowrate: 35 mL/min, Mobile phase: H2O + 0.1% NH4OH / MeOH + 0.1% NH4OH, Temperature: 45°C, Gradient: 20 to 95 % of MeOH within 30 min) to give racemates 16a and 16b. 16a (Rt : 0.84 min) (40 mg, 23%) as a white powder. ¹H NMR (500 MHz, DMSO-*d*₆) δ ppm 7.72 (br s, 1 H), 7.68 (td, *J*=8.80, 6.88 Hz, 1 H), 7.48 (dd, *J*=8.60, 1.03 Hz, 1 H), 7.35 (m, 2 H), 7.29 (m, 3 H), 7.20 (tt, *J*=7.15, 1.51 Hz, 1 H), 7.13 (d, *J*=8.80 Hz, 1 H), 7.11 (s, 1 H), 5.83 (br s, 1 H), 4.26 (m, 2 H), 3.69 (dd, *J*=5.09, 3.99 Hz, 2 H), 3.31 (s, 3 H), 3.24 (d, *J*=13.89 Hz, 1 H), 3.15 (d, *J*=13.89 Hz, 1 H), 1.36 (br s, 2 H). And **16b** (Rt : 0.95 min) (19 mg, 11%) as a white powder. ¹H NMR (500 MHz, DMSO-*d*₆) δ ppm 7.78 (td, *J*=8.84, 6.95 Hz, 1 H), 7.72 (br s, 1 H), 7.49 (dd, *J*=8.67, 1.10 Hz, 1 H), 7.34 (m, 2 H), 7.29 (m, 3 H), 7.20 (tt, *J*=7.02, 1.24 Hz, 1 H), 7.16 (s, 1 H), 7.14 (d, *J*=8.80 Hz, 1 H), 5.83 (br s, 1 H), 4.25 (m, 2 H), 3.69 (m, 2 H), 3.36 (d, *J*=13.76 Hz, 1 H), 3.30 (s, 3 H), 3.11 (d, *J*=13.75 Hz, 1 H), 1.33 (br s, 2 H).

### Example 17: 2-[5-(2-amino-1-hydroxy-1-phenyl-ethyl)-2-chloro-phenyl]-3-chloro-4-methoxy-benzamide

### Step a: 3-chloro-4-methoxybenzonitrile

Under argon atmosphere, 3-chloro-4-fluorobenzonitrile (3.0 g, 19.28 mmol, 1 eq.) was dissolved in dry MeOH (96.0 mL), cooled to 0-5 °C and NaOMe (2.0 g, 38.57 mmol, 2 eq.) was slowly added at 0-5 °C. The resulting mixture was stirred at rt overnight. After completion the mixture was evaporated, the residue was diluted with DCM and washed with water. The organic layer was dried, filtered, and concentrated. The crude product was purified by column chromatography (eluent: 0-25% EtOAc in *n*-heptane) to obtain 3-chloro-4-methoxybenzonitrile (1.5 g, 46%) as a beige solid. ES₊ MS *m*/*z* 168 [M+H]⁺, UPLC-MS using Waters method 4min_normal_A1B1_ELS_EVO (220 nm) 99% (AUC).

### Step b: 3-chloro-2-iodo-4-methoxybenzonitrile

Under argon atmosphere, LDA (1M solution, 2.3 eq.) was added to a solution of 3-Chloro-4-methoxybenzonitrile (1.75 g, 10.44 mmol, 1 eq.) in dry THF (21.0 mL) at -78 °C and stirred at this temperature for 45 min. Then, a solution of I₂ (3.98 g, 15.66 mmol, 1.5 eq.) in dry THF (3.2 mL) was added and stirring was continued at -78°C for 2 h. After completion the reaction mixture was quenched with a saturated aqueous solution of NH₄Cl (3 mUmmol), extracted with EtOAc (2x3 mUmmol) and washed with H₂O. The organic layer was dried over anhydrous Na₂SO₄ and concentrated to 1/3 and the precipitated solid was filtered off to obtain the first crystal of the product. The mother liquor was evaporated and purified by column chromatography (eluent: 0-40% EtOAc in n-heptane) to obtain further product. The two fractions were merged and dried to obtain 3-chloro-2-iodo-4-methoxybenzonitrile (1.51 g, 49%) as a beige solid. ¹H NMR (400 MHz DMSO-*d₆*): δ 7.84 (d, *J =* 8.7 Hz, 1 H), 7.34 (d, *J* = 8.8 Hz, 1 H), 3.95 (s, 3 H).

### Step c: 3-chloro-4-methoxy-2-(4,4,5,5-tetramethyl-1,3,2-dioxaborolan-2-yl)benzonitrile

Under argon atmosphere, 3-chloro-2-iodo-4-methoxybenzonitrile (1.51 g, 5.14 mmol, 1 eq.) was dissolved in dry Et₂O (51.5 mL) and cooled to -78°C with a dry ice/acetone bath. BuLi (2.5 M in hexane, 3.5 mL, 8.74 mmol, 1.7 eq.) was added slowly and stirred at -78°C for 30 min. Then 2-lsopropoxy-4,4,5,5-tetramethyl-1,3,2-dioxaborolane (10, 2.0 ml, 9.77 mmol, 1.9 eq.) was added and the stirring was continued at -78°C for 2 h. After completion the mixture was quenched by the addition of 1N aqueous HCl solution under ice cooling and extracted with EtOAc. The combined organic layers were washed with brine, dried and concentrated.

The crude product was triturated with n-heptane to obtain 3-chloro-4-methoxy-2-(4,4,5,5-tetramethyl-1 ,3,2-dioxaborolan-2-yl)benzonitrile (1.3 g, 86%) as a beige solid. ¹H NMR (500 MHz DMSO-*d₆*): δ 7.85 (d, *J* = 8.6 Hz, 1 H), 7.35 (d, *J* = 8.7 Hz, 1 H), 3.94 (s, 3 H), 1.36 (s, 12 H).

### Step d: tert-butyl (2-(3-bromo-4-chlorophenyl)-2-hydroxy-2-phenylethyl)carbamate

2-amino-1-(3-bromo-4-chloro-phenyl)-1-phenyl-ethanol as HCl salt (Ex 1, step c) (500.0 mg, 1.37 mmol, 1 eq.) was suspended in 1,4-dioxane (12.0 mL) and a 2M aqueous NaOH solution (895.0 µL, 1.79 mmol, 1.3 eq.) was added. The mixture was stirred vigorously and Boc₂O (348.0 mg, 1.5 mmol, 1.1 eq.) was added. The resulting mixture was stirred at 45°C for 4 h. After completion the mixture was concentrated and partitioned between EtOAc and water. The organic layer was separated and washed with brine, dried and concentrated. The crude product was washed with n-heptane and dried to obtain tert-butyl (2-(3-bromo-4-chlorophenyl)-2-hydroxy-2-phenylethyl)carbamate (565.0 mg, 96%) as a white solid. ¹H NMR (500 MHz DMSO-*d₆*): δ 7.77 (d, *J* = 2.2 Hz, 1 H), 7.52 (d, *J* = 8.5 Hz, 1 H), 7.43 - 7.40 (m, 2 H), 7.36 (dd, *J* = 8.5, 2.2 Hz, 1 H), 7.32 (t, *J* = 7.6 Hz, 2 H), 7.23 (t, *J* = 7.3 Hz, 1 H), 6.46 (t, *J* = 5.8 Hz, 1 H), 6.04 (s, 1H), 3.86 (dd, *J* = 13.9, 6.6 Hz, 1 H), 3.63 (dd, *J* = 13.9, 5.1 Hz, 1 H), 1.29 (s, 9 H).

### Step e: tert-butyl N-[2-[4-chloro-3-(2-chloro-6-cyano-3-methoxy-phenyl)phenyl]-2-hydroxy-2-phenyl-ethyl]carbamate

In a pressure vessel, a solution of *tert*-Butyl (2-(3-bromo-4-chlorophenyl)-2-hydroxy-2-phenylethyl)carbamate (150.0 mg, 0.35 mmol, 1 eq.) and 3-Chloro-4-methoxy-2-(4,4,5,5-tetramethyl-1,3,2-dioxaborolan-2-yl)benzonitrile (309.0 mg, 1.05 mmol, 3 eq.) in toluene (3.5 mL) and water (0.7 mL) was degassed for 2 min. Then K₃PO₄ (298.5 mg, 1.40 mmol, 4 eq.), XPHOS PD G2 (28.0 mg, 0.035 mmol, 0.1 eq.) and RuPhos (17.0 mg, 0.035 mmol, 0.1 eq.) were added and the mixture was degassed for 5 min then stirred at 130°C for 2-3 h. After completion the mixture was diluted with a saturated aqueous NaHCO₃ solution and extracted with EtOAc (3x). The combined organic layers were dried over anhydrous Na₂SO₄ and concentrated under reduced pressure. The crude product was purified by Prep-HPLC to obtain *tert*-butyl *N*-[2-[4-chloro-3-(2-chloro-6-cyano-3-methoxy-phenyl)phenyl]-2-hydroxy-2-phenyl-ethyl]carbamate (135.0 mg, 75%) as an off-white solid. ¹H NMR (500 MHz DMSO-*d₆*): δ 7.98 and 7.95 (d, *J =* 8.7 Hz, 1 H), 7.58 and 7.57 (d, *J* = 8.5, 1 H), 7.51 and 7.50 (t, *J* = 8.5, 1 H), 7.46 - 7.37 (m, 4 H), 7.33 - 7.24 (m, 2 H), 7.21 and 7.20 (d, *J* = 7.3, 1 H), 6.43 (q, *J* = 5.8 Hz, 1 H), 6.08 and 6.07 (s, 1 H), 4.01 and 4.00 (s, 3 H), 3.81 and 3.80 (d, *J* = 15.9 Hz, 1H), 3.65 and 3.61 (d, *J* = 15.9 Hz, 1 H), 1.27 and 1.26 (s, 9 H).

### Step f: tert-butyl N-[2-[3-(6-carbamoyl-2-chloro-3-methoxy-phenyl)-4-chloro-phenyl]-2-hydroxy-2-phenyl-ethyl]carbamate

The compound was prepared using the same procedure as detailed in example 2 step c starting from *tert*-butyl *N*-[2-[4-chloro-3-(2-chloro-6-cyano-3-methoxy-phenyl)phenyl]-2-hydroxy-2-phenyl-ethyl]carbamate (129.0 mg, 0.25 mmol, 1 eq.) giving tert-butyl N-[2-[3-(6-carbamoyl-2-chloro-3-methoxy-phenyl)-4-chloro-phenyl]-2-hydroxy-2-phenyl-ethyl]carbamate (104.0 mg, 78%) as a white solid, used without further purification.

### Step g: 2-[5-(2-amino-1-hydroxy-1-phenyl-ethyl)-2-chloro-phenyl]-3-chloro-4-methoxybenzamide

*tert*-butyl *N*-[2-[3-(6-carbamoyl-2-chloro-3-methoxy-phenyl)-4-chloro-phenyl]-2-hydroxy-2-phenyl-ethyl]carbamate (50.0 mg, 0.094 mmol, 1 eq.) was dissolved in 1,4-dioxane (0.47 mL) and a saturated solution of HCl in 1,4-dioxane (0.47 mL) was added. The mixture was stirred at rt for 3-4 h. After completion the solvent was evaporated, the residue was diluted with DCM and evaporated again. It was repeated twice. The product as HCl salt was dissolved in EtOAc and extracted with a saturated aqueous NaHCO₃ solution. The basic layer was extracted with EtOAc. The collected organic layer was dried and evaporated to obtain 5'-(2-Amino-1-hydroxy-1-phenylethyl)-2',6-dichloro-5-methoxy-[1,1'-biphenyl]-2-carboxamide (26 mg, 64%) as an off-white solid. ¹H NMR (500 MHz CDCl₃): δ 7.79 and 7.73 (d, *J* = 8.7 Hz, 1 H), 7.52 - 7.38 (m, 4 H), 7.36 - 7.28 (m, 3 H), 7.25 - 7.20 (m, 1 H), 7.05 and 7.03 (d, *J* = 8.7 Hz, 1 H), 5.49 - 5.18 (m, 2 H), 3.99 and 3.98 (s, 3 H), 3.44 - 3.25 (m, 2 H). mp.: 78-80 °C.

### Example 18: 2-[2-chloro-5-[1-hydroxy-2-(methylamino)-1-phenyl-ethyl]phenyl]-3-fluoro-4-(2-methoxyethoxy)benzenecarbohydroxamic acid

### Step a: methyl 2-bromo-3-fluoro-4-(2-methoxyethoxy)benzoate

In 25 mL round-bottomed flask, under argon atmosphere methoxyethanol (0.700 mL, 8.88 mmol, 1.1 eq.) was dissolved in anhydrous THF (40.0 mL). The reaction mixture was cooled down to 0°C. NaH (60% in oil) (414 mg, 10.4 mmol, 1.3 eq.) was added portionwise. The reaction mixture was stirred at 0°C for 20 min then methyl 2-bromo-3,4-difluoro-benzoate (2 g, 7.97 mmol, 1 eq.) was added. The reaction mixture was stirred at 0°C for 15 min then at rt for 1h45min. The mixture was quenched with a saturated solution of NH₄Cl and extracted with EtOAc (3 times). The organic layers were washed with brine and dried over MgSO₄, filtered and concentrated under reduced pressure to give a beige solid (2.2 g). The crude was purified by chromatography (Cartridge Biotage Sfar 50 g / 60 µm, DCM/(DCM:MeOH 9:1) 100:0 over 5 CV, 100:0 to 75:25 over 5 CV, 75:25 over 20 CV, Flow rate 100 ml / min) to give methyl 2-bromo-3-fluoro-4-(2-methoxyethoxy)benzoate (1.39 g, 56.8%) as a white solid. ¹H NMR (400 MHz, DMSO-*d*₆) δ ppm 7.69 (dd, *J*=8.80, 1.98 Hz, 1 H), 7.32 (dd, *J*=8.80, 8.14 Hz, 1 H), 4.29 (m, 2 H), 3.84 (s, 3 H), 3.70 (m, 2 H), 3.31 (s, 3 H).

### Step b: tert-butyl N-[2-(3-bromo-4-chloro-phenyl)-2-hydroxy-2-phenyl-ethyl]-∼{N}-methylcarbamate

The compound was prepared using the same procedure as detailed in example 17 step d starting from 1-(3-bromo-4-chloro-phenyl)-2-(methylamino)-1-phenyl-ethanol (Ex 7 step c) (510 mg, 1.50 mmol, 1 eq.) giving *tert*-butyl *N*-[2-(3-bromo-4-chloro-phenyl)-2-hydroxy-2-phenyl-ethyl]-*N*-methyl-carbamate(450 mg, 68.2%) as a colorless gum, used without further purification.

### Step c: tert-butyl N-[2-[4-chloro-3-(4,4,5,5-tetramethyl-1,3,2-dioxaborolan-2-yl)phenyl]-2-hydroxy-2-phenyl-ethyl]-N-methyl-carbamate

In a Q-tube, under argon, *tert-*butyl *N*-[2-(3-bromo-4-chloro-phenyl)-2-hydroxy-2-phenylethyl]-*N*-methyl-carbamate (850 mg, 1.93 mmol, 1 eq.) was dissolved in toluene (17 mL). The mixture was degassed under argon for 5 min, then bis(pinacolato)diboron (1.5 g, 5.9 mmol, 3.1 eq.), Pd(dppf)Cl₂,CH₂Cl₂ COMPLEX (157 mg, 0.192 mmol, 0.099 eq.) and AcOK (567 mg, 5.78 mmol, 3 eq.) were added. The mixture was stirred at 100°C for 2h30min. The solvent was removed in vacuo. The crude was purified by chromatography (Cartridge Biotage Sfar 25 g / 60 µm, DCM/(DCM:MeOH 9:1) 100:0 over 5 CV, 100:0 to 0:100 over 5 CV, 0:100 over 20 CV) to give tert-butyl *N*-[2-[4-chloro-3-(4,4,5,5-tetramethyl-1,3,2-dioxaborolan-2-yl)phenyl]-2-hydroxy-2-phenyl-ethyl]-*N*-methyl-carbamate (410 mg, 43.6%) as an orange solid used without further purification.

### Step d: methyl 2-[5-[2-[tert-butoxycarbonyl(methyl)amino]-1-hydroxy-1-phenyl-ethyl]-2-chlorophenyl]-3-fluoro-4-(2-methoxyethoxy)benzoate

In a Q-tube, under argon, a solution of *tert*-butyl *N*-[2-[4-chloro-3-(4,4,5,5-tetramethyl-13,2-dioxaborolan-2-yl)phenyl]-2-hydroxy-2-phenyl-ethyl]-*N*-methyl-carbamate (450 mg, 0.922 mmol, 1 eq.) and methyl 2-bromo-3-fluoro-4-(2-methoxyethoxy)benzoate (340 mg, 1.11 mmol, 1.2 eq.) in toluene (9 mL)/water (1.8 mL) was degassed for 2 min. Then Pd₂(dba)₃ (43 mg, 0.047 mmol, 0.051 eq.), N-Xantphos (50 mg, 0.091 mmol, 0.098 eq.) and K₃PO₄ (588 mg, 2.77 mmol, 3 eq.) were added and the mixture was degassed for 2 min before being stirred at 100°C for 2h30 min. The mixture was diluted with NaHCO₃ and extracted with EtOAc (3x). The combined organic layers were dried over anhydrous MgSO₄, filtered and concentrated. The crude was purified by preparative HPLC (Mobile phase: H2O with 0,1% FA / ACN + 0,1 % FA, Flowrate : 42 ml/min, Column: Kinetex C18,30 x 150mm 5µm (phenomenex)) to give methyl 2-[5-[2-[tert-butoxycarbonyl(methyl)amino]-1-hydroxy-1-phenyl-ethyl]-2-chloro-phenyl]-3-fluoro-4-(2-methoxyethoxy)benzoate (230 mg, 42.4%) as a beige solid, used without further purification. A small portion was Boc cleaved to confirm the structure by NMR (methyl 2-[2-chloro-5-[1-hydroxy-2-(methylamino)-1-phenyl-ethyl]phenyl]-3-fluoro-4-(2-methoxyethoxy)benzoate). ¹H NMR (400 MHz, DMSO-*d*₆) δ ppm 7.78 and 7.76 (dd, *J*=8.67, 1.54 Hz, 1 H), 7.50 - 7.26 (m, 9 H), 7.19 (m, 1 H), 5.67 (br s, 1 H), 4.30 (m, 2 H), 3.71 (m, 2 H), 3.43 and 3.40 (s, 3 H), 3.31 and 3.30 (s, 3 H), 3.18 (m, 2 H), 2.27 and 2.26 (s, 3 H).

### Step e: 2-[5-[2-[tert-butoxycarbonyl(methyl)amino]-1-hydroxy-1-phenyl-ethyl]-2-chlorophenyl]-3-fluoro-4-(2-methoxyethoxy)benzoic acid

A solution of KOH (790 mg, 14.1 mmol, 1.40) in MeOH (1.80 mL, 44.5 mmol, 4.42 eq.) was cooled to 0°C. Methyl 2-[5-[2-[*tert*-butoxycarbonyl(methyl)amino]-1-hydroxy-1-phenyl-ethyl]-2-chloro-phenyl]-3-fluoro-4-(2-methoxyethoxy)benzoate (95.0 mg, 0.162 mmol, 1 eq.) was added and the reaction mixture was stirred at rt overnight. The reaction mixture was concentrated under reduced pressure. The crude was purified by chromatography (Cartridge Biotage Sfar 5 g / 20 µm, DCM/(DCM/MeOH 9:1) 100:0 over 5 CV,100:0 to 0:100 over 30 CV,0:100 over 5 CV, Flow rate 22 ml / min) to give 2-[5-[2-[*tert-*butoxycarbonyl(methyl)amino]-1-hydroxy-1-phenyl-ethyl]-2-chloro-phenyl]-3-fluoro-4-(2-methoxyethoxy)benzoic acid (92.5 mg, 99.7%) as a beige solid, used without further purification.

### Step f: tert-butyl N-[2-[4-chloro-3-[2-fluoro-3-(2-methoxyethoxy)-6-(tetrahydropyran-2-yloxycarbamoyl)phenyl]phenyl]-2-hydroxy-2-phenyl-ethyl]-N-methyl-carbamate

In a 5 mL round bottom flask and under inert atmosphere, were added 2-[5-[2-[*tert-*butoxycarbonyl(methyl)amino]-1-hydroxy-1-phenyl-ethyl]-2-chloro-phenyl]-3-fluoro-4-(2-methoxyethoxy)benzoic acid (48.9 mg, 0.0852 mmol, 1 eq.), dry DMF (0.42 mL,) and DIPEA (0.0600 mL, 0.363 mmol, 4.26 eq.). The reaction was stirred for 1 min. Then EDCI (23.0 mg, 0.120 mmol, 1.41 eq.), HOBt (16.1 mg, 0.119 mmol, 1.40 eq.) and O-(oxan-2-yl)hydroxylamine (14.0 mg, 0.120 mmol, 1.4 eq.) were added. The mixture was stirred at room temperature for 18h30 min. The reaction mixture was concentrated then water and EtOAc were added. The combined organic layers were washed with brine, dried over MgSO₄, filtered and concentrated. The crude was purified by chromatography (Column: Kinetex C18,30 x 150mm 5µm (phenomenex ), Flowrate: 42 ml/min, Mobile phase: H₂O with 0,1% FA / ACN + 0,1 % FA° to give tert-butyl *N*-[2-[4-chloro-3-[2-fluoro-3-(2-methoxyethoxy)-6-(tetrahydropyran-2-yloxycarbamoyl)phenyl]phenyl]-2-hydroxy-2-phenyl-ethyl]-*N*-methylcarbamate (40.2 mg, 70.1%), used without further purification.

### Step g: 2-[2-chloro-5-[1-hydroxy-2-(methylamino)-1-phenyl-ethyl]phenyl]-3-fluoro-4-(2-methoxyethoxy)benzenecarbohydroxamic acid

In a 5 mL round bottom flask, *tert*-butyl *N*-[2-[4-chloro-3-[2-fluoro-3-(2-methoxyethoxy)-6-(tetrahydropyran-2-yloxycarbamoyl)phenyl]phenyl]-2-hydroxy-2-phenyl-ethyl]-*N*-methylcarbamate (40.0 mg, 0.0594 mmol, 1 eq.) was dissolved in 1,4-dioxane (1.3 mL) at rt and a 4.0M solution of HCl in 1,4-dioxane (1.3 mL, 5.2 mmol, 88 eq.) was added. The resulting colorless mixture was stirred at rt for 17h. The mixture was evaporated, and the crude (39 mg) was purified by preparative LCMS (Column: Kinetex C18,30 x 150mm 5µm (phenomenex ), Flowrate : 42 ml/min, Mobile phase: H₂O with 0.1% FA / ACN + 0.1 % FA). Fractions of expected product were collected, concentrated, then water and a saturated solution of NaHCO₃ were added until a white precipitate formed. DCM was then added and extracted (2x). The organic layer was freeze-dried overnight (lyophilization) to give 2-[2-chloro-5-[1-hydroxy-2-(methylamino)-1-phenyl-ethyl]phenyl]-3-fluoro-4-(2-methoxyethoxy)benzenecarbohydroxamic acid (11.4 mg, 39.2%) as a beige solid. ¹H NMR (500 MHz, DMSO-*d*₆) δ ppm 7.57 (m, 1 H), 7.50 (m, 3.5 H), 7.37 (m, 2.5 H), 7.29 (m, 3 H), 4.25 (m, 2 H), 3.92 and 3.90 (d, *J*=13.75 Hz, 1 H), 3.87 and 3.75 (d, *J*=13.34 Hz, 1 H), 3.70 (m, 2 H), 3.32 and 3.31 (s, 3 H), 2.50 and 2.49 (s, 3 H).

### Example 19a: rel-(2aR)-2-[3-[(1S)-2-amino-1-hydroxy-1-phenyl-ethyl]-6-chloro-2-methylphenyl]-3-fluoro-4-(2-methoxyethoxy)benzamide

### Step a: [(1S,2R,5S)-2-isopropyl-5-methyl-cyclohexyl] N-[(2S)-2-(3-bromo-4-chloro-2-methylphenyl)-2-hydroxy-2-phenyl-ethyl]carbamate

Under N₂ atmosphere, 2-amino-1-(3-bromo-4-chloro-2-methyl-phenyl)-1-phenyl-ethanol (Ex 9a, step c) (12 g, 35.2 mmol, 1 eq.) was solubilized in DCM (240 mL) and TEA (10 mL, 71.9 mmol, 2.04 eq.) was added. The solution was cooled to 0°C and (+)-menthyl chloroformate (9 mL, 42.42 mmol, 1.2 eq.) was added dropwise at 0°C. After the end of the addition, the reaction was complete. The solution was diluted with DCM and washed with an aqueous solution of NH₄Cl. The resulting organic layer was dried over MgSO₄, filtered and concentrated to afford a brown foam (21 g). The crude product was dissolved in 63 ml (3V) of ACN and sonicated until a beige precipitate appeared. The precipitate was filtered off and washed with cold ACN (15mL) to afford [(1S,2R,5S)-2-isopropyl-5-methyl-cyclohexyl] *N-*[(2S)-2-(3-bromo-4-chloro-2-methyl-phenyl)-2-hydroxy-2-phenyl-ethyl]carbamate (3.2 g, 17%) as a white powder. LC/MS (Method C): Rt: 2.46, 93.3 %. ¹H NMR (500 MHz, DMSO-*d*₆) δ ppm 7.67 (d, *J*=8.67 Hz, 1 H), 7.49 (d, *J*=8.53 Hz, 1 H), 7.27 (m, 2 H), 7.21 (m, 3 H), 6.61 (br t, *J*=5.85 Hz, 1 H), 5.95 (s, 1 H), 4.22 (td, *J*=10.80, 4.13 Hz, 1 H), 3.93 (dd, *J*=13.62, 6.74 Hz, 1 H), 3.70 (dd, *J*=13.55, 4.88 Hz, 1 H), 2.08 (s, 3 H), 1.72 (dtd, *J*=13.84, 7.01, 7.01, 2.82 Hz, 1 H), 1.58 (m, 3 H), 1.34 (m, 1 H), 1.18 (m, 1 H), 0.95 (m, 1 H), 0.86 (d, *J*=6.46 Hz, 3 H), 0.80 (d, *J*=7.02 Hz, 3 H), 0.73 (m, 2 H), 0.62 (d, *J*=6.88 Hz, 3 H).

### Step b: (1S)-2-amino-1-(3-bromo-4-chloro-2-methyl-phenyl)-1-phenyl-ethanol

In a Q-tube, [(1S,2R,5S)-2-isopropyl-5-methyl-cyclohexyl] N-[(2S)-2-(3-bromo-4-chloro-2-methyl-phenyl)-2-hydroxy-2-phenyl-ethyl]carbamate (3.1 g, 5.93 mmol, 1 eq) was suspended in EtOH (16 mL). KOH (7 g, 124.7646 mmol, 21 eq.) in water (16 mL) was added. The reaction mixture was stirred at 100°C for 20h to afford completion. The reaction mixture was extracted with EtOAc. The organic layer was dried over MgSO₄ and evaporated under reduced pressure. The crude product was purified by chromatography (SiO₂, eluting from 0% to 100 % DCM/MeOH/NH₃: 9/1/0.05 in 8 CV) to give (1S)-2-amino-1-(3-bromo-4-chloro-2-methyl-phenyl)-1-phenyl-ethanol (1.51 g, 74%) as a white solid. The absolute configuration was validated by XR. ¹H NMR (500 MHz, DMSO-*d*₆) δ ppm 7.67 (d, *J*=8.53 Hz, 1 H), 7.50 (d, *J*=8.39 Hz, 1 H), 7.28 (t, *J*=7.84 Hz, 2 H), 7.19 (m, 3 H), 5.77 (br s, 1 H), 3.29 (d, *J*=13.07 Hz, 1 H), 2.97 (d, *J*=13.20 Hz, 1 H), 2.13 (s, 3 H), 1.46 (br s, 2 H). [α]_{d}²² = -64.5° (C=0.38, DCM).

### Step c: 3-fluoro-4-(2-methoxyethoxy)-2-trimethylstannyl-benzonitrile

2-bromo-3-fluoro-4-(2-methoxyethoxy)benzonitrile (Ex 6, step a) (6 g, 21.89 mmol, 1 eq.) was dissolved in THF (60 mL). The solution was cooled down to -78°C then n-BuLi 1.6M in hexane (16 mL, 26 mmol, 1.2 eq.) was slowly added. The mixture became red wine. The reaction mixture was stirred at -78°C for 1h. Me₃SnCl (33 mL, 33 mmol, 1.5 eq.) was added as solid. The reaction mixture was stirred at -78°C for 30 min and then quenched at -78°C with a saturated solution of NH₄Cl. The reaction mixture was extracted with EtOAc (x3), dried over MgSO₄, filtered and concentrated to dryness. The crude product was purified by chromatography (Cartridge 100g silica kp, gradient = cyclohexane/EtOAc 9:1(6cv) to 80:20 (9cv) and 80:20 (5cv), flow rate 40ml/min) to give 3-fluoro-4-(2-methoxyethoxy)-2-trimethylstannyl-benzonitrile (6.25 g, 79.8%) as a colorless oil. ¹H NMR (400 MHz, DMSO-*d*₆) δ ppm 7.63 (dd, *J*=8.36, 0.66 Hz, 1 H), 7.27 (t, *J*=8.80 Hz, 1 H), 4.24 (m, 2 H), 3.68 (m, 2 H), 3.31 (s, 3 H), 0.44 (m, 9 H).

### Step d: rel-(2aR)-2-[3-[(1S)-2-amino-1-hydroxy-1-phenyl-ethyl]-6-chloro-2-methyl-phenyl]-3-fluoro-4-(2-methoxyethoxy)benzonitrile and rel-(2aS)-2-[3-[(1S)-2-amino-1-hydroxy-1-phenylethyl]-6-chloro-2-methyl-phenyl]-3-fluoro-4-(2-methoxyethoxy)benzonitrile

In a 50mL flask, under nitrogen, (1S)-2-amino-1-(3-bromo-4-chloro-2-methyl-phenyl)-1-phenyl-ethanol (Ex 19, step b) (200 mg, 0.59 mmol, 1 eq.), 3-fluoro-4-(2-methoxyethoxy)-2-trimethylstannyl-benzonitrile (420 mg, 1.17 mmol, 2 eq.), and Bis(tri-t-butylphosphine)palladium(0) (150 mg, 0.29 mmol, 0.5 eq.) in DMF (13 mL) were degassed under nitrogen. The mixture was stirred and warmed at 130°C for 2 h. The reaction mixture was concentrated to dryness to give a crude product as a black oil. The crude was portioned between water and EtOAc and extracted twice with EtOAc. The combined organic layers were washed with brine, dried over MgSO₄, filtered and concentrated in vacuo. The crude product was purified by Preparative LC (method B) to give rel-(2aR)-2-[3-[(1S)-2-amino-1-hydroxy-1-phenyl-ethyl]-6-chloro-2-methyl-phenyl]-3-fluoro-4-(2-methoxyethoxy)benzonitrile (26 mg, 6.2 %) as a pale yellow oil. LC/MS (method A) Rt = 6.29 min. ¹H NMR (400 MHz, DMSO-*d*₆) δ ppm 7.79 (d, *J*=8.69 Hz, 1 H), 7.75 (dd, *J*=8.69, 1.43 Hz, 1 H), 7.53 (d, *J*=8.58 Hz, 1 H), 7.39 (t, *J*=8.53 Hz, 1 H), 7.24 (m, 2 H), 7.18 (m, 3 H), 5.77 (br s, 1 H), 4.32 (m, 2 H), 3.70 (m, 2 H), 3.35 (d, 1 H), 3.30 (s, 3 H), 3.01 (d, *J*=13.20 Hz, 1 H), 1.69 (s, 3 H), and rel-(2aS)-2-[3-[(1S)-2-amino-1-hydroxy-1-phenyl-ethyl]-6-chloro-2-methyl-phenyl]-3-fluoro-4-(2-methoxyethoxy)benzonitrile (26 mg, 6.2 %) as a colorless oil. LC/MS (Method A) Rt = 6.29 min. ¹H NMR (400 MHz, CHLOROFORM-d) δ ppm 7.53 (d, *J*=8.47 Hz, 1 H), 7.49 (dd, *J*=8.58, 1.65 Hz, 1 H), 7.41 (d, *J*=8.36 Hz, 1 H), 7.27 (m, 5 H), 7.04 (dd, *J*=8.58, 7.81 Hz, 1 H), 4.23 (td, *J*=4.65, 1.05 Hz, 2 H), 3.77 (m, 2 H), 3.55 (d, *J*=12.85 Hz, 1 H), 3.42 (s, 3 H), 3.18 (d, *J*=12.87 Hz, 1 H), 1.83 (s, 3 H).

### Step e: rel-(2aR)-2-[3-[(1S)-2-amino-1-hydroxy-1-phenyl-ethyl]-6-chloro-2-methyl-phenyl]-3-fluoro-4-(2-methoxyethoxy)benzamide

The compound was prepared using the same procedure as detailed in example 2 step c starting from rel-(2aR)-2-[3-[(1S)-2-amino-1-hydroxy-1-phenyl-ethyl]-6-chloro-2-methylphenyl]-3-fluoro-4-(2-methoxyethoxy)benzonitrile (24 mg, 0.0528 mmol, 1 eq.) giving rel-(2aR)-2-[3-[(1S)-2-amino-1-hydroxy-1-phenyl-ethyl]-6-chloro-2-methyl-phenyl]-3-fluoro-4-(2-methoxyethoxy)benzamide (15.6 mg, 62.5%) as a white foam. LC/MS (Method A) Rt = 0.84 min. ¹H NMR (500 MHz, DMSO-*d*₆) δ ppm 7.63 (d, *J*=8.67 Hz, 1 H), 7.43 (dd, *J*=8.60, 0.89 Hz, 1 H), 7.35 (d, *J*=8.53 Hz, 1 H), 7.25 (m, 2 H), 7.18 (m, 5 H), 6.98 (br s, 1 H), 5.65 (br s, 1 H), 4.23 (dd, *J*=5.30, 3.65 Hz, 2 H), 3.69 (m, 2 H), 3.31 (s, 3 H), 3.30 (d, *J*=13.2 Hz, 1 H), 3.01 (d, *J*=13.20 Hz, 1 H), 1.64 (s, 3 H).

### Example 19b: rel-(2aS)-2-[3-[(1S)-2-amino-1-hydroxy-1-phenyl-ethyl]-6-chloro-2-methylphenyl]-3-fluoro-4-(2-methoxyethoxy)benzamide

The compound was prepared using the same procedure detailed in example 2 step c starting from rel-(2aS)-2-[3-[(1S)-2-amino-1-hydroxy-1-phenyl-ethyl]-6-chloro-2-methyl-phenyl]-3-fluoro-4-(2-methoxyethoxy)benzonitrile (Ex 19a, step d) (10 mg, 0.022 mmol, 1 eq.) giving rel-2-(2aS)-[3-[(1S)-2-amino-1-hydroxy-1-phenyl-ethyl]-6-chloro-2-methyl-phenyl]-3-fluoro-4-(2-methoxyethoxy)benzamide (6.0 mg, 58 %) as a white foam. LC/MS (Method A) Rt = 0.86min. ¹H NMR (500 MHz, DMSO-*d*₆) δ ppm 7.58 (d, *J*=8.67 Hz, 1 H), 7.44 (m, 1 H), 7.33 (br s, 1 H), 7.33 (d, *J*=8.25 Hz, 1 H), 7.25 (m, 2 H), 7.19 (m, 4 H), 6.99 (br s, 1 H), 5.66 (br s, 1 H), 4.19 (q, *J*=3.99 Hz, 2 H), 3.66 (t, *J*=4.47 Hz, 2 H), 3.31 (d, *J*=12.93 Hz, 16 H), 3.28 (s, 3 H), 3.01 (d, *J*=12.93 Hz, 1 H), 1.66 (s, 3 H).

### Example 20a: rel-(2-aS)-2-[3-[(1R)-2-amino-1-hydroxy-1-phenyl-ethyl]-6-chloro-2-methyl-phenyl]-3-fluoro-4-(2-methoxyethoxy)benzamide

### Step a: [(1R,2S,5R)-2-isopropyl-5-methyl-cyclohexyl] N-[(2R)-2-(3-bromo-4-chloro-2-methylphenyl)-2-hydroxy-2-phenyl-ethyl]carbamate

The compound was prepared using the same procedure as detailed in example 13 step a starting from 2-amino-1-(3-bromo-4-chloro-2-methyl-phenyl)-1-phenyl-ethanol (Ex 9a, step c) (7.75 g, 22.8 mmol, 1 eq.) and (-)-menthyl chloroformate (9 mL, 42.42 mmol, 1.2 eq.) giving [(1R,2S,5R)-2-isopropyl-5-methyl-cyclohexyl] *N*-[(2R)-2-(3-bromo-4-chloro-2-methyl-phenyl)-2-hydroxy-2-phenyl-ethyl]carbamate (1.53 g, 12.9%) as an off-white powder. LC/MS (Method C) : Rt : 2.46 min. ¹H NMR (500 MHz, DMSO-*d*₆) δ ppm 7.67 (d, *J*=8.53 Hz, 1 H), 7.48 (d, *J*=8.67 Hz, 1 H), 7.27 (m, 2 H), 7.21 (m, 3 H), 6.61 (br t, *J*=5.78 Hz, 1 H), 5.95 (s, 1 H), 4.22 (td, *J*=10.80, 4.26 Hz, 1 H), 3.93 (m, 1 H), 3.70 (dd, *J*=13.75, 4.95 Hz, 1 H), 2.08 (s, 3 H), 1.72 (dtd, *J*=1 3.89, 6.88, 6.88, 2.20 Hz, 1 H), 1.58 (m, 3 H), 1.34 (m, 1 H), 1.18 (m, 1 H), 0.95 (m, 1 H), 0.86 (d, *J*=6.46 Hz, 3 H), 0.80 (d, *J*=7.02 Hz, 4 H), 0.73 (m, 2 H), 0.62 (d, *J*=7.02 Hz, 3 H)

### Step b: (1R)-2-amino-1-(3-bromo-4-chloro-2-methyl-phenyl)-1-phenyl-ethanol

The compound was prepared using the same procedure detailed in example 13 step b starting from [(1R,2S,5R)-2-isopropyl-5-methyl-cyclohexyl] *N*-[(2R)-2-(3-bromo-4-chloro-2-methyl-phenyl)-2-hydroxy-2-phenyl-ethyl]carbamate (1.53 g, 2.93 mmol, 1 eq.) giving (1R)-2-amino-1-(3-bromo-4-chloro-2-methyl-phenyl)-1-phenyl-ethanol (635 mg, 63.7%) as a white powder. ¹H NMR (500 MHz, DMSO-*d*₆) δ ppm 7.67 (d, *J*=8.53 Hz, 1 H), 7.50 (d, *J*=8.39 Hz, 1 H), 7.28 (m, 2 H), 7.19 (m, 3 H), 5.76 (br s, 1 H), 3.29 (d, *J*=13.07 Hz, 1 H), 2.97 (d, *J*=13.07 Hz, 1 H), 2.13 (s, 3 H), 1.46 (br s, 2 H).

### Step c: 2-[(2R)-2-(3-bromo-4-chloro-2-methyl-phenyl)-2-hydroxy-2-phenyl-ethyl]isoindoline-1,3-dione

The compound was prepared using the same procedure as detailed in example 16 step d starting from (1R)-2-amino-1-(3-bromo-4-chloro-2-methyl-phenyl)-1-phenyl-ethanol (825 mg, 2.42 mmol, 1 eq.) giving 2-[(2R)-2-(3-bromo-4-chloro-2-methyl-phenyl)-2-hydroxy-2-phenylethyl]isoindoline-1,3-dione (1.14 g, 78.7%) as a white powder. ¹H NMR (400 MHz, DMSO-*d*₆) δ ppm 7.78 (m, 5 H), 7.46 (m, 1 H), 7.19 (m, 5 H), 5.97 (s, 1 H), 4.51 (d, *J*=13.31 Hz, 1 H), 4.43 (d, *J*=13.75 Hz, 1 H), 2.05 (s, 3 H).

### Step d: 2-[6-chloro-3-[(1R)-2-(1,3-dioxoisoindolin-2-yl)-1-hydroxy-1-phenyl-ethyl]-2-methylphenyl]-3-fluoro-4-(2-methoxyethoxy)benzonitrile

The compound was prepared using the same procedure as detailed in example 19a step d starting from 2-[(2R)-2-(3-bromo-4-chloro-2-methyl-phenyl)-2-hydroxy-2-phenylethyl]isoindoline-1,3-dione (410 mg, 0.87 mmol, 1 eq.) and 3-fluoro-4-(2-methoxyethoxy)-2-trimethylstannyl-benzonitrile (Ex 19a, step c) (623 mg, 1.74 mmol, 2 eq.) giving 2-[6-chloro-3-[(1R)-2-(1,3-dioxoisoindolin-2-yl)-1-hydroxy-1-phenyl-ethyl]-2-methyl-phenyl]-3-fluoro-4-(2-methoxyethoxy)benzonitrile (256 mg, 50.2 %) as a pale yellow solid. ¹H NMR (400 MHz, DMSO-*d*₆) δ ppm 7.98 and 7.91 (d, *J*=8.80 Hz, 1 H), 7.78 (m, 5 H), 7.55 and 7.48 (dd, *J*=8.69, 0.55 Hz, 1 H), 7.39 (t, *J*=7.81 Hz, 1 H), 7.20 (m, 2 H), 7.12 (m, 3 H), 6.02 and 6.01 (s, 1 H), 4.55 and 4.52 (d, *J*=13.4 Hz, 1H), 4.51 and 4.46 (d, *J*=13.4 Hz, 1H) 4.31 (m, 2 H), 3.70 (dt, *J*=5.89, 2.89 Hz, 2 H), 3.30 and 3.29 (s, 3 H), 1.61 and 1.60 (s, 3 H)

### Step e: rel-(2aS)-2-[3-[(1R)-2-amino-1-hydroxy-1-phenyl-ethyl]-6-chloro-2-methyl-phenyl]-3-fluoro-4-(2-methoxyethoxy)benzonitrile and rel-(2aR)-2-[3-[(1R)-2-amino-1-hydroxy-1-phenylethyl]-6-chloro-2-methyl-phenyl]-3-fluoro-4-(2-methoxyethoxy)benzonitrile

The compound was prepared using the same procedure as detailed in example 16 step f starting from 2-[6-chloro-3-[(1R)-2-(1,3-dioxoisoindolin-2-yl)-1-hydroxy-1-phenyl-ethyl]-2-methyl-phenyl]-3-fluoro-4-(2-methoxyethoxy)benzonitrile (254 mg, 0.43 mmol, 1 eq.) giving after separation by LC/MS rel-(2aS)-2-[3-[(1R)-2-amino-1-hydroxy-1-phenyl-ethyl]-6-chloro-2-methyl-phenyl]-3-fluoro-4-(2-methoxyethoxy)benzonitrile (43.0 mg, 21.8%) as a white powder. LC/MS (Method C): Rt : 7.06 min. ¹H NMR (400 MHz, DMSO-*d*₆) δ ppm 7.79 (d, *J*=8.69 Hz, 1 H), 7.75 (dd, *J*=8.69, 1.32 Hz, 1 H), 7.53 (d, *J*=8.58 Hz, 1 H), 7.39 (t, *J*=8.53 Hz, 1 H), 7.24 (m, 2 H), 7.18 (m, 3 H), 5.77 (br s, 1 H), 4.32 (m, 2 H), 3.70 (m, 2 H), 3.35 (d, *J*=13.09 Hz, 1 H), 3.30 (s, 3 H), 3.01 (d, *J*=13.09 Hz, 1 H), 1.69 (s, 3 H); and rel-(2aR)-2-[3-[(1R)-2-amino-1-hydroxy-1-phenyl-ethyl]-6-chloro-2-methyl-phenyl]-3-fluoro-4-(2-methoxyethoxy)benzonitrile (42.0 mg, 21.3%) as a white powder. LC/MS (Method C): Rt : 7.13 min. ¹H NMR (400 MHz, DMSO-*d*₆) δ ppm 7.80 (td, *J*=8.69, 1.43 Hz, 1 H), 7.78 (d, *J*=8.69 Hz, 1 H), 7.51 (d, *J*=9.02 Hz, 1 H), 7.39 (t, *J*=8.53 Hz, 1 H), 7.27 (m, 2 H), 7.19 (m, 3 H), 5.78 (br s, 1 H), 4.29 (q, *J*=3.92 Hz, 2 H), 3.67 (m, 2 H), 3.35 (d, *J*=12.98 Hz, 2 H), 3.28 (s, 3 H), 3.04 (d, *J*=13.20 Hz, 1 H), 1.69 (s, 3 H).

### Step f: rel-(2aS)-[6-chloro-3-[(1R)-1-hydroxy-2-(methylamino)-1-phenyl-ethyl]-2-methylphenyl]-3-fluoro-4-(2-methoxyethoxy)benzamide

The compound was prepared using the same procedure as detailed in example 2 step c starting from rel-(2aS)-2-[3-[(1R)-2-amino-1-hydroxy-1-phenyl-ethyl]-6-chloro-2-methylphenyl]-3-fluoro-4-(2-methoxyethoxy)benzonitrile (41 mg, 0.09 mmol, 1 eq.) giving rel-(2aS)-2-[3-[(1R)-2-amino-1-hydroxy-1-phenyl-ethyl]-6-chloro-2-methyl-phenyl]-3-fluoro-4-(2-methoxyethoxy)benzamide (36 mg, 84.4%) as a white powder. ¹H NMR (500 MHz, DMSO-*d*₆) δ ppm 7.63 (d, *J*=8.67 Hz, 1 H), 7.43 (d, *J*=8.25 Hz, 1 H), 7.35 (d, *J*=8.53 Hz, 1 H), 7.24 (m, 3 H), 7.18 (m, 4 H), 6.98 (br s, 1 H), 5.65 (br s, 1 H), 4.23 (m, 2 H), 3.69 (m, 2 H), 3.33 (d, *J*=13.20 Hz, 1 H), 3.30 (s, 3 H), 3.01 (d, *J*=13.20 Hz, 1 H), 1.64 (s, 3 H). [α]_{d}^{21.2} = -22.1° (C=0.46, MeOH).

### Example 20b: rel-(2aR)-2-[3-[(1R)-2-amino-1-hydroxy-1-phenyl-ethyl]-6-chloro-2-methyl-phenyl]-3-fluoro-4-(2-methoxyethoxy)benzamide

The compound was prepared using the same procedure detailed in example 2 step c starting from rel-(2aR)-2-[3-[(1R)-2-amino-1-hydroxy-1-phenyl-ethyl]-6-chloro-2-methyl-phenyl]-3-fluoro-4-(2-methoxyethoxy)benzonitrile (Ex 20a, step e) (40 mg, 0.088 mmol, 1 eq.) giving rel-(2aR)-2-[3-[(1R)-2-amino-1-hydroxy-1-phenyl-ethyl]-6-chloro-2-methyl-phenyl]-3-fluoro-4-(2-methoxyethoxy)benzamide (33 mg, 79.3%) as a white powder. ¹H NMR (500 MHz, DMSO-*d*₆) δ ppm 7.58 (d, *J*=8.53 Hz, 1 H), 7.44 (d, *J*=8.12 Hz, 1 H), 7.34 (br s, 1 H), 7.33 (d, *J*=8.39 Hz, 1 H), 7.25 (m, 2 H), 7.19 (m, 4 H), 6.99 (br s, 1 H), 5.68 (br s, 1 H), 4.19 (q, *J*=4.13 Hz, 2 H), 3.66 (t, *J*=4.47 Hz, 2 H), 3.33 (d, *J*=13.07 Hz, 1 H), 3.28 (s, 3 H), 3.02 (d, *J*=13.07 Hz, 1 H), 1.66 (s, 3 H). [α]_{d}^{20.7} = +33.1 ° (C=0.36, MeOH).

### Example 21: 2-[6-chloro-3-[2-(cyclohexylamino)-1-hydroxy-1-phenyl-ethyl]-2-fluorophenyl]-3-fluoro-4-(2-methoxyethoxy)benzamide

### Step a: 1-(3-bromo-4-chloro-2-fluoro-phenyl)-2-(cyclohexylamino)-1-phenyl-ethanol

The compound was prepared using the same procedure as detailed in example 1 step c starting from 2-(3-bromo-4-chloro-2-fluoro-phenyl)-2-phenyl-oxirane (Ex 10, step b) (250 mg, 0.76 mmol, 1 eq) and cyclohexylamine (1.75 mL, 15.26 mmol, 20 eq.) giving 1-(3-bromo-4-chloro-2-fluoro-phenyl)-2-(cyclohexylamino)-1-phenyl-ethanol (231 mg, 71%) as an orange oil. ¹H NMR (400 MHz, DMSO-*d*₆) δ ppm 7.79 (d, *J*=8.80 Hz, 1 H), 7.49 (d, *J*=8.58 Hz, 1 H), 7.35 (m, 2 H), 7.29 (m, 2 H), 7.22 (tt, *J*=7.04, 1.32 Hz, 1 H), 5.93 (br s, 1 H), 3.44 (d, *J*=11.88 Hz, 1 H), 3.21 (d, *J*=11.88Hz, 1 H), 2.30 (m, 1 H), 1.76 (m, 2 H), 1.61 (m, 2 H), 1.50 (m, 2 H), 1.12 (m, 2 H), 0.91 (m, 2 H).

### Step b: tert-butyl N-[2-(3-bromo-4-chloro-2-fluoro-phenyl)-2-hydroxy-2-phenyl-ethyl]-N-cyclohexyl-carbamate

The compound was prepared using the same procedure as detailed in example 17 step d starting from 1-(3-bromo-4-chloro-2-fluoro-phenyl)-2-(cyclohexylamino)-1-phenyl-ethanol (230 mg, 0.539 mmol, 1 eq.) giving tert-butyl N-[2-(3-bromo-4-chloro-2-fluoro-phenyl)-2-hydroxy-2-phenyl-ethyl]-N-cyclohexyl-carbamate (157 mg, 55.3%) as a colorless oil. ¹H NMR (500 MHz, DMSO-*d*₆) δ ppm 7.79 (t, *J*=8.32 Hz, 1 H), 7.46 (dd, *J*=8.53, 1.10 Hz, 1 H), 7.33 (m, 2 H), 7.30 (m, 2 H), 7.25 (tt, *J*=7.15, 1.51 Hz, 1 H), 6.02 (br s, 1 H), 4.50 (d, *J*=14.31 Hz, 1 H), 3.86 (d, *J*=14.31 Hz, 1 H), 3.08 (m, 1 H), 1.80 (m, 3 H), 1.67 (m, 2 H), 1.52 (br d, *J*=12.38 Hz, 1 H), 1.44 (m, 1 H), 1.19 (s, 9 H), 1.08 (m, 2 H), 0.97 (m, 1 H).

### Step c: 2-[6-chloro-3-[2-(cyclohexylamino)-1-hydroxy-1-phenyl-ethyl]-2-fluoro-phenyl]-3-fluoro-4-(2-methoxyethoxy)benzonitrile

The compound was prepared using the same procedure as detailed in example 19a step d starting from tert-butyl N-[2-(3-bromo-4-chloro-2-fluoro-phenyl)-2-hydroxy-2-phenyl-ethyl]-N-cyclohexyl-carbamate (330 mg, 0.62 mmol, 1 eq.) and 3-fluoro-4-(2-methoxyethoxy)-2-trimethylstannyl-benzonitrile (Ex 19a, step c) (448 mg., 1.25 mmol, 20 eq) giving 2-[6-chloro-3-[2-(cyclohexylamino)-1-hydroxy-1-phenyl-ethyl]-2-fluoro-phenyl]-3-fluoro-4-(2-methoxyethoxy)benzonitrile (50 mg, 14.8 %) as a colorless oil. ¹H NMR (400 MHz, DMSO-*d*₆) δ ppm 7.96 and 7.93 (t, *J*=8.54 Hz, 1 H), 7.83 and 7.81 (dd, *J*=8.69, 1.38 Hz, 1 H), 7.58 and 7.56 (dd, *J*=2.09, 0.88 Hz, 1 H), 7.47 (t, *J*=8.36 Hz, 1 H), 7.36 (s, 1 H), 7.35 (s, 1 H), 7.27 (m, 2 H), 7.21 (m, 1 H), 5.97 and 5.92 (s, 1 H), 4.33 (m, 2 H), 3.70 (m, 2 H), 3.40 and 3.36 (d, *J*=11.44 Hz, 1 H), 3.30 and 3.29 (s, 2 H), 3.25 and 3.23 (d, *J*=11.77 Hz, 1 H), 2.30 (m, 1 H), 1.73 (m, 2 H), 1.59 (m, 2 H), 1.49 (m, 2 H), 1.13 (m, 2 H), 0.98 (m, 2 H).

### Step d: 2-[6-chloro-3-[2-(cyclohexylamino)-1-hydroxy-1-phenyl-ethyl]-2-fluoro-phenyl]-3-fluoro-4-(2-methoxyethoxy)benzamide

The compound was prepared using the same procedure as detailed in example 2 step c starting from 2-[6-chloro-3-[2-(cyclohexylamino)-1-hydroxy-1-phenyl-ethyl]-2-fluoro-phenyl]-3-fluoro-4-(2-methoxyethoxy)benzonitrile (50.0 mg, 0.0924 mmol, 1 eq.) giving 2-[6-chloro-3-[2-(cyclohexylamino)-1-hydroxy-1-phenyl-ethyl]-2-fluoro-phenyl]-3-fluoro-4-(2-methoxyethoxy)benzamide (15 mg, 20 %) as a white powder. ¹H NMR (500 MHz, DMSO-*d*₆) δ ppm 7.76 and 7.68 (t, *J*=8.46 Hz, 1 H), , 7.64 and 7.61 (br s, 1 H), 7.50 and 7.49 (d, *J*=4.54 Hz, 1 H), 7.35 (m, 3 H), 7.27 (m, 3 H), 7.19 (m, 1 H), 7.07 and 7.03 (br s, 1 H), 4.25 (m, 2 H), 3.69 (m, 2 H), 3.35 and 3.28 (d, 1 H), 3.31 and 3.30 (s, 3 H), 3.21 and 3.18 (br d, *J*=12.79 Hz, 1 H), 2.33 and 2.25 (m, 1 H), 1.79 (m, 1 H), 1.70 (m, 1 H), 1.59 (m, 2 H), 1.51 (m, 1 H), 1.13 (m, 3 H), 0.96 (m, 2 H).

### Examples 22a and 22b: rel-(2aS)-2-[6-chloro-2-fluoro-3-[(1S)-1-hydroxy-2-(methylamino)-1-phenyl-ethyl]phenyl]-3-fluoro-4-(2-methoxyethoxy)benzamide and rel-(2aR)-2-[6-chloro-2-fluoro-3-[(1S)-1-hydroxy-2-(methylamino)-1-phenyl-ethyl]phenyl]-3-fluoro-4-(2-methoxyethoxy)benzamide

### Step a: 1-(3-bromo-4-chloro-2-fluoro-phenyl)-2-(methylamino)-1-phenyl-ethanol

The compound was prepared using the same procedure as detailed in example 12 step c starting from 2-(3-bromo-4-chloro-2-fluoro-phenyl)-2-phenyl-oxirane (Ex 10, step b) (2.4 g, 7.3 mmol, 1 eq.) and MeNH₂/EtOH (33%) (18 mL, 145 mmol, 20 eq.) giving 1-(3-bromo-4-chloro-2-fluoro-phenyl)-2-(methylamino)-1-phenyl-ethanol (1 g, 38%) as a beige powder. ¹H NMR (400 MHz, DMSO-*d*₆) δ ppm 7.79 (t, *J*=8.47 Hz, 1 H), 7.50 (dd, *J*=8.58, 1.54 Hz, 1 H), 7.34 (m, 2 H), 7.30 (m, 2 H), 7.23 (m, 1 H), 5.95 (m, 1 H), 3.36 (d, *J*=11.88Hz, 1 H), 3.17 (m, 1 H), 2.28 (s, 3 H).

### Step b: [(1S,2R,5S)-2-isopropyl-5-methyl-cyclohexyl] N-[(2S)-2-(3-bromo-4-chloro-2-fluorophenyl)-2-hydroxy-2-phenyl-ethyl]-N-methyl-carbamate and [(1S,2R,5S)-2-isopropyl-5-methyl-cyclohexyl] N-[(2R)-2-(3-bromo-4-chloro-2-fluoro-phenyl)-2-hydroxy-2-phenyl-ethyl]-N-methyl-carbamate

The compound was prepared using the same procedure as detailed in example 19a step a starting from 1-(3-bromo-4-chloro-2-fluoro-phenyl)-2-(methylamino)-1-phenyl-ethanol (2 g, 5.57 mmol, 1 eq.) and (+)-menthyl chloroformate (1470 mg, 6.72 mmol, 1.2 eq). The diastereoisomers were separated by chromatography (100g HP SiO₂ cartridge; eluting from 30 to 100% cyclohexane / DCM) to give [(1S,2R,5S)-2-isopropyl-5-methyl-cyclohexyl] N-[(2S)-2-(3-bromo-4-chloro-2-fluoro-phenyl)-2-hydroxy-2-phenyl-ethyl]-N-methyl-carbamate (1.08 g, 35.8% Yield) as a white solid. ¹H NMR (500 MHz, DMSO-*d*₆) δ ppm 7.77 (t, *J*=8.32 Hz, 1 H), 7.51 and 7.45 (d, *J*=8.67 Hz, 1 H), 7.32 (m, 4 H), 7.28 (m, 1 H), 6.17 and 6.14 (s, 1 H), 4.76 and 4.43 (d, *J*=13.89 Hz, 1 H), 4.24 and 4.06 (td, *J*=1 0.66, 3.85 Hz, 1 H), 3.95 and 3.68 (d, *J*=13.89 Hz, 1 H), 2.89 (m, 3 H), 1.78 and 1.56 (m, 1 H), 1.55 (m, 2 H), 1.25 (m, 3 H), 0.93 (m, 1 H), 0.85 (d, *J*=6.33 Hz, 3 H), 0.80 (m, 3 H), 0.74 (m, 2 H), 0.63 (m, 3 H), 0.49 (m, 1 H) (2 rotamers of carbamate). And [(1S,2R,5S)-2-isopropyl-5-methyl-cyclohexyl] N-[(2R)-2-(3-bromo-4-chloro-2-fluoro-phenyl)-2-hydroxy-2-phenyl-ethyl]-N-methyl-carbamate (1.1 g, 36%) as a white solid. The absolute configuration was determined by XR. ¹H NMR (500 MHz, DMSO-*d*₆) δ ppm 7.79 (m, 1 H), 7.51 and 7.43 (d, *J*=8.67 Hz, 1 H), 7.31 (m, 5 H), 6.15 and 6.14 (s, 1 H), 4.84 and 4.71 (d, *J*=14.31 Hz, 1 H), 4.16 and 4.04 (td, *J*=10.80, 4.13 Hz, 1 H), 3.71 and 3.65 (d, *J*=14.03 Hz, 1 H), 3.00 and 2.92 (s, 3 H), 1.55 (m, 3 H), 1.33 (m, 2 H), 1.21 (m, 2 H), 0.85 (m, 4 H), 0.77 and 0.72 (d, *J*=6.88 Hz, 3 H), 0.74 (m, 1 H), 0.67 and 0.48 (d, *J*=6.74 Hz, 3 H) (2 rotamers of carbamate).

### Step c: (1S)-1-(3-bromo-4-chloro-2-fluoro-phenyl)-2-(methylamino)-1-phenyl-ethanol

The compound was prepared using the same procedure as detailed in example 19a step b starting from [(1S,2R,5S)-2-isopropyl-5-methyl-cyclohexyl] N-[(2S)-2-(3-bromo-4-chloro-2-fluoro-phenyl)-2-hydroxy-2-phenyl-ethyl]-N-methyl-carbamate (1.05 g, 1.94 mmol, 1 eq.) giving (1S)-1-(3-bromo-4-chloro-2-fluoro-phenyl)-2-(methylamino)-1-phenyl-ethanol (525 mg, 75.4 %) as a white solid. ¹H NMR (400 MHz, DMSO-*d*₆) δ ppm 7.79 (t, *J*=8.42 Hz, 1 H), 7.50 (dd, *J*=8.69, 1.54 Hz, 1 H), 7.34 (m, 2 H), 7.30 (m, 2 H), 7.22 (tt, *J*=6.93, 1.54 Hz, 1 H), 6.05 (br s, 1 H), 3.36 (dd, *J*=11.94, 1.71 Hz, 1 H), 3.17 (d, *J*=11.88Hz, 1 H), 2.28 (s, 3 H). [α]_{d}²² = -34.6° (C=0.64, DCM).

### Step d: tert-butyl N-[(2S)-2-(3-bromo-4-chloro-2-fluoro-phenyl)-2-hydroxy-2-phenyl-ethyl]-N-methyl-carbamate

The compound was prepared using the same procedure as detailed in example 17 step d starting from (1S)-1-(3-bromo-4-chloro-2-fluoro-phenyl)-2-(methylamino)-1-phenyl-ethanol (780 mg, 2.17 mmol, 1 eq.) giving tert-butyl N-[(2S)-2-(3-bromo-4-chloro-2-fluoro-phenyl)-2-hydroxy-2-phenyl-ethyl]-N-methyl-carbamate (898 mg, 90%) as a white foam. ¹H NMR (400 MHz, DMSO-*d*₆) δ ppm 7.79 (t, *J*=8.36 Hz, 1 H), 7.54 and 7.46 (m, 1 H), 7.31 (m, 5 H), 6.12 (s, 1 H), 4.69 and 4.58 (br d, *J*=12.87 Hz, 1 H), 3.74 and 3.60 (br d, *J*=12.87 Hz, 1 H), 2.92 and 2.86 (s, 1 H). (2 rotamers of carbamate).

### Step e: tert-butyl N-[(2S)-2-[4-chloro-3-[6-cyano-2-fluoro-3-(2-methoxyethoxy)phenyl]-2-fluoro-phenyl]-2-hydroxy-2-phenyl-ethyl]-N-methyl-carbamate

The compound was prepared using the same procedure as detailed in example 19a step d starting from tert-butyl N-[(2S)-2-(3-bromo-4-chloro-2-fluoro-phenyl)-2-hydroxy-2-phenylethyl]-N-methyl-carbamate (866 mg, 1.89 mmol, 1 eq.) and 3-fluoro-4-(2-methoxyethoxy)-2-trimethylstannyl-benzonitrile (Ex 19a, step c) (1.35 g, 3.78 mmol, 2 eq.) giving tert-butyl N-[(2S)-2-[4-chloro-3-[6-cyano-2-fluoro-3-(2-methoxyethoxy)phenyl]-2-fluoro-phenyl]-2-hydroxy-2-phenyl-ethyl]-N-methyl-carbamate (820 mg, 75.8%) as a yellow gum, used in next step without further purification.

### Step f: tert-butyl N-[(2S)-2-[3-[6-carbamoyl-2-fluoro-3-(2-methoxyethoxy)phenyl]-4-chloro-2-fluoro-phenyl]-2-hydroxy-2-phenyl-ethyl]-N-methyl-carbamate

The compound was prepared using the same procedure as detailed in example 2 step c starting from tert-butyl N-[(2S)-2-[4-chloro-3-[6-cyano-2-fluoro-3-(2-methoxyethoxy)phenyl]-2-fluoro-phenyl]-2-hydroxy-2-phenyl-ethyl]-N-methyl-carbamate (820 mg, 1.43 mmol, 1 eq.) giving tert-butyl N-[(2S)-2-[3-[6-carbamoyl-2-fluoro-3-(2-methoxyethoxy)phenyl]-4-chloro-2-fluoro-phenyl]-2-hydroxy-2-phenyl-ethyl]-N-methyl-carbamate (540 mg, 63.8%) as a white foam (mixture of rotamers and atropoisomers).

### Step g: rel-(2aS)-2-[6-chloro-2-fluoro-3-[(1S)-1-hydroxy-2-(methylamino)-1-phenylethyl]phenyl]-3-fluoro-4-(2-methoxyethoxy)benzamide and rel-(2aR)-2-[6-chloro-2-fluoro-3-[(1S)-1-hydroxy-2-(methylamino)-1-phenyl-ethyl]phenyl]-3-fluoro-4-(2-methoxyethoxy)benzamide

The compound was prepared using the same procedure as detailed in example 17 step g starting from tert-butyl N-[(2S)-2-[3-[6-carbamoyl-2-fluoro-3-(2-methoxyethoxy)phenyl]-4-chloro-2-fluoro-phenyl]-2-hydroxy-2-phenyl-ethyl]-N-methyl-carbamate (540 mg, 0.91 mmol, 1 eq.) giving a crude mixture of atropoisomers (400 mg). The two atropoisomers were separated by chromatography LC/UV (Column: Xbridge C18, 30 x 100mm 5µm, Flowrate: 35 mL/min, Mobile phase: H2O + 0.1% NH₄OH / MeOH + 0.1% NH₄OH, Temperature: 45°C, Gradient: 20-95 % in 30 min). The samples were freeze-dried to give 22a: rel-(2aS)-2-[6-chloro-2-fluoro-3-[(1S)-1-hydroxy-2-(methylamino)-1-phenyl-ethyl]phenyl]-3-fluoro-4-(2-methoxyethoxy)benzamide (140 mg, 35%) as a white solid. ¹H NMR (500 MHz, DMSO-*d*₆) δ ppm 7.68 (d, *J*=8.53 Hz, 1 H), 7.66 (br s, 1 H), 7.50 (br d, *J*=8.53 Hz, 1 H), 7.36 (m, 3 H), 7.27 (m, 3 H), 7.19 (m, 1 H), 7.07 (br s, 1 H), 5.84 (br s, 1 H), 4.25 (m, 2 H), 3.70 (br t, *J*=3.99 Hz, 2 H), 3.31 (s, 3 H), 3.19 (d, *J*=12.38 Hz, 1 H), 3.16 (d, *J*=11.83 Hz, 1 H), 2.23 (s, 3 H).

[α]_{d}²²⁷ = -73.5° (C=0.5, MeOH). And **22b:** rel-(2aR)-2-[6-chloro-2-fluoro-3-[(1S)-1-hydroxy-2-(methylamino)-1-phenyl-ethyl]phenyl]-3-fluoro-4-(2-methoxyethoxy)benzamide (78 mg, 19.5%) as a white solid. ¹H NMR (500 MHz, DMSO-*d*₆) δ ppm 7.77 (t, *J*=8.32 Hz, 1 H), 7.62 (br s, 1 H), 7.49 (d, *J*=8.53 Hz, 1 H), 7.35 (m, 3 H), 7.27 (m, 3 H), 7.19 (t, *J*=7.15 Hz, 1 H), 7.09 (br s, 1 H), 5.78 (s, 1 H), 4.24 (m, 2 H), 3.68 (m, 2 H), 3.30 (s, 3 H), 3.29 (d, *J*=12.10 Hz, 1 H), 3.18 (d, *J*=12.10 Hz, 1 H), 2.27 (s, 3 H). [α]_{d}^{22.7} = -19.3° (C=0.51, MeOH).

### Examples 23a and 23b: (2aR)-2-[6-chloro-2-fluoro-3-[(1R)-1-hydroxy-2-(methylamino)-1-phenyl-ethyl]phenyl]-3-fluoro-4-(2-methoxyethoxy)benzamide and (2aS)-2-[6-chloro-2-fluoro-3-[(1 R)-1-hydroxy-2-(methylamino)-1-phenyl-ethyl]phenyl]-3-fluoro-4-(2-methoxyethoxy)benzamide

### Step a: (1R)-1-(3-bromo-4-chloro-2-fluoro-phenyl)-2-(methylamino)-1-phenyl-ethanol

The compound was prepared using the same procedure as detailed in example 19a step b starting from [(1S,2R,5S)-2-isopropyl-5-methyl-cyclohexyl] N-[(2R)-2-(3-bromo-4-chloro-2-fluoro-phenyl)-2-hydroxy-2-phenyl-ethyl]-N-methyl-carbamate (1.1 g, 2.0 mmol, 1.0 eq.) giving (1R)-1-(3-bromo-4-chloro-2-fluoro-phenyl)-2-(methylamino)-1-phenyl-ethanol (475 mg, 65%) as a white solid. ¹H NMR (400 MHz, DMSO-*d*₆) δ ppm 7.79 (t, *J*=8.42 Hz, 1 H), 7.50 (dd, *J*=8.69, 1.54 Hz, 1 H), 7.34 (m, 2 H), 7.30 (m, 2 H), 7.23 (m, 1 H), 3.37 (dd, *J*=11.88, 1.65 Hz, 1 H), 3.17 (m, 1 H), 2.28 (s, 3 H). [α]_{d}²² = +34.5° (C=0.9, DCM).

### Step b: tert-butyl N-[(2R)-2-(3-bromo-4-chloro-2-fluoro-phenyl)-2-hydroxy-2-phenyl-ethyl]-N-methyl-carbamate

The compound was prepared using the same procedure detailed in example 18 step b starting from (1R)-1-(3-bromo-4-chloro-2-fluoro-phenyl)-2-(methylamino)-1-phenyl-ethanol (685 mg, 1.91 mmol, 1 eq.) giving tert-butyl N-[(2R)-2-(3-bromo-4-chloro-2-fluoro-phenyl)-2-hydroxy-2-phenyl-ethyl]-N-methyl-carbamate (870 mg, 99%) as a white powder. ¹H NMR (400 MHz, DMSO-*d*₆) δ ppm 7.79 (t, *J*=8.31 Hz, 1 H), 7.53 and 7.47 (br d, *J*=8.14 Hz, 1 H), 7.31 (m, 5 H), 4.70 and 4.57 (br d, *J*=14.41 Hz, 1 H), 3.76 and 3.60 (br d, *J*=14.09 Hz, 1 H), 2.93 and 2.85 (s, 3 H) (2 rotamers of carbamate).

### Step c: tert-butyl N-[(2R)-2-[4-chloro-3-[6-cyano-2-fluoro-3-(2-methoxyethoxy)phenyl]-2-fluoro-phenyl]-2-hydroxy-2-phenyl-ethyl]-N-methyl-carbamate

The compound was prepared using the same procedure as detailed in example 19a step d starting from tert-butyl N-[(2R)-2-(3-bromo-4-chloro-2-fluoro-phenyl)-2-hydroxy-2-phenylethyl]-N-methyl-carbamate (820 mg, 1.78 mmol, 1 eq.) and 3-fluoro-4-(2-methoxyethoxy)-2-trimethylstannyl-benzonitrile (Ex 19a, step c) (1.28 g, 3.58 mmol, 2 eq.) giving tert-butyl N-[(2R)-2-[4-chloro-3-[6-cyano-2-fluoro-3-(2-methoxyethoxy)phenyl]-2-fluoro-phenyl]-2-hydroxy-2-phenyl-ethyl]-N-methyl-carbamate (175 mg, 17%) as a yellow gum, used in next step without further purification.

### Step d: tert-butyl N-[(2R)-2-[3-[6-carbamoyl-2-fluoro-3-(2-methoxyethoxy)phenyl]-4-chloro-2-fluoro-phenyl]-2-hydroxy-2-phenyl-ethyl]-N-methyl-carbamate

The compound was prepared using the same procedure as detailed in example 2 step c starting from tert-butyl N-[(2R)-2-[4-chloro-3-[6-cyano-2-fluoro-3-(2-methoxyethoxy)phenyl]-2-fluoro-phenyl]-2-hydroxy-2-phenyl-ethyl]-N-methyl-carbamate (170 mg, 0.29 mmol, 1 eq.) giving tert-butyl N-[(2R)-2-[3-[6-carbamoyl-2-fluoro-3-(2-methoxyethoxy)phenyl]-4-chloro-2-fluoro-phenyl]-2-hydroxy-2-phenyl-ethyl]-N-methyl-carbamate (175 mg, 99.8%) as an off-white foam (mixture of rotamers and atropoisomers).

### Step e: (2aR)-2-[6-chloro-2-fluoro-3-[(1R)-1-hydroxy-2-(methylamino)-1-phenyl-ethyl]phenyl]-3-fluoro-4-(2-methoxyethoxy)benzamide and rel-(2aS)-2-[6-chloro-2-fluoro-3-[(1R)-1-hydroxy-2-(methylamino)-1-phenyl-ethyl]phenyl]-3-fluoro-4-(2-methoxyethoxy)benzamide

The compound was prepared using the same procedure detailed in example 17 step g starting from tert-butyl N-[(2R)-2-[3-[6-carbamoyl-2-fluoro-3-(2-methoxyethoxy)phenyl]-4-chloro-2-fluoro-phenyl]-2-hydroxy-2-phenyl-ethyl]-N-methyl-carbamate (175 mg, 0.29 mmol, 1 eq.) giving a crude mixture of atropoisomers. The two atropoisomers were separated by chromatography LC/UV (Column: Xbridge C18, 30 x 100mm 5µm, Flowrate: 35 mL/min, Mobile phase: H₂O + 0.1% NH₄OH / MeOH + 0.1% NH₄OH, Temperature: 45°C, Gradient: 20-95 % in 30 min). The samples were freeze-dried to give **23a:** (2aR)-2-[6-chloro-2-fluoro-3-[(1R)-1-hydroxy-2-(methylamino)-1-phenyl-ethyl]phenyl]-3-fluoro-4-(2-methoxyethoxy)benzamide (60 mg, 41%) as an off-white solid. ¹H NMR (500 MHz, DMSO-*d*₆) δ ppm 7.68 (d, *J*=8.25 Hz, 1 H), 7.65 (m, 1 H), 7.50 (d, *J*=8.39 Hz, 1 H), 7.36 (t, *J*=8.87 Hz, 3 H), 7.27 (m, 3 H), 7.19 (m, 1 H), 7.07 (br s, 1 H), 5.84 (br s, 1 H), 4.25 (m, 2 H), 3.70 (t, *J*=4.47 Hz, 2 H), 3.31 (s, 3 H), 3.19 (d, *J*=12.93 Hz, 1 H), 3.16 (d, *J*=12.38 Hz, 1 H), 2.23 (s, 3 H). [α]_{d}^{22.3} = +71.7° (C=0.42, MeOH). And **23b:** (2aS)-2-[6-chloro-2-fluoro-3-[(1R)-1-hydroxy-2-(methylamino)-1-phenyl-ethyl]phenyl]-3-fluoro-4-(2-methoxyethoxy)benzamide (78 mg, 19.5%) as an off-white solid. ¹H NMR (500 MHz, DMSO-*d*₆) δ ppm 7.77 (t, *J*=8.53 Hz, 1 H), 7.62 (br s, 1 H), 7.49 (d, *J*=8.53 Hz, 1 H), 7.35 (m, 3 H), 7.28 (m, 3 H), 7.19 ((t, *J*=7.15 Hz, 1 H), 7.09 (br s, 1 H), 5.77 (br s, 1 H), 4.24 (m, 2 H), 3.68 (t, *J*=4.47 Hz, 2 H), 3.30 (s, 4 H), 3.29 (d, *J*=12.10 Hz, 1 H), 3.17 (d, *J*=12.10 Hz, 1 H), 2.27 (s, 3 H). [α]_{d}^{22.3} = +18.5° (C=0.35, MeOH). The stereochemistry was defined by XR for compound **23a.**

### Example 24: 2-[2-chloro-5-[(R)-hydroxy-phenyl-[(2S)-pyrrolidin-2-yl]methyl]phenyl]-3-methyl-benzamide

### Step a: tert-butyl (2S)-2-[(3-bromo-4-chloro-phenyl)-hydroxy-methyl]pyrrolidine-1-carboxylate

To a 5 mL round bottom flask, flame dried, under argon, 1-bromo-2-chloro-5-iodobenzene (1.59 g, 5.01 mmol, 2 eq.) was dissolved in THF (3.50 mL) (dry, Acroseal) and the mixture was cooled to 0°C. Isopropylmagnesium chloride lithium chloride complex (3.90 mL, 5.07 mmol, 2 eq.) was added dropwise and the mixture was stirred at 0°C for 1h. The mixture was cooled to -70°C in a cold bath, taken with a syringe and added dropwise to a solution of tert-butyl (2S)-2-formylpyrrolidine-1-carboxylate (500 mg, 2.51 mmol, 1 eq) in THF (7.00 mL) at - 70°C. The mixture was left to return to rt and stirred at rt overnight. The reaction mixture was quenched with 30 mL of an aqueous saturated solution of NH₄Cl and extracted three times with 30 mL of EtOAc. The combined organic layers were dried over MgSO₄, filtered and concentrated under reduced pressure to give 1.55 g of a yellow oil. The crude was purified by chromatography (Cartridge Biotage Sfär 50 g 20 µm silica, cyclohexane-EtOAc 100-0 / 2 CV,100-0 to 80-20 / 10 CV, 80-20 / 15 CV, flow rate 50 to 80 ml / min) to give tert-butyl (2S)-2-[(3-bromo-4-chloro-phenyl)-hydroxy-methyl]pyrrolidine-1-carboxylate (578 mg, 59.0%) as a colorless gum, used directly in the next step.

### Step b: tert-butyl (2S)-2-(3-bromo-4-chloro-benzoyl)pyrrolidine-1-carboxylate

To a 50 mL round bottom flask, tert-butyl (2S)-2-[(3-bromo-4-chloro-phenyl)-hydroxymethyl]pyrrolidine-1-carboxylate (573 mg, 1.47 mmol, 1 eq.) was dissolved in DCM (6.00 mL, 93.6 mmol, 63.8) and the solution was cooled to 0°C. Dess-Martin periodinane (3.95 mL, 1.90 mmol, 1.3 eq.) was added dropwise and the mixture was stirred at 0°C for 5 min. The mixture was left to return to rt and stirred at rt overnight. The reaction mixture was diluted with 50 mL of DCM and washed with 50 mL of an aqueous saturated solution of NaHCO₃. The aqueous layer was again extracted twice with 50 mL of DCM. The combined organic layers were dried over MgSO₄, filtered and concentrated under reduced pressure to give 683 mg of a white gum. The crude was purified by chromatography (Cartridge Biotage Sfär 25 g 60 µm silica, cyclohexane-EtOAc 100-0 / 2 CV, 100-0 to 80-20 / 10 CV, 80-20 / 15 CV, flow rate 82 ml / min) to give tert-butyl (2S)-2-(3-bromo-4-chloro-benzoyl)pyrrolidine-1-carboxylate (454 mg, 79.6%) as a white powder. ¹H NMR (400 MHz, DMSO-*d*₆) δ ppm 8.32 (m, 1 H), 8.03 (m, 1 H), 7.82 (dd, *J*=8.36, 2.75 Hz, 1 H), 5.31 (dt, *J*=8.86, 4.59 Hz, 1 H), 3.41 (m, 2 H), 2.33 (m, 1 H), 1.85 (m, 1 H), 1.78 (m, 2 H), 1.38 and 1.17 (s, 9 H).

### Step c: tert-butyl (2S)-2-[(R)-(3-bromo-4-chloro-phenyl)-hydroxy-phenyl-methyl]pyrrolidine-1-carboxylate

To a 10 mL round bottom flask, flame dried, under argon, tert-butyl (2S)-2-(3-bromo-4-chlorobenzoyl)pyrrolidine-1-carboxylate (454 mg, 1.17 mmol, 1 eq.) was dissolved in THF (5.50 mL) (dry, Acroseal) and the mixture was cooled to 0°C. Phenylmagnesium bromide (2.35 mL, 2.35 mmol, 2.01 eq.) was added dropwise, then the mixture was left to return to rt and stirred at rt overnight. The reaction mixture was quenched with an aqueous saturated solution of NH₄Cl (50 mL) at rt and extracted with EtOAc (3 x 50 mL). The combined organic layers were dried over anhydrous MgSO₄. After filtration, the filtrate was concentrated under reduced pressure to give 729 mg of a colorless oil. The crude was purified by chromatography (Cartridge Biotage Sfär 25 g 20 µm silica, cyclohexane-EtOAc 100-0 / 2 CV, 100-0 to 90-10 / 10 CV, 90-10 / 20 CV, flow rate 50 ml / min) to give tert-butyl tert-butyl (2S)-2-[(R)-(3-bromo-4-chloro-phenyl)-hydroxy-phenyl-methyl]pyrrolidine-1-carboxylate (458 mg, 84.% Yield) as a white powder. The absolute configuration was determined by XR of the deprotected product. ¹H NMR (400 MHz, DMSO-*d*₆) δ ppm 7.82 (d, *J*=1.98 Hz, 1 H), 7.62 (d, *J*=8.47 Hz, 1 H), 7.53 (d, *J*=8.80 Hz, 1 H), 7.24 (m, 4 H), 7.18 (m, 1 H), 6.00 (br s, 1 H), 4.80 and 4.79 (m, 1 H), 3.51 (m, 1 H), 3.31 (m, 2 H), 1.91 (m, 1 H), 1.69 (m, 2 H), 1.11 (s, 9 H).

### Step d: tert-butyl (2S)-2-[(R)-[4-chloro-3-(2-cyano-6-methyl-phenyl)phenyl]-hydroxy-phenylmethyl]pyrrolidine-1-carboxylate

The compound was prepared using the same procedure as detailed in example 2 step b starting from (2S)-2-[(R)-(3-bromo-4-chloro-phenyl)-hydroxy-phenyl-methyl]pyrrolidine-1-carboxylate (250 mg, 0.536 mmol, 1 eq.) and 3-methyl-2-(4,4,5,5-tetramethyl-1,3,2-dioxaborolan-2-yl)benzonitrile (Ex 2, step a) (260 mg, 1.07 mmol, 2 eq.) with APHOS PD G3 (17337, 68.0 mg, 0.107 mmol, 0.200 eq.) as catalyst giving tert-butyl (2S)-2-[(R)-[4-chloro-3-(2-cyano-6-methyl-phenyl)phenyl]-hydroxy-phenyl-methyl]pyrrolidine-1-carboxylate (299 mg, 88.8%) as a yellow gum used directly in the next step.

### Step e: tert-butyl (2S)-2-[(R)-[3-(2-carbamoyl-6-methyl-phenyl)-4-chloro-phenyl]-hydroxyphenyl-methyl]pyrrolidine-1-carboxylate

The compound was prepared using the same procedure as detailed in example 2 step c starting from tert-butyl (2S)-2-[(R)-[4-chloro-3-(2-cyano-6-methyl-phenyl)phenyl]-hydroxyphenyl-methyl]pyrrolidine-1-carboxylate (200 mg, 0.318 mmol, 1 eq.) giving tert-butyl (2S)-2-[(R)-[3-(2-carbamoyl-6-methyl-phenyl)-4-chloro-phenyl]-hydroxy-phenyl-methyl]pyrrolidine-1-carboxylate (135 mg, 81.5%) as a white powder used directly in the next step.

### Step f: 2-[2-chloro-5-[(R)-hydroxy-phenyl-[(2S)-pyrrolidin-2-yl]methyl]phenyl]-3-methyl-benzamide

The compound was prepared using the same procedure as detailed in example 17 step g starting from tert-butyl (2S)-2-[(R)-[3-(2-carbamoyl-6-methyl-phenyl)-4-chloro-phenyl]-hydroxy-phenyl-methyl]pyrrolidine-1-carboxylate (130 mg, 0.249 mmol, 1 eq.) giving 2-[2-chloro-5-[(R)-hydroxy-phenyl-[(2S)-pyrrolidin-2-yl]methyl]phenyl]-3-methyl-benzamide (60.0 mg, 57.1%) as a white powder. ¹H NMR (500 MHz, DMSO-*d*₆) δ ppm 7.55 and 7.54 (d, *J*=4.13 Hz, 2 H), 7.41 and 7.36 (m, 2 H), 7.33 (m, 4 H), 7.26 (m, 3 H), 7.15 (m, 1 H), 7.07 and 7.02 (br s, 1 H), 5.17 (br s, 1 H), 4.17 and 4.12 (t, *J*=7.43 Hz, 1 H), 2.82 (m, 1 H), 2.80 and 2.77 (m, 1 H), 1.92 and 1.80 (s, 3 H), 1.57 (m, 2 H), 1.44 (m, 2 H). [α]_{d}^{21.7} = -37.3° (C=0.31, DCM).

### Example 25: 2-[rel-(5R)-5-[[(2S)-azetidin-2-yl]-hydroxy-phenyl-methyl]-2-chloro-phenyl]-3-fluoro-4-(2-methoxyethoxy)benzamide

### Step a: tert-butyl (2S)-2-(3-bromo-4-chloro-benzoyl)azetidine-1-carboxylate

To a 50 mL three-necked round bottom flask, flame dried, under argon, 1-bromo-2-chloro-5-iodobenzene (5.2 g, 16 mmol, 2 eq.) was dissolved in THF (10 mL) (dry, Acroseal) and the mixture was cooled to 0°C. Isopropylmagnesium chloride lithium chloride complex (12.6 mL, 16.4 mmol, 2 eq.) was added dropwise and the mixture was stirred at 0°C for 45min. This mixture was cooled to -70°C and added dropwise to a solution of (S)-tert-butyl 2-(methoxy(methyl)carbamoyl)azetidine-1-carboxylate (2.00 g, 8.19 mmol, 1 eq) in THF (20 mL) at -70°C (15 min of addition). The resulting yellow mixture was left to return to rt and stirred at rt for 45min. The mixture was diluted with a saturated solution of NH₄Cl and extracted with EtOAc (x3). The combined organic layers were washed with brine and dried over anhydrous MgSO₄. After filtration, the filtrate was concentrated under reduced pressure to give a crude which was purified by chromatography (Cartridge Biotage Sfär 100 g 20 µm silica, Cyclohexane/EtOAc 100-0 / 3 CV, 100-0 to 80-20 / 15 CV, 80-20 / 6 CV, flow rate 18 ml / min) giving tert-butyl (2S)-2-(3-bromo-4-chloro-benzoyl)azetidine-1-carboxylate (1.9 g, 57%) as a yellow gum. ¹H NMR (400 MHz, DMSO-*d*₆) δ ppm 8.25 (d, *J*=1.98 Hz, 1 H), 7.94 (dd, *J*=8.36, 2.09 Hz, 1 H), 7.83 (d, *J*=8.36 Hz, 1 H), 5.67 (dt, *J*=6.66, 1.68 Hz, 1 H), 3.82 (m, 2 H), 2.65 (m, 1 H), 2.03 (m, 1 H), 1.33 (br s, 9 H).

### Step b: tert-butyl (2S)-2-[rel-(R)-(3-bromo-4-chloro-phenyl)-hydroxy-phenyl-methyl]azetidine-1-carboxylate

To a 5 mL round bottom flask, flame dried, under argon, tert-butyl (2S)-2-(3-bromo-4-chlorobenzoyl)azetidine-1-carboxylate (1.9 g, 4.7 mmol, 1 eq.) was dissolved in THF (30 mL) (dry, Acroseal) and the mixture was cooled to 5°C. Phenylmagnesium bromide (23 mL, 23.0 mmol, 4.9 eq.) was added dropwise (the mixture was kept at 5°C, 5 minutes of addition), then the mixture was left to return to rt and stirred at rt for 2h. The reaction mixture was slowly quenched with water at rt and extracted with EtOAc. The combined organic layers were dried over anhydrous MgSO₄. After filtration, the filtrate was concentrated under reduced pressure to give a crude which was purified by chromatography (Cartridge Biotage Sfär 50 g 20 µm silica, Cyclohexane/EtOAc 100-0 / 4 CV,100-0 to 85-15 / 15 CV, 85-15 / 6 CV, flow rate 80 ml / min) giving tert-butyl (2S)-2-[rel-(R)-(3-bromo-4-chloro-phenyl)-hydroxyphenyl-methyl]azetidine-1-carboxylate (970 mg, 42%) as a white powder. ¹H NMR (400 MHz, DMSO-*d*₆) δ ppm 7.75 (d, *J*=2.09 Hz, 1 H), 7.54 (d, *J*=8.36 Hz, 1 H), 7.40 (m, 3 H), 7.27 (m, 2 H), 7.20 (m, 1 H), 6.24 (s, 1 H), 5.16 (dd, *J*=8.80, 5.06 Hz, 1 H), 3.66 (ddd, *J*=9.77, 8.23, 5.34 Hz, 1 H), 3.52 (m, 1 H), 2.41 (m, 1 H), 1.87 (m, 1 H), 1.14 (br s, 9 H).

### Step c: tert-butyl (2S)-2-[rel-(R)-[4-chloro-3-[6-cyano-2-fluoro-3-(2-methoxyethoxy)-phenyl]phenyl]-hydroxy-phenyl-methyl]azetidine-1-carboxylate

The compound was prepared using the same procedure as detailed in example 2 step b starting from tert-butyl (2S)-2-[rel-(R)-(3-bromo-4-chloro-phenyl)-hydroxy-phenylmethyl]azetidine-1-carboxylate (435 mg, 0.961 mmol, 1 eq.) and 3-fluoro-4-(2-methoxyethoxy)-2-(4,4,5,5-tetramethyl-1,3,2-dioxaborolan-2-yl)benzonitrile (1.8 g, 5.76 mmol, 6 eq.) giving tert-butyl (2S)-2-[rel-(R)-[4-chloro-3-[6-cyano-2-fluoro-3-(2-methoxyethoxy)phenyl]phenyl]-hydroxy-phenyl-methyl]azetidine-1-carboxylate (515 mg, 54.8%) as a yellow gum. ¹H NMR (400 MHz, DMSO-*d*₆) δ ppm 7.92 (m, 1 H), 7.81 (ddd, *J*=8.75, 7.59, 1.49 Hz, 1 H), 7.59 (m, 1 H), 7.52 (m, 1 H), 7.43 (m, 3 H), 7.26 (m, 2 H), 7.20 (m, 1 H), 6.27 (m, 1 H), 5.16 (m, 1 H), 4.33 (m, 2 H), 3.91 (s, 3 H), 3.71 (m, 2 H), 3.66 (m, 1 H), 3.34 (m, 1 H), 2.39 (m, 1 H), 1.93 (m, 1 H), 1.13 (m, 6 H).

### Step d: tert-butyl (2S)-2-[rel-(R)-[3-[6-carbamoyl-2-fluoro-3-(2-methoxyethoxy)phenyl]-4-chloro-phenyl]-hydroxy-phenyl-methyl]azetidine-1-carboxylate

The compound was prepared using the same procedure as detailed in example 2 step c starting from tert-butyl (2S)-2-[rel-(R)-[4-chloro-3-[6-cyano-2-fluoro-3-(2-methoxyethoxy)phenyl]phenyl]-hydroxy-phenyl-methyl]azetidine-1-carboxylate (515 mg, 0.527 mmol, 1.00 eq.) giving tert-butyl (2S)-2-[rel-(2R)-[3-[6-carbamoyl-2-fluoro-3-(2-methoxyethoxy)phenyl]-4-chloro-phenyl]-hydroxy-phenyl-methyl]azetidine-1-carboxylate (190 mg, 61.7%) as a white powder used directly in the next step

### Step e: 2-[rel-(5R)-5-[[(2S)-azetidin-2-yl]-hydroxy-phenyl-methyl]-2-chloro-phenyl]-3-fluoro-4-(2-methoxyethoxy)benzamide

The compound was prepared using the same procedure as detailed in example 17 step g starting from tert-butyl (2S)-2-[rel-(R)-[3-[6-carbamoyl-2-fluoro-3-(2-methoxyethoxy)phenyl]-4-chloro-phenyl]-hydroxy-phenyl-methyl]azetidine-1-carboxylate (190 mg, 0.325 mmol, 1.00 eq.) giving 2-[rel-(5R)-5-[[(2S)-azetidin-2-yl]-hydroxy-phenyl-methyl]-2-chloro-phenyl]-3-fluoro-4-(2-methoxyethoxy)benzamide (21 mg, 13.4%) as a white powder. ¹H NMR (500 MHz, DMSO-*d*₆) δ ppm 7.47 (m, 1 H), 7.41 (m, 3 H), 7.36 (m, 2 H), 7.27 (m, 3 H), 7.22 and 7.20 (s, 1 H), 7.18 (m, 1 H), 7.14 and 7.09 (br s, 1 H), 5.56 (br s, 1 H), 4.79 and 4.74 (br t, *J*=7.63 Hz, 1 H), 4.24 (m, 2 H), 3.69 (m, 2 H), 3.41 (m, 1 H), 3.31 and 3.30 (s, 3 H), 3.04 (m, 1 H), 2.12 (m, 1 H), 2.03 (m, 1 H), 1.87 (m, 1 H).

### Example 26: 2-[3-(2-amino-1-hydroxy-1-phenyl-ethyl)-2,6-dichloro-phenyl]-3-fluoro-4-(2-methoxyethoxy)benzamide

### Step a: (3-bromo-2,4-dichloro-phenyl)-phenyl-methanone

The compound was prepared using the same procedure as detailed in example 10 step a starting from 3-bromo-2,4-dichlorobenzoic acid (2 g, 7.4099 mmol, 1 eq.) giving (3-bromo-2,4-dichloro-phenyl)-phenyl-methanone (1.60 g, 66.6%) as a yellow oil. ¹H NMR (400 MHz, DMSO-*d*₆) δ ppm 7.80 (d, *J*=8.14 Hz, 1 H), 7.77 (m, 2 H), 7.73 (m, 1 H), 7.58 (d, *J*=8.36 Hz, 1 H), 7.57 (m, 2 H).

### Step b: 2-(3-bromo-2,4-dichloro-phenyl)-2-phenyl-oxirane

The compound was prepared using the same procedure as detailed in example 1 step b starting from (3-bromo-2,4-dichloro-phenyl)-phenyl-methanone (1.50 g, 4.55 mmol, 1 eq.) giving 2-(3-bromo-2,4-dichloro-phenyl)-2-phenyl-oxirane (1.6 g, quant.) as a yellow oil. ¹H NMR (400 MHz, DMSO-*d*₆) δ ppm 7.73 (m, 1 H), 7.68 (m, 1 H), 7.36 (m, 1 H), 7.33 (m, 2 H), 7.17 (m, 2 H), 3.48 (d, *J*=4.84 Hz, 1 H), 3.33 (d, *J*=4.95 Hz, 1 H).

### Step c: 2-amino-1-(3-bromo-2,4-dichloro-phenyl)-1-phenyl-ethanol

The compound was prepared using the same procedure as detailed in example 1 step c starting from 2-(3-bromo-2,4-dichloro-phenyl)-2-phenyl-oxirane (1.6 g, 4.7 mmol, 1 eq.) giving 2-amino-1-(3-bromo-2,4-dichloro-phenyl)-1-phenyl-ethanol (1.1 g, 64.7%) as a beige powder. ¹H NMR (400 MHz, DMSO-*d*₆) δ ppm 7.98 (d, *J*=8.69 Hz, 1 H), 7.67 (d, *J*=8.58 Hz, 1 H), 7.27 (m, 2 H), 7.21 (m, 2 H), 5.87 (br s, 1 H), 3.56 (d, *J*=13.09 Hz, 1 H), 3.28 (d, *J*=13.09 Hz, 1 H).

### Step d: 2-[3-(2-amino-1-hydroxy-1-phenyl-ethyl)-2,6-dichloro-phenyl]-3-fluoro-4-(2-methoxyethoxy)benzonitrile

The compound was prepared using the same procedure as detailed in example 19a step d starting from 2-amino-1-(3-bromo-2,4-dichloro-phenyl)-1-phenyl-ethanol (420 mg, 1.16 mmol, 1 eq.) and 3-fluoro-4-(2-methoxyethoxy)-2-trimethylstannyl-benzonitrile (Ex 19a, step c) (832 mg, 2.32 mmol, 2 eq.) giving 2-[3-(2-amino-1-hydroxy-1-phenyl-ethyl)-2,6-dichloro-phenyl]-3-fluoro-4-(2-methoxyethoxy)benzonitrile (22 mg, 4 %) as a colorless oil, used directly in the next step.

### Step e: 2-[3-(2-amino-1-hydroxy-1-phenyl-ethyl)-2,6-dichloro-phenyl]-3-fluoro-4-(2-methoxyethoxy)benzamide

The compound was prepared using the same procedure as detailed in example 2 step c starting from 2-[3-(2-amino-1-hydroxy-1-phenyl-ethyl)-2,6-dichloro-phenyl]-3-fluoro-4-(2-methoxyethoxy)benzonitrile (22 mg, 0.046 mmol, 1 eq.) giving 2-[3-(2-amino-1-hydroxy-1-phenyl-ethyl)-2,6-dichloro-phenyl]-3-fluoro-4-(2-methoxyethoxy)benzamide (4.1 mg, 18%) as a white powder. ¹H NMR (500 MHz, DMSO-*d*₆) δ ppm 7.96 and 7.88 (d, *J*=8.67 Hz, 1 H), 7.58 and 7.53 (br s, 1 H), 7.54 (dd, *J*=9.90, 8.67 Hz, 1 H), 7.52 (m, 1H), 7.29-7.17 (m, 6 H), 7.05 and 7.00 (br s, 1 H), 5.80 (br s, 1 H), 4.23 (m, 2 H), 3.69 (m, 2 H), 3.66 and 3.51 (d, *J*=13.34 Hz, 1 H), 3.30 (s, 3 H), 3.27 and 3.25 (br d, *J*=13.89 Hz, 1 H).

### Example 27: trans-2-[2-chloro-5-[1-hydroxy-2-[(4-hydroxycyclohexyl)amino]-1-phenylethyl]phenyl]-3-fluoro-4-(2-methoxyethoxy)benzamide

### Step a: trans-4-[[2-(3-bromo-4-chloro-phenyl)-2-hydroxy-2-phenyl-ethyl]amino]cyclohexanol

The compound was prepared using the same procedure as detailed in example 1 step c starting from 2-(3-bromo-4-chloro-phenyl)-2-phenyl-oxirane (200 mg, 0.594 mmol, 1 eq.) and trans-4-Aminocyclohexanol (69 mg, 0.60 mmol, 1 eq.) giving trans-4-[[2-(3-bromo-4-chlorophenyl)-2-hydroxy-2-phenyl-ethyl]amino]cyclohexanol (143 mg, 56.6%) as a colorless oil. ¹H NMR (400 MHz, DMSO-*d*₆) δ ppm 7.79 (d, *J*=2.09 Hz, 1 H), 7.51 (d, *J*=8.47 Hz, 1 H), 7.42 (m, 3 H), 7.28 (m, 2 H), 7.19 (tt, *J*=7.26, 1.32 Hz, 1 H), 5.77 (br s, 1 H), 4.42 (d, *J*=4.40 Hz, 1 H), 3.35 (m, 1 H), 3.28 (d, *J*=12.65 Hz, 1 H), 3.19 (d, *J*=12.65 Hz, 1 H), 2.25 (m, 1 H), 1.78 (m, 4 H), 1.07 (m, 2 H), 0.98 (m, 2 H).

### Step b: trans-2-[2-chloro-5-[1-hydroxy-2-[(4-hydroxycyclohexyl)amino]-1-phenylethyl]phenyl]-3-fluoro-4-(2-methoxyethoxy)benzonitrile

The compound was prepared using the same procedure as detailed in example 2 step b starting from trans-4-[[2-(3-bromo-4-chloro-phenyl)-2-hydroxy-2-phenylethyl]amino]cyclohexanol (120 mg, 0.283 mmol, 1 eq.) and 3-fluoro-4-(2-methoxyethoxy)-2-(4,4,5,5-tetramethyl-1,3,2-dioxaborolan-2-yl)benzonitrile (272 mg, 0.847 mmol, 3 eq.) giving trans-2-[2-chloro-5-[1-hydroxy-2-[(4-hydroxycyclohexyl)amino]-1-phenyl-ethyl]phenyl]-3-fluoro-4-(2-methoxyethoxy)benzonitrile (49 mg, 31%) as a beige powder. ¹H NMR (400 MHz, DMSO-*d*₆) δ ppm 7.80 (dd, *J*=8.69, 1.32 Hz, 1 H), 7.56 (m, 3 H), 7.43 (m, 3 H), 7.28 (m, 3 H), 7.18 (m, 1 H), 5.72 and 5.68 (m, 1 H), 4.41 and 4.40 (d, *J*=5.51 Hz, 1 H), 4.34 (m, 2 H), 3.71 (m, 2 H), 3.34 (m, 1H), 3.31 and 3.30 (s, 3 H), 3.29 (d, *J*=11.33 Hz, 1 H), 3.24 (d, *J*=11.33 Hz, 1 H), 2.25 (m, 1 H), 1.75 (m, 4 H), 1.07 (m, 2 H), 0.98 (m, 2H).

### Step c: trans-2-[2-chloro-5-[1-hydroxy-2-[(4-hydroxycyclohexyl)amino]-1-phenylethyl]phenyl]-3-fluoro-4-(2-methoxyethoxy)benzamide

The compound was prepared using the same procedure as detailed in example 2 step c?? starting from trans-2-[2-chloro-5-[1-hydroxy-2-[(4-hydroxycyclohexyl)amino]-1-phenylethyl]phenyl]-3-fluoro-4-(2-methoxyethoxy)benzonitrile (48 mg, 0.089 mmol, 1 eq.) giving trans-2-[2-chloro-5-[1-hydroxy-2-[(4-hydroxycyclohexyl)amino]-1-phenyl-ethyl]phenyl]-3-fluoro-4-(2-methoxyethoxy)benzamide (23 mg, 46%) as a white powder. ¹H NMR (500 MHz, DMSO-*d*₆) δ ppm 7.47 and 7.40 (br s, 1 H), 7.40 (m, 5 H), 7.33 (m, 1 H), 7.27 (m, 3 H), 7.17 (m, 1 H), 7.10 and 7.07 (br s, 1 H), 5.61 and 5.53 (br s, 1 H), 4.41 (br s, 1 H), 4.24 (m, 2 H), 3.70 and 3.69 (t, *J*=4.40 Hz, 2 H), 3.32 (m, 1 H), 3.31 and 3.30 (s, 3H), 3.24 and 3.16 (d, *J*=12.10 Hz, 1 H), 3.15 (br d, *J*=13.62 Hz, 1 H), 2.25 (m, 1 H), 1.75 (m, 4 H), 1.31 (m, 1 H), 1.08 (m, 2 H), 0.97 (m, 2 H).

### Examples 28a and 28b: rel-(2aR)-2-[3-[(1R)-2-amino-1-hydroxy-1-phenyl-ethyl]-6-chloro-2-fluoro-phenyl]-3-fluoro-4-(2-methoxyethoxy)benzamide and rel-(2aS)-2-[3-[(1R)-2-amino-1-hydroxy-1-phenyl-ethyl]-6-chloro-2-fluoro-phenyl]-3-fluoro-4-(2-methoxyethoxy)benzamide

### Step a: [(1S,2R,5S)-2-isopropyl-5-methyl-cyclohexyl] N-[(2R)-2-(3-bromo-4-chloro-2-fluorophenyl)-2-hydroxy-2-phenyl-ethyl]carbamate and [(1S,2R,5S)-2-isopropyl-5-methylcyclohexyl] N-[(2S)-2-(3-bromo-4-chloro-2-fluoro-phenyl)-2-hydroxy-2-phenylethyl]carbamate

The compound was prepared using the same procedure as detailed in example 19a step a starting from 2-amino-1-(3-bromo-4-chloro-2-fluoro-phenyl)-1-phenyl-ethanol (3.5 g, 10 mmol, 1 eq) and (+)-menthyl chloroformate (2.7 g, 12 mmol, 1.2 eq.). The diastereoisomers were separated by chromatography (100g HP SiO₂ cartridge; eluting from 30 to 100% cyclohexane / DCM) to give [(1S,2R,5S)-2-isopropyl-5-methyl-cyclohexyl] N-[(2R)-2-(3-bromo-4-chloro-2-fluoro-phenyl)-2-hydroxy-2-phenyl-ethyl]carbamate (1.45 g, 27 %) as a white powder and [(1S,2R,5S)-2-isopropyl-5-methyl-cyclohexyl] N-[(2S)-2-(3-bromo-4-chloro-2-fluoro-phenyl)-2-hydroxy-2-phenyl-ethyl]carbamate (1.68 g, 31 %), both isomers were used directly for menthyl cleavage.

### Step b: (1R)-2-amino-1-(3-bromo-4-chloro-2-fluoro-phenyl)-1-phenyl-ethanol

The compound was prepared using the same procedure as detailed in example 19a step b starting from [(1S,2R,5S)-2-isopropyl-5-methyl-cyclohexyl] N-[(2R)-2-(3-bromo-4-chloro-2-fluoro-phenyl)-2-hydroxy-2-phenyl-ethyl]carbamate (1.68 g, 3.19 mmol, 1 eq.) giving (1R)-2-amino-1-(3-bromo-4-chloro-2-fluoro-phenyl)-1-phenyl-ethanol (0.55 g, 50%) as a white solid. ¹H NMR (400 MHz, DMSO-*d*₆) δ ppm 7.78 (t, *J*=8.42 Hz, 1 H), 7.50 (dd, *J*=8.58, 1.54 Hz, 1 H), 7.32 (m, 4 H), 7.22 (tt, *J*=7.04, 1.65 Hz, 1 H), 5.91 (br s, 1 H), 3.38 (d, *J*=13.20 Hz, 1 H), 3.32 (br s, 2 H), 3.25 (d, *J*=13.20 Hz, 1 H, 1 H). [α]_{d}²² = +35.3° (C=0.28, DCM). ee = 93% (method D).

### Step c: 2-[(2R)-2-(3-bromo-4-chloro-2-fluoro-phenyl)-2-hydroxy-2-phenyl-ethyl]isoindoline-1,3-dione

The compound was prepared using the same procedure as detailed in example 16 step d starting from (1R)-2-amino-1-(3-bromo-4-chloro-2-fluoro-phenyl)-1-phenyl-ethanol (695 mg, 2.01 mmol, 1 eq.) giving 2-[(2R)-2-(3-bromo-4-chloro-2-fluoro-phenyl)-2-hydroxy-2-phenylethyl]isoindoline-1,3-dione (748 mg, 78.1%) as a white powder. ¹H NMR (400 MHz, DMSO-*d*₆) δ ppm 7.80 (m, 4 H), 7.61 (t, *J*=8.42 Hz, 1 H), 7.42 (m, 2 H), 7.37 (m, 2 H), 7.34 (m, 1 H), 7.30 (m, 1 H), 6.22 (br s, 1 H), 4.65 (d, *J*=13.97 Hz, 1 H), 4.49 (d, *J*=13.87 Hz, 1 H).

### Step d: 2-[6-chloro-3-[(1R)-2-(1,3-dioxoisoindolin-2-yl)-1-hydroxy-1-phenyl-ethyl]-2-fluorophenyl]-3-fluoro-4-(2-methoxyethoxy)benzonitrile

The compound was prepared using the same procedure as detailed in example 19a step d starting from 2-[(2R)-2-(3-bromo-4-chloro-2-fluoro-phenyl)-2-hydroxy-2-phenylethyl]isoindoline-1,3-dione (400 mg, 0.84 mmol, 1 eq.) and 3-fluoro-4-(2-methoxyethoxy)-2-trimethylstannyl-benzonitrile (Ex 19a, step c) (603 mg, 1.68 mmol, 2 eq.) giving 2-[6-chloro-3-[(1R)-2-(1,3-dioxoisoindolin-2-yl)-1-hydroxy-1-phenyl-ethyl]-2-fluoro-phenyl]-3-fluoro-4-(2-methoxyethoxy)benzonitrile (279 mg, 56.2%) as an off-white solid, used in next step without further purification.

### Step e: rel-(2aR)-2-[3-[(1R)-2-amino-1-hydroxy-1-phenyl-ethyl]-6-chloro-2-fluoro-phenyl]-3-fluoro-4-(2-methoxyethoxy)benzonitrile and rel-(2aS)-2-[3-[(1R)-2-amino-1-hydroxy-1-phenylethyl]-6-chloro-2-fluoro-phenyl]-3-fluoro-4-(2-methoxyethoxy)benzonitrile

The compounds were prepared using the same procedure as detailed in example 16 step f starting from 2-[6-chloro-3-[(1R)-2-(1,3-dioxoisoindolin-2-yl)-1-hydroxy-1-phenyl-ethyl]-2-fluoro-phenyl]-3-fluoro-4-(2-methoxyethoxy)benzonitrile (278 mg, 0.47 mmol, 1 eq.). The two atropoisomers were separated by chromatography (Column: Xbridge C18, 30 x 100mm 5µm, Flowrate: 35 mL/min,Mobile phase: H2O + 0.1% NH4OH / MeOH + 0.1% NH4OH, Temperature: 45°C) to give rel-(2aR)-2-[3-[(1R)-2-amino-1-hydroxy-1-phenyl-ethyl]-6-chloro-2-fluoro-phenyl]-3-fluoro-4-(2-methoxyethoxy)benzonitrile (96 mg, 44%) as a white solid. ¹H NMR (400 MHz, DMSO-*d*₆) δ ppm 7.90 (t, *J*=8.58 Hz, 1 H), 7.83 (dd, *J*=8.69, 1.43 Hz, 1 H), 7.58 (dd, *J*=8.69, 0.99 Hz, 1 H), 7.48 (t, *J*=8.53 Hz, 1 H), 7.34 (m, 2 H), 7.26 (m, 2 H), 7.21 (tt, *J*=7.04, 1.65 Hz, 1 H), 5.97 (br s, 1 H), 4.34 (m, 2 H), 3.70 (m, 2 H), 3.31 (d, *J*=13.42 Hz, 1 H), 3.30 (s, 3 H), 3.24 (d, *J*=13.09 Hz, 1 H). And rel-(2aS)-2-[3-[(1R)-2-amino-1-hydroxy-1-phenyl-ethyl]-6-chloro-2-fluoro-phenyl]-3-fluoro-4-(2-methoxyethoxy)benzonitrile (44 mg, 20%) as a white solid. ¹H NMR (400 MHz, DMSO-*d*₆) δ ppm 7.96 (t, *J*=8.64 Hz, 1 H), 7.84 (dd, *J*=8.69, 1.43 Hz, 1 H), 7.59 (dd, *J*=8.69, 0.99 Hz, 1 H), 7.48 (t, *J*=8.53 Hz, 1 H), 7.34 (m, 2 H), 7.29 (m, 2 H), 7.21 (tt, *J*=7.26, 1.43 Hz, 1 H), 5.97 (br s, 1 H), 4.33 (m, 2 H), 3.70 (dd, *J*=4.73, 4.18 Hz, 2 H), 3.35 (d, *J*=13.64 Hz, 1 H), 3.30 (s, 3 H), 3.25 (d, *J*=13.53 Hz, 1 H).

### Step f: rel-(2aR)-2-[3-[(1R)-2-amino-1-hydroxy-1-phenyl-ethyl]-6-chloro-2-fluoro-phenyl]-3-fluoro-4-(2-methoxyethoxy)benzamide (Ex 28a)

The compound was prepared using the same procedure as detailed in example 2 step c starting from rel-(2aR)-2-[3-[(1R)-2-amino-1-hydroxy-1-phenyl-ethyl]-6-chloro-2-fluorophenyl]-3-fluoro-4-(2-methoxyethoxy)benzonitrile (86 mg, 0.19 mmol, 1 eq.) giving rel-(2aR)-2-[3-[(1R)-2-amino-1-hydroxy-1-phenyl-ethyl]-6-chloro-2-fluoro-phenyl]-3-fluoro-4-(2-methoxyethoxy)benzamide (84.0 mg, 94.0%) as a white powder. ¹H NMR (500 MHz, DMSO-*d*₆) δ ppm 7.69 (br s, 1 H), 7.66 (t, *J*=8.46 Hz, 1 H), 7.51 (d, *J*=8.39 Hz, 1 H), 7.36 (m, 3 H), 7.28 (m, 3 H), 7.19 (t, *J*=7.29 Hz, 1 H), 7.09 (br s, 1 H), 5.87 (br s, 1 H), 4.27 (dt, *J*=10.87, 4.54 Hz, 1 H), 4.24 (dt, *J*=10.87, 4.54 Hz, 1 H), 3.70 (t, *J*=4.47 Hz, 2 H), 3.31 (s, 3 H), 3.23 (d, *J*=13.48 Hz, 1 H), 3.15 (d, *J*=13.34 Hz, 1 H). [α]_{d}^{22.8} = +91.9° (C=0.39, MeOH).

### Step g: rel-(2aS)-2-[3-[(1R)-2-amino-1-hydroxy-1-phenyl-ethyl]-6-chloro-2-fluoro-phenyl]-3-fluoro-4-(2-methoxyethoxy)benzamide (Ex 28b)

The compound was prepared using the same procedure as detailed in example 2 step c starting from rel-(2aS)-2-[3-[(1R)-2-amino-1-hydroxy-1-phenyl-ethyl]-6-chloro-2-fluorophenyl]-3-fluoro-4-(2-methoxyethoxy)benzonitrile (44 mg, 0.096 mmol, 1 eq.) giving rel-(2aS)-2-[3-[(1R)-2-amino-1-hydroxy-1-phenyl-ethyl]-6-chloro-2-fluoro-phenyl]-3-fluoro-4-(2-methoxyethoxy)benzamide (36 mg, 78%) as a white powder. ¹H NMR (500 MHz, DMSO-*d*₆) δ ppm 7.76 (t, *J*=8.46 Hz, 1 H), 7.72 (br s, 1 H), 7.51 (m, 1 H), 7.38 (d, *J*=8.53 Hz, 1 H), 7.34 (m, 2 H), 7.29 (m, 3 H), 7.20 (tt, *J*=7.43, 1.38 Hz, 1 H), 7.13 (br s, 1 H), 5.87 (br s, 1 H), 4.27 (dt, *J*=11.1, 4.40 Hz, 1 H), 4.23 (dt, *J*=11.1, 4.40 Hz, 1 H), 3.69 (t, *J*=4.54 Hz, 2 H), 3.37 (dd, *J*=13.48, 2.34 Hz, 1 H), 3.30 (s, 3 H), 3.09 (d, *J*=13.89 Hz, 1 H). [α]_{d}^{22.8} = +39° (C=0.46, MeOH).

### Examples 29a and 29b: rel-(2aR)-2-[3-[(1S)-2-amino-1-hydroxy-1-phenyl-ethyl]-6-chloro-2-fluoro-phenyl]-3-fluoro-4-(2-methoxyethoxy)benzamide and rel-(2aS)-2-[3-[(1S)-2-amino-1-hydroxy-1-phenyl-ethyl]-6-chloro-2-fluoro-phenyl]-3-fluoro-4-(2-methoxyethoxy)benzamide

### Step a: (1S)-2-amino-1-(3-bromo-4-chloro-2-fluoro-phenyl)-1-phenyl-ethanol

The compound was prepared using the same procedure as detailed in example 19a step b starting from [(1S,2R,5S)-2-isopropyl-5-methyl-cyclohexyl] N-[(2S)-2-(3-bromo-4-chloro-2-fluoro-phenyl)-2-hydroxy-2-phenyl-ethyl]carbamate (Ex 28a, step a) (1.4 g, 2.67 mmol, 1 eq.) giving (1S)-2-amino-1-(3-bromo-4-chloro-2-fluoro-phenyl)-1-phenyl-ethanol (0.425 g, 46%) as a white solid. ¹H NMR (400 MHz, DMSO-*d*₆) δ ppm 7.77 (t, *J*=8.42 Hz, 1 H), 7.50 (dd, *J*=8.64, 1.49 Hz, 1 H), 7.33 (m, 2 H), 7.30 (m, 2 H), 7.23 (m, 1 H), 5.91 (br s, 1 H), 3.38 (dd, *J*=13.09, 1.65 Hz, 1 H), 3.24 (dd, *J*=12.98, 0.77 Hz, 1 H). [α]_{d}²² = -29.2° (C=0.25, DCM). ee = 99% (method D).

### Step b: 2-[(2S)-2-(3-bromo-4-chloro-2-fluoro-phenyl)-2-hydroxy-2-phenyl-ethyl]isoindoline-1,3-dione

The compound was prepared using the same procedure as detailed in example 16 step d starting from (1S)-2-amino-1-(3-bromo-4-chloro-2-fluoro-phenyl)-1-phenyl-ethanol (430 mg, 1.24 mmol, 1 eq.) giving 2-[(2S)-2-(3-bromo-4-chloro-2-fluoro-phenyl)-2-hydroxy-2-phenylethyl]isoindoline-1,3-dione (470 mg, 79.3%) as a white powder. ¹H NMR (400 MHz, DMSO-*d*₆) δ ppm 7.80 (m, 4 H), 7.61 (t, *J*=8.42 Hz, 1 H), 7.42 (m, 2 H), 7.38 (m, 1 H), 7.34 (m, 2 H), 7.29 (t, *J*=7.15 Hz, 1 H), 6.22 (s, 1 H), 4.65 (d, *J*=13.86 Hz, 1 H), 4.50 (d, *J*=13.75 Hz, 1 H). Step c: 2-[6-chloro-3-[(1S)-2-(1,3-dioxoisoindolin-2-yl)-1-hydroxy-1-phenyl-ethyl]-2-fluorophenyl]-3-fluoro-4-(2-methoxyethoxy)benzonitrile

The compound was prepared using the same procedure as detailed in example 19a step d starting from 2-[(2S)-2-(3-bromo-4-chloro-2-fluoro-phenyl)-2-hydroxy-2-phenylethyl]isoindoline-1,3-dione (470 mg, 0.99 mmol, 1 eq.) and 3-fluoro-4-(2-methoxyethoxy)-2-trimethylstannyl-benzonitrile (Ex 19a, step c) (709 mg, 1.98 mmol, 2 eq.) giving 2-[6-chloro-3-[(1S)-2-(1,3-dioxoisoindolin-2-yl)-1-hydroxy-1-phenyl-ethyl]-2-fluoro-phenyl]-3-fluoro-4-(2-methoxyethoxy)benzonitrile (322 mg, 55.2%), used directly in the next step of deprotection. Step d: rel-(2aR)-2-[3-[(1S)-2-amino-1-hydroxy-1-phenyl-ethyl]-6-chloro-2-fluoro-phenyl]-3-fluoro-4-(2-methoxyethoxy)benzonitrile and rel-(2aS)-2-[3-[(1S)-2-amino-1-hydroxy-1-phenylethyl]-6-chloro-2-fluoro-phenyl]-3-fluoro-4-(2-methoxyethoxy)benzonitrile The compounds were prepared using the same procedure as detailed in example 16 step f starting from 2-[6-chloro-3-[(1S)-2-(1,3-dioxoisoindolin-2-yl)-1-hydroxy-1-phenyl-ethyl]-2-fluoro-phenyl]-3-fluoro-4-(2-methoxyethoxy)benzonitrile (320 mg, 0.54 mmol, 1 eq.). The two atropoisomers were separated by chromatography (Column: Xbridge C18, 30 x 100mm 5µm, Flowrate: 35 mL/min,Mobile phase: H2O + 0.1% NH4OH / MeOH + 0.1% NH4OH, Temperature: 45°C) to give rel-(2aR)-2-[3-[(1S)-2-amino-1-hydroxy-1-phenyl-ethyl]-6-chloro-2-fluoro-phenyl]-3-fluoro-4-(2-methoxyethoxy)benzonitrile (34 mg, 13%) as a colorless gum. ¹H NMR (400 MHz, DMSO-*d*₆) δ ppm 7.96 (t, *J*=8.58 Hz, 1 H), 7.84 (dd, *J*=8.69, 1.32 Hz, 1 H), 7.60 (d, *J*=8.58 Hz, 1 H), 7.48 (t, *J*=8.58 Hz, 1 H), 7.35 (m, 2 H), 7.28 (m, 2 H), 7.21 (m, 1 H), 5.98 (br s, 1 H), 4.33 (m, 2 H), 3.70 (t, *J*=4.40 Hz, 2 H), 3.36 (d, *J*=13.53 Hz, 2 H), 3.30 (s, 3 H), 3.25 (d, *J*=13.42 Hz, 1 H). And rel-(2aS)-2-[3-[(1S)-2-amino-1-hydroxy-1-phenylethyl]-6-chloro-2-fluoro-phenyl]-3-fluoro-4-(2-methoxyethoxy)benzonitrile as a colorless gum. ¹H NMR (400 MHz, DMSO-*d*₆) δ ppm 7.91 (m, 1 H), 7.83 (d, *J*=8.80 Hz, 1 H), 7.58 (d, *J*=8.58 Hz, 1 H), 7.48 (t, *J*=8.47 Hz, 1 H), 7.34 (m, 2 H), 7.27 (m, 2 H), 7.21 (m, 1 H), 5.95 (br s, 1 H), 4.34 (m, 2 H), 3.71 (t, *J*=3.63 Hz, 2 H), 3.31 (d, *J*=13.64 Hz, 1 H), 3.30 (br s, 3 H), 3.24 (d, *J*=13.64 Hz, 1 H).

### Step e: rel-(2aR)-2-[3-[(1S)-2-amino-1-hydroxy-1-phenyl-ethyl]-6-chloro-2-fluoro-phenyl]-3-fluoro-4-(2-methoxyethoxy)benzamide (Ex 29a)

The compound was prepared using the same procedure as detailed in example 2 step c starting from rel-(2aR)-2-[3-[(1S)-2-amino-1-hydroxy-1-phenyl-ethyl]-6-chloro-2-fluorophenyl]-3-fluoro-4-(2-methoxyethoxy)benzonitrile (31 mg, 0.068 mmol, 1 eq.) giving rel-(2aR)-2-[3-[(1S)-2-amino-1-hydroxy-1-phenyl-ethyl]-6-chloro-2-fluoro-phenyl]-3-fluoro-4-(2-methoxyethoxy)benzamide (29 mg, 90%) as a white powder. ¹H NMR (500 MHz, DMSO-*d*₆) δ ppm 7.76 (t, *J*=8.46 Hz, 1 H), 7.72 (br s, 1 H), 7.51 (dd, *J*=8.67, 1.10 Hz, 1 H), 7.38 (d, *J*=8.39 Hz, 1 H), 7.34 (m, 2 H), 7.31 (m, 1 H), 7.28 (m, 2 H), 7.20 (tt, *J*=7.43, 1.51 Hz, 1 H), 7.13 (br s, 1 H), 5.87 (s, 1 H), 4.27 (dt, *J*=11.1, 4.40 Hz, 1 H), 4.23 (dt, *J*=11.1, 4.40 Hz, 1 H), 3.69 (t, *J*=4.54 Hz, 2 H), 3.38 (dd, *J*=13.62, 2.48 Hz, 1 H), 3.30 (s, 3 H), 3.09 (d, *J*=13.34 Hz, 1 H). [α]_{d}^{21.9} = -39.1° (C=0.37, MeOH).

### Step f: rel-(2aS)-2-[3-[(1S)-2-amino-1-hydroxy-1-phenyl-ethyl]-6-chloro-2-fluoro-phenyl]-3-fluoro-4-(2-methoxyethoxy)benzamide (Ex 29b)

The compound was prepared using the same procedure as detailed in example 2 step c starting from rel-(2aS)-2-[3-[(1S)-2-amino-1-hydroxy-1-phenyl-ethyl]-6-chloro-2-fluorophenyl]-3-fluoro-4-(2-methoxyethoxy)benzonitrile (68 mg, 0.15 mmol, 1 eq.) giving rel-(2aS)-2-[3-[(1S)-2-amino-1-hydroxy-1-phenyl-ethyl]-6-chloro-2-fluoro-phenyl]-3-fluoro-4-(2-methoxyethoxy)benzamide (62 mg, 87.7%) as a white powder ¹H NMR (500 MHz, DMSO-*d*₆) δ ppm 7.69 (br s, 1 H), 7.66 (t, *J*=8.39 Hz, 1 H), 7.51 (d, *J*=8.67 Hz, 1 H), 7.36 (m, 3 H), 7.28 (m, 3 H), 7.20 (t, *J*=7.29 Hz, 1 H), 7.09 (br s, 1 H), 5.88 (br s, 1 H), 4.27 (dt, *J*=10.87, 4.54 Hz, 1 H), 4.24 (dt, *J*=10.87, 4.54 Hz, 1 H), 3.70 (t, *J*=4.33 Hz, 2 H), 3.31 (s, 3 H), 3.23 (d, *J*=13.62 Hz, 1 H), 3.15 (d, *J*=13.75 Hz, 1 H). [α]_{d}²² = -84.9° (C=0.46, MeOH).

### Example 30: 2-[6-chloro-3-[2-(cyclopropylmethylamino)-1-hydroxy-1-phenyl-ethyl]-2-fluoro-phenyl]-3-fluoro-4-(2-methoxyethoxy)benzamide

### Step a: 1-(3-bromo-4-chloro-2-fluoro-phenyl)-2-(cyclopropylmethylamino)-1-phenyl-ethanol

2-amino-1-(3-bromo-4-chloro-2-fluoro-phenyl)-1-phenyl-ethanol (Ex 10, step c) (785 mg, 2.28 mmol, 1 eq.) was solubilized in MeOH (9.22 mL) and cyclopropanecarboxaldehyde (0.187 mL, 2.5 mmol 1.1 eq.) was added. The solution was stirred at rt for 2h. Then, the solution was cooled to 0°C and NaBH₄ (0.17 g, 4.59 mmol 2.00 eq.) was added. The reaction mixture was stirred at rt for 1h. The solution was diluted with EtOAc and washed with water. The resulting organic layer was dried over MgSO₄, filtered and concentrated. The crude product was purified by chromatography (CH₂Cl₂/MeOH 100:0 to 98:2) to give 1-(3-bromo-4-chloro-2-fluoro-phenyl)-2-(cyclopropylmethylamino)-1-phenyl-ethanol (566 mg, 62.3%) as a pale-yellow oil. ¹H NMR (400 MHz, DMSO-*d*₆) δ ppm 7.78 (t, *J*=8.42 Hz, 1 H), 7.48 (dd, *J*=8.69, 1.21 Hz, 1 H), 7.32 (m, 2 H), 7.27 (m, 2 H), 7.20 (tt, *J*=7.04, 1.43 Hz, 1 H), 5.95 (br s, 1 H), 3.40 (d, *J*=12.87 Hz, 1 H), 3.23 (d, *J*=11.88Hz, 1 H), 2.36 (m, 2 H), 0.80 (m, 1 H), 0.34 (m, 2 H), 0.00 (m, 2 H).

### Step b: tert-butyl N-[2-(3-bromo-4-chloro-2-fluoro-phenyl)-2-hydroxy-2-phenyl-ethyl]-N-(cyclopropylmethyl)carbamate

The compound was prepared using the same procedure as detailed in example 17 step b starting from 1-(3-bromo-4-chloro-2-fluoro-phenyl)-2-(cyclopropylmethylamino)-1-phenyl-ethanol (565 mg, 1.4mmol, 1 eq.) giving tert-butyl N-[2-(3-bromo-4-chloro-2-fluoro-phenyl)-2-hydroxy-2-phenyl-ethyl]-N-(cyclopropylmethyl)carbamate (666 mg, 94.2%) as a colorless oil. The compound is used in the next step without further purification.

### Step c: 2-[6-chloro-3-[2-(cyclopropylmethylamino)-1-hydroxy-1-phenyl-ethyl]-2-fluoro-phenyl]-3-fluoro-4-(2-methoxyethoxy)benzonitrile

The compound was prepared using the same procedure as detailed in example 19a step d starting from tert-butyl N-[2-(3-bromo-4-chloro-2-fluoro-phenyl)-2-hydroxy-2-phenyl-ethyl]-N-(cyclopropylmethyl)carbamate (660 mg, 1.32 mmol, 1 eq.) and 3-fluoro-4-(2-methoxyethoxy)-2-trimethylstannyl-benzonitrile (Ex 19a, step c) (0.94 g, 2.6 mmol, 2.0 eq.) giving the crude product. The deprotection was done using the same procedure as detailed in example 17 step g giving 2-[6-chloro-3-[2-(cyclopropylmethylamino)-1-hydroxy-1-phenyl-ethyl]-2-fluorophenyl]-3-fluoro-4-(2-methoxyethoxy)benzonitrile. ¹H NMR (400 MHz, CHLOROFORM-d) δ ppm 7.96 and 7.85 (t, J=8.47 Hz, 1 H), 7.51 and 7.49 (m, 1 H), 7.45 and 7.43 (dt, J=2.70, 1.29 Hz, 2 H), 7.39 and 7.37 (dd, J=6.16, 1.10 Hz, 1 H), 7.32 and 7.30 (m, 2 H), 7.23 (m, 1 H), 7.09 (m, 1 H), 4.27 (m, 2 H), 3.80 (m, 2 H), 3.72 and 3.55 (dd, J=12.27, 1.71 Hz, 1 H), 3.45 and 3.44 (s, 3 H), 3.35 and 3.27 (dd, J=12.16, 2.15 Hz, 1 H), 2.48 (m, 2 H), 0.89 (m, 1 H), 0.44 (m, 2 H), 0.08 (m, 2 H).

### Step d: 2-[6-chloro-3-[2-(cyclopropylmethylamino)-1-hydroxy-1-phenyl-ethyl]-2-fluoro-phenyl]-3-fluoro-4-(2-methoxyethoxy)benzamide

The compound was prepared using the same procedure as detailed in example 2 step c starting from 2-[6-chloro-3-[2-(cyclopropylmethylamino)-1-hydroxy-1-phenyl-ethyl]-2-fluorophenyl]-3-fluoro-4-(2-methoxyethoxy)benzonitrile (138 mg, 0.269 mmol, 1 eq.) giving 2-[6-chloro-3-[2-(cyclopropylmethylamino)-1-hydroxy-1-phenyl-ethyl]-2-fluoro-phenyl]-3-fluoro-4-(2-methoxyethoxy)benzamide (67.4 mg, 47%) as a white powder. ¹H NMR (500 MHz, DMSO-*d*₆) δ ppm 7.77 and 7.68 (t, *J*=8.39 Hz, 1 H), 7.66 and 7.61 (br s, 1 H), 7.50 and 7.49 (s, 1 H), 7.36 (m, 3 H), 7.28 (m, 3 H), 7.19 (m, 1 H), 7.07 and 7.02 (br s, 1 H), 5.86 and 5.77 (br s, 1 H), 4.24 (m, 2 H), 3.69 (m, 2 H), 3.36 (m , 1H), 3.31 and 3.30 (s, 3 H), 3.25 and 3.22 (d, , *J*=12.3 Hz, 1 H), 2.34 (m, 2 H), 0.82 (m, 1 H), 0.34 (m, 2 H), 0.04 and 0.02 (m, 2 H).

### Example 31: 2-[6-chloro-2-fluoro-3-[1-hydroxy-2-(2-methoxyethylamino)-1-phenylethyl]phenyl]-3-fluoro-4-(2-methoxyethoxy)benzamide

### Step a: 1-(3-bromo-4-chloro-2-fluoro-phenyl)-2-(2-methoxyethylamino)-1-phenyl-ethanol

The compound was prepared using the same procedure as detailed in example 1 step c starting from 2-(3-bromo-4-chloro-2-fluoro-phenyl)-2-phenyl-oxirane (1.00 g, 3.05 mmol, 1 eq.) and 2 methoxy ethylamine (4.59 g, 61.1 mmol, 20.0 eq.) giving 1-(3-bromo-4-chloro-2-fluoro-phenyl)-2-(2-methoxyethylamino)-1-phenyl-ethanol (780 mg, 63.4%) as a yellow oil. ¹H NMR (400 MHz, DMSO-*d*₆) δ ppm 7.79 (t, *J*=8.42 Hz, 1 H), 7.50 (dd, *J*=8.64, 1.49 Hz, 1 H), 7.35 (m, 2 H), 7.30 (m, 2 H), 7.22 (tt, *J*=7.15, 1.54 Hz, 1 H), 5.97 (br s, 1 H), 3.42 (br d, *J*=12.32 Hz, 1 H), 3.33 (m, 2 H), 3.25 (d, *J*=12.43 Hz, 1 H), 3.20 (s, 3 H), 2.67 (m, 2 H).

### Step b: tert-butyl N-[2-(3-bromo-4-chloro-2-fluoro-phenyl)-2-hydroxy-2-phenyl-ethyl]-N-(2-methoxyethyl)carbamate

The compound was prepared using the same procedure as detailed in example 17 step d starting from 1-(3-bromo-4-chloro-2-fluoro-phenyl)-2-(2-methoxyethylamino)-1-phenyl-ethanol (780 mg, 1.9 mmol, 1 eq.) giving tert-butyl N-[2-(3-bromo-4-chloro-2-fluoro-phenyl)-2-hydroxy-2-phenyl-ethyl]-N-(2-methoxyethyl)carbamate (973 mg, 99.9%) as a pale-yellow oil. ¹H NMR (400 MHz, DMSO-*d*₆) δ ppm 8.31 (s, 1 H), 7.76 (m, 1 H), 7.54 and 7.46 (m, 1 H), 7.30 (m, 5 H), 6.15 (s, 1 H), 4.64 and 4.52 (d, *J*=13.53 Hz, 1 H), 3.87 and 3.73 (d, *J*=16.62 Hz, 1 H), 3.48 (m, 4 H), 3.24 (m, 3 H), 1.13 and 1.12 (s, 9 H).

### Step c: 2-[6-chloro-2-fluoro-3-[1-hydroxy-2-(2-methoxyethylamino)-1-phenyl-ethyl]phenyl]-3-fluoro-4-(2-methoxyethoxy)benzonitrile

The compound was prepared using the same procedure as detailed in example 19a step d starting from tert-butyl N-[2-(3-bromo-4-chloro-2-fluoro-phenyl)-2-hydroxy-2-phenyl-ethyl]-N-(2-methoxyethyl)carbamate (600 mg, 1.19 mmol, 1 eq.) and 3-fluoro-4-(2-methoxyethoxy)-2-trimethylstannyl-benzonitrile (Ex 19a, step c) (0.85 g, 2.4 mmol, 2.0 eq.) giving the crude product. The deprotection was done using the same procedure as detailed in example 17 step g giving 2-[6-chloro-2-fluoro-3-[1-hydroxy-2-(2-methoxyethylamino)-1-phenylethyl]phenyl]-3-fluoro-4-(2-methoxyethoxy)benzonitrile (165 mg; 26.7%) as a colorless oil. ¹H NMR (400 MHz, CHLOROFORM-d) δ ppm 7.94 and 7.84 (t, *J*=8.47 Hz, 1 H), 7.50 (dd, *J*=8.64, 1.60 Hz, 1 H), 7.43 (m, 2 H), 7.38 and 7.36 (dd, *J*=5.50, 1.10 Hz, 1 H), 7.32 and 7.29 (m, 2 H), 7.23 (m, 1 H), 7.09 (m, 1 H), 5.30 (s, 1 H), 4.27 (m, 2 H), 3.80 (m, 2 H), 3.64 and 3.54 (dd, *J*=12.43, 1.43 Hz, 1 H), 3.45 and 3.44 (s, 3 H), 3.42 (m, 2 H), 3.35 (m, 1 H), 3.32 (s, 3 H), 2.78 (m, 2 H).

### Step d: 2-[6-chloro-2-fluoro-3-[1-hydroxy-2-(2-methoxyethylamino)-1-phenyl-ethyl]phenyl]-3-fluoro-4-(2-methoxyethoxy)benzamide

The compound was prepared using the same procedure as detailed in example 2 step c starting from 2-[6-chloro-2-fluoro-3-[1-hydroxy-2-(2-methoxyethylamino)-1-phenylethyl]phenyl]-3-fluoro-4-(2-methoxyethoxy)benzonitrile (165 mg, 0.319 mmol, 1 eq.) giving 2-[6-chloro-2-fluoro-3-[1-hydroxy-2-(2-methoxyethylamino)-1-phenyl-ethyl]phenyl]-3-fluoro-4-(2-methoxyethoxy)benzamide (87.6 mg, 51 %) as a white solid. ¹H NMR (500 MHz, DMSO-*d*₆) δ ppm 7.76 and 7.67 (d, *J*=8.39 Hz, 1 H), 7.60 and 7.58 (br s, 1 H), 7.50 and 7.48 (m, 1 H), 7.35 (m, 3 H), 7.27 (m, 3 H), 7.19 (m, 1 H), 7.07 and 7.04 (br s, 1 H), 5.85 and 5.77 (s, 1 H), 4.25 and 4.24 (q, *J*=4.05 Hz, 2 H), 3.69 and 3.68 (t, *J*=4.57 Hz, 2 H), 3.33 (m, 2 H), 3.31 and 3.30 (s, 3 H), 3.29 and 3.27 (m, 1 H), 3.26 and 3.21 (d, *J*=13.20 Hz, 1 H), 3.20 and 3.18 (s, 3 H), 2.64 and 2.57 (m, 2 H).

### Example 32: 2-[6-chloro-3-[2-(dimethylamino)-1-hydroxy-1-phenyl-ethyl]-2-fluorophenyl]-3-fluoro-4-(2-methoxyethoxy)benzamide

### Step a: tert-butyl N-[2-(3-bromo-4-chloro-2-fluoro-phenyl)-2-hydroxy-2-phenyl-ethyl]-N-methyl-carbamate

The compound was prepared using the same procedure as detailed in example 17 step d starting from 1-(3-bromo-4-chloro-2-fluoro-phenyl)-2-(methylamino)-1-phenyl-ethanol (Ex 22, step a) (800 mg, 2.23 mmol, 1.00 eq.) giving tert-butyl N-[2-(3-bromo-4-chloro-2-fluorophenyl)-2-hydroxy-2-phenyl-ethyl]-N-methyl-carbamate (860 mg, 84.3 %) as a colorless oil. ¹H NMR (400 MHz, CHLOROFORM-d) δ ppm 7.79 (t, *J*=8.09 Hz, 1 H), 7.36 (m, 2 H), 7.32 (m, 3 H), 7.26 (m, 1 H), 6.50 (br s, 1 H), 4.34 (d, *J*=14.64 Hz, 1 H), 4.08 (d, *J*=14.75 Hz, 1 H), 2.53 (br s, 3 H), 1.42 (s, 9 H).

### Step b: 2-[6-chloro-2-fluoro-3-[1-hydroxy-2-(methylamino)-1-phenyl-ethyl]phenyl]-3-fluoro-4-(2-methoxyethoxy)benzonitrile

The compound was prepared using the same procedure as detailed in example 19a step d starting from tert-butyl N-[2-(3-bromo-4-chloro-2-fluoro-phenyl)-2-hydroxy-2-phenyl-ethyl]-N-methyl-carbamate (850 mg, 1.85 mmol, 1.00 eq.) and 3-fluoro-4-(2-methoxyethoxy)-2-trimethylstannyl-benzonitrile (Ex 19a, step c) (1.1 g, 3.1 mmol, 1.7 eq.) giving tert-butyl N-[2-[4-chloro-3-[6-cyano-2-fluoro-3-(2-methoxyethoxy)phenyl]-2-fluoro-phenyl]-2-hydroxy-2-phenyl-ethyl]-N-methyl-carbamate which is directly deprotected using the same procedure as described in example 17 step g to give 2-[6-chloro-2-fluoro-3-[1-hydroxy-2-(methylamino)-1-phenyl-ethyl]phenyl]-3-fluoro-4-(2-methoxyethoxy)benzonitrile (546 mg, 62.3 %) as a yellow oil. ¹H NMR (400 MHz, CHLOROFORM-d) δ ppm 7.95 and 7.84 (t, *J*=8.47 Hz, 1 H), 7.50 (m, 1 H), 7.43 (m, 2 H), 7.38 and 7.36 (dd, *J*=5.94, 1.10 Hz, 1 H), 7.31 (m, 2 H), 7.24 (m, 1 H), 7.10 (td, *J*=8.23, 1.27 Hz, 1 H), 4.27 (m, 2 H), 3.80 (m, 2 H), 3.66 and 3.49 (dd, *J*=12.10, 1.76 Hz, 1 H), 3.45 and 3.44 (s, 3 H), 3.29 and 3.21 (dd, *J*=12.10, 2.09 Hz, 1 H), 2.45 and 2.43 (s, 3 H).

### Step c: 2-[6-chloro-3-[2-(dimethylamino)-1-hydroxy-1-phenyl-ethyl]-2-fluoro-phenyl]-3-fluoro-4-(2-methoxyethoxy)benzonitrile

2-[6-chloro-2-fluoro-3-[1-hydroxy-2-(methylamino)-1-phenyl-ethyl]phenyl]-3-fluoro-4-(2-methoxyethoxy)benzonitrile (545 mg, 1.15 mmol, 1.00 eq.) was solubilized in 1,4-dioxane (8.00 mL). HCOH/ 37% in H₂O (1.80 mL, 23.1 mmol, 20.0 eq.) and formic acid (0.87 mL, 23 mmol, 20 eq.) were added and the reaction mixture was stirred at 60°C for 4h. The solution was diluted with DCM and washed with NaOH (1N). The resulting organic layer was dried over MgSO₄, filtered and concentrated under reduced pressure. The crude product was purified by chromatography (DCM/MeOH 100:0 to 95:5 v/v) to give 2-[6-chloro-3-[2-(dimethylamino)-1-hydroxy-1-phenyl-ethyl]-2-fluoro-phenyl]-3-fluoro-4-(2-methoxyethoxy)benzonitrile (102 mg, 18.2 %) as a colorless oil. ¹H NMR (400 MHz, CHLOROFORM-d) δ ppm 8.03 and 7.94 (t, *J*=8.53 Hz, 1 H), 7.52 and 7.50 (dd, *J*=2.75, 1.65 Hz, 1 H), 7.47 (m, 2 H), 7.38 and 7.36 (dd, *J*=4.35, 1.05 Hz, 1 H), 7.29 (m, 2 H), 7.21 (m, 1 H), 7.10 (ddd, *J*=8.64, 7.81, 1.93 Hz, 1 H), 4.27 (m, 2 H), 3.81 (m, 2 H), 3.51 and 3.43 (d, *J*=12.87 Hz, 1 H), 3.46 and 3.44 (s, 3 H), 3.28 and 3.23 (d, *J*=13.64 Hz, 1 H), 2.24 (s, 3 H), 2.20 (s, 3 H).

### Step d: 2-[6-chloro-3-[2-(dimethylamino)-1-hydroxy-1-phenyl-ethyl]-2-fluoro-phenyl]-3-fluoro-4-(2-methoxyethoxy)benzamide

The compound was prepared using the same procedure as detailed in example 12 step e starting from 2-[6-chloro-3-[2-(dimethylamino)-1-hydroxy-1-phenyl-ethyl]-2-fluoro-phenyl]-3-fluoro-4-(2-methoxyethoxy)benzonitrile (102 mg, 0.209 mmol, 1.00 eq.) giving 2-[6-chloro-3-[2-(dimethylamino)-1-hydroxy-1-phenyl-ethyl]-2-fluoro-phenyl]-3-fluoro-4-(2-methoxyethoxy)benzamide (50 mg, 47.2 %) as a white powder. ¹H NMR (500 MHz, DMSO-*d*₆) δ ppm 7.80 and 7.72 (t, *J*=8.53 Hz, 1 H), 7.69 and 7.45 (br s, 1 H), 7.50 (d, J*=*8.53 Hz, 1 H), 7.36 (m, 3 H), 7.28 (m, 3 H), 7.20 (tt, *J*=7.43, 1.38 Hz, 1 H), 7.11 and 7.10 (br s, 1 H), 5.74 and 5.73 (br s, 1 H), 4.25 (m, 2 H), 3.70 and 3.68 (t, *J*=4.54 Hz, 2 H), 3.31 and 3.29 (s, 3 H), 3.25 and 3.14 (d, *J*=14.58 Hz, 1 H), 3.03 and 2.95 (d, *J*=13.48 Hz, 1 H), 2.14 (s, 3 H), 2.13 (s, 3 H)

### Example 33: 2-[2-chloro-5-(1-hydroxy-1-phenyl-2-pyrrolidin-1-yl-ethyl)phenyl]-3-fluoro-4-(2-methoxyethoxy)benzamide

### Step a: 1-(3-bromo-4-chloro-phenyl)-1-phenyl-2-pyrrolidin-1-yl-ethanol

The compound was prepared using the same procedure as detailed in example 1 step c starting from 2-(3-bromo-4-chloro-phenyl)-2-phenyl-oxirane (Ex 1, step b) (393 mg, 1.27 mmol, 1 eq.) and pyrrolidine (1 mL, 12.7 mmol, 10 eq.) giving 1-(3-bromo-4-chloro-phenyl)-1-phenyl-2-pyrrolidin-1-yl-ethanol (377.0 mg 78%) used directly in the next step.

### Step b: 2-[2-chloro-5-(1-hydroxy-1-phenyl-2-pyrrolidin-1-yl-ethyl)phenyl]-3-fluoro-4-(2-methoxyethoxy)benzonitrile

The compound was prepared using the same procedure as detailed in example 3? step d starting from 1-(3-bromo-4-chloro-phenyl)-1-phenyl-2-pyrrolidin-1-yl-ethanol (250 mg, 0.66 mmol, 1 eq.) and 3-fluoro-4-(2-methoxyethoxy)-2-(4,4,5,5-tetramethyl-1,3,2-dioxaborolan-2-yl)benzonitrile (Ex 6, step b) (633 mg, 1.97 mmol, 3 eq.) giving 2-[2-chloro-5-(1-hydroxy-1-phenyl-2-pyrrolidin-1-yl-ethyl)phenyl]-3-fluoro-4-(2-methoxyethoxy)benzonitrile (199.0 mg 61% yield) as a brown semisolid.

### Step c: 2-[2-chloro-5-(1-hydroxy-1-phenyl-2-pyrrolidin-1-yl-ethyl)phenyl]-3-fluoro-4-(2-methoxyethoxy)benzamide

The compound was prepared using the same procedure as detailed in example 2 step c starting from 2-[2-chloro-5-(1-hydroxy-1-phenyl-2-pyrrolidin-1-yl-ethyl)phenyl]-3-fluoro-4-(2-methoxyethoxy)benzonitrile (50.0 mg, 0.1 mmol, 1.0 eq.) giving 2-[2-chloro-5-(1-hydroxy-1-phenyl-2-pyrrolidin-1-yl-ethyl)phenyl]-3-fluoro-4-(2-methoxyethoxy)benzamide (18.5 mg 36% yield) as a pale yellow solid. ¹H NMR (400 MHz CDCl₃): δ 7.71 (d, J = 9.0 Hz, 1 H), 7.59 (ddd, J = 25.7, 8.5, 2.3 Hz, 1 H), 7.52 - 7.41 (m, 3 H), 7.29 (td, J = 7.7, 1.7 Hz, 2 H), 7.21 (dd, J = 7.5, 2.2 Hz, 1H), 7.09 (t, J = 8.3 Hz, 1 H), 5.14 (m, 2 H), 4.27 (t, J = 4.7 Hz, 2 H), 3.81 (td, J = 4.2, 1.1 Hz, 2 H), 3.46 (s, 3 H), 3.41 (m, 2 H), 2.45 (m, 4 H), 1.80 (m, 2 H), 1.60 (m, 2 H). mp: 90-95°C.

### Example 34: 2-[2-chloro-5-[1-hydroxy-1-phenyl-2-(1-piperidyl)ethyl]phenyl]-3-fluoro-4-(2-methoxyethoxy)benzamide

### Step a: 1-(3-bromo-4-chloro-phenyl)-1-phenyl-2-(1-piperidyl)ethanol

The compound was prepared using the same procedure as detailed in example 1 step c starting from 2-(3-bromo-4-chloro-phenyl)-2-phenyl-oxirane (Ex 1, step b) (393 mg, 1.27 mmol, 1 eq.) and piperidine (1.26 mL, 12.7 mmol, 10.0 eq.) giving 1-(3-bromo-4-chlorophenyl)-1-phenyl-2-(1-piperidyl)ethanol (438.0 mg 87%) used directly in the next step.

### Step b: 2-[2-chloro-5-[1-hydroxy-1-phenyl-2-(1-piperidyl)ethyl]phenyl]-3-fluoro-4-(2-methoxyethoxy)benzonitrile

The compound was prepared using the same procedure as detailed in example 3? step d starting from 1-(3-bromo-4-chloro-phenyl)-1-phenyl-2-(1-piperidyl)ethanol (275 mg, 0.697 mmol, 1 eq.) and 3-fluoro-4-(2-methoxyethoxy)-2-(4,4,5,5-tetramethyl-1,3,2-dioxaborolan-2-yl)benzonitrile (Ex 6, step b) (671 mg, 2.09 mmol, 3 eq.) giving 2-[2-chloro-5-[1-hydroxy-1-phenyl-2-(1-piperidyl)ethyl]phenyl]-3-fluoro-4-(2-methoxyethoxy)benzonitrile (95.0 mg 27% yield) as a beige solid.

### Step c: 2-[2-chloro-5-[1-hydroxy-1-phenyl-2-(1-piperidyl)ethyl]phenyl]-3-fluoro-4-(2-methoxyethoxy)benzamide

The compound was prepared using the same procedure as detailed in example 2 step c starting from 2-[2-chloro-5-[1-hydroxy-1-phenyl-2-(1-piperidyl)ethyl]phenyl]-3-fluoro-4-(2-methoxyethoxy)benzonitrile (94 mg, 0.18 mmol, 1 eq.) giving 2-[2-chloro-5-[1-hydroxy-1-phenyl-2-(1-piperidyl)ethyl]phenyl]-3-fluoro-4-(2-methoxyethoxy)benzamide (32 mg 33%) as a white solid. ¹H NMR (500 MHz CDCl₃): δ 7.72 (dd, J = 8.7, 1.6 Hz, 1H), 7.56 (ddd, J = 33.7, 8.4, 2.3 Hz, 1H), 7.51 - 7.40 (m, 3H), 7.28 (td, J = 7.7, 2.1 Hz, 2H), 7.18 (td, J = 7.4, 3.0 Hz, 1H), 7.09 (t, J = 8.3 Hz, 1H), 5.65 (br s, 1H), 5.11 (br s, 1H), 4.27 (t, J = 4.7 Hz, 2H), 3.80 (dt, J = 4.3, 2.5 Hz, 2H), 3.46 (s, 3H), 3.23 - 3.13 (m, 2H), 2.31 (q, J = 5.1 Hz, 4H), 1.45 (m, 4H), 1.38 - 1.33 (m, 2H). mp: 78-80 °C.

### Example 35: 2-[5-(2-amino-1-hydroxy-1-phenyl-ethyl)-2,4-dichloro-phenyl]-3-methyl-benzamide

### Step a: (5-bromo-2,4-dichloro-phenyl)-phenyl-methanone

The compound was prepared using the same procedure as detailed in example 10 step a starting from 5-bromo-2,4-dichlorobenzoic acid (500 mg, 1.85 mmol, 1 eq.) giving (5-bromo-2,4-dichloro-phenyl)-phenyl-methanone (587 mg, 96%) as a white powder. ¹H NMR (400 MHz, DMSO-*d*₆) δ ppm 8.02 (s, 2 H), 7.77 (m, 2 H), 7.73 (m, 1 H), 7.58 (m, 2 H).

### Step b: 2-(5-bromo-2,4-dichloro-phenyl)-2-phenyl-oxirane

The compound was prepared using the same procedure as detailed in example 1 step b starting from (5-bromo-2,4-dichloro-phenyl)-phenyl-methanone (582 mg, 1.76 mmol, 1 eq.) giving 2-(5-bromo-2,4-dichloro-phenyl)-2-phenyl-oxirane (710 mg, 99.4%) as a yellow oil. ¹H NMR (400 MHz, DMSO-*d*₆) δ ppm 8.02 (s, 1 H), 7.91 (s, 1 H), 7.34 (m, 3 H), 7.19 (m, 2 H), 3.43 (d, *J*=4.84 Hz, 1 H), 3.34 (d, *J*=4.84 Hz, 1 H).

### Step c: 2-amino-1-(5-bromo-2,4-dichloro-phenyl)-1-phenyl-ethanol

The compound was prepared using the same procedure as detailed in example 1 step c starting from 2-(5-bromo-2,4-dichloro-phenyl)-2-phenyl-oxirane (705 mg, 1.74 mmol, 1 eq.) giving 2-amino-1-(5-bromo-2,4-dichloro-phenyl)-1-phenyl-ethanol (445 mg, 60.1%) as a pale-yellow gum. ¹H NMR (400 MHz, DMSO-*d*₆) δ ppm 8.25 (s, 1 H), 7.62 (s, 1 H), 7.25 (m, 5 H), 5.94 (br s, 1 H), 3.57 (d, *J*=13.09 Hz, 1 H), 3.34 (d, *J*=13.20 Hz, 1 H).

### Step d: 2-[5-(2-amino-1-hydroxy-1-phenyl-ethyl)-2,4-dichloro-phenyl]-3-methyl-benzonitrile

The compound was prepared using the same procedure detailed in example 3? step d starting from 2-amino-1-(5-bromo-2,4-dichloro-phenyl)-1-phenyl-ethanol (289 mg, 0.680 mmol, 1 eq.) and 3-methyl-2-(4,4,5,5-tetramethyl-1,3,2-dioxaborolan-2-yl)benzonitrile (Ex2, step a) (496 mg, 2.04 mmol, 3 eq;) giving 2-[5-(2-amino-1-hydroxy-1-phenyl-ethyl)-2,4-dichloro-phenyl]-3-methyl-benzonitrile (122 mg, 21.2%) as a colorless gum. ¹H NMR (400 MHz, DMSO-*d*₆) δ ppm 7.89 and 7.87 (s, 1 H), 7.85 and 7.83 (m, 1 H), 7.75 and 7.73 (m, 1 H), 7.68 and 7.67 (s, 1 H), 7.58 (m, 2 H), 7.29 and 7.28 (m, 2 H), 7.23 (m, 1 H), 5.87 (br s, 1 H), 3.60 and 3.50 (d, *J*=13.09 Hz, 1 H), 3.33 and 3.27 (d, *J*=13.31 Hz, 1 H), 2.17 and 2.14 (s, 3 H).

### Step e: 2-[5-(2-amino-1-hydroxy-1-phenyl-ethyl)-2,4-dichloro-phenyl]-3-methyl-benzamide

The compound was prepared using the same procedure as detailed in example 2 step c starting from 2-[5-(2-amino-1-hydroxy-1-phenyl-ethyl)-2,4-dichloro-phenyl]-3-methyl-benzonitrile (117 mg, 0.138 mmol, 1 eq.) giving 2-[5-(2-amino-1-hydroxy-1-phenyl-ethyl)-2,4-dichloro-phenyl]-3-methyl-benzamide (10.0 mg, 17.4%) as a white powder. ¹H NMR (400 MHz, DMSO-*d*₆) δ ppm 7.72 (d, *J*=6.27 Hz, 1 H), 7.57 and 7.56 (br s, 1 H), 7.45 (d, *J*=3.63 Hz, 1 H), 7.40 (m, 3 H), 7.28 (m, 4 H), 7.21 (m, 1 H), 7.16 (br d, *J*=0.88 Hz, 1 H), 5.71 (br s, 1 H), 3.57 and 3.40 (d, *J*=13.20 Hz, 1 H), 3.17 and 3.26 (d, *J*=13.20 Hz, 1 H), 2.09 and 2.03 (s, 3 H).

### Example 36: 2-[5-(2-amino-1-hydroxy-1-phenyl-ethyl)-2-chloro-4-fluoro-phenyl]-3-methyl-benzamide

### Step a: (5-bromo-4-chloro-2-fluoro-phenyl)-phenyl-methanone

The compound was prepared using the same procedure as detailed in example 10 step a starting from 5-bromo-2-fluoro-4-chlorobenzoic acid (1.00 g, 3.95 mmol, 1 eq.) giving (5-bromo-4-chloro-2-fluoro-phenyl)-phenyl-methanone (1.14 g, 86.6%) as a brown powder. ¹H NMR (400 MHz, DMSO-*d*₆) δ ppm 8.01 (d, *J*=6.82 Hz, 1 H), 7.90 (d, *J*=9.46 Hz, 1 H), 7.81 (dt, *J*=8.39, 1.14 Hz, 2 H), 7.73 (m, 1 H), 7.58 (m, 2 H).

### Step b: 2-(5-bromo-4-chloro-2-fluoro-phenyl)-2-phenyl-oxirane

The compound was prepared using the same procedure as detailed in example 1 step b starting from (5-bromo-4-chloro-2-fluoro-phenyl)-phenyl-methanone (1.13 g, 3.39 mmol, 1 eq.) giving 2-(5-bromo-4-chloro-2-fluoro-phenyl)-2-phenyl-oxirane (1.12 g, 90.8%) as a yellow oil. ¹H NMR (400 MHz, DMSO-*d*₆) δ ppm 8.00 (d, *J*=7.04 Hz, 1 H), 7.75 (d, *J*=9.68 Hz, 1 H), 7.35 (m, 3 H), 7.23 (m, 2 H), 3.39 (d, *J*=4.95 Hz, 1 H), 3.31 (d, *J*=4.84 Hz, 1 H).

### Step c: 2-amino-1-(5-bromo-4-chloro-2-fluoro-phenyl)-1-phenyl-ethanol

The compound was prepared using the same procedure as detailed in example 1 step c starting from 2-(5-bromo-4-chloro-2-fluoro-phenyl)-2-phenyl-oxirane (1.12 g, 3.42 mmol, 1 eq.) giving 2-amino-1-(5-bromo-4-chloro-2-fluoro-phenyl)-1-phenyl-ethanol (634 mg, 50.6%) as a yellow powder. ¹H NMR (400 MHz, DMSO-*d*₆) δ ppm 8.04 (dd, *J*=7.65, 1.49 Hz, 1 H), 7.51 (dd, *J*=11.06, 1.71 Hz, 1 H), 7.32 (m, 4 H), 7.24 (m, 1 H), 5.99 (br s, 1 H), 3.37 (d, *J*=13.31 Hz, 1 H), 3.26 (d, *J*=13.20 Hz, 1 H).

### Step d: 2-[5-(2-amino-1-hydroxy-1-phenyl-ethyl)-2-chloro-4-fluoro-phenyl]-3-methyl-benzonitrile

The compound was prepared using the same procedure as detailed in example 3? step d starting from 2-amino-1-(5-bromo-4-chloro-2-fluoro-phenyl)-1-phenyl-ethanol (200.0 mg, 0.58 mmol, 1 eq.) and 3-methyl-2-(4,4,5,5-tetramethyl-1,3,2-dioxaborolan-2-yl)benzonitrile(Ex2, step a) (743 mg, 1.74 mmol, 3 eq.) giving 2-[5-(2-amino-1-hydroxy-1-phenyl-ethyl)-2-chloro-4-fluoro-phenyl]-3-methyl-benzonitrile (49.0 mg, 22.2%) as a pale-yellow solid. ¹H NMR (400 MHz, DMSO-*d*₆) δ ppm 7.81 (m, 1 H), 7.71 (m, 1 H), 7.68 (dd, *J*=8.36, 1.43 Hz, 1 H), 7.56 (m, 1 H), 7.52 and 7.49 (d, *J*=5.06 Hz, 1 H), 7.36 (m, 2 H), 7.30 (m, 2 H), 7.24 (m, 1 H), 5.87 (br s, 1 H), 3.39 and 3.33 (d, *J*=13.64 Hz, 1 H), 3.30 and 3.26 (d, *J*=13.53 Hz, 1 H), 2.11 and 2.09 (s, 3 H).

### Step e: 2-[5-(2-amino-1-hydroxy-1-phenyl-ethyl)-2-chloro-4-fluoro-phenyl]-3-methyl-benzamide

The compound was prepared using the same procedure as detailed in example 2 step c starting from 2-[5-(2-amino-1-hydroxy-1-phenyl-ethyl)-2-chloro-4-fluoro-phenyl]-3-methyl-benzonitrile (48.0 mg, 0.126 mmol, 1 eq.) giving 2-[5-(2-amino-1-hydroxy-1-phenyl-ethyl)-2-chloro-4-fluoro-phenyl]-3-methyl-benzamide (32 mg, 63.6%) as a white powder. ¹H NMR (500 MHz, DMSO-*d*₆) δ ppm 7.56 (m, 1 H), 7.50 (br s, 1 H), 7.41 -7.26 (m, 8 H), 7.22 (m, 1 H), 7.14 and 7.11 (br s, 1 H), 5.74 (br s, 1 H), 3.37 and 3.26 (br d, *J*=13.34 Hz, 1 H), 3.20 and 3.19 (d, *J*=13.07 Hz, 1 H), 2.02 and 2.00 (m, 3 H).

### Example 37a and 37b: rel-(2aS)-2-[6-chloro-3-[(1S)-2-(cyclohexylamino)-1-hydroxy-1-phenyl-ethyl]-2-fluoro-phenyl]-3-fluoro-4-(2-methoxyethoxy)benzamide and rel-(2aR)-2-[6-chloro-3-[(1S)-2-(cyclohexylamino)-1-hydroxy-1-phenyl-ethyl]-2-fluoro-phenyl]-3-fluoro-4-(2-methoxyethoxy)benzamide

### Step a: (1S)-1-(3-bromo-4-chloro-2-fluoro-phenyl)-2-(cyclohexylamino)-1-phenyl-ethanol

In a 5 mL flask under nitrogen, (1S)-2-amino-1-(3-bromo-4-chloro-2-fluoro-phenyl)-1-phenyl-ethanol (500 mg, 1.451 mmol, 1 eq.) was solubilized in MeOH (5 mL) and dried molecular sieves 4A was added. Cyclohexanone (165 µL, 1.59 mmol, 1.10 eq.) was added and the mixture was stirred at 35°C for 1h. The mixture was cooled. The reaction mixture was poured in a new flask under nitrogen to remove molecular sieves. NaBH₄ (165 mg, 4.3613 mmol, 3.006 eq.) was added and the reaction mixture was stirred at rt for 1h30. The reaction mixture was hydrolyzed with a saturated aqueous NaHCO₃ solution and MeOH was removed under reduced pressure. The residue was partitioned between water and EtOAc. The combined organic layer was washed with brine and dried over MgSO₄, filtered and concentrated in vacuo to give 670mg of crude product. The crude was purified by chromatography (Column Biotage HC 25g (20µm), Flow rate : 18 ml/min, Eluate A : cyclohexane eluate B: EtOAc, Gradient :A/B : 100/0 (iso) on 1CV, then go to 70/30 in 10CV and 70/30 (iso) on 6CV) to give (1S)-1-(3-bromo-4-chloro-2-fluoro-phenyl)-2-(cyclohexylamino)-1-phenyl-ethanol (452 mg, 61.3%) as a colorless oil used directly in next step.

### Step b: tert-butyl N-[(2S)-2-(3-bromo-4-chloro-2-fluoro-phenyl)-2-hydroxy-2-phenyl-ethyl]-N-cyclohexyl-carbamate

The compound was prepared using the same procedure as detailed in example 17 step d starting from (1S)-1-(3-bromo-4-chloro-2-fluoro-phenyl)-2-(cyclohexylamino)-1-phenyl-ethanol (450 mg, 1.05 mmol, 1 eq.) giving tert-butyl N-[(2S)-2-(3-bromo-4-chloro-2-fluorophenyl)-2-hydroxy-2-phenyl-ethyl]-N-cyclohexyl-carbamate (462 mg, 83.2%) as a colorless oil. LC/MS (method A): Rt = 2.76 min (94%)

### Step c: tert-butyl N-[(2S)-2-[4-chloro-3-[6-cyano-2-fluoro-3-(2-methoxyethoxy)phenyl]-2-fluoro-phenyl]-2-hydroxy-2-phenyl-ethyl]-N-cyclohexyl-carbamate

The compound was prepared using the same procedure as detailed in example 19a step d starting from tert-butyl N-[(2S)-2-(3-bromo-4-chloro-2-fluoro-phenyl)-2-hydroxy-2-phenylethyl]-N-cyclohexyl-carbamate (460 mg, 0.87 mmol, 1 eq.) and 3-fluoro-4-(2-methoxyethoxy)-2-trimethylstannyl-benzonitrile (625 mg, 1.74 mmol, 1.99 eq.) giving tert-butyl N-[(2S)-2-[4-chloro-3-[6-cyano-2-fluoro-3-(2-methoxyethoxy)phenyl]-2-fluoro-phenyl]-2-hydroxy-2-phenylethyl]-N-cyclohexyl-carbamate (316 mg, 56.4%) as a colorless gum, LC/MS (method A): Rt = 2.46 min (77%), used directly in the next step.

### Step d: tert-butyl N-[(2S)-2-[3-[6-carbamoyl-2-fluoro-3-(2-methoxyethoxy)phenyl]-4-chloro-2-fluoro-phenyl]-2-hydroxy-2-phenyl-ethyl]-N-cyclohexyl-carbamate

The compound was prepared using the same procedure as detailed in example 2 step c starting from tert-butyl N-[(2S)-2-[4-chloro-3-[6-cyano-2-fluoro-3-(2-methoxyethoxy)phenyl]-2-fluoro-phenyl]-2-hydroxy-2-phenyl-ethyl]-N-cyclohexyl-carbamate (200 mg, 0.32 mmol, 1 eq.) giving tert-butyl N-[(2S)-2-[3-[6-carbamoyl-2-fluoro-3-(2-methoxyethoxy)phenyl]-4-chloro-2-fluoro-phenyl]-2-hydroxy-2-phenyl-ethyl]-N-cyclohexyl-carbamate (160 mg, 77.8%) as a white solid. Purification by LC/MS (method E) gave a mixture of atropoisomers and rotamers 64% (Rt : 6.94 min) and 36 % (Rt = 7.13 min).

### Step e: rel-(2aS)-2-[6-chloro-3-[(1R)-2-(cyclohexylamino)-1-hydroxy-1-phenyl-ethyl]-2-fluorophenyl]-3-fluoro-4-(2-methoxyethoxy)benzamide and rel-(2aR)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide

The compounds were prepared using the same procedure as detailed in example 17 step g starting from tert-butyl N-[(2S)-2-[3-[6-carbamoyl-2-fluoro-3-(2-methoxyethoxy)phenyl]-4-chloro-2-fluoro-phenyl]-2-hydroxy-2-phenyl-ethyl]-N-cyclohexyl-carbamate (160 mg, 0.24 mmol, 1 eq.) giving a mixture of atropoisomers (160 mg). The two atropoisomers were separated by chromatography (Column: Kinetex 150x30 mm, 5µm, 100 A, Flowrate: 30 mL/min,Mobile phase: H2O + 0.1% Acetic Acid / MeOH + 0.1% Acetic Acid, Temperature: 45 °C, Gradient: B 20% to 95% in 30 min) to give 37a, rel-(2aS)-2-[6-chloro-3-[(1S)-2-(cyclohexylamino)-1-hydroxy-1-phenyl-ethyl]-2-fluoro-phenyl]-3-fluoro-4-(2-methoxyethoxy)benzamide (32.0 mg, 23.6%) as a white powder. ¹H NMR (500 MHz, DMSO-*d*₆) δ ppm 7.68 (t, *J*=8.53 Hz, 1 H), 7.64 (br s, 1 H), 7.49 (dd, *J*=8.67, 0.83 Hz, 1 H), 7.37 (d, *J*=7.70 Hz, 2 H), 7.34 (d, *J*=8.67 Hz, 1 H), 7.27 (m, 3 H), 7.19 (tt, *J*=7.29, 1.10 Hz, 1 H), 7.07 (br s, 1 H), 5.83 (br s, 1 H), 4.25 (m, 2 H), 3.69 (m, 2 H), 3.31 (s, 3 H), 3.28 (d, *J* =12.10 Hz, 1 H), 3.17 (d, *J*=12.10 Hz, 1 H), 2.24 (m, 1 H), 1.73 (m, 1 H), 1.67 (m, 1 H), 1.59 (m, 2 H), 1.48 (m, 1 H), 1.42 (br s, 1 H), 1.11 (m, 3 H), 0.94 (m, 2 H). [α]_{d}^{20.7} = -43° (C=0.346, DCM), LC/MS (method A) Rt = 0.98 min (100%). And **37b,** rel-(2aR)- 2-[6-chloro-3-[(1S)-2-(cyclohexylamino)-1-hydroxy-1-phenyl-ethyl]-2-fluoro-phenyl]-3-fluoro-4-(2-methoxyethoxy)benzamide (24.0 mg, 17.7%) as a white powder. ¹H NMR (500 MHz, DMSO-*d*₆) δ ppm 7.76 (t, *J*=8.46 Hz, 1 H), 7.60 (br s, 1 H), 7.50 (d, *J*=7.98 Hz, 1 H), 7.35 (m, 3 H), 7.27 (m, 3 H), 7.19 (tt, *J*=7.29, 1.10 Hz, 1 H), 7.03 (br s, 1 H), 5.70 (br s, 1 H), 4.24 (m, 2 H), 3.68 (t, *J*=4.47 Hz, 2 H), 3.35 (d, *J*=12.1 Hz, 1 H), 3.30 (s, 3 H), 3.20 (d, *J*=12.1 Hz, 1 H), 2.32 (m, 1 H), 1.78 (m, 2 H), 1.62 (m, 2 H), 1.51 (m, 1 H), 1.34 (br s, 1 H), 1.15 (m, 3 H), 0.99 (m, 2 H). [α]_{d}^{20.7} = -31° (C=0.331, DCM), LC/MS (method A) Rt = 1.04 min (98.9%).

### Example 38a and 38b: rel-(2aR)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide and rel-(2aS)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide

Step a: (1R)-1-(3-bromo-4-chloro-2-fluoro-phenyl)-2-(cyclohexylamino)-1-phenyl-ethanol The compound was prepared using the same procedure as detailed in example 37a step a starting from (1R)-2-amino-1-(3-bromo-4-chloro-2-fluoro-phenyl)-1-phenyl-ethanol (Ex 28a, step b) (520 mg, 1.51 mmol, 1 eq.) and cyclohexanone (172 µL, 1.66 mmol, 1.10 eq.) giving (1R)-1-(3-bromo-4-chloro-2-fluoro-phenyl)-2-(cyclohexylamino)-1-phenyl-ethanol (413 mg, 53.9%) as a colorless oil. ¹H NMR (400 MHz, DMSO-*d*₆) δ ppm 7.79 (t, *J*=8.42 Hz, 1 H), 7.50 (dd, *J*=8.64, 1.49 Hz, 1 H), 7.35 (m, 2 H), 7.29 (m, 2 H), 7.22 (m, 1 H), 5.93 (br s, 1 H), 3.44 (d, *J*=11.33 Hz, 1 H), 3.21 (d, *J*=11.55 Hz, 1 H), 2.32 (m, 1 H), 1.76 (m, 2 H), 1.61 (m, 2 H), 1.49 (m, 2 H), 1.18 (m, 2 H), 0.95 (m, 2 H).

### Step b: tert-butyl N-[(2R)-2-(3-bromo-4-chloro-2-fluoro-phenyl)-2-hydroxy-2-phenyl-ethyl]-N-cyclohexyl-carbamate

The compound was prepared using the same procedure as detailed in example 17 step d starting from (1R)-1-(3-bromo-4-chloro-2-fluoro-phenyl)-2-(cyclohexylamino)-1-phenyl-ethanol (413 mg, 0.968 mmol, 1 eq.) giving tert-butyl N-[(2R)-2-(3-bromo-4-chloro-2-fluorophenyl)-2-hydroxy-2-phenyl-ethyl]-N-cyclohexyl-carbamate (447 mg, 87.7%) as a colorless gum. LC/MS (method A) Rt = 2.77 min (94%).

### Step c: tert-butyl N-[(2R)-2-[4-chloro-3-[6-cyano-2-fluoro-3-(2-methoxyethoxy)phenyl]-2-fluoro-phenyl]-2-hydroxy-2-phenyl-ethyl]-N-cyclohexyl-carbamate

The compound was prepared using the same procedure detailed in example 19a step d starting from tert-butyl N-[(2R)-2-(3-bromo-4-chloro-2-fluoro-phenyl)-2-hydroxy-2-phenylethyl]-N-cyclohexyl-carbamate (447 mg, 0.848 mmol, 1 eq.) and 3-fluoro-4-(2-methoxyethoxy)-2-trimethylstannyl-benzonitrile (Ex 19a, step c) (607 mg, 1.70 mmol, 2 eq.) giving tert-butyl N-[(2R)-2-[4-chloro-3-[6-cyano-2-fluoro-3-(2-methoxyethoxy)phenyl]-2-fluorophenyl]-2-hydroxy-2-phenyl-ethyl]-N-cyclohexyl-carbamate (337 mg, 61.9% ) as a pale-yellow oil. LC/MS (method A) Rt = 2.47 min (79%).

### Step d: tert-butyl N-[(2R)-2-[3-[6-carbamoyl-2-fluoro-3-(2-methoxyethoxy)phenyl]-4-chloro-2-fluoro-phenyl]-2-hydroxy-2-phenyl-ethyl]-N-cyclohexyl-carbamate

The compound was prepared using the same procedure detailed in example 2 step c starting from tert-butyl N-[(2R)-2-[4-chloro-3-[6-cyano-2-fluoro-3-(2-methoxyethoxy)phenyl]-2-fluorophenyl]-2-hydroxy-2-phenyl-ethyl]-N-cyclohexyl-carbamate (215 mg, 0.335 mmol, 1 eq.) giving tert-butyl N-[(2R)-2-[3-[6-carbamoyl-2-fluoro-3-(2-methoxyethoxy)phenyl]-4-chloro-2-fluoro-phenyl]-2-hydroxy-2-phenyl-ethyl]-N-cyclohexyl-carbamate (165 mg, 74.6%) as a white gum. Purification by LC/MS (method E) gave a mixture of atropoisomers and rotamers 65% (Rt: 6.97 min) and 31% (Rt = 7.16 min).

### Step e: rel-(2aR)-2-[6-chloro-3-[(1R)-2-(cyclohexylamino)-1-hydroxy-1-phenyl-ethyl]-2-fluorophenyl]-3-fluoro-4-(2-methoxyethoxy)benzamide and rel-(2aS)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide

The compounds were prepared using the same procedure as detailed in example 17 step g starting from tert-butyl N-[(2R)-2-[3-[6-carbamoyl-2-fluoro-3-(2-methoxyethoxy)phenyl]-4-chloro-2-fluoro-phenyl]-2-hydroxy-2-phenyl-ethyl]-N-cyclohexyl-carbamate (165 mg, 0.25 mmol, 1 eq.) giving **38a** rel-(2aR)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide (47.5 mg, 33.9%) as a white powder. ¹H NMR (500 MHz, DMSO-*d*₆) δ ppm 7.67 (d, *J*=8.53 Hz, 1 H), 7.64 (br s, 1 H), 7.49 (dd, *J*=8.60, 0.89 Hz, 1 H), 7.37 (d, *J*=7.70 Hz, 2 H), 7.34 (d, *J*=8.67 Hz, 1 H), 7.27 (m, 3 H), 7.19 (tt, *J*=7.29, 1.10 Hz, 1 H), 7.06 (br s, 1 H), 5.84 (br s, 1 H), 4.25 (m, 2 H), 3.69 (t, *J*=4.54 Hz, 2 H), 3.31 (s, 3 H), 3.29 (d, *J*=12.2 Hz, 1 H), 3.17 (d, *J*=12.38 Hz, 1 H), 2.24 (m, 1 H), 1.74 (m, 1 H), 1.67 (m, 1 H), 1.59 (m, 2 H), 1.48 (m, 1 H), 1.42 (br s, 1 H), 1.10 (m, 3 H), 0.94 (m, 2 H). [α]_{d}^{20.7} = +43° (C=0.322, DCM), LC/MS (method A) Rt = 0.99 min (100%). And **38b** rel-(2aS)-2-[6-chloro-3-[(1R)-2-(cyclohexylamino)-1-hydroxy-1-phenylethyl]-2-fluoro-phenyl]-3-fluoro-4-(2-methoxyethoxy)benzamide (25.0 mg, 17.9%) as a white powder. ¹H NMR (500 MHz, DMSO-*d*₆) δ ppm 7.76 (t, *J*=8.53 Hz, 1 H), 7.60 (br s, 1 H), 7.50 (dd, *J*=8.67, 0.96 Hz, 1 H), 7.35 (m, 3 H), 7.27 (m, 3 H), 7.19 (tt, *J*=7.29, 1.10 Hz, 1 H), 7.03 (br s, 1 H), 5.70 (br s, 1 H), 4.24 (m, 2 H), 3.68 (t, *J*=4.47 Hz, 2 H), 3.35 (d, *J*=10.7 Hz, 1 H), 3.30 (s, 3 H), 3.20 (d, *J*=10.73 Hz, 1 H), 2.32 (m, 1 H), 1.78 (m, 2 H), 1.62 (m, 2 H), 1.51 (m, 1 H), 1.34 (br s, 1 H), 1.14 (m, 3 H), 0.99 (m, 2 H). [α]_{d}^{20.9} = +28.1° (C=0.361, DCM), LC/MS (method A) Rt = 1.05 min (97.3%).

### Example 39: 2-[6-chloro-2-fluoro-3-[1-hydroxy-2-(methylamino)-1-phenyl-ethyl]phenyl]-3-fluoro-4-(5-methylisoxazol-3-yl)oxy-benzamide

### Step a: 2-bromo-3-fluoro-4-(5-methylisoxazol-3-yl)oxy-benzonitrile

At 0°C, in a 250ml flask, NaH (60% in oil) (23 mg, 0.5751 mmol, 1 eq.) was added to a solution of 5-methylisoxazol-3-ol (45 mg, 0.454 mmol, 1 eq.) in DMF (1.3 mL) under Ar. After 5 min, 2-bromo-3,4-difluorobenzonitrile (0.1 g, 0.5 mmol, 1 eq.) was added and the reaction mixture was stirred at 0°C for 30min. The reaction mixture was stirred at rt for 18h. The reaction mixture was quenched with a saturated solution of NH₄Cl and extracted with EtOAc (three times). The combined organic layers were washed with brine and dried over anhydrous Na₂SO₄. After filtration, the filtrate was concentrated under reduced pressure. The crude product was purified by chromatography (Column Biotage HC 10g (20µm), Flow : 30 ml/mn, Eluate A :cyclohexane eluate B: EtOAc, Gradient :A/B : 100/0 (iso) 3CV, then go to 90/10 in 4CV 90:10 (iso) 5cv then go to 80/20 in 3CV and 80/20 (iso) 15CV) to give 2-bromo-3-fluoro-4-(5-methylisoxazol-3-yl)oxy-benzonitrile (77 mg, 60%) as a white solid. ¹H NMR (500 MHz, DMSO-*d*₆) δ ppm 7.92 (dd, *J*=8.67, 1.65 Hz, 1 H), 7.71 (t, *J*=8.12 Hz, 1 H), 6.37 (s, 1 H), 2.40 (s, 3 H).

### Step b: 3-fluoro-4-(5-methylisoxazol-3-yl)oxy-2-trimethylstannyl-benzonitrile

The compound was prepared using the same procedure as detailed in example 19a step c starting from 2-bromo-3-fluoro-4-(5-methylisoxazol-3-yl)oxy-benzonitrile (244 mg, 0.821 mmol, 1 eq.) giving 3-fluoro-4-(5-methylisoxazol-3-yl)oxy-2-trimethylstannyl-benzonitrile (173 mg, 55.2%) as a colorless oil. ¹H NMR (400 MHz, DMSO-*d*₆) 6 ppm 7.75 (dd, *J*=8.31, 0.61 Hz, 1 H), 7.56 (t, *J*=8.47 Hz, 1 H), 6.28 (q, *J*=0.81 Hz, 1 H), 2.39 (d, *J*=0.88 Hz, 3 H), 0.48 (s, 9 H).

### Step c: tert-butyl N-[2-[4-chloro-3-[6-cyano-2-fluoro-3-(5-methylisoxazol-3-yl)oxy-phenyl]-2-fluoro-phenyl]-2-hydroxy-2-phenyl-ethyl]-N-methyl-carbamate

The compound was prepared using the same procedure as detailed in example 19a step d starting from tert-butyl N-[2-(3-bromo-4-chloro-2-fluoro-phenyl)-2-hydroxy-2-phenyl-ethyl]-N-methyl-carbamate (Ex 32, step a) (139 mg, 0.302 mmol, 1 eq.) and 3-fluoro-4-(5-methylisoxazol-3-yl)oxy-2-trimethylstannyl-benzonitrile (173 mg, 0.4541 mmol, 1.5 eq.) giving tert-butyl N-[2-[4-chloro-3-[6-cyano-2-fluoro-3-(5-methylisoxazol-3-yl)oxy-phenyl]-2-fluorophenyl]-2-hydroxy-2-phenyl-ethyl]-N-methyl-carbamate (113 mg, 62.5%) as a brown foam used directly in the next step.

### Step d: tert-butyl N-[2-[3-[6-carbamoyl-2-fluoro-3-(5-methylisoxazol-3-yl)oxy-phenyl]-4-chloro-2-fluoro-phenyl]-2-hydroxy-2-phenyl-ethyl]-N-methyl-carbamate

The compound was prepared using the same procedure as detailed in example 2 step c starting from tert-butyl N-[2-[4-chloro-3-[6-cyano-2-fluoro-3-(5-methylisoxazol-3-yl)oxyphenyl]-2-fluoro-phenyl]-2-hydroxy-2-phenyl-ethyl]-N-methyl-carbamate (145 mg, 0.243 mmol, 1 eq.) giving tert-butyl N-[2-[3-[6-carbamoyl-2-fluoro-3-(5-methylisoxazol-3-yl)oxyphenyl]-4-chloro-2-fluoro-phenyl]-2-hydroxy-2-phenyl-ethyl]-N-methyl-carbamate (134 mg, 89.7%) as a white foam, used directly in the next step.

### Step e: 2-[6-chloro-2-fluoro-3-[1-hydroxy-2-(methylamino)-1-phenyl-ethyl]phenyl]-3-fluoro-4-(5-methylisoxazol-3-yl)oxy-benzamide

The compound was prepared using the same procedure as detailed in example 17 step g starting from tert-butyl N-[2-[3-[6-carbamoyl-2-fluoro-3-(5-methylisoxazol-3-yl)oxy-phenyl]-4-chloro-2-fluoro-phenyl]-2-hydroxy-2-phenyl-ethyl]-N-methyl-carbamate (130 mg, 0.211 mmol, 1 eq.) giving 2-[6-chloro-2-fluoro-3-[1-hydroxy-2-(methylamino)-1-phenyl-ethyl]phenyl]-3-fluoro-4-(5-methylisoxazol-3-yl)oxy-benzamide (8 mg, 7.4%) as a white solid. ¹H NMR (500 MHz, DMSO-*d*₆) δ ppm 7.86 and 7.81 (br s, 1 H), 7.81 and 7.70 (t, *J*=8.39 Hz, 1 H), 7.61 and 7.60 (t, *J*=8.08 Hz, 1 H), 7.54 and 7.53 (d, *J*=3.85 Hz, 1 H), 7.40 and 7.38 (m, 1 H), 7.35 and 7.34 (m, 2 H), 7.28 (m, 3 H), 7.20 and 7.19 (m, 1 H), 6.24 and 6.22 (d, *J*=0.83 Hz, 1 H), 3.29 and 3.20 (m, 2 H), 3.18 and 3.16 (m, 1 H), 2.38 and 2.37 (d, *J*=0.69 Hz, 3 H), 2.28 and 2.22 (s, 3 H).

### Example 40 : 2-[5-(2-amino-1-hydroxy-1-pyrimidin-2-yl-ethyl)-2-chloro-phenyl]-3-fluoro-4-(2-methoxyethoxy)benzamide

### Step a : N-methoxy-N-methyl-pyrimidine-2-carboxamide

In 500mL round bottom flask, pyrimidine-2-carboxylic acid (3.0 g, 24 mmol, 1 eq.), triethylamine (3500 µL, 25.2 mmol, 1 eq.), HOBt (980 mg, 7.25 mmol, 0.3 eq.) and EDCI (5.6 g, 29 mmol, 1.2 eq.) were dissolved in dichloromethane (130 mL). The solution was stirred at rt for 10 min. Then, O,N dimethyl hydroxylamine hydrochloride (2.5 g, 26 mmol, 1.1 eq.) was added and the reaction mixture was stirred at rt over night. Water was added and the aqueous layer was basified to pH=7 with a solution of NaHCO3 sat and extracted two times with dichloromethane. The combined organic layers were dried on MgSO4, filtered and concentrated under vacuum to give 4.38g of crude. The crude product was purified by chromatography (Cartridge Biotage Sfär HC 50g / 20µm silica, cyclohexane/EtOAc , flow rate 100ml/min) to give N-methoxy-N-methyl-pyrimidine-2-carboxamide (1.59 g, 39 %) as a yellow oil. ¹H NMR (400 MHz, DMSO-*d*₆) δ ppm 8.90 (d, *J*=4.95 Hz, 2 H), 7.61 (t, *J*=4.95 Hz, 1 H), 3.58 (s, 3 H), 3.28 (m, H)

### Step b : (3-bromo-4-chloro-phenyl)-pyrimidin-2-yl-methanone

In a 2-neckround bottom flask, flame dried, under argon, 1-bromo-2-chloro-5-iodobenzene (A, 2.95 g, 9.30 mmol, 1 eq.)was dissolved in tetrahydrofuran (3890 µL). The solution was cooled at 0°C. Then, isopropylmagnesium chloride lithium chloride complex (7150 µL, 9.30 mmol, 1 eq.) was add dropwise. The reaction mixture was stirred at -70°C. In an other 3-neckround bottom flask, flame dried, under argon, N-methoxy-N-methyl-pyrimidine-2-carboxamide (777 mg, 4.65 mmol, 1.00 eq.) was solubilized in tetrahydrofuran (7770 µL). The mixture was cooled at -70°C. The Grignard solution was add dropwise to the second solution and the reaction mixture was stirred at rt over night. A solution of NH₄Cl sat was added and the aqueous layer was extracted two times with EtOAc. The combined organic layers were dried on MgSO4, filtered and concentrated under vacuum to give : 2.24g of crude. The crude product was purified by chromotagraphy (Cartridge Biotage Sfär HC 50g / 20µm silica, Cyclohexane-EtOAc,
flow rate 100ml/min) to give (3-bromo-4-chloro-phenyl)-pyrimidin-2-yl-methanone (617 mg, 1.91 mmol, 41. %) as an off white powder. ¹H NMR (400 MHz, DMSO-*d*₆) δ ppm 9.05 (d, *J*=4.95 Hz, 2 H), 8.29 (d, *J*=1.76 Hz, 1 H), 7.94 (dd, *J*=8.36, 1.98 Hz, 1 H), 7.83 (d, *J*=8.36 Hz, 1 H), 7.78 (t, *J*=4.95 Hz, 1 H).

### Step c : 2-[2-(3-bromo-4-chloro-phenyl)oxiran-2-yl]pyrimidine

The compound was prepared using the same procedure detailed in example 1 step b starting from (3-bromo-4-chloro-phenyl)-pyrimidin-2-yl-methanone (1.139 g, 3.66 mmol, 1 eq.) giving 2-[2-(3-bromo-4-chloro-phenyl)oxiran-2-yl]pyrimidine (1.04 g, 79.3 %) as an orange oil. ¹H NMR (400 MHz, DMSO-*d*₆) δ ppm 8.83 (d, *J*=4.84 Hz, 2 H), 7.78 (d, *J*=1.87 Hz, 1 H), 7.64 (d, *J*=8.25 Hz, 1 H), 7.52 (t, *J*=4.90 Hz, 1 H), 7.43 (dd, *J*=8.25, 1.98 Hz, 1 H), 3.79 (d, *J*=5.94 Hz, 1 H), 3.24 (d, *J*=5.94 Hz, 1 H).

### Step d : 2-amino-1-(3-bromo-4-chloro-phenyl)-1-pyrimidin-2-yl-ethanol

The compound was prepared using the same procedure detailed in example 1 step c starting from 2-[2-(3-bromo-4-chloro-phenyl)oxiran-2-yl]pyrimidine (1.04 g, 2.90 mmol, 1.0 eq.) giving 2-amino-1-(3-bromo-4-chloro-phenyl)-1-pyrimidin-2-yl-ethanol (415 mg, 42.2 %) as a yellow oil. ¹H NMR (400 MHz, DMSO-*d*₆) δ ppm 8.85 (d, *J*=4.95 Hz, 2 H), 7.92 (d, *J*=2.42 Hz, 1 H), 7.53 (m, 2 H), 7.44 (t, *J*=4.90 Hz, 1 H), 6.04 (br s, 1 H), 3.45 (d, *J*=13.09 Hz, 1 H), 3.09 (d, *J*=13.09 Hz, 1 H).

### Step e : 2-[5-(2-amino-1-hydroxy-1-pyrimidin-2-yl-ethyl)-2-chloro-phenyl]-3-fluoro-4-(2-methoxyethoxy)benzonitrile

The compound was prepared using the same procedure detailed in example 2 step b starting from 2-amino-1-(3-bromo-4-chloro-phenyl)-1-pyrimidin-2-yl-ethanol (300 mg, 0.913 mmol, 1.0 eq.) and 3-fluoro-4-(2-methoxyethoxy)-2-(4,4,5,5-tetramethyl-1,3,2-dioxaborolan-2-yl)benzonitrile (880 mg, 2.74 mmol, 3.0 eq.) giving 2-[5-(2-amino-1-hydroxy-1-pyrimidin-2-yl-ethyl)-2-chloro-phenyl]-3-fluoro-4-(2-methoxyethoxy)benzonitrile (48 mg, 0.11 mmol, 12 %) as a white powder. ¹H NMR (400 MHz, DMSO-*d*₆) δ ppm 8.86 and 8.85 (d, *J*=1.65 Hz, 2 H), 7.81 and 7.79 (dd, *J*=4.57, 1.49 Hz, 1 H), 7.74 and 7.66 (dd, *J*=8.53, 2.26 Hz, 1 H), 7.63 (m, 1 H), 7.60 (m, 1 H), 7.45 (m, 2 H), 4.34 (m, 2 H), 3.71 (td, *J*=4.40, 1.65 Hz, 2 H), 3.62 and 3.61 (d, *J*=13.09 Hz, 1 H), 3.31 and 3.30 (s, 3H), 3.18 and 3.15 (d, *J*=13.09 Hz, 1 H).

### Step f : 2-[5-(2-amino-1-hydroxy-1-pyrimidin-2-yl-ethyl)-2-chloro-phenyl]-3-fluoro-4-(2-methoxyethoxy)benzamide

The compound was prepared using the same procedure detailed in example 2 step c starting from 2-[5-(2-amino-1-hydroxy-1-pyrimidin-2-yl-ethyl)-2-chloro-phenyl]-3-fluoro-4-(2-methoxyethoxy)benzonitrile (48 mg, 0.11 mmol, 1.0 eq.) giving 2-[5-(2-amino-1-hydroxy-1-pyrimidin-2-yl-ethyl)-2-chloro-phenyl]-3-fluoro-4-(2-methoxyethoxy)benzamide (32 mg, 63 %) as a white powder.¹H NMR (500 MHz, DMSO-*d*₆) δ ppm 8.85 and 8.83(d, *J*=4.95 Hz, 1 H), 7.63 (dd, *J*=8.46, 2.27 Hz, 0.5 H), 7.52 (d, *J*=2.20 Hz, 0.5 H), 7.53 and 7.47 (br s, 1 H), 7.41 (m, 4 H), 7.26 (td, *J*=8.39, 3.03 Hz, 1 H), 7.11 and 7.03 (br s, 1 H), 5.88 and 5.87 (br s, 1 H), 4.24 (m, 2 H), 3.69 (q, *J*=4.08 Hz, 2 H), 3.42 and 3.38 (d, *J*=13.34 Hz, 1 H), 3.31 and 3.31 (s, 3 H), 3.09 and 3.01 (d, *J*=13.20 Hz, 1 H)

### In-vitro Biochemical assay

The assay is based on a TR-FRET sandwich immunoassay using 6His-humanYAP⁶⁰⁻¹⁰⁰ and FLAG-AVI-HumanTEAD4²¹⁷⁴³⁴. TEAD4 protein was produced using a process based on *Hau et al.,* ChemBioChem 2013, 14(10): 1218-1225. YAP was obtained via peptide synthesis. The compounds were dissolved at 50mM in 100% DMSO and serial dilutions were made in 100% DMSO. The diluted compound solutions were incubated at the same time than 6His-humanYAP⁶⁰⁻¹⁰⁰ (1.5nM in reaction buffer) and ⁻ FLAG-AVI-HumanTEAD4²¹⁷⁻⁴³⁴ (1.5nM in reaction buffer) in white 384-well plates (Greiner). The final DMSO concentrations in the assay was 1%. The mix reaction was incubated for 120 min at room temperature. After 120 min of incubation, anti-His Europium cryptate labelled antibody and Streptavidin d2 were added to the reaction mix for 120 min more at room temperature. The plate was read with an Envision microplate reader. The data analyses were carried out by using the TR-FRET module. Two sequential measurements were carried out: at 620nm for the cryptate emission and at 665nm for the signal emitted by the d2 acceptor. The ratio of the two fluorescence intensities 665/620 enables the calculation of Delta F (%) which represents the relative energy transfer rate for each sample. The IC₅₀ values were estimated by fitting the data by nonlinear fit regression (GraphPad Prism).

The results are presented in Table 1.

**Table 1**

| **Example** | **FRET IC₅₀ (µM)** | **Example** | **FRET IC₅₀ (µM)** |
|---|---|---|---|
| 1 | >200 | 20a | 183 |
| 1a | 161.2 | 20b | >200 |
| 1b | 676.2 | 21 | 5.7 |
| 2 | 75.9 | 22a | 8.7 |
| 2a | 11.3 | 22b | 82.2 |
| 2b | >200 | 23a | 82.7 |
| 3 | 70.1 | 23b | 4.2 |
| 4 | 189.2 | 24 | 50.2 |
| 4a | 111.5 | 25 | 83 |
| 4b | >200 | 26 | 12.8 |
| 5 | 80.4 | 27 | 24.2 |
| 6 | 36.7 | 28a | >200 |
| 7 | 18 | 28b | 5.2 |
| 8 | 53.3 | 29a | >200 |
| 9a | >200 | 29b | 19.8 |
| 9b | 87.2 | 30 | 7.4 |
| 10 | 186.8 | 31 | 17.8 |
| 11 | 93.8 | 32 | >100 |
| 12 | 171.4 | 33 | >100 |
| 13 | 112.1 | 34 | >100 |
| 14 | 59.8 | 35 | >100 |
| 15 | 45.6 | 36 | 181.5 |
| 16a | 105.5 | 37a | >100 |
| 16b | 14.8 | 37b | >20 |
| 17 | 78.3 | 38a | >100 |
| 18 | 4.8 | 38b | 1.4 |
| 19a | >200 | 39 | 12 |
| 19b | 73.6 | 40 | 15 |

| | | | |
|---|---|---|---|
| nd = not determined | | | |

### Inhibition of malignant mesothelioma tumor cell growth

The tumor cell growth inhibitory activity of the compounds of formula (I) was evaluated in NCI-H2052 mesothelioma cell line harboring a NF2 mutation. 4,000 cells/well were plated in a 96-well black plate with clear flat bottom TC-Treated Imaging plate (Greiner Bio one #655090) in regular medium (as suggested from ATCC for each cell line) with serum, which was replaced the day after with a starvation medium containing 1 % serum. After one day growth in the starvation medium, cells were incubated with the test compounds. The starting concentration was 30 µM and serial dilutions in DMSO and medium were performed until 0.1µM to achieve a final DMSO concentration of 0.5%. The cells were then allowed to grow for 3 days, and then, EdU (Invitrogen, Molecular Probe) was added in each well at a final concentration of 10 µM and the cells were returned to the incubator for an additional 24h. The starvation medium was removed and 100 µL of PFA 4% containing Hoechst dye was adding in each well to fix the cells. Plates were then incubated at rt for 15 min, washed twice with PBS and the cells were permeabilized by adding 100 µL per well of triton-100 0.3%. After 20 min the cells were washed with PBS and EdU detection was performed according to the instructions of the manufacturer. Image acquisition was performed using the ImageXpress Micro and analyzed using the MetaXpress software (Molecular Device). Results were expressed as a percent of inhibition (%) of the cell proliferation values obtained with 0.5 % DMSO treatment alone. The cellular response was determined by fitting the concentration response curves using three parameters curve fit equation and determining the concentration that inhibited cell growth between 50% and 100%. The results are presented in Table 2.

**Table 2**

| **Example** | **Prolif H2052 IC50 (µM)** |
|---|---|
| **16b** | **7.1** |
| **18** | **2.2** |
| **21** | **3.2** |
| **22a** | **7.3** |
| **23b** | **4.1** |
| **27** | **7.7** |
| **28b** | **3.0** |
| **30** | **7.8** |
| **38b** | **0.87** |

Aspects of the present disclosure are further illustrated by reference to the following, nonlimiting embodiments.
1. A compound of formula (I): 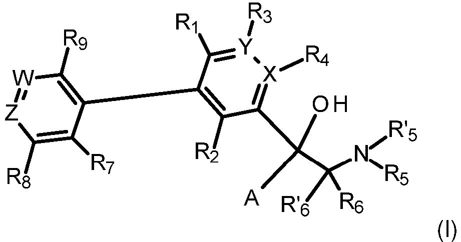 or a pharmaceutically acceptable salt, enantiomers, diastereomers, tautomers, solvates, isotope substituents, polymorphs, prodrugs or metabolites thereof, wherein:
   A is a phenyl optionally substituted with 1 to 3 substituents selected from halogen, (C₁₋C₆)alkyl and (C₁-C₆)alkoxy; a 4- to 7-membered unsaturated, monocyclic group comprising from 1 to 3 heteroatoms, preferably 1 or 2 heteroatoms, chosen from nitrogen, oxygen and sulfur;;
   W and Z are each independently CR₁₅ or N;
   X and Y are each independently C or N;
   R₁ is H, halogen, (C₁-C₆)alkyl or (C₁-C₆)alkoxy;
   R₂ is H, halogen, (C₁-C₆)alkyl or (C₁-C₆)alkoxy;
   R₃ is absent when Y is N, or R₃ is H, halogen, -OH, -COCH₃, (C₁-C₆)alkyl or (C₁-C₆)alkoxy when Y is C;
   R₄ is absent when X is N, or R₄ is H, halogen, -OH, -COCH₃, (C₁-C₆)alkyl or (C₁-C₆)alkoxy when X is C;
   R₅ and R'₅ are each independently H; (C₁-C₆)alkyl optionally substituted with halogen, -OH, - CONHR₁₂, -CONH₂, -NR₁₂R₁₃, (C₁-C₆)alkoxy or (C₃-C₆)cycloalkyl; or (C₃-C₆)cycloalkyl optionally substituted with -OR₁₂, -CONHR₁₂, -CONH₂, -NR₁₂R₁₃;
   or R₅ and R'₅, together with the nitrogen atom to which are bound, form a 4- to 7-membered ring;
   R₆ and R'₆ are each independently H or (C₁-C₆)alkyl; or R₆ and R'₆ together form a (C₃-C₆)cycloalkyl;
   or R₅ and R₆ together form a (C₃-C₆)cycloalkyl;
   R₇ is H, halogen, (C₁-C₆)alkyl or (C₁-C₆)alkoxy;
   R₈ is H, halogen, (C₁-C₆)alkyl, (C₁-C₆)alkyl optionally substituted with halogen, (C₁-C₆)alkoxy, (C₁-C₆)alkoxy optionally substituted with halogen, (C₁-C₆)alkoxy optionally substituted with (C₁-C₆)alkoxy,
   -OR₁₀ , -OCH₂R₁₀ , -NHR₁₀ , -N(CH₃)R₁₀, or 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₉ is (C₁-C₆)alkyl, (C₁-C₆)alkoxy, -CONHRₙ, -C(NH)NHOH, -C(NH)NHOCH₃ or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₁₀ is H, (C₁-C₆)alkyl, (C₁-C₆)alkyl optionally substituted with halogen, (C₁-C₆)alkoxy, or a 4-to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
   R₁₁ is H, -OH, -NH₂, (C₁-C₆)alkyl, (C₁-C₆)alkoxy, and -NHR₁₄ ;
   R₁₂ is H or (C₁-C₆)alkyl;
   R₁₃ is H or (C₁-C₆)alkyl;
   R₁₄ is (C₁-C₆)alkyl;
   R₁₅ is H, halogen, (C₁-C₆)alkyl or (C₁-C₆)alkoxy.
2. The compound of item 1, or a pharmaceutically acceptable salt, enantiomers, diastereomers, tautomers, solvates, isotope substituents, polymorphs, prodrugs or metabolites thereof, wherein A is a phenyl optionally substituted with 1 to 3 substituents selected from halogen, (C₁-C₆)alkyl and (C₁-C₆)alkoxy.
3. The compound of item 1, or a pharmaceutically acceptable salt, enantiomers, diastereomers, tautomers, solvates, isotope substituents, polymorphs, prodrugs or metabolites thereof, wherein A is a 4- to 7-membered unsaturated monocyclic group comprising from 1 to 3 heteroatoms, preferably 1 or 2 heteroatoms, chosen from nitrogen, oxygen and sulfur.
4. The compound of any preceding item, or a pharmaceutically acceptable salt, enantiomers, diastereomers, tautomers, solvates, isotope substituents, polymorphs, prodrugs or metabolites thereof, wherein W and Z are each CH.
5. The compound of any preceding item, or a pharmaceutically acceptable salt, enantiomers, diastereomers, tautomers, solvates, isotope substituents, polymorphs, prodrugs or metabolites thereof, wherein X and Y are each C.
6. The compound of any preceding item, or a pharmaceutically acceptable salt, enantiomers, diastereomers, tautomers, solvates, isotope substituents, polymorphs, prodrugs or metabolites thereof, wherein R₁ is halogen or (C₁-C₆)alkyl.
7. The compound of any preceding item, or a pharmaceutically acceptable salt, enantiomers, diastereomers, tautomers, solvates, isotope substituents, polymorphs, prodrugs or metabolites thereof, wherein R₂ is H, halogen or (C₁-C₆)alkyl.
8. The compound of any preceding item, or a pharmaceutically acceptable salt, enantiomers, diastereomers, tautomers, solvates, isotope substituents, polymorphs, prodrugs or metabolites thereof, wherein R₃ when present is H or halogen.
9. The compound of any preceding item, or a pharmaceutically acceptable salt, enantiomers, diastereomers, tautomers, solvates, isotope substituents, polymorphs, prodrugs or metabolites thereof, wherein R₄ when present is H.
10. The compound of any preceding item, or a pharmaceutically acceptable salt, enantiomers, diastereomers, tautomers, solvates, isotope substituents, polymorphs, prodrugs or metabolites thereof, wherein R'₅ is H or (C₁-C₆)alkyl or (C₃-C₆)cycloalkyl
11. The compound of any preceding item, or a pharmaceutically acceptable salt, enantiomers, diastereomers, tautomers, solvates, isotope substituents, polymorphs, prodrugs or metabolites thereof, wherein R₇ is H, halogen or (C₁-C₆)alkyl.
12. The compound of any preceding item, or a pharmaceutically acceptable salt, enantiomers, diastereomers, tautomers, solvates, isotope substituents, polymorphs, prodrugs or metabolites thereof, wherein R₈ is H or (C₁-C₆)alkoxy optionally substituted with (C₁-C₆)alkoxy or OR₁₀
13. The compound of any preceding item, or a pharmaceutically acceptable salt, enantiomers, diastereomers, tautomers, solvates, isotope substituents, polymorphs, prodrugs or metabolites thereof, wherein R₁₁ is H or OH.
14. The compound of item 1, which is selected from:
   2-[2-chloro-5-[rel-(1S)-2-amino-1-hydroxy-1-phenyl-ethyl]phenyl]benzamide;
   2-[2-chloro-5-[rel-(1R)-2-amino-1-hydroxy-1-phenyl-ethyl]phenyl]benzamide;
   2-[5-(2-amino-1-hydroxy-1-phenyl-ethyl)-2-chloro-phenyl]-3-methyl-benzamide;
   2-[5-(2-amino-1-hydroxy-1-phenyl-ethyl)-2-chloro-phenyl]-3-methyl-benzamide;
   2-[5-(2-amino-1-hydroxy-1-phenyl-ethyl)-2-chloro-phenyl]-3-methyl-benzamide;
   2-[5-(2-amino-1-hydroxy-1-phenyl-ethyl)-2-chloro-phenyl]-4-methoxy-3-methyl-benzamide;
   2-[5-(2-amino-1-hydroxy-1-phenyl-ethyl)-2-chloro-phenyl]-4-(2-methoxyethoxy)-3-methyl-benzamide;
   2-[5-(2-amino-1-hydroxy-1-phenyl-ethyl)-2-chloro-phenyl]-4-(2-methoxyethoxy)-3-methyl-benzamide;
   rac2 2-[5-(2-amino-1-hydroxy-1-phenyl-ethyl)-2-chloro-phenyl]-4-(2-methoxyethoxy)-3-methyl-benzamide;
   2-[5-(2-amino-1-hydroxy-1-phenyl-ethyl)-2-chloro-phenyl]-3-fluoro-benzamide;
   2-[5-(2-amino-1-hydroxy-1-phenyl-ethyl)-2-chloro-phenyl]-3-fluoro-4-(2-methoxyethoxy)benzamide;
   2-[2-chloro-5-[1-hydroxy-2-(methylamino)-1-phenyl-ethyl]phenyl]-3-fluoro-4-(2-methoxyethoxy)benzamide;
   2-[5-(2-amino-1-hydroxy-1-phenyl-ethyl)-2-chloro-phenyl]-3-chloro-benzamide;
   2-[3-(2-amino-1-hydroxy-1-phenyl-ethyl)-6-chloro-2-methyl-phenyl]benzamide;
   2-[3-(2-amino-1-hydroxy-1-phenyl-ethyl)-6-chloro-2-methyl-phenyl]benzamide;
   2-[3-(2-amino-1-hydroxy-1-phenyl-ethyl)-6-chloro-2-fluoro-phenyl]benzamide;
   2-[5-(2-amino-1-hydroxy-1-phenyl-ethyl)-2-chloro-3-fluoro-phenyl]-3-methyl-benzamide;
   2-[5-(2-amino-1-hydroxy-1-phenyl-ethyl)-2-methyl-phenyl]-3-fluoro-4-(2-methoxyethoxy)benzamide;
   2-[5-[(1S)-2-amino-1-hydroxy-1-phenyl-ethyl]-2-chloro-phenyl]-3-fluoro-4-(2-methoxyethoxy)benzamide;
   2-[5-[(1R)-2-amino-1-hydroxy-1-phenyl-ethyl]-2-chloro-phenyl]-3-fluoro-4-(2-methoxyethoxy)benzamide;
   2-[5-[(1-aminocyclopropyl)-hydroxy-phenyl-methyl]-2-chloro-phenyl]-3-fluoro-4-(2-methoxyethoxy)benzamide;
   2-[3-(2-amino-1-hydroxy-1-phenyl-ethyl)-2,6-difluoro-phenyl]-3-fluoro-4-(2-methoxyethoxy)benzamide;
   2-[3-(2-amino-1-hydroxy-1-phenyl-ethyl)-2,6-difluoro-phenyl]-3-fluoro-4-(2-methoxyethoxy)benzamide;
   2-[5-(2-amino-1-hydroxy-1-phenyl-ethyl)-2-chloro-phenyl]-3-chloro-4-methoxy-benzamide;
   2-[2-chloro-5-[1-hydroxy-2-(methylamino)-1-phenyl-ethyl]phenyl]-3-fluoro-4-(2-methoxyethoxy)benzenecarbohydroxamic acid;
   rel-(2aR)-2-[3-[(1S)-2-amino-1-hydroxy-1-phenyl-ethyl]-6-chloro-2-methyl-phenyl]-3-fluoro-4-(2-methoxyethoxy)benzamide;
   rel-(2aS)-2-[3-[(1S)-2-amino-1-hydroxy-1-phenyl-ethyl]-6-chloro-2-methyl-phenyl]-3-fluoro-4-(2-methoxyethoxy)benzamide;
   rel-(2-aS)-2-[3-[(1R)-2-amino-1-hydroxy-1-phenyl-ethyl]-6-chloro-2-methyl-phenyl]-3-fluoro-4-(2-methoxyethoxy)benzamide;
   rel-(2aR)-2-[3-[(1R)-2-amino-1-hydroxy-1-phenyl-ethyl]-6-chloro-2-methyl-phenyl]-3-fluoro-4-(2-methoxyethoxy)benzamide;
   2-[6-chloro-3-[2-(cyclohexylamino)-1-hydroxy-1-phenyl-ethyl]-2-fluoro-phenyl]-3-fluoro-4-(2-methoxyethoxy)benzamide;
   rel-(2aS)-2-[6-chloro-2-fluoro-3-[(1S)-1-hydroxy-2-(methylamino)-1-phenyl-ethyl]phenyl]-3-fluoro-4-(2-methoxyethoxy)benzamide;
   rel-(2aR)-2-[6-chloro-2-fluoro-3-[(1S)-1-hydroxy-2-(methylamino)-1-phenyl-ethyl]phenyl]-3-fluoro-4-(2-methoxyethoxy)benzamide;
   rel-(2aR)-2-[6-chloro-2-fluoro-3-[(1R)-1-hydroxy-2-(methylamino)-1-phenyl-ethyl]phenyl]-3-fluoro-4-(2-methoxyethoxy)benzamide;
   rel-(2aS)-2-[6-chloro-2-fluoro-3-[(1R)-1-hydroxy-2-(methylamino)-1-phenyl-ethyl]phenyl]-3-fluoro-4-(2-methoxyethoxy)benzamide;
   2-[2-chloro-5-[(R)-hydroxy-phenyl-[(2S)-pyrrolidin-2-yl]methyl]phenyl]-3-methyl-benzamide;
   2-[rel-(5R)-5-[[(2S)-azetidin-2-yl]-hydroxy-phenyl-methyl]-2-chloro-phenyl]-3-fluoro-4-(2-methoxyethoxy)benzamide;
   2-[3-(2-amino-1-hydroxy-1-phenyl-ethyl)-2,6-dichloro-phenyl]-3-fluoro-4-(2-methoxyethoxy)benzamide;
   trans-2-[2-chloro-5-[1-hydroxy-2-[(4-hydroxycyclohexyl)amino]-1-phenyl-ethyl]phenyl]-3-fluoro-4-(2-methoxyethoxy)benzamide;
   rel-(2aR)-2-[3-[(1R)-2-amino-1-hydroxy-1-phenyl-ethyl]-6-chloro-2-fluoro-phenyl]-3-fluoro-4-(2-methoxyethoxy)benzamide;
   rel-(2aS)-2-[3-[(1R)-2-amino-1-hydroxy-1-phenyl-ethyl]-6-chloro-2-fluoro-phenyl]-3-fluoro-4-(2-methoxyethoxy)benzamide;
   rel-(2aR)-2-[3-[(1S)-2-amino-1-hydroxy-1-phenyl-ethyl]-6-chloro-2-fluoro-phenyl]-3-fluoro-4-(2-methoxyethoxy)benzamide;
   rel-(2aS)-2-[3-[(1S)-2-amino-1-hydroxy-1-phenyl-ethyl]-6-chloro-2-fluoro-phenyl]-3-fluoro-4-(2-methoxyethoxy)benzamide;
   2-[6-chloro-3-[2-(cyclopropylmethylamino)-1-hydroxy-1-phenyl-ethyl]-2-fluoro-phenyl]-3-fluoro-4-(2-methoxyethoxy)benzamide;
   2-[6-chloro-2-fluoro-3-[1-hydroxy-2-(2-methoxyethylamino)-1-phenyl-ethyl]phenyl]-3-fluoro-4-(2-methoxyethoxy)benzamide;
   2-[6-chloro-3-[2-(dimethylamino)-1-hydroxy-1-phenyl-ethyl]-2-fluoro-phenyl]-3-fluoro-4-(2-methoxyethoxy)benzamide;
   2-[2-chloro-5-(1-hydroxy-1-phenyl-2-pyrrolidin-1-yl-ethyl)phenyl]-3-fluoro-4-(2-methoxyethoxy)benzamide;
   2-[2-chloro-5-[1-hydroxy-1-phenyl-2-(1-piperidyl)ethyl]phenyl]-3-fluoro-4-(2-methoxyethoxy)benzamide;
   2-[5-(2-amino-1-hydroxy-1-phenyl-ethyl)-2,4-dichloro-phenyl]-3-methyl-benzamide;
   2-[5-(2-amino-1-hydroxy-1-phenyl-ethyl)-2-chloro-4-fluoro-phenyl]-3-methyl-benzamide rel-(2aS)-2-[6-chloro-3-[(1S)-2-(cyclohexylamino)-1-hydroxy-1-phenyl-ethyl]-2-fluoro-phenyl]-3-fluoro-4-(2-methoxyethoxy)benzamide;
   rel-(2aR)-2-[6-chloro-3-[(1S)-2-(cyclohexylamino)-1 -hydroxy-1-phenyl-ethyl]-2-fluoro-phenyl]-3-fluoro-4-(2-methoxyethoxy)benzamide;
   rel-(2aR)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide;
   rel-(2aS)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide;
   2-[6-chloro-2-fluoro-3-[1-hydroxy-2-(methylamino)-1-phenyl-ethyl]phenyl]-3-fluoro-4-(5-methylisoxazol-3-yl)oxy-benzamide;
   2-[5-(2-amino-1-hydroxy-1-pyrimidin-2-yl-ethyl)-2-chloro-phenyl]-3-fluoro-4-(2-methoxyethoxy)benzamide ;
   and pharmaceutically acceptable salts, enantiomers, diastereomers, tautomers, solvates, isotope substituents, polymorphs, prodrugs or metabolites thereof..
15. The compound of item 15, which is selected from:
   2-[5-(2-amino-1-hydroxy-1-phenyl-ethyl)-2-chloro-phenyl]-3-methyl-benzamide;
   2-[5-(2-amino-1-hydroxy-1-phenyl-ethyl)-2-chloro-phenyl]-3-fluoro-4-(2-methoxyethoxy)benzamide;
   2-[2-chloro-5-[1-hydroxy-2-(methylamino)-1-phenyl-ethyl]phenyl]-3-fluoro-4-(2-methoxyethoxy)benzamide;
   2-[3-(2-amino-1-hydroxy-1-phenyl-ethyl)-6-chloro-2-methyl-phenyl]benzamide;
   2-[5-[(1R)-2-amino-1-hydroxy-1-phenyl-ethyl]-2-chloro-phenyl]-3-fluoro-4-(2-methoxyethoxy)benzamide;
   2-[5-[(1-aminocyclopropyl)-hydroxy-phenyl-methyl]-2-chloro-phenyl]-3-fluoro-4-(2-methoxyethoxy)benzamide;
   2-[3-(2-amino-1-hydroxy-1-phenyl-ethyl)-2,6-difluoro-phenyl]-3-fluoro-4-(2-methoxyethoxy)benzamide;
   2-[2-chloro-5-[1-hydroxy-2-(methylamino)-1-phenyl-ethyl]phenyl]-3-fluoro-4-(2-methoxyethoxy)benzenecarbohydroxamic acid
   rel-(2aS)-2-[3-[(1S)-2-amino-1-hydroxy-1-phenyl-ethyl]-6-chloro-2-methyl-phenyl]-3-fluoro-4-(2-methoxyethoxy)benzamide;
   rel-(2-aS)-2-[3-[(1R)-2-amino-1-hydroxy-1-phenyl-ethyl]-6-chloro-2-methyl-phenyl]-3-fluoro-4-(2-methoxyethoxy)benzamide;
   2-[6-chloro-3-[2-(cyclohexylamino)-1-hydroxy-1-phenyl-ethyl]-2-fluoro-phenyl]-3-fluoro-4-(2-methoxyethoxy)benzamide;
   rel-(2aS)-2-[6-chloro-2-fluoro-3-[(1S)-1-hydroxy-2-(methylamino)-1-phenyl-ethyl]phenyl]-3-fluoro-4-(2-methoxyethoxy)benzamide;
   rel-(2aS)-2-[6-chloro-2-fluoro-3-[(1R)-1-hydroxy-2-(methylamino)-1-phenyl-ethyl]phenyl]-3-fluoro-4-(2-methoxyethoxy)benzamide;
   2-[3-(2-amino-1-hydroxy-1-phenyl-ethyl)-2,6-dichloro-phenyl]-3-fluoro-4-(2-methoxyethoxy)benzamide;
   trans-2-[2-chloro-5-[1-hydroxy-2-[(4-hydroxycyclohexyl)amino]-1-phenyl-ethyl]phenyl]-3-fluoro-4-(2-methoxyethoxy)benzamide;
   and pharmaceutically acceptable salts, enantiomers, diastereomers, tautomers, solvates, isotope substituents, polymorphs, prodrugs or metabolites thereof.
16. A pharmaceutical composition comprising a compound according to any one of items 1 to 16, or a pharmaceutically acceptable salt, enantiomers, diastereomers, tautomers, solvates, isotope substituents, polymorphs, prodrugs or metabolites thereof, and at least one pharmaceutically acceptable excipient.
17. The pharmaceutical composition of item 16, which further comprises at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and combinations thereof.
18. The pharmaceutical composition according to item 16 or item 17, for use for the prevention or the treatment of a cancer.
19. The pharmaceutical composition according to item 16, for use for the prevention or the treatment of a fibrosis related disease.
20. A compound according to any one of items 1 to 15, or a pharmaceutically acceptable salt, enantiomers, diastereomers, tautomers, solvates, isotope substituents, polymorphs, prodrugs or metabolites thereof, for use as a medicament.
21. A compound according to any one of items 1 to 15, or a pharmaceutically acceptable salt, enantiomers, diastereomers, tautomers, solvates, isotope substituents, polymorphs, prodrugs or metabolites thereof, for use for the treatment of a cancer.
22. A compound according to any one of items 1 to 15, or a pharmaceutically acceptable salt, enantiomers, diastereomers, tautomers, solvates, isotope substituents, polymorphs, prodrugs or metabolites thereof, for use for the prevention of a cancer.
23. Use of a compound according to any one of items 1 to 15, or a pharmaceutically acceptable salt, enantiomers, diastereomers, tautomers, solvates, isotope substituents, polymorphs, prodrugs or metabolites thereof, in the manufacture of a medicament for the treatment of a cancer.
24. Use of a compound according to any one of items 1 to 15, or a pharmaceutically acceptable salt, enantiomers, diastereomers, tautomers, solvates, isotope substituents, polymorphs, prodrugs or metabolites thereof, in the manufacture of a medicament for the prevention of a cancer.
25. A method of treating a cancer in a patient, comprising administering to the patient an effective amount of a compound of formula (I) according to any one of items 1 to 15, or a pharmaceutically acceptable salt, enantiomers, diastereomers, tautomers, solvates, isotope substituents, polymorphs, prodrugs or metabolites thereof.
26. A method of preventing a cancer in a patient, comprising administering to the patient an effective amount of a compound of formula (I) according to any one of items 1 to 15, or a pharmaceutically acceptable salt, enantiomers, diastereomers, tautomers, solvates, isotope substituents, polymorphs, prodrugs or metabolites thereof.
27. The method according to item 25 or item 26, which further comprises administering to the patient at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and combinations thereof.
28. The compound for use, use or method according to any one of items 20 to 27, wherein the cancer is selected from: adrenal cancer, adrenocortical cancer, anal cancer, bile duct cancer, bladder cancer, blood related cancer, bone cancer, brain cancer, breast cancer, male breast cancer, cervical cancer, colon cancer, colorectal cancer, embryonal cancer, endometrial cancer, uterine cancer, esophageal cancer, eye cancer, gastric cancer, head and neck cancer, kidney cancer, liver cancer, lung cancer, mesothelioma, ovarian cancer, pancreatic cancer, peripheral nervous system cancer, prostate cancer, gallbladder cancer, rhabdoid tumors, soft tissue sarcoma and sarcoma, skin cancer, melanoma, penile cancer, rectal cancer, small intestine cancer, testicular cancer, urethral cancer, germ cell tumors, childhood cancer and thyroid cancer.
29. A compound according to any one of items 1 to 15, or a pharmaceutically acceptable salt, enantiomers, diastereomers, tautomers, solvates, isotope substituents, polymorphs, prodrugs or metabolites thereof, for use for the treatment of a fibrosis related disease.
30. A compound according to any one of items 1 to 15, or a pharmaceutically acceptable salt, enantiomers, diastereomers, tautomers, solvates, isotope substituents, polymorphs, prodrugs or metabolites thereof, for use for the prevention of a fibrosis related disease.
31. Use of a compound according to any one of items 1 to 15, or a pharmaceutically acceptable salt, enantiomers, diastereomers, tautomers, solvates, isotope substituents, polymorphs, prodrugs or metabolites thereof, in the manufacture of a medicament for the treatment of a fibrosis related disease.
32. Use of a compound according to any one of items 1 to 15, or a pharmaceutically acceptable salt, enantiomers, diastereomers, tautomers, solvates, isotope substituents, polymorphs, prodrugs or metabolites thereof, in the manufacture of a medicament for the prevention of a fibrosis related disease.
33. A method of treating a fibrosis related disease in a patient, comprising administering to the patient an effective amount of a compound of formula (I) according to any one of items 1 to 15, or a pharmaceutically acceptable salt, enantiomers, diastereomers, tautomers, solvates, isotope substituents, polymorphs, prodrugs or metabolites thereof.
34. A method of preventing a fibrosis related disease in a patient, comprising administering to the patient an effective amount of a compound of formula (I) according to any one of items 1 to 15, or a pharmaceutically acceptable salt, enantiomers, diastereomers, tautomers, solvates, isotope substituents, polymorphs, prodrugs or metabolites thereof.
35. The compound for use, use or method according to any one of items 29 to 34, wherein the fibrosis related disease is selected from: skin fibrosis (such as for example hypertrophic scar or systemic sclerosis), heart fibrosis (such as for example cardiac fibrosis, hypertrophic cardiomyopathy, valvular diseases), bone marrow fibrosis (such as for example myelofibrosis, myelodysplatic syndrome), liver fibrosis (such as for example non-alcoholic steatohepatitis, Alcoholic liver disease, cirrhosis, portal hypertension), retroperitoneum fibrosis, gut fibrosis (such as for example intestinal fibrosis, Inflammatory bowel disease, enteropathies), joint fibrosis (such as for example arthrofibrosis), brain fibrosis (such as glial scar), nervous system fibrosis, eye fibrosis (such as for example subretinal fibrosis, epiretinal fibrosis, vision loss), lung fibrosis (such as for example idiopathic pulmonary fibrosis, ANCA-associated vasculitis, pulmonary hypertension), cystic fibrosis, mediastinum fibrosis, pancreas fibrosis (such as for example pancreatic fibrosis, chronic pancreatitis, duct obstruction) and kidney fibrosis (such as for example renal fibrosis, ANCA-associated vasculitis, nephrogenic systemic fibrosis, chronic kidney disease)..
36. A pharmaceutical composition according to item 16 or item 17, for use as a medicament.
37. Use of a pharmaceutical composition according to item 16 or item 17 in the manufacture of a medicament for the treatment or prevention of a cancer.
38. A method of treating or preventing a cancer in a patient, comprising administering to the patient a pharmaceutical composition according to item 16, wherein the pharmaceutical composition comprises an effective amount of a compound of formula (I) or a pharmaceutically acceptable salt, enantiomer, diastereomer, tautomer, solvate, isotope substituent, polymorph, prodrug or metabolite thereof.
39. The method according to item 38, which further comprises administering to the patient at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic and combinations thereof.
40. The pharmaceutical composition for use, use or method according to any one of items 18 and 37 to 39, wherein the cancer is selected from: adrenal cancer, adrenocortical cancer, anal cancer, bile duct cancer, bladder cancer, blood related cancer, bone cancer, brain cancer, breast cancer, male breast cancer, cervical cancer, colon cancer, colorectal cancer, embryonal cancer, endometrial cancer, uterine cancer, esophageal cancer, eye cancer, gastric cancer, head and neck cancer, kidney cancer, liver cancer, lung cancer, mesothelioma, ovarian cancer, pancreatic cancer, peripheral nervous system cancer, prostate cancer, gallbladder cancer, rhabdoid tumors, soft tissue sarcoma and sarcoma, skin cancer, melanoma, penile cancer, rectal cancer, small intestine cancer, testicular cancer, urethral cancer, germ cell tumors, childhood cancer and thyroid cancer.
41. Use of a pharmaceutical composition according to item 16 in the manufacture of a medicament for the treatment or prevention of a fibrosis related disease.
42. A method of treating or preventing a fibrosis related disease in a patient, comprising administering to the patient a pharmaceutical composition according to item 16, wherein the pharmaceutical composition comprises an effective amount of a compound of formula (I) or a pharmaceutically acceptable salt, enantiomer, diastereomer, tautomer, solvate, isotope substituent, polymorph, prodrug or metabolite thereof.
43. The pharmaceutical composition for use, use or method according to any one of items 19, 41 or 42 wherein the fibrosis related disease is selected from: skin fibrosis (such as for example hypertrophic scar or systemic sclerosis), heart fibrosis (such as for example cardiac fibrosis, hypertrophic cardiomyopathy, valvular diseases), bone marrow fibrosis (such as for example myelofibrosis, myelodysplatic syndrome), liver fibrosis (such as for example non-alcoholic steatohepatitis, Alcoholic liver disease, cirrhosis, portal hypertension), retroperitoneum fibrosis, gut fibrosis (such as for example intestinal fibrosis, Inflammatory bowel disease, enteropathies), joint fibrosis (such as for example arthrofibrosis), brain fibrosis (such as glial scar), nervous system fibrosis, eye fibrosis (such as for example subretinal fibrosis, epiretinal fibrosis, vision loss), lung fibrosis (such as for example idiopathic pulmonary fibrosis, ANCA-associated vasculitis, pulmonary hypertension), cystic fibrosis, mediastinum fibrosis, pancreas fibrosis (such as for example pancreatic fibrosis, chronic pancreatitis, duct obstruction) and kidney fibrosis (such as for example renal fibrosis, ANCA-associated vasculitis, nephrogenic systemic fibrosis, chronic kidney disease)..
44. A method of modulating one or more of proteins encompassed by, or related to, the Hippo pathway in a subject, comprising administering to a subject in need thereof a compound of formula (I), or a pharmaceutically acceptable salt, enantiomer, diastereomer, tautomer, solvate, isotope substituent, polymorph, prodrug or metabolite thereof.
45. A method of inhibiting one or more of proteins encompassed by, or related to, the Hippo pathway in a subject, comprising administering to a subject in need thereof a compound of formula (I), or a pharmaceutically acceptable salt, enantiomer, diastereomer, tautomer, solvate, isotope substituent, polymorph, prodrug or metabolite thereof.
46. A method of activating one or more of proteins encompassed by, or related to, the Hippo pathway in a subject, comprising administering to a subject in need thereof a compound of formula (I), or a pharmaceutically acceptable salt, enantiomer, diastereomer, tautomer, solvate, isotope substituent, polymorph, prodrug or metabolite thereof.
47. A compound of formula (I) according to any one of items 1 to 15 or a pharmaceutically acceptable salt thereof, or the pharmaceutical composition, or an enantiomer, a diastereomer, a tautomer, a solvate, an isotope substituent, a polymorph, a prodrug or a metabolite thereof as described herein, for treating or preventing a disease or disorder by modulating or inhibiting or activating one or more of proteins encompassed by, or related to, the Hippo pathway, in a subject in need thereof.
48. Uses of a compound of formula (I) according to any one of items 1 to 15 or a pharmaceutically acceptable salt thereof, or the pharmaceutical composition, or an enantiomer, a diastereomer, a tautomer, a solvate, an isotope substituent, a polymorph, a prodrug or a metabolite thereof, in the preparation of a medicament for treating or preventing a disease or disorder by modulating or inhibiting or activating one or more of proteins encompassed by, or related to, the Hippo pathway, in a subject in need thereof.

## Claims

1. A compound of formula (I): 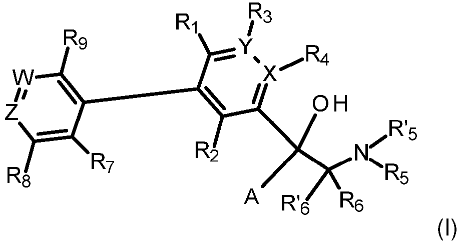
or a pharmaceutically acceptable salt, enantiomer, diastereomer, tautomer, solvate, isotope substituent, polymorph, prodrug or metabolite thereof,
wherein:
A is a phenyl optionally substituted with 1 to 3 substituents selected from halogen, (C₁-C₆)alkyl and (C₁-C₆)alkoxy; a 4- to 7-membered unsaturated, monocyclic group comprising from 1 to 3 heteroatoms, preferably 1 or 2 heteroatoms, chosen from nitrogen, oxygen and
sulfur;
W and Z are each independently CR₁₅ or N;
X and Y are each independently C or N;
R₁ is H, halogen, (C₁-C₆)alkyl or (C₁-C₆)alkoxy;
R₂ is H, halogen, (C₁-C₆)alkyl or (C₁-C₆)alkoxy;
R₃ is absent when Y is N, or R₃ is H, halogen, -OH, -COCH₃, (C₁-C₆)alkyl or (C₁-C₆)alkoxy when Y is C;
R₄ is absent when X is N, or R₄ is H, halogen, -OH, -COCH₃, (C₁-C₆)alkyl or (C₁-C₆)alkoxy when X is C;
R₅ and R'₅ are each independently H; (C₁-C₆)alkyl optionally substituted with halogen, -OH, - CONHR₁₂, -CONH₂, -NR₁₂R₁₃, (C₁-C₆)alkoxy or (C₃-C₆)cycloalkyl; or (C₃-C₆)cycloalkyl optionally substituted with -OR₁₂, -CONHR₁₂, -CONH₂, -NR₁₂R₁₃;
or R₅ and R'₅, together with the nitrogen atom to which are bound, form a 4- to 7-membered ring;
R₆ and R'₆ are each independently H or (C₁-C₆)alkyl; or R₆ and R'₆ together form a (C₃-C₆)cycloalkyl;
or R₅ and R₆ together form a (C₃-C₆)cycloalkyl;
R₇ is H, halogen, (C₁-C₆)alkyl or (C₁-C₆)alkoxy;
R₈ is H, halogen, (C₁-C₆)alkyl, (C₁-C₆)alkyl optionally substituted with halogen, (C₁-C₆)alkoxy, (C₁-C₆)alkoxy optionally substituted with halogen, (C₁-C₆)alkoxy optionally substituted with (C₁-C₆)alkoxy ,
-OR₁₀ , -OCH₂R₁₀ , -NHR₁₀ , -N(CH₃)R₁₀, or 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
R₉ is (C₁-C₆)alkyl , (C₁-C₆)alkoxy, -CONHR₁₁, -C(NH)NHOH, -C(NH)NHOCH₃ or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen;
R₁₀ is H, (C₁-C₆)alkyl, (C₁-C₆)alkyl optionally substituted with halogen, (C₁-C₆)alkoxy or a 4- to 7-membered saturated, unsaturated or partially unsaturated monocyclic group comprising from 1 to 4 heteroatoms chosen from nitrogen, oxygen and sulfur, said monocyclic group being optionally substituted with 1 to 3 substituents selected from halogen,;
R₁₁ is H, -OH, -NH₂, (C₁-C₆)alkyl, (C₁-C₆)alkoxy, and -NHR₁₄ ;
R₁₂ is H or (C₁-C₆)alkyl;
R₁₃ is H or (C₁-C₆)alkyl;
R₁₄ is (C₁-C₆)alkyl;
R₁₅ is H, halogen, (C₁-C₆)alkyl or (C₁-C₆)alkoxy.

2. The compound of claim 1, or a pharmaceutically acceptable salt, enantiomer, diastereomer, tautomer, solvate, isotope substituent, polymorph, prodrug or metabolite thereof, wherein A is a phenyl optionally substituted with 1 to 3 substituents selected from halogen, (C₁-C₆)alkyl and (C₁-C₆)alkoxy.

3. The compound of claim 1 or claim 2, or a pharmaceutically acceptable salt, enantiomer, diastereomer, tautomer, solvate, isotope substituent, polymorph, prodrug or metabolite thereof, wherein W and Z are each CH.

4. The compound of any preceding claim, or a pharmaceutically acceptable salt, enantiomer, diastereomer, tautomer, solvate, isotope substituent, polymorph, prodrug or metabolite thereof, wherein X and Y are each C.

5. The compound of any preceding claim, or a pharmaceutically acceptable salt, enantiomer, diastereomer, tautomer, solvate, isotope substituent, polymorph, prodrug or metabolite thereof, wherein R₁ is halogen or (C₁-C₆)alkyl.

6. The compound of any preceding claim, or a pharmaceutically acceptable salt, enantiomer, diastereomer, tautomer, solvate, isotope substituent, polymorph, prodrug or metabolite thereof, wherein R₂ is H, halogen or (C₁-C₆)alkyl.

7. The compound of any preceding claim, or a pharmaceutically acceptable salt, enantiomer, diastereomer, tautomer, solvate, isotope substituent, polymorph, prodrug or metabolite thereof, wherein R₃ when present is H or halogen.

8. The compound of any preceding claim, or a pharmaceutically acceptable salt, enantiomer, diastereomer, tautomer, solvate, isotope substituent, polymorph, prodrug or metabolite thereof, wherein R₄ when present is H.

9. The compound of any preceding claim, or a pharmaceutically acceptable salt, enantiomer, diastereomer, tautomer, solvate, isotope substituent, polymorph, prodrug or metabolite thereof, wherein R'₅ is H or (C₁-C₆)alkyl or (C₃-C₆)cycloalkyl

10. The compound of any preceding claim, or a pharmaceutically acceptable salt, enantiomer, diastereomer, tautomer, solvate, isotope substituent, polymorph, prodrug or metabolite thereof, wherein R₇ is H, halogen or (C₁-C₆)alkyl.

11. The compound of any preceding claim, or pharmaceutically acceptable salt, enantiomer, diastereomer, tautomer, solvate, isotope substituent, polymorph, prodrug or metabolite thereof, wherein R₈ is H or (C₁-C₆)alkoxy optionally substituted with (C₁-C₆)alkoxy or -OR₁₀.

12. The compound of any preceding claim, or pharmaceutically acceptable salt, enantiomer, diastereomer, tautomer, solvate, isotope substituent, polymorph, prodrug or metabolite thereof, wherein R₁₁ is H or OH.

13. The compound of claim 1, which is selected from:
2-[2-chloro-5-[rel-(1S)-2-amino-1-hydroxy-1-phenyl-ethyl]phenyl]benzamide;
2-[2-chloro-5-[rel-(1R)-2-amino-1-hydroxy-1-phenyl-ethyl]phenyl]benzamide;
2-[5-(2-amino-1-hydroxy-1-phenyl-ethyl)-2-chloro-phenyl]-3-methyl-benzamide;
2-[5-(2-amino-1-hydroxy-1-phenyl-ethyl)-2-chloro-phenyl]-3-methyl-benzamide;
2-[5-(2-amino-1-hydroxy-1-phenyl-ethyl)-2-chloro-phenyl]-3-methyl-benzamide;
2-[5-(2-amino-1-hydroxy-1-phenyl-ethyl)-2-chloro-phenyl]-4-methoxy-3-methyl-benzamide;
2-[5-(2-amino-1-hydroxy-1-phenyl-ethyl)-2-chloro-phenyl]-4-(2-methoxyethoxy)-3-methylbenzamide;
2-[5-(2-amino-1-hydroxy-1-phenyl-ethyl)-2-chloro-phenyl]-4-(2-methoxyethoxy)-3-methylbenzamide;
rac2 2-[5-(2-amino-1-hydroxy-1-phenyl-ethyl)-2-chloro-phenyl]-4-(2-methoxyethoxy)-3-methyl-benzamide;
2-[5-(2-amino-1-hydroxy-1-phenyl-ethyl)-2-chloro-phenyl]-3-fluoro-benzamide;
2-[5-(2-amino-1-hydroxy-1-phenyl-ethyl)-2-chloro-phenyl]-3-fluoro-4-(2-methoxyethoxy)benzamide;
2-[2-chloro-5-[1-hydroxy-2-(methylamino)-1-phenyl-ethyl]phenyl]-3-fluoro-4-(2-methoxyethoxy)benzamide;
2-[5-(2-amino-1-hydroxy-1-phenyl-ethyl)-2-chloro-phenyl]-3-chloro-benzamide;
2-[3-(2-amino-1-hydroxy-1-phenyl-ethyl)-6-chloro-2-methyl-phenyl]benzamide;
2-[3-(2-amino-1-hydroxy-1-phenyl-ethyl)-6-chloro-2-methyl-phenyl]benzamide;
2-[3-(2-amino-1-hydroxy-1-phenyl-ethyl)-6-chloro-2-fluoro-phenyl]benzamide;
2-[5-(2-amino-1-hydroxy-1-phenyl-ethyl)-2-chloro-3-fluoro-phenyl]-3-methyl-benzamide;
2-[5-(2-amino-1-hydroxy-1-phenyl-ethyl)-2-methyl-phenyl]-3-fluoro-4-(2-methoxyethoxy)benzamide;
2-[5-[(1S)-2-amino-1-hydroxy-1-phenyl-ethyl]-2-chloro-phenyl]-3-fluoro-4-(2-methoxyethoxy)benzamide;
2-[5-[(1R)-2-amino-1-hydroxy-1-phenyl-ethyl]-2-chloro-phenyl]-3-fluoro-4-(2-methoxyethoxy)benzamide;
2-[5-[(1-aminocyclopropyl)-hydroxy-phenyl-methyl]-2-chloro-phenyl]-3-fluoro-4-(2-methoxyethoxy)benzamide;
2-[3-(2-amino-1-hydroxy-1-phenyl-ethyl)-2,6-difluoro-phenyl]-3-fluoro-4-(2-methoxyethoxy)benzamide;
2-[3-(2-amino-1-hydroxy-1-phenyl-ethyl)-2,6-difluoro-phenyl]-3-fluoro-4-(2-methoxyethoxy)benzamide;
2-[5-(2-amino-1-hydroxy-1-phenyl-ethyl)-2-chloro-phenyl]-3-chloro-4-methoxy-benzamide;
2-[2-chloro-5-[1-hydroxy-2-(methylamino)-1-phenyl-ethyl]phenyl]-3-fluoro-4-(2-methoxyethoxy)benzenecarbohydroxamic acid;
rel-(2aR)-2-[3-[(1S)-2-amino-1-hydroxy-1-phenyl-ethyl]-6-chloro-2-methyl-phenyl]-3-fluoro-4-(2-methoxyethoxy)benzamide;
rel-(2aS)-2-[3-[(1S)-2-amino-1-hydroxy-1-phenyl-ethyl]-6-chloro-2-methyl-phenyl]-3-fluoro-4-(2-methoxyethoxy)benzamide;
rel-(2-aS)-2-[3-[(1R)-2-amino-1-hydroxy-1-phenyl-ethyl]-6-chloro-2-methyl-phenyl]-3-fluoro-4-(2-methoxyethoxy)benzamide;
rel-(2aR)-2-[3-[(1R)-2-amino-1-hydroxy-1-phenyl-ethyl]-6-chloro-2-methyl-phenyl]-3-fluoro-4-(2-methoxyethoxy)benzamide;
2-[6-chloro-3-[2-(cyclohexylamino)-1-hydroxy-1-phenyl-ethyl]-2-fluoro-phenyl]-3-fluoro-4-(2-methoxyethoxy)benzamide;
rel-(2aS)-2-[6-chloro-2-fluoro-3-[(1S)-1-hydroxy-2-(methylamino)-1-phenyl-ethyl]phenyl]-3-fluoro-4-(2-methoxyethoxy)benzamide;
rel-(2aR)-2-[6-chloro-2-fluoro-3-[(1S)-1-hydroxy-2-(methylamino)-1-phenyl-ethyl]phenyl]-3-fluoro-4-(2-methoxyethoxy)benzamide;
rel-(2aR)-2-[6-chloro-2-fluoro-3-[(1R)-1-hydroxy-2-(methylamino)-1-phenyl-ethyl]phenyl]-3-fluoro-4-(2-methoxyethoxy)benzamide;
rel-(2aS)-2-[6-chloro-2-fluoro-3-[(1R)-1-hydroxy-2-(methylamino)-1-phenyl-ethyl]phenyl]-3-fluoro-4-(2-methoxyethoxy)benzamide;
2-[2-chloro-5-[(R)-hydroxy-phenyl-[(2S)-pyrrolidin-2-yl]methyl]phenyl]-3-methyl-benzamide;
2-[rel-(5R)-5-[[(2S)-azetidin-2-yl]-hydroxy-phenyl-methyl]-2-chloro-phenyl]-3-fluoro-4-(2-methoxyethoxy)benzamide;
2-[3-(2-amino-1-hydroxy-1-phenyl-ethyl)-2,6-dichloro-phenyl]-3-fluoro-4-(2-methoxyethoxy)benzamide;
trans-2-[2-chloro-5-[1-hydroxy-2-[(4-hydroxycyclohexyl)amino]-1-phenyl-ethyl]phenyl]-3-fluoro-4-(2-methoxyethoxy)benzamide;
rel-(2aR)-2-[3-[(1R)-2-amino-1-hydroxy-1-phenyl-ethyl]-6-chloro-2-fluoro-phenyl]-3-fluoro-4-(2-methoxyethoxy)benzamide;
rel-(2aS)-2-[3-[(1R)-2-amino-1-hydroxy-1-phenyl-ethyl]-6-chloro-2-fluoro-phenyl]-3-fluoro-4-(2-methoxyethoxy)benzamide;
rel-(2aR)-2-[3-[(1S)-2-amino-1-hydroxy-1-phenyl-ethyl]-6-chloro-2-fluoro-phenyl]-3-fluoro-4-(2-methoxyethoxy)benzamide;
rel-(2aS)-2-[3-[(1S)-2-amino-1-hydroxy-1-phenyl-ethyl]-6-chloro-2-fluoro-phenyl]-3-fluoro-4-(2-methoxyethoxy)benzamide;
2-[6-chloro-3-[2-(cyclopropylmethylamino)-1-hydroxy-1-phenyl-ethyl]-2-fluoro-phenyl]-3-fluoro-4-(2-methoxyethoxy)benzamide;
2-[6-chloro-2-fluoro-3-[1-hydroxy-2-(2-methoxyethylamino)-1-phenyl-ethyl]phenyl]-3-fluoro-4-(2-methoxyethoxy)benzamide;
2-[6-chloro-3-[2-(dimethylamino)-1-hydroxy-1-phenyl-ethyl]-2-fluoro-phenyl]-3-fluoro-4-(2-methoxyethoxy)benzamide;
2-[2-chloro-5-(1-hydroxy-1-phenyl-2-pyrrolidin-1-yl-ethyl)phenyl]-3-fluoro-4-(2-methoxyethoxy)benzamide;
2-[2-chloro-5-[1-hydroxy-1-phenyl-2-(1-piperidyl)ethyl]phenyl]-3-fluoro-4-(2-methoxyethoxy)benzamide;
2-[5-(2-amino-1-hydroxy-1-phenyl-ethyl)-2,4-dichloro-phenyl]-3-methyl-benzamide;
2-[5-(2-amino-1-hydroxy-1-phenyl-ethyl)-2-chloro-4-fluoro-phenyl]-3-methyl-benzamide rel-(2aS)-2-[6-chloro-3-[(1S)-2-(cyclohexylamino)-1-hydroxy-1-phenyl-ethyl]-2-fluoro-phenyl]-3-fluoro-4-(2-methoxyethoxy)benzamide;
rel-(2aR)-2-[6-chloro-3-[(1S)-2-(cyclohexylamino)-1-hydroxy-1-phenyl-ethyl]-2-fluoro-phenyl]-3-fluoro-4-(2-methoxyethoxy)benzamide;
rel-(2aR)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide;
rel-(2aS)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide;
2-[6-chloro-2-fluoro-3-[1-hydroxy-2-(methylamino)-1-phenyl-ethyl]phenyl]-3-fluoro-4-(5-methylisoxazol-3-yl)oxy-benzamide;
2-[5-(2-amino-1-hydroxy-1-pyrimidin-2-yl-ethyl)-2-chloro-phenyl]-3-fluoro-4-(2-methoxyethoxy)benzamide;
and pharmaceutically acceptable salts, enantiomers, diastereomers, tautomers, solvates, isotope substituents, polymorphs, prodrugs or metabolites thereof.

14. The compound of claim 1, which is selected from:
2-[5-(2-amino-1-hydroxy-1-phenyl-ethyl)-2-chloro-phenyl]-3-methyl-benzamide;
2-[5-(2-amino-1-hydroxy-1-phenyl-ethyl)-2-chloro-phenyl]-3-fluoro-4-(2-methoxyethoxy)benzamide;
2-[2-chloro-5-[1-hydroxy-2-(methylamino)-1-phenyl-ethyl]phenyl]-3-fluoro-4-(2-methoxyethoxy)benzamide;
2-[3-(2-amino-1-hydroxy-1-phenyl-ethyl)-6-chloro-2-methyl-phenyl]benzamide;
2-[5-[(1R)-2-amino-1-hydroxy-1-phenyl-ethyl]-2-chloro-phenyl]-3-fluoro-4-(2-methoxyethoxy)benzamide;
2-[5-[(1-aminocyclopropyl)-hydroxy-phenyl-methyl]-2-chloro-phenyl]-3-fluoro-4-(2-methoxyethoxy)benzamide;
2-[3-(2-amino-1-hydroxy-1-phenyl-ethyl)-2,6-difluoro-phenyl]-3-fluoro-4-(2-methoxyethoxy)benzamide;
2-[2-chloro-5-[1-hydroxy-2-(methylamino)-1-phenyl-ethyl]phenyl]-3-fluoro-4-(2-methoxyethoxy)benzenecarbohydroxamic acid;
rel-(2aS)-2-[3-[(1S)-2-amino-1-hydroxy-1-phenyl-ethyl]-6-chloro-2-methyl-phenyl]-3-fluoro-4-(2-methoxyethoxy)benzamide;
rel-(2-aS)-2-[3-[(1R)-2-amino-1-hydroxy-1-phenyl-ethyl]-6-chloro-2-methyl-phenyl]-3-fluoro-4-(2-methoxyethoxy)benzamide;
2-[6-chloro-3-[2-(cyclohexylamino)-1-hydroxy-1-phenyl-ethyl]-2-fluoro-phenyl]-3-fluoro-4-(2-methoxyethoxy)benzamide;
rel-(2aS)-2-[6-chloro-2-fluoro-3-[(1S)-1-hydroxy-2-(methylamino)-1-phenyl-ethyl]phenyl]-3-fluoro-4-(2-methoxyethoxy)benzamide;
rel-(2aS)-2-[6-chloro-2-fluoro-3-[(1R)-1-hydroxy-2-(methylamino)-1-phenyl-ethyl]phenyl]-3-fluoro-4-(2-methoxyethoxy)benzamide;
2-[3-(2-amino-1-hydroxy-1-phenyl-ethyl)-2,6-dichloro-phenyl]-3-fluoro-4-(2-methoxyethoxy)benzamide;
trans-2-[2-chloro-5-[1-hydroxy-2-[(4-hydroxycyclohexyl)amino]-1-phenyl-ethyl]phenyl]-3-fluoro-4-(2-methoxyethoxy)benzamide;
rel-(2aS)-2-[3-[(1R)-2-amino-1-hydroxy-1-phenyl-ethyl]-6-chloro-2-fluoro-phenyl]-3-fluoro-4-(2-methoxyethoxy)benzamide;
rel-(2aS)-2-[3-[(1S)-2-amino-1-hydroxy-1-phenyl-ethyl]-6-chloro-2-fluoro-phenyl]-3-fluoro-4-(2-methoxyethoxy)benzamide;
2-[6-chloro-3-[2-(cyclopropylmethylamino)-1-hydroxy-1-phenyl-ethyl]-2-fluoro-phenyl]-3-fluoro-4-(2-methoxyethoxy)benzamide;
2-[6-chloro-2-fluoro-3-[1-hydroxy-2-(2-methoxyethylamino)-1-phenyl-ethyl]phenyl]-3-fluoro-4-(2-methoxyethoxy)benzamide;
rel-(2aS)-2-[6-chloro-3-[(1R)-2-(cyclohexylamino)-1-hydroxy-1-phenyl-ethyl]-2-fluoro-phenyl]-3-fluoro-4-(2-methoxyethoxy)benzamide;
2-[6-chloro-2-fluoro-3-[1-hydroxy-2-(methylamino)-1-phenyl-ethyl]phenyl]-3-fluoro-4-(5-methylisoxazol-3-yl)oxy-benzamide;
and pharmaceutically acceptable salts, enantiomers, diastereomers, tautomers, solvates, isotope substituents, polymorphs, prodrugs or metabolites thereof.

15. A pharmaceutical composition comprising a compound according to any preceding claim, or a pharmaceutically acceptable salt, enantiomer, diastereomer, tautomer, solvate, isotope substituent, polymorph, prodrug or metabolite, thereof, and one or more pharmaceutically acceptable excipient(s).

16. The pharmaceutical composition of claim 15, which further comprises at least one other active pharmaceutical ingredient selected from KRAS inhibitors, SHP2 inhibitors, EGFR inhibitors, HER2 inhibitors, PI3K inhibitors, MEK inhibitors, ERK inhibitors, MDM2 inhibitors, Raf inhibitors, CDK4/6 inhibitors, cMET inhibitors, VEGFR inhibitors, chemotherapeutic agent, radiotherapeutic treatment, immunotherapy agent, immunotherapy drug based on oncolytic viruses and a combination thereof.

17. The compound of any one of claims 1 to 14, or a pharmaceutically acceptable salt, enantiomer, diastereomer, tautomer, solvate, isotope substituent, polymorph, prodrug or metabolite thereof, for use as a medicament.

18. The compound of any one of claims 1 to 14, or a pharmaceutically acceptable salt, enantiomer, diastereomer, tautomer, solvate, isotope substituent, polymorph, prodrug or metabolite thereof, for use for the treatment or prevention of a cancer or of a fibrosis related disease.

19. The compound for use of claim 18, wherein the cancer is selected from : adrenal cancer, adrenocortical cancer, anal cancer, bile duct cancer, bladder cancer, blood related cancer, bone cancer, brain cancer, breast cancer, male breast cancer, cervical cancer, colon cancer, colorectal cancer, embryonal cancer, endometrial cancer, uterine cancer, esophageal cancer, eye cancer, gastric cancer, head and neck cancer, kidney cancer, liver cancer, lung cancer, mesothelioma, ovarian cancer, pancreatic cancer, peripheral nervous system cancer, prostate cancer, gallbladder cancer, rhabdoid tumors, soft tissue sarcoma and sarcoma, skin cancer, melanoma, penile cancer, rectal cancer, small intestine cancer, testicular cancer, urethral cancer, germ cell tumors, childhood cancer and thyroid cancer.

20. The compound for use of claim 18, wherein the fibrosis related disease is selected from: skin fibrosis (such as for example hypertrophic scar or systemic sclerosis), heart fibrosis (such as for example cardiac fibrosis, hypertrophic cardiomyopathy, valvular diseases), bone marrow fibrosis (such as for example myelofibrosis, myelodysplatic syndrome), liver fibrosis (such as for example non-alcoholic steatohepatitis, Alcoholic liver disease, cirrhosis, portal hypertension), retroperitoneum fibrosis, gut fibrosis (such as for example intestinal fibrosis, Inflammatory bowel disease, enteropathies), joint fibrosis (such as for example arthrofibrosis), brain fibrosis (such as glial scar), nervous system fibrosis, eye fibrosis (such as for example subretinal fibrosis, epiretinal fibrosis, vision loss), lung fibrosis (such as for example idiopathic pulmonary fibrosis, ANCA-associated vasculitis, pulmonary hypertension), cystic fibrosis, mediastinum fibrosis, pancreas fibrosis (such as for example pancreatic fibrosis, chronic pancreatitis, duct obstruction) and kidney fibrosis (such as for example renal fibrosis, ANCA-associated vasculitis, nephrogenic systemic fibrosis, chronic kidney disease)..

21. A method of modulating one or more of proteins encompassed by, or related to, the Hippo pathway in a subject, comprising administering to a subject in need thereof a compound of formula (I) of any one of claims 1 to 14, or the pharmaceutical composition according to any one of claims 15 to 16, or a pharmaceutically acceptable salt, enantiomer, diastereomer, tautomer, solvate, isotope substituent, polymorph, prodrug or metabolite thereof.

22. A compound of formula (I) according to any one of claims 1 to 14, or a pharmaceutically acceptable salt thereof, or the pharmaceutical composition according to any one of claims 15 to 16 or, enantiomer, diastereomer, tautomer, solvate, isotope substituent, polymorph, prodrug or metabolite thereof, for treating or preventing a disease or disorder by modulating or inhibiting or activating one or more of proteins encompassed by, or related to, the Hippo pathway, in a subject in need thereof.

23. Uses of a compound of formula (I) according to any one of claims 1 to 14 or a pharmaceutically acceptable salt thereof, or the pharmaceutical composition according to any one of claims 15 to 16 or, enantiomer, diastereomer, tautomer, solvate, isotope substituent, polymorph, prodrug or metabolite thereof, in the preparation of a medicament for treating or preventing a disease or disorder by modulating or inhibiting or activating one or more of proteins encompassed by, or related to, the Hippo pathway, in a subject in need thereof.
